# EUROPEAN PATENT SPECIFICATION

(11) **EP 1 223 941 B1**
(45) Date of publication and mention of the grant of the patent: **05.11.2008**
(21) Application number: 00970511.2
(22) Date of filing: 28.09.2000
(51) Int. Cl.: A61K 31/565, A61K 31/566, A61P 7/06

(54) **THERAPEUTIC TREATMENTS FOR BLOOD CELL DEFICIENCIES USING SREROIDS**
THERAPEUTISCHE VERWENDUNGEN VON STEROIDEN BEI BLUTZELLMANGELZUSTAENDEN
TRAITEMENTS THERAPEUTIQUES POUR DES DEFICIENCES EN CELLULES SANGUINES PAR DES STEROIDES

(30) Priority: 25.10.1999 US 161453 P
(43) Date of publication of application: 24.07.2002
(73) Proprietor: Hollis-Eden Pharmaceuticals Inc., San Diego, CA 92121 (US)
(72) Inventor: FRINCKE, James, Martin, San Diego, CA 92192 (US); READING, Christopher, L., San Diego, CA 92112-3511 (US); PRENDERGAST, Patrick, T., County Kildare (IE)
(74) Representative: Kaiser, Jürgen
(86) International application number: PCT/US2000/026771
(87) International publication number: WO 2001/030802

(56) References cited:
- WO-A-97/13500
- WO-A-97/17992
- US-A- 4 602 008
- US-A- 5 763 433
- SINGER J W ET AL: "STEROIDS AND HEMATOPOIESIS PART 1 THE EFFECT OF STEROIDS ON IN-VITRO ERYTHROID COLONY GROWTH STRUCTURE ACTIVITY RELATIONSHIPS" JOURNAL OF CELLULAR PHYSIOLOGY, vol. 88, no. 2, 1976, pages 127-134, XP001015349 ISSN: 0021-9541
- LAFAYE, PIERRE ET AL: "The 7.alpha.-hydroxysteroids produced in human tonsils enhance the immune response to tetanus toxoid and Bordetella pertussis antigens" BIOCHIM. BIOPHYS. ACTA (1999), 1472(1-2), 222-231 , XP001014347
- DATABASE BIOSIS [Online] BIOSCIENCES INFORMATION SERVICE, PHILADELPHIA, PA, US; 1983 FEDOROVSKAYA N A ET AL: "CHARACTERISTICS OF IMMUNO HEMATOLOGIC PARAMETERS IN PATIENTS WITH HYPOPLASTIC ANEMIA DURING REMISSION" Database accession no. PREV198477064932 XP002174053 & GEMATOLOGIYA I TRANSFUZIOLOGIYA, vol. 28, no. 7, 1983, pages 29-32, ISSN: 0234-5730
- PIERELLI LUCA ET AL: "Erythropoietin addition to granulocyte colony-stimulating factor abrogates life-threatening neutropenia and increases peripheral-blood progenitor-cell mobilization after epirubicin, paclitaxel, and cisplatin combination chemotherapy: Results of a randomized comparison." JOURNAL OF CLINICAL ONCOLOGY, vol. 17, no. 4, April 1999 (1999-04), pages 1288-1295, XP001015368 ISSN: 0732-183X
- ALONZI TONINO ET AL: "Interleukin-6 and CAAT/enhancer binding protein beta-deficient mice act as tools to dissect the IL-6 signalling pathway and IL-6 regulation." IMMUNOBIOLOGY, vol. 198, no. 1-3, December 1997 (1997-12), pages 144-156, XP001015352 ISSN: 0171-2985

## Description

### FIELD OF THE INVENTION

The present invention relates to a use of a compound for the preparation of a medicament for the prophylaxis or treatment of neutropenia and thrombocytopenia in a human or primate subject, in accordance with claim 1.

### BACKGROUND OF THE INVENTION

Hemopoiesis is the formation and development of the various types of blood cells and their progenitor cells. Mature cells are found in circulation or tissues such as the lymph nodes or the thymus. Many of the stem cells that give rise to mature forms reside in the bone marrow, although some may circulate in the blood for some time. Clinical blood cell deficiencies such as thrombocytopenia, neutropenia or erythropenia can arise from causes such as impaired hemopoiesis or abnormal loss or destruction of mature or immature blood cells.

Thrombocytopenia ("TP"), abnormally low platelet counts, can arise from impaired platelet production, sequestration of platelets in the spleen or abnormal loss of circulating platelets. Impaired production can result from causes such as chemotherapies or radiation therapies. Abnormal loss of circulating platelets is often associated with autoreactive antibodies that bind to platelets and reduce their life span. These underlying causes give rise to the various clinical forms of TP, such as autoimmune neonatal TP, immune thrombocytopenic purpra, radiation induced TP, chemotherapy induced TP and amegakaryocitic TP.

A number of treatment options are available and the selection of a treatment typically depends on the source of the disorder and its severity. Treatments include administering one or more of glucocorticoid steroids (e.g., prednisone, prednisolone), human IgG antibodies, anti-Rh(D)⁺ antibodies for Rh(D)⁺ patients, an androgen such as danazol, vinca alkaloids (e.g., vincristine, vinblastine), thrombopoietin and immunosuppresants (e.g., azathioprine, cyclophosphamide). Splenectomy is also indicated, for example when first line treatments fail. The goal of treatment is typically to increase platelet counts to 20,000 mm⁻³ or more typically to at least about 50,000 mm⁻³ and to maintain these levels.

Although the treatment options to increase platelet levels are generally known, they usually have a number of drawbacks. For example, infusion of IgG antibodies is not always effective and the treatment is relatively expensive. Other treatments, such as prednisone are also not always effective and they typically are discontinued or tapered off after several weeks due to toxicity. Splenectomy, which is relatively expensive and invasive, is also not always effective.

The sources of thrombocytopenia and treatment options have been described. See, e.g., Hematology - Basic Principles and Practice, 3rd edition, R. Hoffman, E.J. Benz Jr. et al., editors, Churchill Livingstone, New York, 2000 (see, e.g., Chapters 126-129 and 131 at pages 2096-2154 and 2172-2186), PCT publication WO 200035466.

Neutropenia ("NP"), is considered to exist clinically when neutrophils drop to below a level considered normal. NP can arise from impaired production of neutrophil precursors or mature neutrophils, movement of neutrophils from the circulation to tissue, abnormal circulating neutrophil loss or a combination of these causes. Impaired neutrophil production can be acquired from, e.g., treatment with a cytotoxic or cytostatic drug, chemotherapy, radiation therapy or an autoimmune response. The abnormal loss of circulating neutrophils in autoimmunity is associated with autoreactive antibodies that bind to the cells and reduce their life span. These underlying causes give rise to the various clinical forms of NP, such as postinfectious NP, drug-induced NP, autoimmune NP, or chronic idiopathic NP.

The sources of NP and treatment options have been described. See, e.g., Hematology - Basic Principles and Practice, 3rd edition, R. Hoffman, E.J. Benz Jr. et al., editors, Churchill Livingstone, New York, 2000 (see, e.g., Chapters 19, 41, 51, 79, 134 and 137 at pages 297-331, 720-762, 939-979, 1443-1500, 2220-2248 and 2257-2263).

The use of 3β-hydroxyandrost-5-ene-17-one, 3β,17β-dihydroxyandrost-5-ene and other steroids to modulate immune functions or to stimulate myelopoiesis has been described, see, e.g., M.H. Whitnall et al., Int'l. J. Immunopharmacology 2000 22:1-14. U.S. patents 5,162,198, 5,206,008, 5,292,730, 5,407,684, 5,461,042, 5,461,768, 5,478,566, 5,585,371, 5,635,496, 5,641,766, 5,753,237, 5,837,269, 5,885,977 and 5,919,465, PCT publication nos. WO93/20696 and WO99/25333. I. Porsova-Dutoit et al., Physiological Res. 2000 49(Suppl. 1):S43-S56, R.L. Jesse et al., Ann. N. Y. Acad. Sci. 1995 774:281-290 and U.S. patent 5,532,230, 5,811,418 and 5,846,963 discuss the capacity of 3β-hydroxyandrost-5-ene-17-one, its 3-sulfate derivative and other steroids to affect platelet and neutrophil aggregation or their adhesion to endothelial cells.

U.S. patents 4,908,358 and 4,902,681 describe the capacity of compounds such as 5α-pregnan-3,20-dione, cortexolone, 17-hydroxyprogesterone and 16α-methylprogesterone to inhibit the clearance of antibody-coated cells from circulation in disorders such as immune thrombocytopenic purpura or immune hemolytic anemia.

U.S. patents 5,532,230, 5,686,438, 5,753,640 and 5,811,418 and J. Bratt and M. Heimburger, Scand. J. Rheumatol. 1999 28:308-313 describe the capacity of compounds such as 3β,7β-dihydroxyandrost-5-ene-17-one, prednisolone, and 3β-hydroxyandrost-5-ene-17-one to limit tissue damage in ischemic tissues by inhibiting adhesion of cells such as neutrophils to endothelial cells or to treat pulmonary hypertension.

U.S. patent 5,859,000 describes the capacity of compounds such as 3β,7β-dihydroxyandrost-5-ene-17-one and 3β-hydroxyandrost-5-ene-17-one to reduce mast cell mediated allergic reactions.

U.S. patent 5,763,433 and PCT publication WO 96/35428 describe the capacity of compounds related to dehydroepiandrosterone and 16α-halodehydroepiandrosterone to modulate immune responses and to treat conditions certain immune related conditions such as systemic lupus erythematosus.

U.S. patents 5,925,630, 5,939,545 and 5,962,443 describe the capacity of 19-nur-pregnane steroids, 3α-hydroxy-5a-pregnan-20-one and related steroids to modulate certain neurological activities such as hypothalamic function and GABA receptor activity.

Some proteins such as interleukin-6 ("IL-6"), erythropoietin ("EPO") and thrombopoietin ("TPO") have been examined for their capacity to enhance various aspects hemopoiesis, e.g., Hematology - Basic Principles and Practice, 3rd edition, R. Hoffman, E.J. Benz Jr. et al., editors, Churchill Livingstone, New York, 2000 (see, e.g., Chapter 14 at pages 154-202), O.J. Borge et al., Blood 1996 88:2859-2870, M. Cremer et al., Ann. Hematol. 1999 78:401-407, Y. Sasaki et al., Blood 1999 94:1952-1960, U.S. patent 5,879,673. Recombinant IL-6 was shown in model systems to affect platelet counts in peripheral circulation, e.g., Stahl et al., Blood 1991 78:1467-1475, although significant toxicities are associated with its administration to humans, e.g., Andus et al., FEBS Lett. 1987 221:18, J. Gauldie et al., P.N.A.S. U.S.A. 1987 84:7251-7255, T. Geiger et al., Eur. J. Immunol. 1988 18:717-721. The IL-6 molecule has been described in detail, e.g., publication no. WO 88/00206. Administration of proteins is typically expensive, given factors such as the complexity of producing pharmaceutical grade material.

The capacity of various compounds or agents such as deuterium oxide, lithium and butyrate to affect or to participate in biological functions of cells such as neutrophils has been described. See, e.g., M.F. Tsan and R.M. Turkall, Inflammation 1982 6:387-396, M. Nakamura et al., Exp. Cell Res. 1976 102:429-431, P. Blier et al., Int. Clin. Psychopharmacol. 1998 13:137-140, N. Turkozkan et al., Int. J. Biochem. 1993 25:1501-1504, L.V. Deriy et al., Biochem. Biophys. Res. Commun. 2000 275:241-246, M.T. Elghetany et al., Leuk. Res. 1997 21:801-806, E. Brandt et al., J. Leukocyte Biol. 2000 68:125-130, M. Boussac and J. Garin, Electrophoresis 2000 21:665-672, M. Niwa et al., Life Sci. 2000 18:1525-1534, D.A. Moulding et al., J. Leukocyte Biol. 1999 65:875-882 and D. Moulding et al., Biologicals 1996 24:301-306.

In addition, U.S. patent 4,602,008 discloses erythropoietic activity of 16-alkylated-7-hydroxy-5β-androstane-3-ol-17-one and its derivatives in mice and chicken.

Furthermore, Singer et al., J. Cell Physiol. 1976, 88, 127-134 describe the effect of steroids, in particular 17a-methyl-5 α-androstane-3β,11β,17β-triol, on in vitro erythroid colony growth

There is a current need for cost-effective pharmaceutical agents or treatment methods that are more effective in treating deficiencies of blood cells or reducing their symptoms. The present invention provides therapeutic compounds to treat hemopoiesis deficiencies and disorders such as thrombocytopenia and neutropenia. The invention is thus useful to reduce one or more symptoms associated with any of these conditions. Also, the use of the invention agents and uses can be combined with one or more conventional treatments for these disorders.

### SUMMARY OF THE INVENTION

Accordingly, the invention provides a use according to claim 1 and a compound in accordance with claim 10 to enhance hemopoiesis in a human or primate subject in need thereof.

The invention may be more fully understood with reference to the following description. 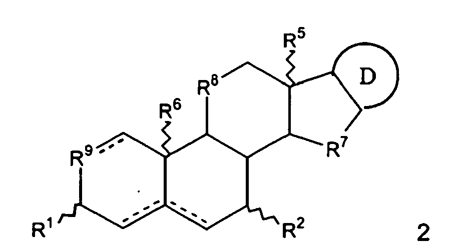
R⁷ is -CHR¹⁰-, -CHR¹⁰-CHR¹⁰-, -CHR¹⁰-CHR¹⁰-CHR¹⁰-, -CHR¹⁰-O-CHR¹⁰-, -CHR¹⁰-S-CHR¹⁰-, -CHR¹⁰-NR^{PR}-CHR¹⁰-, -O-, -O-CHR¹⁰-, -S-, -S-CHR¹⁰-, -NR^{PR}- or - NR^{PR}-CHR¹⁰-;
R⁸ and R⁹ independently are -CHR¹⁰-, -CHR¹⁰-CHR¹⁰-, -O-, -O-CHR¹⁰-, -S-, - S-CHR¹⁰-, -NR^{PR}- or -NR^{PR}-CHR¹⁰-, or R⁸ or R⁹ independently is absent, leaving a 5-membered ring;
R¹³ independently is C₁₋₆ alkyl;
R¹⁶ independently are -CH₂-, -O-, -S- or -NH-;
D is a heterocycle or a 4-, 5-, 6- or 7-membered ring that comprises saturated carbon atoms, wherein 1, 2 or 3 ring carbon atoms of the 4-, 5-, 6- or 7-membered ring are optionally independently substituted with -O-, -S- or -NR^{PR}- or where 1, 2 or 3 hydrogen atoms of the heterocycle or where 1 or 2 hydrogen atoms of the 4-, 5-, 6-or 7-membered ring are substituted with -OR^{PR}, -SR^{PR}, -N(R^{PR})₂, -O-Si-(R¹³)₃, -CN, - NO₂, an ester, a thioester, a phosphoester, a phosphothioester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted monosaccharide, an optionally substituted oligosaccharide, a nucleoside, a nucleotide, an oligonucleotide or a polymer, or,
one more of the ring carbons are substituted with =O or =S,
or D comprises two 5- or 6-membered rings, wherein the rings are fused or are linked by 1 or 2 bonds, provided that the compound is not 5-androstene-3β-ol-17-one, 5-androstene-3β,17β-diol, 5-androstene-3β,7β,17β-triol or a derivative of any of these three compounds that can convert to these compounds by hydrolysis.

Related aspects include a method to enhance thrombopoiesis, myelopoiesis or erythropoiesis in a subject by administering to the subject, or delivering to the subject's tissues, an effective amount of a formula 1 compound.

The invention may be more fully understood with reference to the following description. The invention is not limited to the exemplary embodiments and should be recognized as contemplating all modifications within the skill of an ordinary artisan.

### DETAILED DESCRIPTION

Definitions. As used herein the following terms or phrases have the definitions given here, unless otherwise expressly stated or implied by context.

An "excipient" , "carrier", "pharmaceutically acceptable carrier" and the like mean a component or an ingredient that is acceptable in the sense of being compatible with the other ingredients of invention compositions or formulations and not overly deleterious to the patient or animal to which the formulation is to be administered. As used here, "excipients" include solids and liquids, such as lactose, sucrose, starch, microcrystalline cellulose, cellulose, benzyl benzoate, cottonseed oil, N,N-dimethylacetamide, a C₂₋₁₂ alcohol (e.g., ethanol), glycerol, peanut oil, a polyethylene glycol ("PEG"), vitamin E, poppyseed oil, propylene glycol, safflower oil, sesame oil, soybean oil and vegetable oil. Excipients comprise one or more components typically used in the pharmaceutical formulation arts, e.g., fillers, binders, disintegrants and lubricants.

A "subject" means a human or animal. Usually the animal is a vertebrate such as a primate, rodent, domestic animal or game animal. Primates include chimpanzees, cynomologous monkeys, spider monkeys, and macaques, e.g., *Rhesus*. Rodents include mice, rats, woodchucks, ferrets, rabbits and hamsters. Domestic and game animals include cows, horses, pigs, deer, bison, buffalo, felines, e.g., domestic cat, canines, e.g., dog, avian species, e.g., chicken, emu, ostrich, and fish, e.g., trout, catfish and salmon. Subject includes any subset of the foregoing, e.g., all of the above, but excluding one or more groups or species such as humans, primates or rodents.

Expressions that refer to "a formula 1 compound(s)", "a formula 1 compound" and the like mean that one or more than one formula 1 compound is used or is present, typically 1, 2, 3 or 4. Generally the formula 1 compound is present in a composition or formulation that contains a pharmaceutically acceptable carrier.

"Alkyl" as used here means linked normal, secondary, tertiary or cyclic carbon atoms, i.e., linear, branched or cyclic. The number of carbon atoms in an alkyl group or moiety is 1 to about 20, unless otherwise specified, e.g., C₁₋₈ alkyl means an alkyl moiety containing 1, 2, 3, 4, 5, 6, 7 or 8 carbon atoms. Examples include methyl, ethyl, 1-propyl (*n*-propyl), 2-propyl (*i*-propyl, -CH(CH₃)₂), 1-butyl (*n*-butyl), 2-methyl-1-propyl (*i*-butyl, -CH₂CH(CH₃)₂), 2-butyl (*s*-butyl, -CH(CH₃)CH₂CH₃), 2-methyl-2-propyl (*t*-butyl, -C(CH₃)₃), 1-pentyl (*n*-pentyl), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl, 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), cyclopropyl, cyclobutyl, cyclopentyl and cyclohexyl.

"Alkenyl" means linked normal, secondary, tertiary or cyclic carbon atoms where one or more double bonds (e.g., -CH=CH-) are present, typically 1, 2 or 3, usually 1 or 2. The number of carbon atoms in an alkenyl group or moiety is 2 to about 20, unless otherwise specified, e.g., C₁₋₈ alkenyl means an alkenyl moiety containing 1, 2, 3, 4, 5, 6, 7 or 8 carbon atoms.

"Alkynyl" means linked normal, secondary, tertiary or cyclic carbon atoms where one or more triple bonds (-C≡C-) are present, typically 1, 2 or 3, usually 1. The number of carbon atoms in an alkynyl group or moiety is 2 to about 20, unless otherwise specified, e.g., C₁₋₈ alkynyl means an alkynyl moiety containing 1, 2, 3, 4, 5, 6, 7 or 8 carbon atoms.

"Aryl" means phenyl or naphthyl.

"Substituted alkyl", "substituted alkenyl" and "substituted alkynyl" mean an alkyl, alkenyl or alkynyl group that has a substituent(s) linked to a carbon atom or substituent(s) that interrupt a carbon atom chain. Substituents include ethers (-O-), ketones (-C(O)-), -OR^{PR}, -C(O)OR^{PR}, -C(O)O-, -C(S)OR^{PR}, -C(S)O-, -OC(O)-, - C(O)H, -OCH₂-, -OCH₂CH₂-, -OCH₂O-, -OCH₂CH₂O-, -NR^{PR}-, -N(R^{PR})₂, - NHR^{PR}, -NHC(O)-, -CH₂-NR^{PR}-, -CH₂-NHR^{PR}, -CH₂-NHC(O)-, -C(O)NH-, - C(O)NHR^{PR}, -OC(O)NRPR-, -OC(O)NHR^{PR}, -NR^{PR}C(O)NR^{PR}-, - NR^{PR}C(O)NHR^{PR}, -NR^{PR}CH₂-, -NR^{PR}CH₂CH₂-, -S-, -SR^{PR}, -S(O)-, -S(O)(O)-, - S(O)OR^{PR}, -S(O)H, -CN, -NO₂, halogen, and combinations of these moieties where R^{PR} independently is hydrogen, a protecting group or both R^{PR} together are a protecting group. Substituents are independently chosen when more than one is present. Alkenyl and alkynyl groups that comprise a substituent(s), are typically substituted at a carbon that is one or more methylene moiety removed from the double bond, e.g., separated at least by one, two or more -CH₂- moieties.

"Heterocycle" or "heterocyclic" means by way of example and not limitation the heterocycles described in Paquette, Leo A.; "Principles of Modem Heterocyclic Chemistry" (W. A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. 1960, 82:5566.

Examples of heterocycles include by way of example and not limitation pyridyl, thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2dithiazinyl, thienyl, thianthrenyl, pyranyl, isobenzofuranyl, chromenyl, xanthenyl, phenoxathiinyl, 2H-pyrrolyl, isothiazolyl, isoxazolyl, pyrazinyl, pyridazinyl, indolizinyl, isoindolyl, 3H-indolyl, 1 H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, and isatinoyl.

By way of example and not limitation, carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5--pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1 H-indazole, position 2 of a isoindole, or isoindoline, position 4 of a morpholine, and position 9 of a carbazole, or β-carboline. Typically, nitrogen bonded heterocycles include 1-aziridyl, 1-azetedyl, 1-pyrrolyl, 1-imidazolyl, 1-pyrazolyl, and 1-piperidinyl.

"Heteroaryl" means an aromatic ring or two or more fused rings that contain one or more aromatic rings where the ring or fused rings comprise 1, 2, 3 or more heteroatoms, usually oxygen (-O-), nitrogen (-NX-) or sulfur (-S-) where X is -H, a protecting group or C₁₋₆ alkyl, usually -H. Examples are as described for heterocycle.

"Alcohol" as used herein means an alcohol that comprises a C₂₋₁₂ alkyl moiety substituted at a hydrogen atom with one hydroxyl group. Alcohols include ethanol, *n*-propanol, *i*-propanol, n-butanol, *i*-butanol, s-butanol, *t*-butanol, *n*-pentanol, *i-*pentanol, *n*-hexanol, cyclohexanol, *n*-heptanol, *n*-octanol, *n*-nonanol and *n*-decanol. The carbon atoms in alcohols can be straight, branched or cyclic. Alcohol includes any subset of the foregoing, e.g., C₂₋₄ alcohols (alcohols having 2, 3 or 4 carbon atoms).

"Halogen" means fluorine, chlorine, bromine or iodine.

"Protecting group" means a moiety that prevents the atom to which it is linked from participating in unwanted reactions. For example, for -OR^{PR}, R^{PR} may be hydrogen or a protecting group for the oxygen atom found in a hydroxyl, while for - C(O)-OR^{PR}, R^{PR} may be hydrogen or a carboxyl protecting group, for -SR^{PR}, R^{PR} may be hydrogen or a protecting group for sulfur in thiols for instance, and for - NHR^{PR} or -N(R^{PR})₂-, R^{PR} may be hydrogen or a nitrogen atom protecting group for primary or secondary amines. Hydroxyl, amine and other reactive groups are found in formula 1 compounds at, e.g., R¹ or R². These groups may require protection against reactions taking place elsewhere in the molecule. The protecting groups for oxygen, sulfur or nitrogen atoms are usually used to prevent unwanted reactions with electrophilic compounds, such as acylating used, e.g., in steroid chemistry.

"Ester" means a moiety that comprises a -C(O)-O- structure that is bonded to the steroid nucleus. Typically, esters as used here comprise an organic moiety containing about 1-50 carbon atoms (e.g., about 2-20 carbon atoms) and 0 to about 10 independently selected heteroatoms (e.g., O, S, N, P, Si), where the organic moiety is bonded to a formula 1 steroid nucleus at, e.g., R¹-R⁶, R¹⁰, R¹⁵, R¹⁷ or R¹⁸, through the -C(O)-O- structure, e.g., organic moiety-C(O)-O-steroid or organic moiety-O-C(O)-steroid. The organic moiety usually comprises one or more of any of the organic groups described above, e.g., C₁₋₂₀ alkyl moieties, C₂₋₂₀ alkenyl moieties, C₂₋₂₀ alkynyl moieties, aryl moieties, C₂₋₉ heterocycles or substituted derivatives of any of these, e.g., comprising 1, 2, 3, 4 or more substituents, where each substituent is independently chosen. Typical substitutions for hydrogen or carbon atoms in these organic groups include 1, 2, 3, 4 or more, usually 1, 2, or 3 -O-, -S-, -NR^{PR}- (including -NH-), -C(O)-, =O, =S, =N-OH, -N(R^{PR})₂ (including -NH₂), -C(O)OR^{PR} (including -C(O)OH), -OC(O)R^{PR} (including -O-C(O)-H), -OR^{PR} (including -OH), -SR^{PR} (including -SH), -NO₂, -CN, -NHC(O)-, -C(O)NH-, -OC(O)-, -C(O)O-, -O-A8, -S-A8, -C(O)-A8, -OC(O)-A8, - C(O)O-A8, =N-, -N=, =N-OH, =CH₂, =CH-(CH₂)₀₋₄CH₃, -OPO₃(R^{PR})₂, -OPO₃H₂, - OSO₃H₂, halogen, monosaccharide, oligosaccharide or polymer moieties or atoms, where each R^{PR} is -H, an independently selected protecting group or both R^{PR} together comprise a protecting group, and A8 is C₁₋₈ alkyl, C₂₋₈ alkenyl, C₂₋₈ alkynyl, C₁₋₄ alkyl-aryl (e.g., benzyl), aryl (e.g. phenyl) or C₀₋₄ alkyl-C₂₋₉ heterocycle. Substitutions are independently chosen. The organic moiety includes compounds defined by the R₄ variable. The organic moieties exclude obviously unstable moieties, e.g., -O-O-, except where such unstable moieties are transient species that one can use to make a compound with sufficient chemical stability for one or more of the uses described herein. The substitutions listed above are typically substituents that one can use to replace one or more carbon atoms, e.g., -O- or -C(O)-, or one or more hydrogen atom, e.g., halogen, -NH₂ or -OH.

"Thioester" means a moiety that comprises a -C(S)-O- structure. Typically, thioesters as used here comprise an organic moiety containing about 1-50 carbon atoms (e.g., about 2-20 carbon atoms) and 0 to about 10 independently selected heteroatoms (e.g., O, S, N, P, Si), where the organic moiety is bonded to a formula 1 steroid nucleus at, e.g., R¹-R⁶, R¹⁰, R¹⁵, R¹⁷ or R¹⁸, through the -C(S)-O- structure, e.g., organic moiety-C(S)-O-steroid or organic moiety-O-C(S)-steroid. The organic moiety is as described above for esters.

"Thioacetal" means a moiety that comprises a -C(O)-S- structure. Typically, thioacetals as used here comprise an organic moiety containing about 1-50 carbon atoms (e.g., about 2-20 carbon atoms) and 0 to about 10 independently selected heteroatoms (e.g., O, S, N, P, Si), where the organic moiety is bonded to a formula 1 steroid nucleus at one or more of, e.g., R¹-R⁶, R¹⁰, R¹⁵, R¹⁷ or R¹⁸, through the -C(O)-S- structure, e.g., organic moiety-C(O)-S-steroid or organic moiety-S-C(O)-steroid. The organic moiety is as described above for esters.

"Phosphoester" or "phosphate ester" means a moiety that comprises a -O-P(OR^{PR})(O)-O- structure where R^{PR} is hydrogen (-H), a protecting group or an organic moiety as described for esters. Typically, phosphoesters as used here comprise a hydrogen atom, a protecting group or an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the -O-P(O)(O)-O- structure, e.g., organic moiety-OP(O)(OH)-O-steroid. The organic moiety is as described above for esters.

"Phosphothioester" means a moiety that comprises a -O-P(SR^{PR})(O)-O-structure where R^{PR} is -H, a protecting group or an organic moiety as described for esters. Typically, phosphothioesters as used here comprise a hydrogen atom, a protecting group or an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the -O-P(O)(O)-O- structure, e.g., organic moiety-O-P(O)(SH)-O-steroid. The organic moiety is as described above for esters.

"Phosphonoester" means a moiety that comprises a -P(OR^{PR})(O)-O- structure where R^{PR} is -H, a protecting group or an organic moiety as described for esters. Typically, phosphonoesters as used here comprise a hydrogen atom, a protecting group or an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the - P(OR^{PR})(O)-O- structure, i.e., organic moiety-P(OR^{PR})(O)-O-steroid or steroid-P(OR^{PR})(O)-O-organic moiety. The organic moiety is as described above for esters.

"Sulfate ester" means a moiety that comprises a -O-S(O)(O)-O- structure. Typically, sulfate esters as used here comprise a hydrogen atom, a protecting group or an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the -O-S(O)(O)-O- structure, e.g., organic moiety-O-S(O)(O)-O-steroid. The organic moiety is as described above for esters.

"Sulfite ester" means a moiety that comprises a -O-S(O)-O- structure. Typically, sulfite esters as used here comprise an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the -O-S(O)-O- structure, e.g., organic moiety-O-S(O)-O-steroid. The organic moiety is as described above for esters.

"Thioacetal" means a moiety that comprises a -S-C(O)- structure. Typically, thioacetal groups as used here comprise an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the -S-C(O)- structure, e.g., organic moiety-S-C(O)-steroid or steroid-S-C(O)-organic moiety. The organic moiety is as described above for esters.

"Amide" means an organic moiety as described for ester that comprises 1, 2, 3, 4 or more -C(O)-NR^{PR}- moieties, usually 1 or 2, where R^{PR} is -H or a protecting group, R^{PR} is usually H. In embodiments where one or more of, e.g., R¹-R⁶, R¹⁰, R¹⁵, R¹⁷ or R¹⁸, is an amide, the -C(O)NR^{PR}- group is linked to the steroid nucleus at R¹-R⁶, R¹⁰, R¹⁵, R¹⁷ or R¹⁸, i.e., organic moiety-C(O)NR^{PR}-steroid or steroid-C(O)NR^{PR}-organic moiety.

"Ether" means an organic moiety as described for ester that comprises 1, 2, 3, 4 or more -O- moieties, usually 1 or 2. In embodiments where one or more of, e.g., R¹-R⁶, R¹⁰, R¹⁵, R¹⁷ or R¹⁸, is an ether, the -O- group is bonded to the steroid nucleus at R¹-R⁶, R¹⁰, R¹⁵, R¹⁷ or R¹⁸, i.e., organic moiety-O-steroid.

"Thioether" means an organic moiety as described for ester that comprises 1, 2, 3, 4 or more -S- moieties, usually 1 or 2. In embodiments where one or more of, e.g., R¹-R⁶, R¹⁰, R¹⁵, R¹⁷ or R¹⁸, is an ether, the -S- group is bonded to the steroid nucleus at R¹-R⁶, R¹⁰, R¹⁵, R¹⁷ or R¹⁸, i.e., organic moiety-S-steroid.

"Acyl group" means an organic moiety as described for ester that comprises 1, 2, 3, 4 or more -C(O)- groups. In some embodiments, the -C(O)- group is linked to the steroid nucleus at one or more of, e.g., R¹-R⁶, R¹⁰, R¹⁵, R¹⁷ or R¹⁸, e.g., organic moiety-C(O)-steroid.

"Thioacyl" means an organic moiety as described for ester that comprises 1, 2, 3, 4 or more -C(S)- groups. In some embodiments, the -C(S)- group is linked to the steroid nucleus at one or more of, e.g., R¹-R⁶, R¹⁰, R¹⁵, R¹⁷ or R¹⁸, e.g., organic moiety-C(S)-steroid.

"Carbonate" means an organic moiety as described for ester that comprises 1, 2, 3, 4 or more -O-C(O)-O- structures. Typically, carbonate groups as used here comprise an organic moiety containing about 1-50 carbon atoms and 0 to about 10 independently selected heteroatoms (e.g., O, S, N, P, Si) linked to a formula 1 steroid nucleus at one or more of, e.g., R¹-R⁶, R¹⁰, R¹⁵, R¹⁷ or R¹⁸, through the -O-C(O)-O- structure, e.g., organic moiety-O-C(O)-O-steroid.

"Carbamate" means an organic moiety as described for ester that comprises 1, 2, 3, 4 or more -O-C(O)NR^{PR}- structures where R^{PR} is -H, a protecting group or an organic moiety as described for ester. Typically, carbamate groups as used here comprise an organic moiety containing about 1-50 carbon atoms and 0 to about 10 heteroatoms (e.g., O, S, N, P, Si) linked to a formula 1 steroid nucleus at one or more of, e.g., R¹-R⁶, R¹⁰, R¹⁵, R¹⁷ or R¹⁸, through the -O-C(O)-NR^{PR}- structure, e.g., organic moiety-O-C(O)-NR^{PR}-steroid or steroid-O-C(O)-NR^{PR}-organic moiety.

As used herein, "monosaccharide" means a polyhydroxy aldehyde or ketone having the empirical formula (CH₂O)ₙ where n is 3, 4, 5, 6 or 7. Monosaccharide includes open chain and closed chain forms, but will usually be closed chain forms. Monosaccharide includes hexofuranose, pentofuranose and related sugars such as glucuronic acid, 2'-deoxyribose, ribose, arabinose, xylose, their 2'-deoxy and 3'-deoxy derivatives and their 2',3'-dideoxy derivatives. Monosaccharide also includes the 2',3' dideoxydidehydro derivative of ribose. Monosaccharides can be the D-, L- or DL-isomers. They include glucose, fructose, mannose, idose, galactose, allose, gulose, altrose, talose, fucose, erythrose, threose, lyxose, erythrulose, ribulose, xylulose, ribose, arabinose, xylose, psicose, sorbose, tagatose, glyceraldehyde, dihydroxyacetone and their monodeoxy derivatives such as rhamnose. When one or more monosaccharides are bonded directly to the steroid, they are independently selected and they are linked to the steroid nucleus at one or more of, e.g., R¹-R⁶, R¹⁰, R¹⁵, R¹⁷ or R¹⁸, through, e.g., the -O- ether structure, i.e., steroid-O-monosaccharide. The linkage between the monosaccharide the steroid is in the α or β configuration and the configuration of the atom that is bonded to the steroid is also either in the α or β configuration.

Optionally substituted "monosaccharide" comprises any C3-C7 sugar, D-, L- or DL-configurations, e.g., erythrose, glycerol, ribose, deoxyribose, arabinose, glucose, mannose, galactose, fucose, mannose, glucosamine, N-acetylneuraminic acid, N-acetylglucosamine, N-acetylgalactosamine or glucuronic acid that is optionally substituted at one or more hydroxyl groups. Suitable substitutions include a protecting group for one or more hydroxyl groups, e.g., acetate. Other substitutions include carboxyl, azido, cyano, -O-C₁₋₆ alkyl, -S-C₁₋₆ alkyl, -O-C₂₋₆ alkenyl, -S-C₂₋₆ alkenyl, optionally protected amine, optionally protected carboxyl, halogen, thiol or protected thiol. Independently selected monosaccharides are linked to a formula 1 steroid nucleus at one or more of R¹-R⁶, R¹⁰, R¹⁵, R¹⁷ or R¹⁸, through the -O- ether structure, i.e., steroid-O-substituted monosaccharide. The manner of linkage to the steroid is the same as described for monosaccharides.

Optionally substituted "oligosaccharide" comprises two, three, four or more of any C3-C7 monosaccharides that are covalently linked to each other. The linked sugars may have D-, L- or DL-configurations. Suitable sugars and substitutions are as described for monosaccharides and optionally substituted monosaccharides. Independently selected monosaccharides are linked to a formula 1 steroid nucleus at one or more of, e.g., R¹-R⁶, R¹⁰, R¹⁵, R¹⁷ or R¹⁸, through the -O- ether structure, i.e., steroid-O-optionally substituted oligosaccharide. The manner of linkage to the steroid is the same as described for monosaccharides, as are the linkages between the monosaccharides that comprise the oligosaccharide.

Optionally substituted alkyl group, optionally substituted alkenyl group, optionally substituted alkynyl group, optionally substituted aryl moiety and optionally substituted heterocycle mean substitutions that include C₁₋₂₀ alkyl moieties, C₂₋₂₀ alkenyl moieties, C₂₋₂₀ alkynyl moieties, aryl moieties, C₂₋₉ heterocycles or substituted derivatives of any of these. Typical substitutions for these organic groups include 1, 2, 3, 4 or more, usually 1 or 2, independently selected -O-, -S-, -NR^{PR}-, - C(O)-, -N(R^{PR})₂, -C(O)OR^{PR}, -OC(O)R^{PR}, -OR^{PR}, -SR^{PR}, -NO₂, -CN, -NHC(O)-, - C(O)NH-, -OC(O)-, -C(O)O-, -O-A8, -S-A8, -C(O)-A8, -OC(O)-A8, -C(O)O-A8, =N-, - N=, -OPO₂R^{PR}, -OSO₃H or halogen moieties or atoms, where R^{PR} independently is - H, a protecting group or both R^{PR} together are a protecting group and A8 is C₁₋₈ alkyl, C₁₋₈ alkenyl, C₁₋₈ alkynyl, C₁₋₄ alkyl-aryl (e.g., benzyl), aryl (e.g. phenyl) or C₁₋₄ alkyl-C₁₋₅ heterocycle. Substitutions are independently chosen. The organic moieties as described here, and for other any other moieties described herein, exclude obviously unstable moieties, e.g., -O-O-, except where such unstable moieties are transient species that one can use to make a compound with sufficient chemical stability for the one or more of the uses described herein. When bonded directly to the steroid, these moieties are typically bonded through an ether, ester or other linkage as described above, e.g., optionally substituted C₁₋₈ alkyl -O-steroid, optionally substituted C₁₋₈ alkyl-C(O)-O-steroid or optionally substituted C₁₋₈ alkyl-O-C(O)-O-steroid.

Polymer means biocompatible organic polymers, e.g., PEGs and polyhydroxyalkyl polymers.

PEG means an ethylene glycol polymer that contains about 20 to about 2000000 linked monomers, typically about 50-1000 linked monomers, usually about 100-300. Polyethylene glycols include PEGs containing various numbers of linked monomers, e.g., PEG20, PEG30, PEG40, PEG60, PEG80, PEG100, PEG115, PEG 200, PEG 300, PEG400, PEG500, PEG600, PEG 1000, PEG1500, PEG2000, PEG 3350, PEG4000, PEG4600, PEG5000, PEG6000, PEG8000, PEG11000, PEG12000, PEG2000000 and any mixtures thereof. When a PEG is bonded to the steroid at one or more of R¹-R¹⁶ and R¹⁹ it can be bonded through a terminal or an internal hydroxyl.

"Amino acid" means an amino acid moiety that comprises any naturally-occurring or synthetic amino acid residue, i.e., any moiety comprising at least one carboxyl and at least one amino residue directly linked by one, two three or more carbon atoms, typically one (α) carbon atom. The nature and identity of the intervening structure located between the carboxyl and amino groups can have a variety of structures including those described herein. Typically, amino acids are linked to the steroid through the amine group have sufficient conformation and length to be capable of autocatalytic hydrolysis of the amino acid-steroid bond and release of the steroid. This can occur when the free carboxyl is generated *in vivo* by deesterification, deamidation or peptidolytic cleavage of the precursor containing a linkage between the amino acid's amine group and the steroid. Hydrolysis of the bond between an amino acid's carboxyl or amino group and the steroid can also occur by chemical or enzymatic activity, e.g., esterase cleavage or non-enzymatic hydrolysis.

"Leukopenia" means a deficiency of one or more types of leukocytes in a subject, e.g., a neutrophil, eosinophil, basophil, monocyte or dendritic cell deficiency.

"Myelomonocytic cells" mean basophils, eosinophils, neutrophils, dendritic cells and monocytes. Most of these cells are believed to be derived from the CFU-GM precursor cell, which normally resides in the bone marrow. Earlier stem cells give rise to the CFU-GM progenitor cell.

"Myelopoiesis" means any or all steps in the process that begins with that begin with the earliest precursor or stem cell and ending with the maturation of a myelomonocytic cell, which is typically observed as increased myelomonocytic cells in circulation. "Thrombopoiesis" means any or all steps in the process that begins with that begin with the earliest precursor or stem cell and ending with the maturation of a platelet, which is typically observed as increased platelets in circulation. "Erythropoiesis" means any or all steps in the process that begins with that begin with the earliest precursor or stem cell and ending with the maturation of a red cell, which is typically observed as increased red cells in circulation.

"Neutrophils" as used herein mean mature white blood cells that arise from precursor cells such as myelocytes, metamyelocytes and band forms and that has a cytoplasm that is not acidophilic or basophilic like eosinophils or basophils.

"Platelets" mean the small anucleate circulating cells that are associated with thrombus or clot formation. These cells arise from a line of precursor cells that include BFU-Mk cells, CFU-Mk cells, promegakaryoblasts and megakaryocytes.

"Red cells" or "red blood cells" mean the oxygen carrying cells in blood. They arise from precursors that include BFU-E and CFU-E cells.

Phrases such as a "subject in need" and the like mean a subject having a leukocyte, platelet or red cell deficiency. This means a cell level below that considered normal for the subject. Such deficiencies are typically seen as a reduced number of circulating leukocytes or platelets, or for red cells, a reduced red cell mass, which is typically measured as a reduced hemoglobin value, red cell count or a reduced hematocrit. Typically these deficiencies arise from acquired causes, e.g., autoimmune responses, drug-induced toxicities, etc., and not congenital disorders.

Similarly, a subject or patient that is "subject to" (or a like phrase) a blood cell deficiency means one for which the possibility of deficiency is reasonably anticipated. Such subjects include a patient on a drug regimen with side effects that may lead to a blood cell deficiency, or a patient who will undergo surgery, a radiation therapy or a chemotherapy or who will donate blood or tissue products, e.g., a bone marrow donor (for, e.g., transplant purposes) or an apheresis donor.

When used in the context of tissue that is associated with the development, movement or function of the blood cells, "tissue" means the tissues normally associated with these functions, e.g., blood, bone marrow, lymph nodes, gut-associated lymphoid tissue, skin-associated lymphoid tissue, liver, lung, thymus or spleen.

Unless stated or implied by context, expressions of a percentage of a liquid ingredient, e.g., an excipient, in an invention composition or formulation mean the ingredient's percent by volume (v/v). Thus, 20% propylene glycol means 20% v/v propylene glycol is present in an invention composition or formulation. The amount of excipient indicated in invention compositions is not affected by the form used, e.g., NF or USP grade solvent or excipient. Thus, a composition that comprises about 30% polyethylene glycol 300 NF can instead comprise a USP counterpart.

Proteins that affect hemopoiesis such as granulocyte colony stimulating factor ("G-CSF"), granulocyte macrophage colony stimulating factor ("GM-CSF"), macrophage colony stimulating factor ("M-CSF"), interleukin 2 ("IL-2"), interleukin 3 ("IL-3"), interleukin 4 ("IL-4"), IL-6, interleukin 11 ("IL-11"), EPO, TPO, stem cell factor ("SCF"), interferon and γ-interferon refer to recombinant or naturally-produced human or animal proteins that are glycosylated or deglycosylated or any biologically active fragments, isoforms or homologs of any of these molecules. Unless otherwise specified, the nomenclature for these proteins as used herein is generally is derived from the molecules found in humans, while homologs found in other species may have a different nomenclature. Deglycosylation of any of these proteins may be complete or partial. Fragments or deglycosylated proteins are generally suitable provided that they retain at least some of biological activity, e.g., at least about 20-30% (on a mole basis) of the activity of the native molecule or preferably at least about 60%. Any of these molecules may optionally be bonded to a carrier molecule such as a serum albumin or a polymer such as polyethyleneglycol. See e.g., N.C. Daw et al., Blood 1998 91:466-474, N. Wrighton et al., Science 1996 273:458-463, S. Cwirla et al., Science 1997 276:1696-1699 and U.S. patent 6,121,238.

As used herein terms such as "neutrophil or monocyte stimulator" mean a non-protein agent that can stimulate a biological activity of neutrophils or monocytes or the number of these cells in circulation.

Invention embodiments. The formula 1 compounds encompass numerous genera and species of compounds. Any of these compounds are suitable for preventing or treating any of the conditions disclosed herein or in the cited references.

The compounds include ones wherein, for carbon atoms with two bonded variable groups, one variable group is -H and the other is another moiety. In formula 1 compounds, R⁵ and R⁶ are often independently C₁₋₄ alkyl, e.g., -CH₃, or C₁₋₄ hydroxyalkyl, e.g., -CH₂OH, where the hydroxyl group optionally comprises a moiety that can hydrolyze to give the free -OH moiety. Typically, R⁵ and R⁶ are both -CH₃, which are usually both in the β-configuration. For carbon atoms with two variable groups that are bonded to the same carbon atom (R¹, R², R³ or R⁴ at positions 3, 7, 16 and 17), each variable group is independently chosen and one of the two at each position can be -H, while one, two three or all of the others can be a moiety other than -H, e.g., -OH, -SH, a C₁₋₁₀ ester, a C₁₋₁₀ ether, a C₁₋₁₀ thioether or a carbonate. When a double bond is present at one or more of the 1-2, 4-5, 5-6 or 16-17 positions, the variable group that is bonded to the carbon at these positions is typically -H or optionally substituted C₁₋₆ alkyl, e.g., -CH₃ or -C₂H₅, optionally substituted alkenyl or optionally substituted aryl.

R¹-R⁶, R¹⁰, R¹⁵, R¹⁷ and R¹⁸ include moieties, e.g., esters, thioesters, carbonates, amino acids, peptides and/or carbamates, that are chemically and/or enzymatically hydrolyzable, often under physiological conditions. Such moieties are independently chosen. Typically these moieties will give rise to -OH, -SH or -NH₂ at these positions on the steroid nucleus.

In other embodiments, one or more of R¹-R⁶, R¹⁰, R¹⁵, R¹⁷ or R¹⁸, usually one or two, comprises an amino acid or a peptide, while the remaining groups are independently selected from the moieties defined herein. In these embodiments, the peptides are typically dimers (dipeptides) or trimers (tripeptides). For example one of R¹-R⁴ comprises an amino acid or peptide and the remaining R¹-R⁶, R¹⁰, R¹⁵, R¹⁷ or R¹⁸ independently comprise -OH, =O, an ester, an ether, a carbonate or a carbamate, while R³ is a halogen, hydroxyl or an ester and R⁵ and R⁶ independently are -H, -(CH₂)ₙ-CH₃, -(CH₂)ₙ-CH₂OH, or -(CH₂)ₙ-CH₂F, -(CH₂)₂₋₄-O-(CH₂)₂₋₄-CH₃, where n is 0, 1, 2, 3, 4, 5, 6, 7 or 8 often 0, 1, or 2, usually O. Typically the ester, carbonate or carbamate are hydrolyzable under physiological conditions.

Formula 1 compounds that comprise one or more hydrolyzable moieties typically comprise acyl groups, esters, ethers, thioethers, amides, amino acids, peptides, carbonates, monosaccharides and/or carbamates. In general, the structure of hydrolyzable moieties is not critical and can vary. In some of embodiments, these moieties contain a total of about 4 to about 10 carbon atoms and in other embodiments, these moieties comprise about 10 to about 20 carbon atoms. These hydrolyzable moieties in other embodiments comprise an organic moiety, as described above for ester, that contains 2, 3, 4, 5, 6, 7, 8, 9, 10, 11 or 12 carbon atoms and 1, 2, 3, 4, 5 or 6 independently selected heteroatoms, e.g., oxygen, nitrogen or sulfur. These hydrolyzable moieties can comprise no groups that are charged in plasma, blood, intracellular cytoplasm or in the gut, or they can comprise 1, 2, 3 or more positive, negative or positive and negative charges under one or more of these conditions. The charges may be fractional depending on the group and the conditions it is under. These hydrolyzable moieties may comprise 1, 2, 3, 4 or more substitutions at a hydrogen atom(s) and/or a carbon atom(s), e.g., -OH, protected hydroxyl, -SH, protected thiol, carboxyl, protected carboxyl, amine, protected amine, -O-, -S-, -CO-, -CS-, alkoxy, alkylthio, alkenyloxy, aryl, -OP(O)(O)-O-, -OS(O)(O)-O- and/or heterocycle. Such substitutions are independently selected.

Formula 1 compounds that comprise a hydrolyzable moiety(ies) may include one or more independently chosen -O-CHR²⁴C(O)OR²⁵, -S-CHR²⁴C(O)OR²⁵, -NH-CHR²⁴C(O)OR²⁵, -O-CHR²⁴C(S)OR²⁵, -S-CHR²⁴C(S)OR²⁵, -NH-CHR²⁴C(S)OR²⁵, -O-CHR²⁴OC(O)R²⁵, -S-CHR²⁴OC(O)R²⁵, -NH-CHR²⁴OC(O)R²⁵, -O-CHR²⁴C(O)N(R²⁵)₂, -S-CHR²⁴C(O)N(R²⁵)₂, -NH-CHR²⁴C(O)N(R²⁵)₂, -O-CHR²⁴OR²⁵, -S-CHR²⁴OR²⁵, -NH-CHR²⁴OR²⁵, -O-CHR²⁴C(R²⁵)₂CH₂OX, -S-CHR²⁴C(R²⁵)₂CH₂OX, -NH-CHR²⁴C(R²⁵)₂CH₂OX, -O-CHR²⁴C(R²⁵)₂OX, -S-CHR²⁴C(R²⁵)₂OX or -NH-CHR²⁴C(R²⁵)₂OX, groups that one or more of, e.g., R¹-R⁶, R¹⁰, R¹⁵, R¹⁷ or R¹⁸, comprise. For these hydrolyzable moieties, R²⁴ independently is -H, -CH₂-C₆H₅, - CH₂CH₂-C₆H₅, C₁₋₈ alkyl, C₂₋₈ alkenyl, aryl or heterocycle where each alkyl, alkenyl, aryl and heterocycle moiety is independently optionally substituted with 1, 2, or 3, usually 1, -O-, -S-, -NH-, halogen, aryl, -OX, -SX, -NHX, ketone (=O) or -CN moieties or the C₁₋₈ alkyl is optionally substituted with 3, 4, 5 or 6 halogens, and X is -H or a protecting group. Exemplary R²⁴ are -H, -CH₃, -C₂H₅, -CH₂-C₁₋₅ optionally substituted alkyl, -CH₂CH₂-C₁₋₄ optionally substituted alkyl and -CH₂CH₂-O-C₁₋₄ optionally substituted alkyl. R²⁵ independently is -H, -CH₂-C₆H₅, -CH₂CH₂-C₆H₅, C₁₋₁₂ alkyl, C₂₋₁₂ alkenyl, aryl, heterocycle, -CH₂-heterocycle or -CH₂-aryl, where each alkyl alkenyl, aryl, heterocycle, -CH₂-heterocycle or -CH₂-aryl moiety is independently optionally substituted with 1 or 2, usually 1, -O-, -S-, -NH-, halogen, aryl, -OX, -SX, -NHX, ketone (=O), -C(O)OX or -CN moieties or the C₁₋₁₂ alkyl, C₂₋₁₂ alkenyl or aryl, are optionally independently substituted with 3, 4, 5 or 6 halogens, where X is -H or a protecting group, or the aryl, heterocycle, -CH₂-heterocycle or -CH₂-aryl moieties are optionally independently substituted with 1, 2 or 3 C₁₋₄ alkyl moieties or with 1, 2 or 3 C₁₋₄ alkoxy moieties at the aryl moiety or at the heterocycle, usually at a ring carbon. Exemplary R²⁵ are -H, -CH₃, -C₂H₅, -C₃H₇, -C₄H₉, -C₆H₁₃, -C₆H₅, -C₆H₄OH, - C₆H₄OCH₃, -C₆H₄F, -CH₂-C₁₋₅ optionally substituted alkyl, -CH₂CH₂-C₁₋₄ optionally substituted alkyl and -CH₂CH₂-O-C₁₋₄ optionally substituted alkyl.

In some embodiments, the formula 1 compounds have the structure 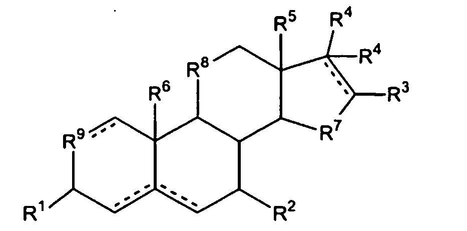 wherein, R¹, R², R³, R⁴, R⁵, R⁶ and R¹⁰ independently are -H, -OR^{PR}, -SR^{PR}, - N(R^{PR})₂, -O-Si-(R¹³)₃, -CN, -SCN -NO₂, an ester, a thioester, a phosphoester, a phosphothioester, a phosphonoester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted monosaccharide, an optionally substituted oligosaccharide, a nucleoside, a nucleotide, an oligonucleotide, a polymer, or, one more of R¹, R², R³, R⁴, R⁵, R⁶, R¹⁰, R¹⁵, R¹⁷ and R¹⁸ are =O or =S and the hydrogen atom that is bonded to the same carbon atom is absent, or, R³ and both R⁴ together comprise a structure of formula 2.

Embodiments of formula 1 compounds include or exclude any subset, species or genus of compounds within the definition of formula 1, provided that at least one compound remains. Compounds that are optionally excluded from the formula 1 compounds are one or more of 5-androstene-3β-ol-17-one (dehydroepiandrosterone), 5-androstene-3β,17β-diol, 5-androstene-3β,7β,17β-triol and derivatives that can hydrolytically convert to these compounds. Compounds that are excluded from formula 1 compounds comprises one or all compounds that are disclosed in one or more prior art references or publications, e.g., one or more compounds that are disclosed in any reference cited herein.

Exemplary embodiments of species and genera of formula 1 compounds are named as described below.

Group 1. Exemplary embodiments include the formula 1 compounds named according to the compound structures shown in Tables A and B below. Each compound named in Table B is depicted as a compound having formula B 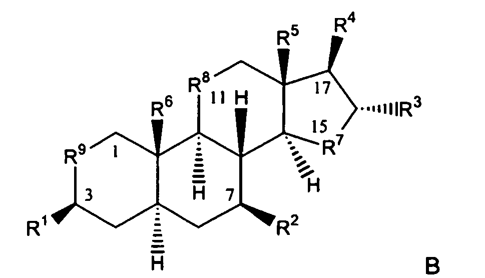 where R⁵ and R⁶ are both -CH₃, there is no double bond at the 1-2, 4-5, 5-6 or 16-17 positions, one each of R¹, R², R³ and R⁴ is hydrogen, R⁷, R⁸ and R⁹ are all -CH₂- and the other R¹, R², R³ and R⁴ are the substituents shown in Table A. The compounds named according to Tables A and B are referred to as "group 1" compounds.

Compounds named in Table B are named by numbers assigned to R¹, R², R³ and R⁴ according to the following compound naming convention, R¹.R².R³.R⁴, based on the numbered chemical substituents depicted in Table A. Each Table A number specifies a different structure for each of R¹, R², R³ and R⁴. When R¹, R², R³ or R⁴ is a divalent moiety, e.g., =O, the hydrogen at the corresponding position is absent. Thus, the group 1 compound named 1.2.1.1 is a formula B structure with a β-hydroxyl bonded to carbons at the 3- and 7-positions (the variable groups R¹ and R² respectively), an α-bromine bonded to carbon 16 (the variable group R³) and double bonded oxygen (=O) at carbon 17 (the variable group R⁴), i.e., 1.2.1.1 has the structure shown below. 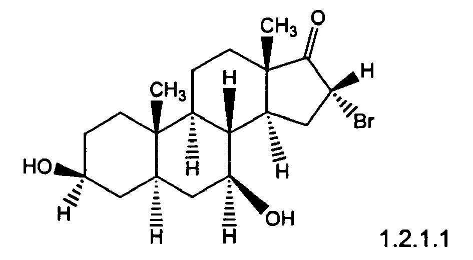 Similarly, the group 1 compound named 10.3.6.2 has the structure shown below.

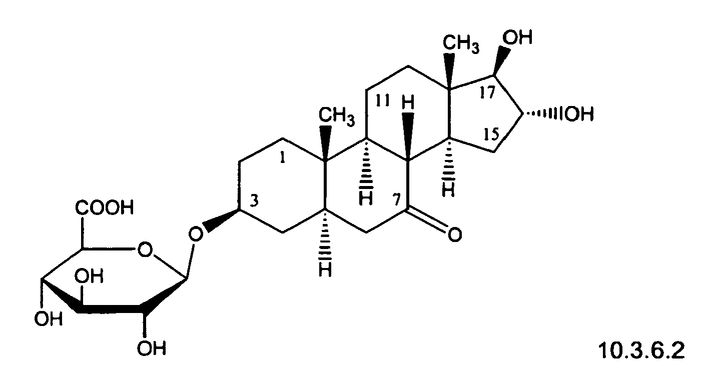

Table B names the group 1 compounds.

**TABLE A**

| **R¹** | **R²** |
|---|---|
| 1 -OH | 1 -H |
| 2 =O | 2 -OH |
| 3 -SH | 3 =O |
| 4 =S | 4 -SH |
| 5 -O-CH₃ | 5 =S |
| 6 -O-S(O)(O)-O⁻Na⁺ | 6 -O-C(O)CH₃ |
| 7 -O-S(O)(O)-O-C₂H₅ | 7 -CH₃ |
| 8 -CH₃ | 8 -OCH₃ |
| 9 -H | 9 -Cl |
| 10 -O-β-glucuronide | 10 -Br |

| **R³** | **R⁴** |
|---|---|
| 1 -Br | 1 =O |
| 2 -Cl | 2 -OH |
| 3 -I | 3 -H |
| 4 -F | 4 -SH |
| 5 -H | 5 =S |
| 6 -OH | 6 -Br |
| 7 =O | 7 -I |
| 8 -O-C(O)-CH₃ | 8 -O-C(O)-CH₃ |
| 9 -SH | 9 -O-C(O)-CH₂CH₃ |
| 10 =S | 10 -O-β-glucuronide |

**TABLE B**

| |
|---|
| 1.1.1.1, 1.1.1.2, 1.1.1.3, 1.1.1.4, 1.1.1.5, 1.1.1.6, 1.1.1.7, 1.1.1.8, 1.1.1.9, 1.1.1.10, 1.1.2.1, 1.1.2.2, 1.1.2.3, 1.1.2.4, 1.1.2.5, 1.1.2.6, 1.1.2.7, 1.1.2.8, 1.1.2.9, 1.1.2.10, 1.1.3.1, 1.1.3.2, 1.1.3.3, 1.1.3.4, 1.1.3.5, 1.1.3.6, 1.1.3.7, 1.1.3.8, 1.1.3.9, 1.1.3.10, 1.1.4.1, 1.1.4.2, 1.1.4.3, 1.1.4.4, 1.1.4.5, 1.1.4.6, 1.1.4.7, 1.1.4.8, 1.1.4.9, 1.1.4.10, 1.1.5.1, 1.1.5.2, 1.1.5.3, 1.1.5.4, 1.1.5.5, 1.1.5.6, 1.1.5.7, 1.1.5.8, 1.1.5.9, 1.1.5.10, 1.1.6.1, 1.1.6.2, 1.1.6.3, 1.1.6.4, 1.1.6.5, 1.1.6.6, 1.1.6.7, 1.1.6.8, 1.1.6.9, 1.1.6.10, 1.1.7.1, 1.1.7.2, 1.1.7.3, 1.1.7.4, 1.1.7.5, 1.1.7.6, 1.1.7.7, 1.1.7.8, 1.1.7.9, 1.1.7.10, 1.1.8.1, 1.1.8.2, 1.1.8.3, 1.1.8.4, 1.1.8.5, 1.1.8.6, 1.1.8.7, 1.1.8.8, 1.1.8.9, 1.1.8.10, 1.1.9.1, 1.1.9.2, 1.1.9.3, 1.1.9.4, 1.1.9.5, 1.1.9.6, 1.1.9.7, 1.1.9.8, 1.1.9.9, 1.1.9.10, 1.1.10.1, 1.1.10.2, 1.1.10.3, 1.1.10.4, 1.1.10.5, 1.1.10.6, 1.1.10.7, 1.1.10.8, 1.1.10.9, 1.1.10.10, 1.2.1.1, 1.2.1.2, 1.2.1.3, 1.2.1.4, 1.2.1.5, 1.2.1.6, 1.2.1.7, 1.2.1.8, 1.2.1.9, 1.2.1.10, 1.2.2.1, 1.2.2.2, 1.2.2.3, 1.2.2.4, 1.2.2.5, 1.2.2.6, |
| 1.2.2.7, 1.2.2.8, 1.2.2.9, 1.2.2.10, 1.2.3.1, 1.2.3.2, 1.2.3.3, 1.2.3.4, 1.2.3.5, 1.2.3.6, 1.2.3.7, 1.2.3.8, 1.2.3.9, 1.2.3.10, 1.2.4.1, 1.2.4.2, 1.2.4.3, 1.2.4.4, 1.2.4.5, 1.2.4.6, 1.2.4.7, 1.2.4.8, 1.2.4.9, 1.2.4.10, 1.2.5.1, 1.2.5.2, 1.2.5.3, 1.2.5.4, 1.2.5.5, 1.2.5.6, 1.2.5.7, 1.2.5.8, 1.2.5.9, 1.2.5.10, 1.2.6.1, 1.2.6.2, 1.2.6.3, 1.2.6.4, 1.2.6.5, 1.2.6.6, 1.2.6.7, 1.2.6.8, 1.2.6.9, 1.2.6.10, 1.2.7.1, 1.2.7.2, 1.2.7.3, 1.2.7.4, 1.2.7.5, 1.2.7.6, 1.2.7.7, 1.2.7.8, 1.2.7.9, 1.2.7.10, 1.2.8.1, 1.2.8.2, 1.2.8.3, 1.2.8.4, 1.2.8.5, 1.2.8.6, 1.2.8.7, 1.2.8.8, 1.2.8.9, 1.2.8.10, 1.2.9.1, 1.2.9.2, 1.2.9.3, 1.2.9.4, 1.2.9.5, 1.2.9.6, 1.2.9.7, 1.2.9.8, 1.2.9.9, 1.2.9.10, 1.2.10.1, 1.2.10.2, 1.2.10.3, 1.2.10.4, 1.2.10.5, 1.2.10.6, 1.2.10.7, 1.2.10.8, 1.2.10.9, 1.2.10.10, 1.3.1.1, 1.3.1.2, 1.3.1.3, 1.3.1.4, 1.3.1.5, 1.3.1.6, 1.3.1.7, 1.3.1.8, 1.3.1.9, 1.3.1.10, 1.3.2.1, 1.3.2.2, 1.3.2.3, 1.3.2.4, 1.3.2.5, 1.3.2.6, 1.3.2.7, 1.3.2.8, 1.3.2.9, 1.3.2.10, 1.3.3.1, 1.3.3.2, 1.3.3.3, 1.3.3.4, 1.3.3.5, 1.3.3.6, 1.3.3.7, 1.3.3.8, 1.3.3.9, 1.3.3.10, 1.3.4.1, 1.3.4.2, 1.3.4.3, 1.3.4.4, 1.3.4.5, 1.3.4.6, 1.3.4.7, 1.3.4.8, 1.3.4.9, 1.3.4.10, 1.3.5.1, 1.3.5.2, 1.3.5.3, 1.3.5.4, 1.3.5.5, 1.3.5.6, 1.3.5.7, 1.3.5.8, 1.3.5.9, 1.3.5.10, 1.3.6.1, 1.3.6.2, 1.3.6.3, 1.3.6.4, 1.3.6.5, 1.3.6.6, 1.3.6.7, 1.3.6.8, 1.3.6.9, 1.3.6.10, 1.3.7.1, 1.3.7.2, 1.3.7.3, 1.3.7.4, 1.3.7.5, 1.3.7.6, 1.3.7.7, 1.3.7.8, 1.3.7.9, 1.3.7.10, 1.3.8.1, 1.3.8.2, 1.3.8.3, 1.3.8.4, 1.3.8.5, 1.3.8.6, 1.3.8.7, 1.3.8.8, 1.3.8.9, 1.3.8.10, 1.3.9.1, 1.3.9.2, 1.3.9.3, 1.3.9.4, 1.3.9.5, 1.3.9.6, 1.3.9.7, 1.3.9.8, 1.3.9.9, 1.3.9.10, 1.3.10.1, 1.3.10.2, 1.3.10.3, 1.3.10.4, 1.3.10.5, 1.3.10.6, 1.3.10.7, 1.3.10.8, 1.3.10.9, 1.3.10.10, 1.4.1.1, 1.4.1.2, 1.4.1.3, 1.4.1.4, 1.4.1.5, 1.4.1.6, 1.4.1.7, 1.4.1.8, 1.4.1.9, 1.4.1.10, 1.4.2.1, 1.4.2.2, 1.4.2.3, 1.4.2.4, 1.4.2.5, 1.4.2.6, 1.4.2.7, 1.4.2.8, 1.4.2.9, 1.4.2.10, 1.4.3.1, 1.4.3.2, 1.4.3.3, 1.4.3.4, 1.4.3.5, 1.4.3.6, 1.4.3.7, 1.4.3.8, 1.4.3.9, 1.4.3.10, 1.4.4.1, 1.4.4.2, 1.4.4.3, 1.4.4.4, 1.4.4.5, 1.4.4.6, 1.4.4.7, 1.4.4.8, 1.4.4.9, 1.4.4.10, 1.4.5.1, 1.4.5.2, 1.4.5.3, 1.4.5.4, 1.4.5.5, 1.4.5.6, 1.4.5.7, 1.4.5.8, 1.4.5.9, 1.4.5.10, 1.4.6.1, 1.4.6.2, 1.4.6.3, 1.4.6.4, 1.4.6.5, 1.4.6.6, 1.4.6.7, 1.4.6.8, 1.4.6.9, 1.4.6.10, 1.4.7.1, 1.4.7.2, 1.4.7.3, 1.4.7.4, 1.4.7.5, 1.4.7.6, 1.4.7.7, 1.4.7.8, 1.4.7.9, 1.4.7.10, 1.4.8.1, 1.4.8.2, 1.4.8.3, 1.4.8.4, 1.4.8.5, 1.4.8.6, 1.4.8.7, 1.4.8.8, 1.4.8.9, 1.4.8.10, 1.4.9.1, 1.4.9.2, 1.4.9.3, 1.4.9.4, 1.4.9.5, 1.4.9.6, 1.4.9.7, 1.4.9.8, 1.4.9.9, 1.4.9.10, 1.4.10.1, 1.4.10.2, 1.4.10.3, 1.4.10.4, 1.4.10.5, 1.4.10.6, 1.4.10.7, 1.4.10.8, 1.4.10.9, 1.4.10.10, 1.5.1.1, 1.5.1.2, 1.5.1.3, 1.5.1.4, 1.5.1.5, 1.5.1.6, 1.5.1.7, 1.5.1.8, 1.5.1.9, 1.5.1.10, 1.5.2.1, 1.5.2.2, 1.5.2.3, 1.5.2.4, 1.5.2.5, 1.5.2.6, 1.5.2.7, 1.5.2.8, 1.5.2.9, 1.5.2.10, 1.5.3.1, 1.5.3.2, 1.5.3.3, 1.5.3.4, 1.5.3.5, 1.5.3.6, 1.5.3.7, 1.5.3.8, 1.5.3.9, 1.5.3.10, 1.5.4.1, 1.5.4.2, 1.5.4.3, 1.5.4.4, 1.5.4.5, 1.5.4.6, 1.5.4.7, 1.5.4.8, 1.5.4.9, 1.5.4.10, 1.5.5.1, 1.5.5.2, 1.5.5.3, 1.5.5.4, 1.5.5.5, 1.5.5.6, 1.5.5.7, 1.5.5.8, 1.5.5.9, 1.5.5.10, 1.5.6.1, 1.5.6.2, 1.5.6.3, 1.5.6.4, 1.5.6.5, 1.5.6.6, 1.5.6.7, 1.5.6.8, 1.5.6.9, 1.5.6.10, 1.5.7.1, 1.5.7.2, 1.5.7.3, 1.5.7.4, 1.5.7.5, 1.5.7.6, 1.5.7.7, 1.5.7.8, 1.5.7.9, 1.5.7.10, 1.5.8.1, 1.5.8.2, 1.5.8.3, 1.5.8.4, 1.5.8.5, 1.5.8.6, 1.5.8.7, 1.5.8.8, 1.5.8.9, 1.5.8.10, 1.5.9.1, 1.5.9.2, 1.5.9.3, 1.5.9.4, 1.5.9.5, 1.5.9.6, 1.5.9.7, |
| 1.5.9.8, 1.5.9.9, 1.5.9.10, 1.5.10.1, 1.5.10.2, 1.5.10.3, 1.5.10.4, 1.5.10.5, 1.5.10.6, 1.5.10.7, 1.5.10.8, 1.5.10.9, 1.5.10.10, 1.6.1.1, 1.6.1.2, 1.6.1.3, 1.6.1.4, 1.6.1.5, 1.6.1.6, 1.6.1.7, 1.6.1.8, 1.6.1.9, 1.6.1.10, 1.6.2.1, 1.6.2.2, 1.6.2.3, 1.6.2.4, 1.6.2.5, 1.6.2.6, 1.6.2.7, 1.6.2.8, 1.6.2.9, 1.6.2.10, 1.6.3.1, 1.6.3.2, 1.6.3.3, 1.6.3.4, 1.6.3.5, 1.6.3.6, 1.6.3.7, 1.6.3.8, 1.6.3.9, 1.6.3.10, 1.6.4.1, 1.6.4.2, 1.6.4.3, 1.6.4.4, 1.6.4.5, 1.6.4.6, 1.6.4.7, 1.6.4.8, 1.6.4.9, 1.6.4.10, 1.6.5.1, 1.6.5.2, 1.6.5.3, 1.6.5.4, 1.6.5.5, 1.6.5.6, 1.6.5.7, 1.6.5.8, 1.6.5.9, 1.6.5.10, 1.6.6.1, 1.6.6.2, 1.6.6.3, 1.6.6.4, 1.6.6.5, 1.6.6.6, 1.6.6.7, 1.6.6.8, 1.6.6.9, 1.6.6.10, 1.6.7.1, 1.6.7.2, 1.6.7.3, 1.6.7.4, 1.6.7.5, 1.6.7.6, 1.6.7.7, 1.6.7.8, 1.6.7.9, 1.6.7.10, 1.6.8.1, 1.6.8.2, 1.6.8.3, 1.6.8.4, 1.6.8.5, 1.6.8.6, 1.6.8.7, 1.6.8.8, 1.6.8.9, 1.6.8.10, 1.6.9.1, 1.6.9.2, 1.6.9.3, 1.6.9.4, 1.6.9.5, 1.6.9.6, 1.6.9.7, 1.6.9.8, 1.6.9.9, 1.6.9.10, 1.6.10.1, 1.6.10.2, 1.6.10.3, 1.6.10.4, 1.6.10.5, 1.6.10.6, 1.6.10.7, 1.6.10.8, 1.6.10.9, 1.6.10.10, 1.7.1.1, 1.7.1.2, 1.7.1.3, 1.7.1.4, 1.7.1.5, 1.7.1.6, 1.7.1.7, 1.7.1.8, 1.7.1.9, 1.7.1.10, 1.7.2.1, 1.7.2.2, 1.7.2.3, 1.7.2.4, 1.7.2.5, 1.7.2.6, 1.7.2.7, 1.7.2.8, 1.7.2.9, 1.7.2.10, 1.7.3.1, 1.7.3.2, 1.7.3.3, 1.7.3.4, 1.7.3.5, 1.7.3.6, 1.7.3.7, 1.7.3.8, 1.7.3.9, 1.7.3.10, 1.7.4.1, 1.7.4.2, 1.7.4.3, 1.7.4.4, 1.7.4.5, 1.7.4.6, 1.7.4.7, 1.7.4.8, 1.7.4.9, 1.7.4.10, 1.7.5.1, 1.7.5.2, 1.7.5.3, 1.7.5.4, 1.7.5.5, 1.7.5.6, 1.7.5.7, 1.7.5.8, 1.7.5.9, 1.7.5.10, 1.7.6.1, 1.7.6.2, 1.7.6.3, 1.7.6.4, 1.7.6.5, 1.7.6.6, 1.7.6.7, 1.7.6.8, 1.7.6.9, 1.7.6.10, 1.7.7.1, 1.7.7.2, 1.7.7.3, 1.7.7.4, 1.7.7.5, 1.7.7.6, 1.7.7.7, 1.7.7.8, 1.7.7.9, 1.7.7.10, 1.7.8.1, 1.7.8.2, 1.7.8.3, 1.7.8.4, 1.7.8.5, 1.7.8.6, 1.7.8.7, 1.7.8.8, 1.7.8.9, 1.7.8.10, 1.7.9.1, 1.7.9.2, 1.7.9.3, 1.7.9.4, 1.7.9.5, 1.7.9.6, 1.7.9.7, 1.7.9.8, 1.7.9.9, 1.7.9.10, 1.7.10.1, 1.7.10.2, 1.7.10.3, 1.7.10.4, 1.7.10.5, 1.7.10.6, 1.7.10.7, 1.7.10.8, 1.7.10.9, 1.7.10.10, 1.8.1.1, 1.8.1.2, 1.8.1.3, 1.8.1.4, 1.8.1.5, 1.8.1.6, 1.8.1.7, 1.8.1.8, 1.8.1.9, 1.8.1.10, 1.8.2.1, 1.8.2.2, 1.8.2.3, 1.8.2.4, 1.8.2.5, 1.8.2.6, 1.8.2.7, 1.8.2.8, 1.8.2.9, 1.8.2.10, 1.8.3.1, 1.8.3.2, 1.8.3.3, 1.8.3.4, 1.8.3.5, 1.8.3.6, 1.8.3.7, 1.8.3.8, 1.8.3.9, 1.8.3.10, 1.8.4.1, 1.8.4.2, 1.8.4.3, 1.8.4.4, 1.8.4.5, 1.8.4.6, 1.8.4.7, 1.8.4.8, 1.8.4.9, 1.8.4.10, 1.8.5.1, 1.8.5.2, 1.8.5.3, 1.8.5.4, 1.8.5.5, 1.8.5.6, 1.8.5.7, 1.8.5.8, 1.8.5.9, 1.8.5.10, 1.8.6.1, 1.8.6.2, 1.8.6.3, 1.8.6.4, 1.8.6.5, 1.8.6.6, 1.8.6.7, 1.8.6.8, 1.8.6.9, 1.8.6.10, 1.8.7.1, 1.8.7.2, 1.8.7.3, 1.8.7.4, 1.8.7.5, 1.8.7.6, 1.8.7.7, 1.8.7.8, 1.8.7.9, 1.8.7.10, 1.8.8.1, 1.8.8.2, 1.8.8.3, 1.8.8.4, 1.8.8.5, 1.8.8.6, 1.8.8.7, 1.8.8.8, 1.8.8.9, 1.8.8.10, 1.8.9.1, 1.8.9.2, 1.8.9.3, 1.8.9.4, 1.8.9.5, 1.8.9.6, 1.8.9.7, 1.8.9.8, 1.8.9.9, 1.8.9.10, 1.8.10.1, 1.8.10.2, 1.8.10.3, 1.8.10.4, 1.8.10.5, 1.8.10.6, 1.8.10.7, 1.8.10.8, 1.8.10.9, 1.8.10.10, 1.9.1.1, 1.9.1.2, 1.9.1.3, 1.9.1.4, 1.9.1.5, 1.9.1.6, 1.9.1.7, 1.9.1.8, 1.9.1.9, 1.9.1.10, 1.9.2.1, 1.9.2.2, 1.9.2.3, 1.9.2.4, 1.9.2.5, 1.9.2.6, 1.9.2.7, 1.9.2.8, 1.9.2.9, 1.9.2.10, 1.9.3.1, 1.9.3.2, 1.9.3.3, 1.9.3.4, 1.9.3.5, 1.9.3.6, 1.9.3.7, 1.9.3.8, 1.9.3.9, 1.9.3.10, 1.9.4.1, 1.9.4.2, 1.9.4.3, 1.9.4.4, 1.9.4.5, 1.9.4.6, 1.9.4.7, 1.9.4.8, 1.9.4.9, 1.9.4.10, 1.9.5.1, 1.9.5.2, 1.9.5.3, 1.9.5.4, 1.9.5.5, 1.9.5.6, 1.9.5.7, 1.9.5.8, 1.9.5.9, 1.9.5.10, 1.9.6.1, 1.9.6.2, 1.9.6.3, 1.9.6.4, 1.9.6.5, 1.9.6.6, 1.9.6.7, 1.9.6.8, 1.9.6.9, 1.9.6.10, 1.9.7.1, 1.9.7.2, 1.9.7.3, 1.9.7.4, 1.9.7.5, 1.9.7.6, 1.9.7.7, 1.9.7.8, 1.9.7.9, 1.9.7.10, 1.9.8.1, 1.9.8.2, 1.9.8.3, 1.9.8.4, 1.9.8.5, 1.9.8.6, 1.9.8.7, 1.9.8.8, 1.9.8.9, 1.9.8.10, 1.9.9.1, 1.9.9.2, 1.9.9.3, 1.9.9.4, 1.9.9.5, 1.9.9.6, 1.9.9.7, 1.9.9.8, 1.9.9.9, |
| 1.9.9.10, 1.9.10.1, 1.9.10.2, 1.9.10.3, 1.9.10.4, 1.9.10.5, 1.9.10.6, 1.9.10.7, 1.9.10.8, 1.9.10.9, 1.9.10.10, 1.10.1.1, 1.1 0.1.2, 1.1 0.1.3, 1.10.1.4, 1.10.1.5, 1.10.1.6, 1.10.1.7, 1.10.1.8, 1.10.1.9, 1.10.1.10, 1.10.2.1, 1.10.2.2, 1.10.2.3, 1.10.2.4, 1.10.2.5, 1.10.2.6, 1.10.2.7, 1.10.2.8, 1.10.2.9, 1.10.2.10, 1.10.3.1, 1.10.3.2, 1.10.3.3, 1.10.3.4, 1.10.3.5, 1.10.3.6, 1.10.3.7, 1.10.3.8, 1.10.3.9, 1.10.3.10, 1.10.4.1, 1.10.4.2, 1.10.4.3, 1.10.4.4, 1.10.4.5, 1.10.4.6, 1.10.4.7, 1.10.4.8, 1.10.4.9, 1.10.4.10, 1.10.5.1, 1.10.5.2, 1.10.5.3, 1.10.5.4, 1.10.5.5, 1.10.5.6, 1.10.5.7, 1.10.5.8, 1.10.5.9, 1.10.5.10, 1.10.6.1, 1.10.6.2, 1.10.6.3, 1.10.6.4, 1.10.6.5, 1.10.6.6, 1.10.6.7, 1.10.6.8, 1.10.6.9, 1.10.6.10, 1.10.7.1, 1.10.7.2, 1.10.7.3, 1.10.7.4, 1.10.7.5, 1.10.7.6, 1.10.7.7, 1.10.7.8, 1.10.7.9, 1.10.7.10, 1.10.8.1, 1.10.8.2, 1.10.8.3, 1.10.8.4, 1.10.8.5, 1.10.8.6, 1.10.8.7, 1.10.8.8, 1.10.8.9, 1. 10.8.10, 1.10.9.1, 1.10.9.2, 1.10.9.3, 1.10.9.4, 1.10.9.5, 1.10.9.6, 1.10.9.7, 1.10.9.8, 1.10.9.9, 1.10.9.10, 1.10.10.1, 1.10.10.2, 1.10.10.3, 1.10.10.4, 1.10.10.5, 1.10.10.6, 1.10.10.7, 1.10.10.8, 1.10.10.9, 1.10.10.10, 2.1.1.1, 2.1.1.2, 2.1.1.3, 2.1.1.4, 2.1.1.5, 2.1.1.6, 2.1.1.7, 2.1.1.8, 2.1.1.9, 2.1.1.10, 2.1.2.1, 2.1.2.2, 2.1.2.3, 2.1.2.4, 2.1.2.5, 2.1.2.6, 2.1.2.7, 2.1.2.8, 2.1.2.9, 2.1.2.10, 2.1.3.1, 2.1.3.2, 2.1.3.3, 2.1.3.4, 2.1.3.5, 2.1.3.6, 2.1.3.7, 2.1.3.8, 2.1.3.9, 2.1.3.10, 2.1.4.1, 2.1.4.2, 2.1.4.3, 2.1.4.4, 2.1.4.5, 2.1.4.6, 2.1.4.7, 2.1.4.8, 2.1.4.9, 2.1.4.10, 2.1.5.1, 2.1.5.2, 2.1.5.3, 2.1.5.4, 2.1.5.5, 2.1.5.6, 2.1.5.7, 2.1.5.8, 2.1.5.9, 2.1.5.10, 2.1.6.1, 2.1.6.2, 2.1.6.3, 2.1.6.4, 2.1.6.5, 2.1.6.6, 2.1.6.7, 2.1.6.8, 2.1.6.9, 2.1.6.10, 2.1.7.1, 2.1.7.2, 2.1.7.3, 2.1.7.4, 2.1.7.5, 2.1.7.6, 2.1.7.7, 2.1.7.8, 2.1.7.9, 2.1.7.10, 2.1.8.1, 2.1.8.2, 2.1.8.3, 2.1.8.4, 2.1.8.5, 2.1.8.6, 2.1.8.7, 2.1.8.8, 2.1.8.9, 2.1.8.10, 2.1.9.1, 2.1.9.2, 2.1.9.3, 2.1.9.4, 2.1.9.5, 2.1.9.6, 2.1.9.7, 2.1.9.8, 2.1.9.9, 2.1.9.10, 2.1.10.1, 2.1.10.2, 2.1.10.3, 2.1.10.4, 2.1.10.5, 2.1.10.6, 2.1.10.7, 2.1.10.8, 2.1.10.9, 2.1.10.10, 2.2.1.1, 2.2.1.2, 2.2.1.3, 2.2.1.4, 2.2.1.5, 2.2.1.6, 2.2.1.7, 2.2.1.8, 2.2.1.9, 2.2.1.10, 2.2.2.1, 2.2.2.2, 2.2.2.3, 2.2.2.4, 2.2.2.5, 2.2.2.6, 2.2.2.7, 2.2.2.8, 2.2.2.9, 2.2.2.10, 2.2.3.1, 2.2.3.2, 2.2.3.3, 2.2.3.4, 2.2.3.5, 2.2.3.6, 2.2.3.7, 2.2.3.8, 2.2.3.9, 2.2.3.10, 2.2.4.1, 2.2.4.2, 2.2.4.3, 2.2.4.4, 2.2.4.5, 2.2.4.6, 2.2.4.7, 2.2.4.8, 2.2.4.9, 2.2.4.10, 2.2.5.1, 2.2.5.2, 2.2.5.3, 2.2.5.4, 2.2.5.5, 2.2.5.6, 2.2.5.7, 2.2.5.8, 2.2.5.9, 2.2.5.10, 2.2.6.1, 2.2.6.2, 2.2.6.3, 2.2.6.4, 2.2.6.5, 2.2.6.6, 2.2.6.7, 2.2.6.8, 2.2.6.9, 2.2.6.10, 2.2.7.1, 2.2.7.2, 2.2.7.3, 2.2.7.4, 2.2.7.5, 2.2.7.6, 2.2.7.7, 2.2.7.8, 2.2.7.9, |
| 2.2.7.10, 2.2.8.1, 2.2.8.2, 2.2.8.3, 2.2.8.4, 2.2.8.5, 2.2.8.6, 2.2.8.7, 2.2.8.8, 2.2.8.9, 2.2.8.10, 2.2.9.1, 2.2.9.2, 2.2.9.3, 2.2.9.4, 2.2.9.5, 2.2.9.6, 2.2.9.7, 2.2.9.8, 2.2.9.9, 2.2.9.10, 2.2.10.1, 2.2.10.2, 2.2.10.3, 2.2.10.4, 2.2.10.5, 2.2.10.6, 2.2.10.7, 2.2.10.8, 2.2.10.9, 2.2.10.10, 2.3.1.1, 2.3.1.2, 2.3.1.3, 2.3.1.4, 2.3.1.5, 2.3.1.6, 2.3.1.7, 2.3.1.8, 2.3.1.9, 2.3.1.10, 2.3.2.1, 2.3.2.2, 2.3.2.3, 2.3.2.4, 2.3.2.5, 2.3.2.6, 2.3.2.7, 2.3.2.8, 2.3.2.9, 2.3.2.10, 2.3.3.1, 2.3.3.2, 2.3.3.3, 2.3.3.4, 2.3.3.5, 2.3.3.6, 2.3.3.7, 2.3.3.8, 2.3.3.9, 2.3.3.10, 2.3.4.1, 2.3.4.2, 2.3.4.3, 2.3.4.4, 2.3.4.5, 2.3.4.6, 2.3.4.7, 2.3.4.8, 2.3.4.9, 2.3.4.10, 2.3.5.1, 2.3.5.2, 2.3.5.3, 2.3.5.4, 2.3.5.5, 2.3.5.6, 2.3.5.7, 2.3.5.8, 2.3.5.9, 2.3.5.10, 2.3.6.1, 2.3.6.2, 2.3.6.3, 2.3.6.4, 2.3.6.5, 2.3.6.6, 2.3.6.7, 2.3.6.8, 2.3.6.9 2.3.6.10, 2.3.7.1, 2.3.7.2, 2.3.7.3, 2.3.7.4, 2.3.7.5, 2.3.7.6, 2.3.7.7, 2.3.7.8, 2.3.7.9, 2.3.7.10, 2.3.8.1, 2.3.8.2, 2.3.8.3, 2.3.8.4, 2.3.8.5, 2.3.8.6, 2.3.8.7, 2.3.8.8, 2.3.8.9, 2.3.8.10, 2.3.9.1, 2.3.9.2, 2.3.9.3, 2.3.9.4, 2.3.9.5, 2.3.9.6, 2.3.9.7, 2.3.9.8, 2.3.9.9, 2.3.9.10, 2.3.10.1, 2.3.10.2, 2.3.10.3, 2.3.10.4, 2.3.10.5, 2.3.10.6, 2.3.10.7, 2.3.10.8, 2.3.10.9, 2.3.10.10, 2.4.1.1, 2.4.1.2, 2.4.1.3, 2.4.1.4, 2.4.1.5, 2.4.1.6, 2.4.1.7, 2.4.1.8, 2.4.1.9, 2.4.1.10, 2.4.2.1, 2.4.2.2, 2.4.2.3, 2.4.2.4, 2.4.2.5, 2.4.2.6, 2.4.2.7, 2.4.2.8, 2.4.2.9, 2.4.2.10, 2.4.3.1, 2.4.3.2, 2.4.3.3, 2.4.3.4, 2.4.3.5, 2.4.3.6, 2.4.3.7, 2.4.3.8, 2.4.3.9, 2.4.3.10, 2.4.4.1, 2.4.4.2, 2.4.4.3, 2.4.4.4, 2.4.4.5, 2.4.4.6, 2.4.4.7, 2.4.4.8, 2.4.4.9, 2.4.4.10, 2.4.5.1, 2.4.5.2, 2.4.5.3, 2.4.5.4, 2.4.5.5, 2.4.5.6, 2.4.5.7, 2.4.5.8, 2.4.5.9, 2.4.5.10, 2.4.6.1, 2.4.6.2, 2.4.6.3, 2.4.6.4, 2.4.6.5, 2.4.6.6, 2.4.6.7, 2.4.6.8, 2.4.6.9, 2.4.6.10, 2.4.7.1, 2.4.7.2, 2.4.7.3, 2.4.7.4, 2.4.7.5, 2.4.7.6, 2.4.7.7, 2.4.7.8, 2.4.7.9, 2.4.7.10, 2.4.8.1, 2.4.8.2, 2.4.8.3, 2.4.8.4, 2.4.8.5, 2.4.8.6, 2.4.8.7, 2.4.8.8, 2.4.8.9, 2.4.8.10, 2.4.9.1, 2.4.9.2, 2.4.9.3, 2.4.9.4, 2.4.9.5, 2.4.9.6, 2.4.9.7, 2.4.9.8, 2.4.9.9, 2.4.9.10, 2.4.10.1, 2.4.10.2, 2.4.10.3, 2.4.10.4, 2.4.10.5, 2.4.10.6, 2.4.10.7, 2.4.10.8, 2.4.10.9, 2.4.10.10, 2.5.1.1, 2.5.1.2, 2.5.1.3, 2.5.1.4, 2.5.1.5, 2.5.1.6, 2.5.1.7, 2.5.1.8, 2.5.1.9, 2.5.1.10, 2.5.2.1, 2.5.2.2, 2.5.2.3, 2.5.2.4, 2.5.2.5, 2.5.2.6, 2.5.2.7, 2.5.2.8, 2.5.2.9, 2.5.2.10, 2.5.3.1, 2.5.3.2, 2.5.3.3, 2.5.3.4, 2.5.3.5, 2.5.3.6, 2.5.3.7, 2.5.3.8, 2.5.3.9, 2.5.3.10, 2.5.4.1, 2.5.4.2, 2.5.4.3, 2.5.4.4, 2.5.4.5, 2.5.4.6, 2.5.4.7, 2.5.4.8, 2.5.4.9, 2.5.4.10, 2.5.5.1, 2.5.5.2, 2.5.5.3, 2.5.5.4, 2.5.5.5, 2.5.5.6, 2.5.5.7, 2.5.5.8, 2.5.5.9, 2.5.5.10, 2.5.6.1, 2.5.6.2, 2.5.6.3, 2.5.6.4, 2.5.6.5, 2.5.6.6, 2.5.6.7, 2.5.6.8, 2.5.6.9, 2.5.6.10, 2.5.7.1, 2.5.7.2, 2.5.7.3, 2.5.7.4, 2.5.7.5, 2.5.7.6, 2.5.7.7, 2.5.7.8, 2.5.7.9, 2.5.7.10, 2.5.8.1, 2.5.8.2, 2.5.8.3, 2.5.8.4, 2.5.8.5, 2.5.8.6, 2.5.8.7, 2.5.8.8, 2.5.8.9, 2.5.8.10, 2.5.9.1, 2.5.9.2, 2.5.9.3, 2.5.9.4, 2.5.9.5, 2.5.9.6, 2.5.9.7, 2.5.9.8, 2.5.9.9, 2.5.9.10, 2.5.10.1, 2.5.10.2, 2.5.10.3, 2.5.10.4, 2.5.10.5, 2.5.10.6, 2.5.10.7, 2.5.10.8, 2.5.10.9, 2.5.10.10, 2.6.1.1, 2.6.1.2, 2.6.1.3, 2.6.1.4, 2.6.1.5, 2.6.1.6, 2.6.1.7, 2.6.1.8, 2.6.1.9, 2.6.1.10, 2.6.2.1, 2.6.2.2, 2.6.2.3, 2.6.2.4, 2.6.2.5, 2.6.2.6, 2.6.2.7, 2.6.2.8, 2.6.2.9, 2.6.2.10, 2.6.3.1, 2.6.3.2, 2.6.3.3, 2.6.3.4, 2.6.3.5, 2.6.3.6, 2.6.3.7, 2.6.3.8, 2.6.3.9, 2.6.3.10, 2.6.4.1, 2.6.4.2, 2.6.4.3, 2.6.4.4, 2.6.4.5, 2.6.4.6, 2.6.4.7, 2.6.4.8, 2.6.4.9, 2.6.4.10, 2.6.5.1, 2.6.5.2, 2.6.5.3, 2.6.5.4, 2.6.5.5, 2.6.5.6, 2.6.5.7, 2.6.5.8, 2.6.5.9, 2.6.5.10, 2.6.6.1, 2.6.6.2, 2.6.6.3, 2.6.6.4, 2.6.6.5, 2.6.6.6, 2.6.6.7, 2.6.6.8, 2.6.6.9, 2.6.6.10, 2.6.7.1, 2.6.7.2, 2.6.7.3, 2.6.7.4, 2.6.7.5, 2.6.7.6, 2.6.7.7, 2.6.7.8, 2.6.7.9, 2.6.7.10, 2.6.8.1, 2.6.8.2, 2.6.8.3, 2.6.8.4, 2.6.8.5, 2.6.8.6, 2.6.8.7, 2.6.8.8, 2.6.8.9, 2.6.8.10, 2.6.9.1, 2.6.9.2, 2.6.9.3, 2.6.9.4, 2.6.9.5, 2.6.9.6, 2.6.9.7, 2.6.9.8, 2.6.9.9, 2.6.9.10, 2.6.10.1, 2.6.10.2, 2.6.10.3, 2.6.10.4, 2.6.10.5, 2.6.10.6, 2.6.10.7, 2.6.10.8, 2.6.10.9, 2.6.10.10, 2.7.1.1, 2.7.1.2, 2.7.1.3, 2.7.1.4, 2.7.1.5, 2.7.1.6, 2.7.1.7, 2.7.1.8, 2.7.1.9, 2.7.1.10, 2.7.2.1, 2.7.2.2, 2.7.2.3, 2.7.2.4, 2.7.2.5, 2.7.2.6, 2.7.2.7, 2.7.2.8, 2.7.2.9, 2.7.2.10, 2.7.3.1, 2.7.3.2, 2.7.3.3, 2.7.3.4, 2.7.3.5, 2.7.3.6, 2.7.3.7, 2.7.3.8, 2.7.3.9, 2.7.3.10, 2.7.4.1, 2.7.4.2, 2.7.4.3, 2.7.4.4, 2.7.4.5, 2.7.4.6, 2.7.4.7, 2.7.4.8, 2.7.4.9, 2.7.4.10, 2.7.5.1, 2.7.5.2, 2.7.5.3, |
| 2.7.5.4, 2.7.5.5, 2.7.5.6, 2.7.5.7, 2.7.5.8, 2.7.5.9, 2.7.5.10, 2.7.6.1, 2.7.6.2, 2.7.6.3, 2.7.6.4, 2.7.6.5, 2.7.6.6, 2.7.6.7, 2.7.6.8, 2.7.6.9, 2.7.6.10, 2.7.7.1, 2.7.7.2, 2.7.7.3, 2.7.7.4, 2.7.7.5, 2.7.7.6, 2.7.7.7, 2.7.7.8, 2.7.7.9, 2.7.7.10, 2.7.8.1, 2.7.8.2, 2.7.8.3, 2.7.8.4, 2.7.8.5, 2.7.8.6, 2.7.8.7, 2.7.8.8, 2.7.8.9, 2.7.8.10, 2.7.9.1, 2.7.9.2, 2.7.9.3, 2.7.9.4, 2.7.9.5, 2.7.9.6, 2.7.9.7, 2.7.9.8, 2.7.9.9, 2.7.9.10, 2.7.10.1, 2.7.10.2, 2.7.10.3, 2.7.10.4, 2.7.10.5, 2.7.10.6, 2.7.10.7, 2.7.10.8, 2.7.10.9, 2.7.10.10, 2.8.1.1, 2.8.1.2, 2.8.1.3, 2.8.1.4, 2.8.1.5, 2.8.1.6, 2.8.1.7, 2.8.1.8, 2.8.1.9, 2.8.1.10, 2.8.2.1, 2.8.2.2, 2.8.2.3, 2.8.2.4, 2.8.2.5, 2.8.2.6, 2.8.2.7, 2.8.2.8, 2.8.2.9, 2.8.2.10, 2.8.3.1, 2.8.3.2, 2.8.3.3, 2.8.3.4, 2.8.3.5, 2.8.3.6, 2.8.3.7, 2.8.3.8, 2.8.3.9, 2.8.3.10, 2.8.4.1, 2.8.4.2, 2.8.4.3, 2.8.4.4, 2.8.4.5, 2.8.4.6, 2.8.4.7, 2.8.4.8, 2.8.4.9, 2.8.4.10, 2.8.5.1, 2.8.5.2, 2.8.5.3, 2.8.5.4, 2.8.5.5, 2.8.5.6, 2.8.5.7, 2.8.5.8, 2.8.5.9, 2.8.5.10, 2.8.6.1, 2.8.6.2, 2.8.6.3, 2.8.6.4, 2.8.6.5, 2.8.6.6, 2.8.6.7, 2.8.6.8, 2.8.6.9, 2.8.6.10, 2.8.7.1, 2.8.7.2, 2.8.7.3, 2.8.7.4, 2.8.7.5, 2.8.7.6, 2.8.7.7, 2.8.7.8, 2.8.7.9, 2.8.7.10, 2.8.8.1, 2.8.8.2, 2.8.8.3, 2.8.8.4, 2.8.8.5, 2.8.8.6, 2.8.8.7, 2.8.8.8, 2.8.8.9, 2.8.8.10, 2.8.9.1, 2.8.9.2, 2.8.9.3, 2.8.9.4, 2.8.9.5, 2.8.9.6, 2.8.9.7, 2.8.9.8, 2.8.9.9, 2.8.9.10, 2.8.10.1, 2.8.10.2, 2.8.10.3, 2.8.10.4, 2.8.10.5, 2.8.10.6, 2.8.10.7, 2.8.10.8, 2.8.10.9, 2.8.10.10, 2.9.1.1, 2.9.1.2, 2.9.1.3, 2.9.1.4, 2.9.1.5, 2.9.1.6, 2.9.1.7, 2.9.1.8, 2.9.1.9, 2.9.1.10, 2.9.2.1, 2.9.2.2, 2.9.2.3, 2.9.2.4, 2.9.2.5, 2.9.2.6, 2.9.2.7, 2.9.2.8, 2.9.2.9, 2.9.2.10, 2.9.3.1, 2.9.3.2, 2.9.3.3, 2.9.3.4, 2.9.3.5, 2.9.3.6, 2.9.3.7, 2.9.3.8, 2.9.3.9, 2.9.3.10, 2.9.4.1, 2.9.4.2, 2.9.4.3, 2.9.4.4, 2.9.4.5, 2.9.4.6, 2.9.4.7, 2.9.4.8, 2.9.4.9, 2.9.4.10, 2.9.5.1, 2.9.5.2, 2.9.5.3, 2.9.5.4, 2.9.5.5, 2.9.5.6, 2.9.5.7, 2.9.5.8, 2.9.5.9, 2.9.5.10, 2.9.6.1, 2.9.6.2, 2.9.6.3, 2.9.6.4, 2.9.6.5, 2.9.6.6, 2.9.6.7, 2.9.6.8, 2.9.6.9, 2.9.6.10, 2.9.7.1, 2.9.7.2, 2.9.7.3, 2.9.7.4, 2.9.7.5, 2.9.7.6, 2.9.7.7, 2.9.7.8, 2.9.7.9, 2.9.7.10, 2.9.8.1, 2.9.8.2, 2.9.8.3, 2.9.8.4, 2.9.8.5, 2.9.8.6, 2.9.8.7, 2.9.8.8, 2.9.8.9, 2.9.8.10, 2.9.9.1, 2.9.9.2, 2.9.9.3, 2.9.9.4, 2.9.9.5, 2.9.9.6, 2.9.9.7, 2.9.9.8, 2.9.9.9, 2.9.9.10, 2.9.10.1, 2.9.10.2, 2.9.10.3, 2.9.10.4, 2.9.10.5, 2.9.10.6, 2.9.10.7, 2.9.10.8, 2.9.10.9, 2.9.10.10, 2.10.1.1, 2.10.1.2, 2.10.1.3, 2.10.1.4, 2.10.1.5, 2.10.1.6, 2.10.1.7, 2.10.1.8, 2.10.1.9, 2.10.1.10, 2.10.2.1, 2.10.2.2, 2.10.2.3, 2.10.2.4, 2.10.2.5, 2.10.2.6, 2.10.2.7, 2.10.2.8, 2.10.2.9, 2.10.2.10, 2.10.3.1, 2.10.3.2, 2.10.3.3, 2.10.3.4, 2.10.3.5, 2.10.3.6, 2.10.3.7, 2.10.3.8, 2.10.3.9, 2.10.3.10, 2.10.4.1, 2.10.4.2, 2.10.4.3, 2.10.4.4, 2.10.4.5, 2.10.4.6, 2.10.4.7, 2.10.4.8, 2.10.4.9, 2.10.4.10, 2.10.5.1, 2.10.5.2, 2.10.5.3, 2.10.5.4, 2.10.5.5, 2.10.5.6, 2.10.5.7, 2.10.5.8, 2.10.5.9, 2.10.5.10, 2.10.6.1, 2.10.6.2, 2.10.6.3, 2.10.6.4, 2.10.6.5, 2.10.6.6, 2.10.6.7, 2.10.6.8, 2.10.6.9, 2.10.6.10, |
| 2.10.7.1, 2.10.7.2, 2.10.7.3, 2.10.7.4, 2.10.7.5, 2.10.7.6, 2.10.7.7, 2.10.7.8, 2.10.7.9, 2.10.7.10, 2.10.8.1, 2.10.8.2, 2.10.8.3, 2.10.8.4, 2.10.8.5, 2.10.8.6, 2.10.8.7, 2.10.8.8, 2.10.8.9, 2.10.8.10, 2.10.9.1, 2.10.9.2, 2.10.9.3, 2.10.9.4, 2.10.9.5, 2.10.9.6, 2.10.9.7, 2.10.9.8, 2.10.9.9, 2.10.9.10, 2.10.10.1, 2.10.10.2, 2.10.10.3, 2.10.10.4, 2.10.10.5, 2.10.10.6, 2.10.10.7, 2.10.10.8, 2.10.10.9, 2.10.10.10, 3.1.1.1, 3.1.1.2, 3.1.1.3, 3.1.1.4, 3.1.1.5, 3.1.1.6, 3.1.1.7, 3.1.1.8, 3.1.1.9, 3.1.1.10, 3.1.2.1, 3.1.2.2, 3.1.2.3, 3.1.2.4, 3.1.2.5, 3.1.2.6, 3.1.2.7, 3.1.2.8, 3.1.2.9, 3.1.2.10, 3.1.3.1, 3.1.3.2, 3.1.3.3, 3.1.3.4, 3.1.3.5, 3.1.3.6, 3.1.3.7, 3.1.3.8, 3.1.3.9, 3.1.3.10, 3.1.4.1, 3.1.4.2, 3.1.4.3, 3.1.4.4, 3.1.4.5, 3.1.4.6, 3.1.4.7, 3.1.4.8, 3.1.4.9, 3.1.4.10, 3.1.5.1, 3.1.5.2, 3.1.5.3, 3.1.5.4, 3.1.5.5, 3.1.5.6, 3.1.5.7, 3.1.5.8, 3.1.5.9, 3.1.5.10, 3.1.6.1, 3.1.6.2, 3.1.6.3, 3.1.6.4, 3.1.6.5, 3.1.6.6, 3.1.6.7, 3.1.6.8, 3.1.6.9, 3.1.6.10, 3.1.7.1, 3.1.7.2, 3.1.7.3, 3.1.7.4, 3.1.7.5, 3.1.7.6, 3.1.7.7, 3.1.7.8, 3.1.7.9, 3.1.7.10, 3.1.8.1, 3.1.8.2, 3.1.8.3, 3.1.8.4, 3.1.8.5, 3.1.8.6, 3.1.8.7, 3.1.8.8, 3.1.8.9, 3.1.8.10, 3.1.9.1, 3.1.9.2, 3.1.9.3, 3.1.9.4, 3.1.9.5, 3.1.9.6, 3.1.9.7, 3.1.9.8, 3.1.9.9, 3.1.9.10, 3.1.10.1, 3.1.10.2, 3.1.10.3, 3.1.10.4, 3.1.10.5, 3.1.10.6, 3.1.10.7, 3.1.10.8, 3.1.10.9, 3.1.10.10, 3.2.1.1, 3.2.1.2, 3.2.1.3, 3.2.1.4, 3.2.1.5, 3.2.1.6, 3.2.1.7, 3.2.1.8, 3.2.1.9, 3.2.1.10, 3.2.2.1, 3.2.2.2, 3.2.2.3, 3.2.2.4, 3.2.2.5, 3.2.2.6, 3.2.2.7, 3.2.2.8, 3.2.2.9, 3.2.2.10, 3.2.3.1, 3.2.3.2, 3.2.3.3, 3.2.3.4, 3.2.3.5, 3.2.3.6, 3.2.3.7, 3.2.3.8, 3.2.3.9, 3.2.3.10, 3.2.4.1, 3.2.4.2, 3.2.4.3, 3.2.4.4, 3.2.4.5, 3.2.4.6, 3.2.4.7, 3.2.4.8, 3.2.4.9, 3.2.4.10, 3.2.5.1, 3.2.5.2, 3.2.5.3, 3.2.5.4, 3.2.5.5, 3.2.5.6, 3.2.5.7, 3.2.5.8, 3.2.5.9, 3.2.5.10, 3.2.6.1, 3.2.6.2, 3.2.6.3, 3.2.6.4, 3.2.6.5, 3.2.6.6, 3.2.6.7, 3.2.6.8, 3.2.6.9, 3.2.6.10, 3.2.7.1, 3.2.7.2, 3.2.7.3, 3.2.7.4, 3.2.7.5, 3.2.7.6, 3.2.7.7, 3.2.7.8, 3.2.7.9, 3.2.7.10, 3.2.8.1, 3.2.8.2, 3.2.8.3, 3.2.8.4, 3.2.8.5, 3.2.8.6, 3.2.8.7, 3.2.8.8, 3.2.8.9, 3.2.8.10, 3.2.9.1. 3.2.9.2, 3.2.9.3, 3.2.9.4, 3.2.9.5, 3.2.9.6, 3.2.9.7, 3.2.9.8, 3.2.9.9, 3.2.9.10, 3.2.10.1, 3.2.10.2, 3.2.10.3, 3.2.10.4, 3.2.10.5, 3.2.10.6, 3.2.10.7, 3.2.10.8, 3.2.10.9, 3.2.10.10, 3.3.1.1, 3.3.1.2, 3.3.1.3, 3.3.1.4, 3.3.1.5, 3.3.1.6, 3.3.1.7, 3.3.1.8, 3.3.1.9, 3.3.1.10, 3.3.2.1, 3.3.2.2, 3.3.2.3, 3.3.2.4, 3.3.2.5, 3.3.2.6, 3.3.2.7, 3.3.2.8, 3.3.2.9, 3.3.2.10, 3.3.3.1, 3.3.3.2, 3.3.3.3, 3.3.3.4, 3.3.3.5, 3.3.3.6, 3.3.3.7, 3.3.3.8, 3.3.3.9, 3.3.3.10, 3.3.4.1, 3.3.4.2, 3.3.4.3, 3.3.4.4, 3.3.4.5, 3.3.4.6, 3.3.4.7, 3.3.4.8, 3.3.4.9, 3.3.4.10, 3.3.5.1, 3.3.5.2, 3.3.5.3, 3.3.5.4, 3.3.5.5, 3.3.5.6, 3.3.5.7, 3.3.5.8, 3.3.5.9, 3.3.5.10, 3.3.6.1, 3.3.6.2, 3.3.6.3, 3.3.6.4, 3.3.6.5, 3.3.6.6, 3.3.6.7, 3.3.6.8, 3.3.6.9, 3.3.6.10, 3.3.7.1, 3.3.7.2, 3.3.7.3, 3.3.7.4, 3.3.7.5, 3.3.7.6, 3.3.7.7, 3.3.7.8, 3.3.7.9, 3.3.7.10, 3.3.8.1, 3.3.8.2, 3.3.8.3, 3.3.8.4, 3.3.8.5, 3.3.8.6, 3.3.8.7, 3.3.8.8, 3.3.8.9, 3.3.8.10, 3.3.9.1, 3.3.9.2, 3.3.9.3, 3.3.9.4, 3.3.9.5, 3.3.9.6, 3.3.9.7, 3.3.9.8, 3.3.9.9, 3.3.9.10, 3.3.10.1, 3.3.10.2, 3.3.10.3, 3.3.10.4, 3.3.10.5, 3.3.10.6, 3.3.10.7, 3.3.10.8, 3.3.10.9, 3.3.10.10, 3.4.1.1, 3.4.1.2, 3.4.1.3, 3.4.1.4, 3.4.1.5, 3.4.1.6, 3.4.1.7, 3.4.1.8, 3.4.1.9, 3.4.1.10, 3.4.2.1, 3.4.2.2, 3.4.2.3, 3.4.2.4, 3.4.2.5, 3.4.2.6, 3.4.2.7, 3.4.2.8, 3.4.2.9, 3.4.2.10, 3.4.3.1, 3.4.3.2, 3.4.3.3, 3.4.3.4, 3.4.3.5, 3.4.3.6, 3.4.3.7, 3.4.3.8, 3.4.3.9, 3.4.3.10, 3.4.4.1, 3.4.4.2, 3.4.4.3, 3.4.4.4, 3.4.4.5, 3.4.4.6, 3.4.4.7, 3.4.4.8, 3.4.4.9, 3.4.4.10, 3.4.5.1, 3.4.5.2, 3.4.5.3, 3.4.5.4, 3.4.5.5, 3.4.5.6, 3.4.5.7, 3.4.5.8, 3.4.5.9, 3.4.5.10, 3.4.6.1, 3.4.6.2, 3.4.6.3, 3.4.6.4, 3.4.6.5, 3.4.6.6, 3.4.6.7, 3.4.6.8, 3.4.6.9, 3.4.6.10, 3.4.7.1, 3.4.7.2, 3.4.7.3, 3.4.7.4, 3.4.7.5, 3.4.7.6, 3.4.7.7, 3.4.7.8, 3.4.7.9, 3.4.7.10, 3.4.8.1, 3.4.8.2, 3.4.8.3, 3.4.8.4, 3.4.8.5, 3.4.8.6, 3.4.8.7, 3.4.8.8, 3.4.8.9, 3.4.8.10, 3.4.9.1, 3.4.9.2, 3.4.9.3, 3.4.9.4, 3.4.9.5, 3.4.9.6, 3.4.9.7, 3.4.9.8, 3.4.9.9, 3.4.9.10, 3.4.10.1, 3.4.10.2, 3.4.10.3, 3.4.10.4, 3.4.10.5, 3.4.10.6, 3.4.10.7, 3.4.10.8, |
| 3.4.10.9, 3.4.10.10, 3.5.1.1, 3.5.1.2, 3.5.1.3, 3.5.1.4, 3.5.1.5, 3.5.1.6, 3.5.1.7, 3.5.1.8, 3.5.1.9, 3.5.1.10, 3.5.2.1, 3.5.2.2, 3.5.2.3, 3.5.2.4, 3.5.2.5, 3.5.2.6, 3.5.2.7, 3.5.2.8, 3.5.2.9, 3.5.2.10, 3.5.3.1, 3.5.3.2, 3.5.3.3, 3.5.3.4, 3.5.3.5, 3.5.3.6, 3.5.3.7, 3.5.3.8, 3.5.3.9, 3.5.3.10, 3.5.4.1, 3.5.4.2, 3.5.4.3, 3.5.4.4, 3.5.4.5, 3.5.4.6, 3.5.4.7, 3.5.4.8, 3.5.4.9, 3.5.4.10, 3.5.5.1, 3.5.5.2, 3.5.5.3, 3.5.5.4, 3.5.5.5, 3.5.5.6, 3.5.5.7, 3.5.5.8, 3.5.5.9, 3.5.5.10, 3.5.6.1, 3.5.6.2, 3.5.6.3, 3.5.6.4, 3.5.6.5, 3.5.6.6, 3.5.6.7, 3.5.6.8, 3.5.6.9, 3.5.6.10, 3.5.7.1, 3.5.7.2, 3.5.7.3, 3.5.7.4, 3.5.7.5, 3.5.7.6, 3.5.7.7, 3.5.7.8, 3.5.7.9, 3.5.7.10, 3.5.8.1, 3.5.8.2, 3.5.8.3, 3.5.8.4, 3.5.8.5, 3.5.8.6, 3.5.8.7, 3.5.8.8, 3.5.8.9, 3.5.8.10, 3.5.9.1, 3.5.9.2, 3.5.9.3, 3.5.9.4, 3.5.9.5, 3.5.9.6, 3.5.9.7, 3.5.9.8, 3.5.9.9, 3.5.9.10, 3.5.10.1, 3.5.10.2, 3.5.10.3, 3.5.10.4, 3.5.10.5, 3.5.10.6, 3.5.10.7, 3.5.10.8, 3.5.10.9, 3.5.10.10, 3.6.1.1, 3.6.1.2, 3.6.1.3, 3.6.1.4, 3.6.1.5, 3.6.1.6, 3.6.1.7, 3.6.1.8, 3.6.1.9, 3.6.1.10, 3.6.2.1, 3.6.2.2, 3.6.2.3, 3.6.2.4, 3.6.2.5, 3.6.2.6, 3.6.2.7, 3.6.2.8, 3.6.2.9, 3.6.2.10, 3.6.3.1, 3.6.3.2, 3.6.3.3, 3.6.3.4, 3.6.3.5, 3.6.3.6, 3.6.3.7, 3.6.3.8, 3.6.3.9, 3.6.3.10, 3.6.4.1, 3.6.4.2, 3.6.4.3, 3.6.4.4, 3.6.4.5, 3.6.4.6, 3.6.4.7, 3.6.4.8, 3.6.4.9, 3.6.4.10, 3.6.5.1, 3.6.5.2, 3.6.5.3, 3.6.5.4, 3.6.5.5, 3.6.5.6, 3.6.5.7, 3.6.5.8, 3.6.5.9, 3.6.5.10, 3.6.6.1, 3.6.6.2, 3.6.6.3, 3.6.6.4, 3.6.6.5, 3.6.6.6, 3.6.6.7, 3.6.6.8, 3.6.6.9, 3.6.6.10, 3.6.7.1, 3.6.7.2, 3.6.7.3, 3.6.7.4, 3.6.7.5, 3.6.7.6, 3.6.7.7, 3.6.7.8, 3.6.7.9, 3.6.7.10, 3.6.8.1, 3.6.8.2, 3.6.8.3, 3.6.8.4, 3.6.8.5, 3.6.8.6, 3.6.8.7, 3.6.8.8, 3.6.8.9, 3.6.8.10, 3.6.9.1, 3.6.9.2, 3.6.9.3, 3.6.9.4, 3.6.9.5, 3.6.9.6, 3.6.9.7, 3.6.9.8, 3.6.9.9, 3.6.9.10, 3.6.10.1, 3.6.10.2, 3.6.10.3, .6.10.4, 3.6.10.5, 3.6.10.6, 3.6.10.7, 3.6.10.8, 3.6.10.9, 3.6.10.10, 3.7.1.1, 3.7.1.2, 3.7.1.3, 3.7.1.4, 3.7.1.5, 3.7.1.6, 3.7.1.7, 3.7.1.8, 3.7.1.9, 3.7.1.10, 3.7.2.1, 3.7.2.2, 3.7.2.3, 3.7.2.4, 3.7.2.5, 3.7.2.6, 3.7.2.7, 3.7.2.8, 3.7.2.9, 3.7.2.10, 3.7.3.1, 3.7.3.2, 3.7.3.3, 3.7.3.4, 3.7.3.5, 3.7.3.6, 3.7.3.7, 3.7.3.8, 3.7.3.9, 3.7.3.10, 3.7.4.1, 3.7.4.2, 3.7.4.3, 3.7.4.4, 3.7.4.5, 3.7.4.6, 3.7.4.7, 3.7.4.8, 3.7.4.9, 3.7.4.10, 3.7.5.1, 3.7.5.2, 3.7.5.3, 3.7.5.4, 3.7.5.5, 3.7.5.6, 3.7.5.7, 3.7.5.8, 3.7.5.9, 3.7.5.10, 3.7.6.1, 3.7.6.2, 3.7.6.3, 3.7.6.4, 3.7.6.5, 3.7.6.6, 3.7.6.7, 3.7.6.8, 3.7.6.9, 3.7.6.10, 3.7.7.1, 3.7.7.2, 3.7.7.3, 3.7.7.4, 3.7.7.5, 3.7.7.6, 3.7.7.7, 3.7.7.8, 3.7.7.9, 3.7.7.10, 3.7.8.1, 3.7.8.2, 3.7.8.3, 3.7.8.4, 3.7.8.5, 3.7.8.6, 3.7.8.7, 3.7.8.8, 3.7.8.9, 3.7.8.10, 3.7.9.1, |
| 3.7.9.2, 3.7.9.3, 3.7.9.4, 3.7.9.5, 3.7.9.6, 3.7.9.7 ,3.7.9.8, 3.7.9.9, 3.7.9.10, 3.7.10.1, 3.7.10.2, 3.7.10.3, 3.7.10.4, 3.7.10.5, 3.7.10.6, 3.7.10.7, 3.7.10.8, 3.7.10.9, 3.7.10.10, 3.8.1.1, 3.8.1.2, 3.8.1.3, 3.8.1.4, 3.8.1.5, 3.8.1.6, 3.8.1.7, 3.8.1.8, 3.8.1.9, 3.8.1.10, 3.8.2.1, 3.8.2.2, 3.8.2.3, 3.8.2.4, 3.8.2.5, 3.8.2.6, 3.8.2.7, 3.8.2.8, 3.8.2.9, 3.8.2.10, 3.8.3.1, 3.8.3.2, 3.8.3.3, 3.8.3.4, 3.8.3.5, 3.8.3.6, 3.8.3.7, 3.8.3.8, 3.8.3.9, 3.8.3.10, 3.8.4.1, 3.8.4.2, 3.8.4.3, 3.8.4.4, 3.8.4.5, 3.8.4.6, 3.8.4.7, 3.8.4.8, 3.8.4.9, 3.8.4.10, 3.8.5.1, 3.8.5.2, 3.8.5.3, 3.8.5.4, 3.8.5.5, 3.8.5.6, 3.8.5.7, 3.8.5.8, 3.8.5.9, 3.8.5.10, 3.8.6.1, 3.8.6.2, 3.8.6.3, 3.8.6.4, 3.8.6.5, 3.8.6.6, 3.8.6.7, 3.8.6.8, 3.8.6.9, 3.8.6.10, 3.8.7.1, 3.8.7.2, 3.8.7.3, 3.8.7.4, 3.8.7.5, 3.8.7.6, 3.8.7.7, 3.8.7.8, 3.8.7.9, 3.8.7.10, 3.8.8.1, 3.8.8.2, 3.8.8.3, 3.8.8.4, 3.8.8.5, 3.8.8.6, 3.8.8.7, 3.8.8.8, 3.8.8.9, 3.8.8.10, 3.8.9.1, 3.8.9.2, 3.8.9.3, 3.8.9.4, 3.8.9.5, 3.8.9.6, 3.8.9.7, 3.8.9.8, 3.8.9.9, 3.8.9.10, 3.8.10.1, 3.8.10.2, 3.8.10.3, 3.8.10.4, 3.8.10.5, 3.8.10.6, 3.8.10.7, 3.8.10.8, 3.8.10.9, 3.8.10.10, 3.9.1.1, 3.9.1.2, 3.9.1.3, 3.9.1.4, 3.9.1.5, 3.9.1.6, 3.9.1.7, 3.9.1.8, 3.9.1.9, 3.9.1.10, 3.9.2.1, 3.9.2.2, 3.9.2.3, 3.9.2.4, 3.9.2.5, 3.9.2.6, 3.9.2.7, 3.9.2.8, 3.9.2.9, 3.9.2.10, 3.9.3.1, 3.9.3.2, 3.9.3.3, 3.9.3.4, 3.9.3.5, 3.9.3.6, 3.9.3.7, 3.9.3.8, 3.9.3.9, 3.9.3.10, 3.9.4.1, 3.9.4.2, 3.9.4.3, 3.9.4.4, 3.9.4.5, 3.9.4.6, 3.9.4.7, 3.9.4.8, 3.9.4.9, 3.9.4.10, 3.9.5.1, 3.9.5.2, 3.9.5.3, 3.9.5.4, 3.9.5.5, 3.9.5.6, 3.9.5.7, 3.9.5.8, 3.9.5.9, 3.9.5.10, 3.9.6.1, 3.9.6.2, 3.9.6.3, 3.9.6.4, 3.9.6.5, 3.9.6.6, 3.9.6.7, 3.9.6.8, 3.9.6.9, 3.9.6.10, 3.9.7.1, 3.9.7.2, 3.9.7.3, 3.9.7.4, 3.9.7.5, 3.9.7.6, 3.9.7.7, 3.9.7.8, 3.9.7.9, 3.9.7.10, 3.9.8.1, 3.9.8.2, 3.9.8.3, 3.9.8.4, 3.9.8.5, 3.9.8.6, 3.9.8.7, 3.9.8.8, 3.9.8.9, 3.9.8.10, 3.9.9.1, 3.9.9.2, 3.9.9.3, 3.9.9.4, 3.9.9.5, 3.9.9.6, 3.9.9.7, 3.9.9.8, 3.9.9.9, 3.9.9.10, 3.9.10.1, 3.9.10.2, 3.9.10.3, 3.9.10.4, 3.9.10.5, 3.9.10.6, 3.9.10.7, 3.9.10.8, 3.9.10.9, 3.9.10.10, 3.10.1.1, 3.10.1.2, 3.10.1.3, 3.10.1.4, 3.10.1.5, 3.10.1.6, 3.10.1.7, 3.10.1.8, 3.10.1.9, 3.10.1.10, 3.10.2.1, 3.10.2.2, 3.10.2.3, 3.10.2.4, 3.10.2.5, 3.10.2.6, 3.10.2.7, 3.10.2.8, 3.10.2.9, 3.10.2.10, 3.10.3.1, 3.10.3.2, 3.10.3.3, 3.10.3.4, 3.10.3.5, 3.10.3.6, 3.10.3.7, 3.10.3.8, 3.10.3.9, 3.10.3.10, 3.10.4.1, 3.10.4.2, 3.10.4.3, 3.10.4.4, 3.10.4.5, 3.10.4.6, 3.10.4.7, 3.10.4.8, 3.10.4.9, 3.10.4.10, 3.10.5.1, 3.10.5.2, 3.10.5.3, 3.10.5.4, 3.10.5.5, 3.10.5.6, 3.10.5.7, 3.10.5.8, 3.10.5.9, 3.10.5.10, 3.10.6.1, 3.10.6.2, 3.10.6.3, 3.10.6.4, 3.10.6.5, 3.10.6.6, 3.10.6.7, 3.10.6.8, 3.10.6.9, 3.10.6.10, 3.10.7.1, 3.10.7.2, 3.10.7.3, 3.10.7.4, 3.10.7.5, 3.10.7.6, 3.10.7.7, 3.10.7.8, 3.10.7.9, 3.10.7.10, 3.10.8.1, 3.10.8.2, 3.10.8.3, 3.10.8.4, 3.10.8.5, 3.10.8.6, 3.10.8.7, 3.10.8.8, 3.10.8.9, 3.10.8.10, 3.10.9.1, 3.10.9.2, 3.10.9.3, 3.10.9.4, 3.10.9.5, 3.10.9.6, 3.10.9.7, 3.10.9.8, 3.10.9.9, 3.10.9.10, 3.10.10.1, 3.10.10.2, 3.10.10.3, 3.10.10.4, 3.10.10.5, 3.10.10.6, 3.10.10.7, 3.10.10.8, 3.10.10.9, 3.10.10.10, 4.1.1.1, 4.1.1.2, 4.1.1.3, 4.1.1.4, 4.1.1.5, 4.1.1.6, 4.1.1.7, 4.1.1.8, 4.1.1.9, 4.1.1.10, 4.1.2.1, 4.1.2.2, 4.1.2.3, 4.1.2.4, 4.1.2.5, 4.1.2.6, 4.1.2.7, 4.1.2.8, 4.1.2.9, 4.1.2.10, 4.1.3.1, 4.1.3.2, 4.1.3.3, 4.1.3.4, 4.1.3.5, 4.1.3.6, 4.1.3.7, 4.1.3.8, 4.1.3.9, 4.1.3.10, 4.1.4.1, 4.1.4.2, 4.1.4.3, 4.1.4.4, 4.1.4.5, 4.1.4.6, 4.1.4.7, 4.1.4.8, 4.1.4.9, 4.1.4.10, 4.1.5.1, 4.1.5.2, 4.1.5.3, 4.1.5.4, 4.1.5.5, 4.1.5.6, 4.1.5.7, 4.1.5.8, 4.1.5.9, 4.1.5.10, 4.1.6.1, 4.1.6.2, 4.1.6.3, 4.1.6.4, 4.1.6.5, 4.1.6.6, 4.1.6.7, 4.1.6.8, 4.1.6.9, 4.1.6.10, 4.1.7.1, 4.1.7.2, 4.1.7.3, 4.1.7.4, 4.1.7.5, 4.1.7.6, 4.1.7.7, 4.1.7.8, 4.1.7.9, 4.1.7.10, 4.1.8.1, 4.1.8.2, 4.1.8.3, 4.1.8.4, 4.1.8.5, 4.1.8.6, 4.1.8.7, 4.1.8.8, 4.1.8.9, 4.1.8.10, 4.1.9.1, 4.1.9.2, 4.1.9.3, 4.1.9.4, 4.1.9.5, 4.1.9.6, 4.1.9.7, 4.1.9.8, 4.1.9.9, 4.9.10, 4.1.10.1, 4.1.10.2, 4.1.10.3, 4.1.10.4, 4.1.10.5, 4.1.10.6, 4.1.10.7, 4.1.10.8, 4.1.10.9, 4.1.10.10, 4.2.1.1, 4.2.1.2, 4.2.1.3, 4.2.1.4, 4.2.1.5, 4.2.1.6, 4.2.1.7, 4.2.1.8, 4.2.1.9, 4.2.1.10, 4.2.2.1, 4.2.2.2, 4.2.2.3, 4.2.2.4, 4.2.2.5, 4.2.2.6, 4.2.2.7, 4.2.2.8, 4.2.2.9, 4.2.2.10, 4.2.3.1, 4.2.3.2, 4.2.3.3, 4.2.3.4, 4.2.3.5, 4.2.3.6, 4.2.3.7, 4.2.3.8, 4.2.3.9, 4.2.3.10, 4.2.4.1, 4.2.4.2, 4.2.4.3, 4.2.4.4, 4.2.4.5, 4.2.4.6, 4.2.4.7, 4.2.4.8, 4.2.4.9, 4.2.4.10, 4.2.5.1, 4.2.5.2, 4.2.5.3, 4.2.5.4, 4.2.5.5, 4.2.5.6, 4.2.5.7, 4.2.5.8, 4.2.5.9, 4.2.5.10, 4.2.6.1, 4.2.6.2, 4.2.6.3, |
| 4.2.6.4, 4.2.6.5, 4.2.6.6, 4.2.6.7, 4.2.6.8, 4.2.6.9, 4.2.6.10, 4.2.7.1, 4.2.7.2, 4.2.7.3, 4.2.7.4, 4.2.7.5, 4.2.7.6, 4.2.7.7, 4.2.7.8, 4.2.7.9, 4.2.7.10, 4.2.8.1, 4.2.8.2, 4.2.8.3, 4.2.8.4, 4.2.8.5, 4.2.8.6, 4.2.8.7, 4.2.8.8, 4.2.8.9, 4.2.8.10, 4.2.9.1, 4.2.9.2, 4.2.9.3, 4.2.9.4, 4.2.9.5, 4.2.9.6, 4.2.9.7, 4.2.9.8, 4.2.9.9, 4.2.9.10, 4.2.10.1, 4.2.10.2, 4.2.10.3, 4.2.10.4, 4.2.10.5, 4.2.10.6, 4.2.10.7, 4.2.10.8, 4.2.10.9, 4.2.10.10, 4.3.1.1, 4.3.1.2, 4.3.1.3, 4.3.1.4, 4.3.1.5, 4.3.1.6, 4.3.1.7, 4.3.1.8, 4.3.1.9, 4.3.1.10, 4.3.2.1, 4.3.2.2, 4.3.2.3, 4.3.2.4, 4.3.2.5, 4.3.2.6, 4.3.2.7, 4.3.2.8, 4.3.2.9, 4.3.2.10, 4.3.3.1, 4.3.3.2, 4.3.3.3, 4.3.3.4, 4.3.3.5, 4.3.3.6, 4.3.3.7, 4.3.3.8, 4.3.3.9, 4.3.3.10, 4.3.4.1, 4.3.4.2, 4.3.4.3, 4.3.4.4, 4.3.4.5, 4.3.4.6, 4.3.4.7, 4.3.4.8, 4.3.4.9, 4.3.4.10, 4.3.5.1, 4.3.5.2, 4.3.5.3, 4.3.5.4, 43.5.5, 4.3.5.6, 4.3.5.7, 4.3.5.8, 4.3.5.9, 4.3.5.10, 4.3.6.1, 4.3.6.2, 4.3.6.3, 4.3.6.4, 4.3.6.5, 4.3.6.6, 4.3.6.7, 4.3.6.8, 4.3.6.9, 4.3.6.10, 4.3.7.1, 4.3.7.2, 4.3.7.3, 4.3.7.4, 4.3.7.5, 4.3.7.6, 4.3.7.7, 4.3.7.8, 4.3.7.9, 4.3.7.10, 4.3.8.1, 4.3.8.2, 4.3.8.3, 4.3.8.4, 4.3.8.5, 4.3.8.6, 4.3.8.7, 4.3.8.8, 4.3.8.9, 4.3.8.10, 4.3.9.1, 4.3.9.2, 4.3.9.3, 4.3.9.4, 4.3.9.5, 4.3.9.6, 4.3.9.7, 4.3.9.8, 4.3.9.9, 4.3.9.10, 4.3.10.1, 4.3.10.2, 4.3.10.3, 4.3.10.4, 4.3.10.5, 4.3.10.6, 4.3.10.7, 4.3.10.8, 4.3.10.9, 4.3.10.10, 4.4.1.1, 4.4.1.2, 4.4.1.3, 4.4.1.4, 4.4.1.5, 4.4.1.6, 4.4.1.7, 4.4.1.8, 4.4.1.9, 4.4.1.10, 4.4.2.1, 4.4.2.2, 4.4.2.3, 4.4.2.4, 4.4.2.5, 4.4.2.6, 4.4.2.7, 4.4.2.8, 4.4.2.9, 4.4.2.10, 4.4.3.1, 4.4.3.2, 4.4.3.3, 4.4.3.4, 4.4.3.5, 4.4.3.6, 4.4.3.7, 4.4.3.8, 4.4.3.9, 4.4.3.10, 4.4.4.1, 4.4.4.2, 4.4.4.3, 4.4.4.4, 4.4.4.5, 4.4.4.6, 4.4.4.7, 4.4.4.8, 4.4.4.9, 4.4.4.10, 4.4.5.1, 4.4.5.2, 4.4.5.3, 4.4.5.4, 4.4.5.5, 4.4.5.6, 4.4.5.7, 4.4.5.8, 4.4.5.9, 4.4.5.10, 4.4.6.1, 4.4.6.2, 4.4.6.3, 4.4.6.4, 4.4.6.5, 4.4.6.6, 4.4.6.7, 4.4.6.8, 4.4.6.9, 4.4.6.10, 4.4.7.1, 4.4.7.2, 4.4.7.3, 4.4.7.4, 4.4.7.5, 4.4.7.6, 4.4.7.7, 4.4.7.8, 4.4.7.9, 4.4.7.1 0, 4.4.8.1, 4.4.8.2, 4.4.8.3, 4.4.8.4, 4.4.8.5, 4.4.8.6, 4.4.8.7, 4.4.8.8, 4.4.8.9, 4.4.8.10, 4.4.9.1, 4.4.9.2, 4.4.9.3, 4.4.9.4, 4.4.9.5, 4.4.9.6, 4.4.9.7, 4.4.9.8, 4.4.9.9, 4.4.9.10, 4.4.10.1, 4.4.10.2, 4.4.10.3, 4.4.10.4, 4.4.10.5, 4.4.10.6, 4.4.10.7, 4.4.10.8, 4.4.10.9, 4.4.10.10, 4.5.1.1, 4.5.1.2, 4.5.1.3, 4.5.1.4, 4.5.1.5, 4.5.1.6, 4.5.1.7, 4.5.1.8, 4.5.1.9, 4.5.1.10, 4.5.2.1, 4.5.2.2, 4.5.2.3, 4.5.2.4, 4.5.2.5, 4.5.2.6, 4.5.2.7, 4.5.2.8, 4.5.2.9, 4.5.2.10, 4.5.3.1, 4.5.3.2, 4.5.3.3, 4.5.3.4, 4.5.3.5, 4.5.3.6, 4.5.3.7, 4.5.3.8, 4.5.3.9, 4.5.3.10, 4.5.4.1, 4.5.4.2, 4.5.4.3, 4.5.4.4, 4.5.4.5, 4.5.4.6, 4.5.4.7, 4.5.4.8, 4.5.4.9, 4.5.4.10, 4.5.5.1, 4.5.5.2, 4.5.5.3, 4.5.5.4, 4.5.5.5, 4.5.5.6, 4.5.5.7, 4.5.5.8, 4.5.5.9, 4.5.5.10, 4.5.6.1, 4.5.6.2, 4.5.6.3, 4.5.6.4, 4.5.6.5, 4.5.6.6, 4.5.6.7, 4.5.6.8, 4.5.6.9, 4.5.6.10, 4.5.7.1, 4.5.7.2, 4.5.7.3, 4.5.7.4, 4.5.7.5, |
| 4.5.7.6, 4.5.7.7, 4.5.7.8, 4.5.7.9, 4.5.7.10, 4.5.8.1, 4.5.8.2, 4.5.8.3, 4.5.8.4, 4.5.8.5, 4.5.8.6, 4.5.8.7, 4.5.8.8, 4.5.8.9, 4.5.8.10, 4.5.9.1, 4.5.9.2, 4.5.9.3, 4.5.9.4, 4.5.9.5, 4.5.9.6, 4.5.9.7, 4.5.9.8, 4.5.9.9, 4.5.9.10, 4.5.10.1, 4.5.10.2, 4.5.10.3, 4.5.10.4, 4.5.10.5, 4.5.10.6, 4.5.10.7, 4.5.10.8, 4.5.10.9, 4.5.10.10, 4.6.1.1, 4.6.1.2, 4.6.1.3, 4.6.1.4, 4.6.1.5, 4.6.1.6, 4.6.1.7, 4.6.1.8, 4.6.1.9, 4.6.1.10, 4.6.2.1, 4.6.2.2, 4.6.2.3, 4.6.2.4, 4.6.2.5, 4.6.2.6, 4.6.2.7, 4.6.2.8, 4.6.2.9, 4.6.2.10, 4.6.3.1, 4.6.3.2, 4.6.3.3, 4.6.3.4, 4.6.3.5, 4.6.3.6, 4.6.3.7, 4.6.3.8, 4.6.3.9, 4.6.3.10, 4.6.4.1, 4.6.4.2, 4.6.4.3, 4.6.4.4, 4.6.4.5, 4.6.4.6, 4.6.4.7, 4.6.4.8, 4.6.4.9, 4.6.4.10, 4.6.5.1, 4.6.5.2, 4.6.5.3, 4.6.5.4, 4.6.5.5, 4.6.5.6, 4.6.5.7, 4.6.5.8, 4.6.5.9, 4.6.5.10, 4.6.6.1, 4.6.6.2, 4.6.6.3, 4.6.6.4, 4.6.6.5, 4.6.6.6, 4.6.6.7, 4.6.6.8, 4.6.6.9, 4.6.6.10, 4.6.7.1, 4.6.7.2, 4.6.7.3, 4.6.7.4, 4.6.7.5, 4.6.7.6, 4.6.7.7, 4.6.7.8, 4.6.7.9, 4.6.7.10, 4.6.8.1, 4.6.8.2, 4.6.8.3, 4.6.8.4, 4.6.8.5, 4.6.8.6, 4.6.8.7, 4.6.8.8, 4.6.8.9, 4.6.8.10, 4.6.9.1, 4.6.9.2, 4.6.9.3, 4.6.9.4, 4.6.9.5, 4.6.9.6, 4.6.9.7, 4.6.9.8, 4.6.9.9, 4.6.9.10, 4.6.10.1, 4.6.10.2, 4.6.10.3,4.6.10.4,4.6.10.5,4.6.10.6,4.6.10.7, 4.6.10.8, 4.6.10.9, 4.6.10.10, 4.7.1.1, 4.7.1.2, 4.7.1.3, 4.7.1.4, 4.7.1.5, 4.7.1.6, 4.7.1.7, 4.7.1.8, 4.7.1.9, 4.7.1.10, 4.7.2.1, 4.7.2.2, 4.7.2.3, 4.7.2.4, 4.7.2.5, 4.7.2.6, 4.7.2.7, 4.7.2.8, 4.7.2.9, 4.7.2.10, 4.7.3.1, 4.7.3.2, 4.7.3.3, 4.7.3.4, 4.7.3.5, 4.7.3.6, 4.7.3.7, 4.7.3.8, 4.7.3.9, 4.7.3.10, 4.7.4.1, 4.7.4.2, 4.7.4.3, 4.7.4.4, 4.7.4.5, 4.7.4.6, 4.7.4.7, 4.7.4.8, 4.7.4.9, 4.7.4.10, 4.7.5.1, 4.7.5.2, 4.7.5.3, 4.7.5.4, 4.7.5.5, 4.7.5.6, 4.7.5.7, 4.7.5.8, 4.7.5.9, 4.7.5.10, 4.7.6.1, 4.7.6.2, 4.7.6.3, 4.7.6.4, 4.7.6.5, 4.7.6.6, 4.7.6.7, 4.7.6.8, 4.7.6.9, 4.7.6.10, 4.7.7.1, 4.7.7.2, 4.7.7.3, 4.7.7.4, 4.7.7.5, 4.7.7.6, 4.7.7.7, 4.7.7.8, 4.7.7.9, 4.7.7.10, 4.7.8.1, 4.7.8.2, 4.7.8.3, 4.7.8.4, 4.7.8.5, 4.7.8.6, 4.7.8.7, 4.7.8.8, 4.7.8.9, 4.7.8.10, 4.7.9.1, 4.7.9.2, 4.7.9.3, 4.7.9.4, 4.7.9.5, 4.7.9.6, 4.7.9.7, 4.7.9.8, 4.7.9.9, 4.7.9.10, 4.7.10.1, 4.7.10.2, 4.7.10.3, 4.7.10.4, 4.7.10.5, 4.7.10.6, 4.7.10.7, 4.7.10.8, 4.7.10.9, 4.7.10.10, 4.8.1.1, 4.8.1.2, 4.8.1.3, 4.8.1.4, 4.8.1.5, 4.8.1.6, 4.8.1.7, 4.8.1.8, 4.8.1.9, 4.8.1.10, 4.8.2.1, 4.8.2.2, 4.8.2.3, 4.8.2.4, 4.8.2.5, 4.8.2.6, 4.8.2.7, 4.8.2.8, 4.8.2.9, 4.8.2.10, 4.8.3.1, 4.8.3.2, 4.8.3.3, 4.8.3.4, 4.8.3.5, 4.8.3.6, 4.8.3.7, 4.8.3.8, 4.8.3.9, 4.8.3.10, 4.8.4.1, 4.8.4.2, 4.8.4.3, 4.8.4.4, 4.8.4.5, 4.8.4.6, 4.8.4.7, 4.8.4.8, 4.8.4.9, 4.8.4.10, 4.8.5.1, 4.8.5.2, 4.8.5.3, 4.8.5.4, 4.8.5.5, 4.8.5.6, 4.8.5.7, 4.8.5.8, 4.8.5.9, 4.8.5.10, 4.8.6.1, 4.8.6.2, 4.8.6.3, 4.8.6.4, 4.8.6.5, 4.8.6.6, 4.8.6.7, 4.8.6.8, 4.8.6.9, 4.8.6.10, 4.8.7.1, 4.8.7.2, 4.8.7.3, 4.8.7.4, 4.8.7.5, 4.8.7.6, 4.8.7.7, 4.8.7.8, 4.8.7.9, 4.8.7.10, 4.8.8.1, 4.8.8.2, 4.8.8.3, 4.8.8.4, 4.8.8.5, 4.8.8.6, 4.8.8.7, 4.8.8.8, 4.8.8.9, 4.8.8.10, 4.8.9.1, 4.8.9.2, 4.8.9.3, 4.8.9.4, 4.8.9.5, 4.8.9.6, 4.8.9.7, 4.8.9.8, 4.8.9.9, 4.8.9.10, 4.8.10.1, 4.8.10.2, 4.8.10.3, 4.8.10.4, 4.8.10.5, 4.8.10.6, 4.8.10.7, 4.8.10.8, 4.8.10.9, 4.8.10.10, 4.9.1.1, 4.9.1.2, 4.9.1.3, 4.9.1.4, 4.9.1.5, 4.9.1.6, 4.9.1.7, 4.9.1.8, 4.9.1.9, 4.9.1.10, 4.9.2.1, 4.9.2.2, 4.9.2.3, 4.9.2.4, 4.9.2.5, 4.9.2.6, 4.9.2.7, 4.9.2.8, 4.9.2.9, 4.9.2.10, 4.9.3.1, 4.9.3.2, 4.9.3.3, 4.9.3.4, 4.9.3.5, 4.9.3.6, 4.9.3.7, 4.9.3.8, 4.9.3.9, 4.9.3.10, 4.9.4.1, 4.9.4.2, 4.9.4.3, 4.9.4.4, 4.9.4.5, 4.9.4.6, 4.9.4.7, 4.9.4.8, 4.9.4.9, 4.9.4.10, 4.9.5.1, 4.9.5.2, 4.9.5.3, 4.9.5.4, 4.9.5.5, 4.9.5.6, 4.9.5.7, 4.9.5.8, 4.9.5.9, 4.9.5.10, 4.9.6.1, 4.9.6.2, 4.9.6.3, 4.9.6.4, 4.9.6.5, 4.9.6.6, 4.9.6.7, 4.9.6.8, 4.9.6.9, 4.9.6.10, 4.9.7.1, 4.9.7.2, 4.9.7.3, 4.9.7.4, 4.9.7.5, 4.9.7.6, 4.9.7.7, 4.9.7.8, 4.9.7.9, 4.9.7.10, 4.9.8.1, 4.9.8.2, 4.9.8.3, 4.9.8.4, 4.9.8.5, 4.9.8.6, 4.9.8.7, 4.9.8.8, 4.9.8.9, 4.9.8.10, 4.9.9.1, 4.9.9.2, 4.9.9.3, 4.9.9.4, 4.9.9.5, 4.9.9.6, 4.9.9.7, 4.9.9.8, 4.9.9.9, 4.9.9.10, 4.9.10.1, 4.9.10.2, 4.9.10.3, 4.9.10.4, 4.9.10.5, 4.9.10.6, 4.9.10.7, 4.9.10.8, 4.9.10.9, 4.9.10.10, 4.10.1.1, 4.10.1.2, 4.10.1.3, 4.10.1.4, 4.10.1.5, 4.10.1.6, 4.10.1.7, 4.10.1.8, 4.10.1.9, 4.10.1.10, 4.10.2.1, 4.10.2.2, 4.10.2.3, 4.10.2.4, 4.10.2.5, 4.10.2.6, 4.10.2.7, 4.10.2.8, 4.10.2.9, 4.10.2.10, 4.10.3.1, 4.10.3.2, |
| 4.10.3.3, 4.10.3.4, 4.10.3.5, 4.10.3.6, 4.10.3.7, 4.10.3.8, 4.10.3.9, 4.10.3.10, 4.10.4.1, 4.10.4.2, 4.10.4.3, 4.10.4.4, 4.10.4.5, 4.10.4.6, 4.10.4.7, 4.10.4.8, 4.10.4.9, 4.10.4.10, 4.10.5.1, 4.10.5.2, 4.10.5.3, 4.10.5.4, 4.10.5.5, 4.10.5.6, 4.10.5.7, 4.10.5.8, 4.10.5.9, 4.10.5.10, 4.10.6.1, 4.10.6.2, 4.10.6.3, 4.10.6.4, 4.10.6.5, 4.10.6.6, 4.10.6.7, 4.10.6.8, 4.10.6.9, 4.10.6.10, 4.10.7.1, 4.10.7.2, 4.10.7.3, 4.10.7.4, 4.10.7.5, 4.10.7.6, 4.10.7.7, 4.10.7.8, 4.10.7.9, 4.10.7.10, 4.10.8.1, 4.10.8.2, 4.10.8.3, 4.10.8.4, 4.10.8.5, 4.10.8.6, 4.10.8.7, 4.10.8.8, 4.10.8.9, 4.10.8.10, 4.10.9.1, 4.10.9.2, 4.10.9.3, 4.10.9.4, 4.10.9.5, 4.10.9.6, 4.10.9.7, 4.10.9.8, 4.10.9.9, 4.10.9.10, 4.10.10.1, 4.10.10.2, 4.10.10.3, 4.10.10.4, 4.10.10.5, 4.10.10.6, 4.10.10.7, 4.10.10.8, 4.10.10.9, 4.10.10.10, 5.1.1.1, 5.1.1.2, 5.1.1.3, 5.1.1.4, 5.1.1.5, 5.1.1.6, 5.1.1.7, 5.1.1.8, 5.1.1.9, 5.1.1.10, 5.1.2.1, 5.1.2.2, 5.1.2.3, 5.1.2.4, 5.1.2.5, 5.1.2.6, 5.1.2.7, 5.1.2.8, 5.1.2.9, 5.1.2.10, 5.1.3.1, 5.1.3.2, 5.1.3.3, 5.1.3.4, 5.1.3.5, 5.1.3.6, 5.1.3.7, 5.1.3.8, 5.1.3.9, 5.1.3.10, 5.1.4.1, 5.1.4.2, 5.1.4.3, 5.1.4.4, 5.1.4.5, 5.1.4.6, 5.1.4.7, 5.1.4.8, 5.1.4.9, 5.1.4.10, 5.1.5.1, 5.1.5.2, 5.1.5.3, 5.1.5.4, 5.1.5.5, 5.1.5.6, 5.1.5.7, 5.1.5.8, 5.1.5.9, 5.1.5.10, 5.1.6.1, 5.1.6.2, 5.1.6.3, 5.1.6.4, 5.1.6.5, 5.1.6.6, 5.1.6.7, 5.1.6.8, 5.1.6.9, 5.1.6.10, 5.1.7.1, 5.1.7.2, 5.1.7.3, 5.1.7.4, 5.1.7.5, 5.1.7.6, 5.1.7.7, 5.1.7.8, 5.1.7.9, 5.1.7,10, 5.1.8.1, 5.1.8.2, 5.1.8.3, 5.1.8.4, 5.1.8.5, 5.1.8.6, 5.1.8.7, 5.1.8.8, 5.1.8.9, 5.1.8.10, 5.1.9.1, 5.1.9.2, 5.1.9.3, 5.1.9.4, 5.1.9.5, 5.1.9.6, 5.1.9.7, 5.1.9.8, 5.1.9.9, 5.1.9.10, 5.1.10.1, 5.1.10.2, 5.1.10.3, 5.1.10.4, 5.1.10.5, 5.1.10.6, 5.1.10.7, 5.1.10.8, 5.1.10.9, 5.1.10.10, 5.2.1.1, 5.2.1.2, 5.2.1.3, 5.2.1.4, 5.2.1.5, 5.2.1.6, 5.2.1.7, 5.2.1.8, 5.2.1.9, 5.2.1.10, 5.2.2.1, 5.2.2.2, 5.2.2.3, 5.2.2.4, 5.2.2.5, 5.2.2.6, 5.2.2.7, 5.2.2.8, 5.2.2.9, 5.2.2.10, 5.2.3.1, 5.2.3.2, 5.2.3.3, 5.2.3.4, 5.2.3.5, 5.2.3.6, 5.2.3.7, 5.2.3.8, 5.2.3.9, 5.2.3.10, 5.2.4.1, 5.2.4.2, 5.2.4.3, 5.2.4.4, 5.2.4.5, 5.2.4.6, 5.2.4.7, 5.2.4.8, 5.2.4.9, 5.2.4.10, 5.2.5.1, 5.2.5.2, 5.2.5.3, 5.2.5.4, 5.2.5.5, 5.2.5.6, 5.2.5.7, 5.2.5.8, 5.2.5.9, 5.2.5.10, 5.2.6.1, 5.2.6.2, 5.2.6.3, 5.2.6.4, 5.2.6.5, 5.2.6.6, 5.2.6.7, 5.2.6.8, 5.2.6.9, 5.2.6.10, 5.2.7.1, 5.2.7.2, 5.2.7.3, 5.2.7.4, 5.2.7.5, 5.2.7.6, 5.2.7.7, 5.2.7.8, 5.2.7.9, 5.2.7.10, 5.2.8.1, 5.2.8.2, 5.2.8.3, 5.2.8.4, 5.2.8.5, 5.2.8.6, 5.2.8.7, 5.2.8.8, 5.2.8.9, 5.2.8.10, 5.2.9.1, 5.2.9.2, 5.2.9.3, 5.2.9.4, 5.2.9.5, 5.2.9.6, 5.2.9.7, 5.2.9.8, 5.2.9.9, 5.2.9.10, 5.2.10.1, 5.2.10.2, 5.2.10.3, 5.2.10.4, 5.2.10.5, 5.2.10.6, 5.2.10.7, 5.2.10.8, 5.2.10.9, 5.2.10.10, 5.3.1.1, 5.3.1.2, 5.3.1.3, 5.3.1.4, 5.3.1.5, 5.3.1.6, 5.3.1.7, 5.3.1.8, 5.3.1.9, 5.3.1.10, 5.3.2.1, 5.3.2.2, 5.3.2.3, 5.3.2.4, 5.3.2.5, 5.3.2.6, 5.3.2.7, 5.3.2.8, 5.3.2.9, 5.3.2.10, 5.3.3.1, 5.3.3.2, 5.3.3.3, 5.3.3.4, 5.3.3.5, 5.3.3.6, 5.3.3.7, 5.3.3.8, 5.3.3.9, 5.3.3.10, 5.3.4.1, 5.3.4.2, 5.3.4.3, 5.3.4.4, 5.3.4.5, 5.3.4.6, 5.3.4.7, 5.3.4.8, 5.3.4.9, 5.3.4.10, 5.3.5.1, 5.3.5.2, 5.3.5.3, 5.3.5.4, 5.3.5.5, 5.3.5.6, 5.3.5.7, 5.3.5.8, 5.3.5.9, 5.3.5.10, 5.3.6.1, 5.3.6.2, 5.3.6.3, 5.3.6.4, 5.3.6.5, 5.3.6.6, 5.3.6.7, 5.3.6.8, 5.3.6.9, 5.3.6.10, 5.3.7.1, 5.3.7.2, 5.3.7.3, 5.3.7.4, 5.3.7.5, 5.3.7.6, 5.3.7.7, |
| 5.3.7.8, 5.3.7.9, 5.3.7.10, 5.3.8.1, 5.3.8.2, 5.3.8.3, 5.3.8.4, 5.3.8.5, 5.3.8.6, 5.3.8.7, 5.3.8.8, 5.3.8.9, 5.3.8.10, 5.3.9.1, 5.3.9.2, 5.3.9.3, 5.3.9.4, 5.3.9.5, 5.3.9.6, 5.3.9.7, 5.3.9.8, 5.3.9.9, 5.3.9.10, 5.3.10.1, 5.3.10.2, 5.3.10.3, 5.3.10.4, 5.3.10.5, 5.3.10.6, 5.3.10.7, 5.3.10.8, 5.3.10.9, 5.3.10.10, 5.4.1.1, 5.4.1.2, 5.4.1.3, 5.4.1.4, 5.4.1.5, 5.4.1.6, 5.4.1.7, 5.4.1.8, 5.4.1.9, 5.4.1.10, 5.4.2.1, 5.4.2.2, 5.4.2.3, 5.4.2.4, 5.4.2.5, 5.4.2.6, 5.4.2.7, 5.4.2.8, 5.4.2.9, 5.4.2.10, 5.4.3.1, 5.4.3.2, 5.4.3.3, 5.4.3.4, 5.4.3.5, 5.4.3.6, 5.4.3.7, 5.4.3.8, 5.4.3.9, 5.4.3.10, 5.4.4.1, 5.4.4.2, 5.4.4.3, 5.4.4.4, 5.4.4.5, 5.4.4.6, 5.4.4.7, 5.4.4.8, 5.4.4.9, 5.4.4.10, 5.4.5.1, 5.4.5.2, 5.4.5.3, 5.4.5.4, 5.4.5.5, 5.4.5.6, 5.4.5.7, 5.4.5.8, 5.4.5.9, 5.4.5.10, 5.4.6.1, 5.4.6.2, 5.4.6.3, 5.4.6.4, 5.4.6.5, 5.4.6.6, 5.4.6.7, 5.4.6.8, 5.4.6.9, 5.4.6.10, 5.4.7.1, 5.4.7.2, 5.4.7.3, 5.4.7.4, 5.4.7.5, 5.4.7.6, 5.4.7.7, 5.4.7.8, 5.4.7.9, 5.4.7.10, 5.4.8.1, 5.4.8.2, 5.4.8.3, 5.4.8.4, 5.4.8.5, 5.4.8.6, 5.4.8.7, 5.4.8.8, 5.4.8.9, 5.4.8.10, 5.4.9.1, 5.4.9.2, 5.4.9.3, 5.4.9.4, 5.4.9.5, 5.4.9.6, 5.4.9.7, 5.4.9.8, 5.4.9.9, 5.4.9.10, 5.4.10.1, 5.4.10.2, 5.4.10.3, 5.4.10.4, 5.4.10.5, 5.4.10.6, 5.4.10.7, 5.4.10.8, 5.4.10.9, 5.4.10.10, 5.5.1.1, 5.5.1.2, 5.5.1.3, 5.5.1.4, 5.5.1.5, 5.5.1.6, 5.5.1.7, 5.5.1.8, 5.5.1.9, 5.5.1.10, 5.5.2.1, 5.5.2.2, 5.5.2.3, 5.5.2.4, 5.5.2.5, 5.5.2.6, 5.5.2.7, 5.5.2.8, 5.5.2.9, 5.5.2.10, 5.5.3.1, 5.5.3.2, 5.5.3.3, 5.5.3.4, 5.5.3.5, 5.5.3.6, 5.5.3.7, 5.5.3.8, 5.5.3.9, 5.5.3.10, 5.5.4.1, 5.5.4.2, 5.5.4.3, 5.5.4.4, 5.5.4.5, 5.5.4.6, 5.5.4.7, 5.5.4.8, 5.5.4.9, 5.5.4.10, 5.5.5.1, 5.5.5.2, 5.5.5.3, 5.5.5.4, 5.5.5.5, 5.5.5.6, 5.5.5.7, 5.5.5.8, 5.5.5.9, 5.5.5.10, 5.5.6.1, 5.5.6.2, 5.5.6.3, 5.5.6.4, 5.5.6.5, 5.5.6.6, 5.5.6.7, 5.5.6.8, 5.5.6.9, 5.5.6.10, 5.5.7.1, 5.5.7.2, 5.5.7.3, 5.5.7.4, 5.5.7.5, 5.5.7.6, 5.5.7.7, 5.5.7.8, 5.5.7.9, 5.5.7.10, 5.5.8.1, 5.5.8.2, 5.5.8.3, 5.5.8.4, 5.5.8.5, 5.5.8.6, 5.5.8.7, 5.5.8.8, 5.5.8.9, 5.5.8.10, 5.5.9.1, 5.5.9.2, 5.5.9.3, 5.5.9.4, 5.5.9.5, 5.5.9.6, 5.5.9.7, 5.5.9.8, 5.5.9.9, 5.5.9.10, 5.5.10.1, 5.5.10.2, 5.5.10.3, 5.5.10.4, 5.5.10.5, 5.5.10.6, 5.5.10.7, 5.5.10.8, 5.5.10.9, 5.5.10.10, 5.6.1.1, 5.6.1.2, 5.6.1.3, 5.6.1.4, 5.6.1.5, 5.6.1.6, 5.6.1.7, 5.6.1.8, 5.6.1.9, 5.6.1.10, 5.6.2.1, 5.6.2.2, 5.6.2.3, 5.6.2.4, 5.6.2.5, 5.6.2.6, 5.6.2.7, 5.6.2.8, 5.6.2.9, 5.6.2.10, 5.6.3.1, 5.6.3.2, 5.6.3.3, 5.6.3.4, 5.6.3.5, 5.6.3.6, 5.6.3.7, 5.6.3.8, 5.6.3.9, 5.6.3.10, 5.6.4.1, 5.6.4.2, 5.6.4.3, 5.6.4.4, 5.6.4.5, 5.6.4.6, 5.6.4.7, 5.6.4.8, 5.6.4.9, 5.6.4.10, 5.6.5.1, 5.6.5.2, 5.6.5.3, 5.6.5.4, 5.6.5.5, 5.6.5.6, 5.6.5.7, 5.6.5.8, 5.6.5.9, 5.6.5.10, 5.6.6.1, 5.6.6.2, 5.6.6.3, 5.6.6.4, 5.6.6.5, 5.6.6.6, 5.6.6.7, 5.6.6.8, 5.6.6.9, 5.6.6.10, 5.6.7.1, 5.6.7.2, 5.6.7.3, 5.6.7.4, 5.6.7.5, 5.6.7.6, 5.6.7.7, 5.6.7.8, 5.6.7.9, 5.6.7.10, 5.6.8.1, 5.6.8.2, 5.6.8.3, 5.6.8.4, 5.6.8.5, 5.6.8.6, 5.6.8.7, 5.6.8.8, 5.6.8.9, 5.6.8.10, 5.6.9.1, 5.6.9.2, 5.6.9.3, 5.6.9.4, 5.6.9.5, 5.6.9.6, 5.6.9.7, 5.6.9.8, 5.6.9.9, 5.6.9.10, 5.6.10.1, 5.6.10.2, 5.6.10.3, 5.6.10.4, 5.6.10.5, 5.6.10.6, 5.6.10.7, 5.6.10.8, 5.6.10.9, 5.6.10.10, 5.7.1.1, 5.7.1.2, 5.7.1.3, 5.7.1.4, 5.7.1.5, 5.7.1.6, 5.7.1.7, 5.7.1.8, 5.7.1.9, 5.7.1.10, 5.7.2.1, 5.7.2.2, 5.7.2.3, 5.7.2.4, 5.7.2.5, 5.7.2.6, 5.7.2.7, 5.7.2.8, 5.7.2.9, 5.7.2.10, 5.7.3.1, 5.7.3.2, 5.7.3.3, 5.7.3.4, 5.7.3.5, 5.7.3.6, 5.7.3.7, 5.7.3.8, 5.7.3.9, 5.7.3.10, 5.7.4.1, 5.7.4.2, 5.7.4.3, 5.7.4.4, 5.7.4.5, 5.7.4.6, 5.7.4.7, 5.7.4.8, 5.7.4.9, 5.7.4.10, 5.7.5.1, 5.7.5.2, 5.7.5.3, 5.7.5.4, 5.7.5.5, 5.7.5.6, 5.7.5.7, 5.7.5.8, 5.7.5.9, 5.7.5.10, 5.7.6.1, 5.7.6.2, 5.7.6.3, 5.7.6.4, 5.7.6.5, 5.7.6.6, 5.7.6.7, 5.7.6.8, 5.7.6.9, 5.7.6.10, 5.7.7.1, 5.7.7.2, 5.7.7.3, 5.7.7.4, 5.7.7.5, 5.7.7.6, 5.7.7.7, 5.7.7.8, 5.7.7.9, 5.7.7.10, 5.7.8.1, 5.7.8.2, 5.7.8.3, 5.7.8.4, 5.7.8.5, 5.7.8.6, 5.7.8.7, 5.7.8.8, 5.7.8.9, 5.7.8.10, 5.7.9.1, 5.7.9.2, |
| 5.7.9.3, 5.7.9.4, 5.7.9.5, 5.7.9.6, 5.7.9.7, 5.7.9.8, 5.7.9.9, 5.7.9.10, 5.7.10.1, 5.7.10.2, 5.7.10.3, 5.7.10.4, 5.7.10.5, 5.7.10.6, 5.7.10.7, 5.7.10.8, 5.7.10.9, 5.7.10.10, 5.8.1.1, 5.8.1.2, 5.8.1.3, 5.8.1.4, 5.8.1.5, 5.8.1.6, 5.8.1.7, 5.8.1.8, 5.8.1.9, 5.8.1.10, 5.8.2.1, 5.8.2.2, 5.8.2.3, 5.8.2.4, 5.8.2.5, 5.8.2.6, 5.8.2.7, 5.8.2.8, 5.8.2.9, 5.8.2.10, 5.8.3.1, 5.8.3.2, 5.8.3.3, 5.8.3.4, 5.8.3.5, 5.8.3.6, 5.8.3.7, 5.8.3.8, 5.8.3.9, 5.8.3.10, 5.8.4.1, 5.8.4.2, 5.8.4.3, 5.8.4.4, 5.8.4.5, 5.8.4.6, 5.8.4.7, 5.8.4.8, 5.8.4.9, 5.8.4.10, 5.8.5.1, 5.8.5.2, 5.8.5.3, 5.8.5.4, 5.8.5.5, 5.8.5.6, 5.8.5.7, 5.8.5.8, 5.8.5.9, 5.8.5.10, 5.8.6.1, 5.8.6.2, 5.8.6.3, 5.8.6.4, 5.8.6.5, 5.8.6.6, 5.8.6.7, 5.8.6.8, 5.8.6.9, 5.8.6.10, 5.8.7.1, 5.8.7.2, 5.8.7.3, 5.8.7.4, 5.8.7.5, 5.8.7.6, 5.8.7.7, 5.8.7.8, 5.8.7.9, 5.8.7.10, 5.8.8.1, 5.8.8.2, 5.8.8.3, 5.8.8.4, 5.8.8.5, 5.8.8.6, 5.8.8.7, 5.8.8.8, 5.8.8.9, 5.8.8.10, 5.8.9.1, 5.8.9.2, 5.8.9.3, 5.8.9.4, 5.8.9.5, 5.8.9.6, 5.8.9.7, 5.8.9.8, 5.8.9.9, 5.8.9.10, 5.8.10.1, 5.8.10.2, 5.8.10.3, 5.8.10.4, 5.8.10.5, 5.8.10.6, 5.8.10.7, 5.8.10.8, 5.8.10.9, 5.8.10.10, 5.9.1.1, 5.9.1.2, 5.9.1.3, 5.9.1.4, 5.9.1.5, 5.9.1.6, 5.9.1.7, 5.9.1.8, 5.9.1.9, 5.9.1.10, 5.9.2.1, 5.9.2.2, 5.9.2.3, 5.9.2.4, 5.9.2.5, 5.9.2.6, 5.9.2.7, 5.9.2.8, 5.9.2.9, 5.9.2.10, 5.9.3.1, 5.9.3.2, 5.9.3.3, 5.9.3.4, 5.9.3.5, 5.9.3.6, 5.9.3.7, 5.9.3.8, 5.9.3.9, 5.9.3.10, 5.9.4.1, 5.9.4.2, 5.9.4.3, 5.9.4.4, 5.9.4.5, 5.9.4.6, 5.9.4.7, 5.9.4.8, 5.9.4.9, 5.9.4.10, 5.9.5.1, 5.9.5.2, 5.9.5.3, 5.9.5.4, 5.9.5.5, 5.9.5.6, 5.9.5.7, 5.9.5.8, 5.9.5.9, 5.9.5.10, 5.9.6.1, 5.9.6.2, 5.9.6.3, 5.9.6.4, 5.9.6.5, 5.9.6.6, 5.9.6.7, 5.9.6.8, 5.9.6.9, 5.9.6.10, 5.9.7.1, 5.9.7.2, 5.9.7.3, 5.9.7.4, 5.9.7.5, 5.9.7.6, 5.9.7.7, 5.9.7.8, 5.9.7.9, 5.9.7.10, 5.9.8.1, 5.9.8.2, 5.9.8.3, 5.9.8.4, 5.9.8.5, 5.9.8.6, 5.9.8.7, 5.9.8.8, 5.9.8.9, 5.9.8.10, 5.9.9.1, 5.9.9.2, 5.9.9.3, 5.9.9.4, 5.9.9.5, 5.9.9.6, 5.9.9.7, 5.9.9.8, 5.9.9.9, 5.9.9.10, 5.9.10.1, 5.9.10.2, 5.9.10.3, 5.9.10.4, 5.9.10.5, 5.9.10.6, 5.9.10.7, 5.9.10.8, 5.9.10.9, 5.9.10.10, 5.10.1.1, 5.10.1.2, 5,10.1.3, 5.10.1.4, 5.10.1.5, 5.10.1.6, 5.10.1.7, 5.10.1.8, 5.10.1.9, 5.10.1.10, 5.10.2.1, 5.10.2.2, 5.10.2.3, 5.10.2.4, 5.10.2.5, 5.10.2.6, 5.10.2.7, 5.10.2.8, 5.10.2.9, 5.10.2.10, 5.10.3.1, 5.10.3.2, 5.10.3.3, 5.10.3.4, 5.10.3.5, 5.10.3.6, 5.10.3.7, 5.10.3.8, 5.10.3.9, 5.10.3.10, 5.10.4.1, 5.10.4.2, 5.10.4.3, 5.10.4.4, 5.10.4.5, 5.10.4.6, 5.10.4.7, 5.10.4.8, 5.10.4.9, 5.10.4.10, 5.10.5.1, 5.10.5.2, 5.10.5.3, 5.10.5.4, 5.10.5.5, 5.10.5.6, 5.10.5.7, 5.10.5.8, 5.10.5.9, 5.10.5.10, 5.10.6.1, 5.10.6.2, 5.10.6.3, 5.10.6.4, 5.10.6.5, 5.10.6.6, 5.10.6.7, 5.10.6.8, 5.10.6.9, 5.10.6.10, 5.10.7.1, 5.10.7.2, 5.10.7.3, 5.10.7.4, 5.10.7.5, 5.10.7.6, 5.10.7.7, 5.10.7.8, 5.10.7.9, 5.10.7.10, 5.10.8.1, 5.10.8.2, 5.10.8.3, 5.10.8.4, 5.10.8.5, 5.10.8.6, 5.10.8.7, 5.10.8.8, 5.10.8.9, 5.10.8.10, 5.10.9.1, 5.10.9.2, 5.10.9.3, 5.10.9.4, 5.10.9.5, 5.10.9.6, 5.10.9.7, 5.10.9.8, 5.10.9.9, 5.10.9.10, 5.10.10.1, 5.10.10.2, 5.10.10.3, 5.10.10.4, 5.10.10.5, 5.10.10.6, 5.10.10.7, 5.10.10.8, 5.10.10.9, 5.10.10.10, 6.1.1.1, 6.1.1.2, 6.1.1.3, 6.1.1.4, 6.1.1.5, 6.1.1.6, 6.1.1.7, 6.1.1.8, 6.1.1.9, 6.1.1.10, 6.1.2.1, 6.1.2.2, 6.1.2.3, 6.1.2.4, 6.1.2.5, 6.1.2.6, 6.1.2.7, 6.1.2.8, 6.1.2.9, 6.1.2.10, 6.1.3.1, 6.1.3.2, 6.1.3.3, 6.1.3.4, 6.1.3.5, 6.1.3.6, 6.1.3.7, 6.1.3.8, 6.1.3.9, 6.1.3.10, 6.1.4.1, 6.1.4.2, 6.1.4.3, 6.1.4.4, 6.1.4.5, 6.1.4.6, |
| 6.1.4.7, 6.1.4.8, 6.1.4.9, 6.1.4.10, 6.1.5.1, 6.1.5.2, 6.1.5.3, 6.1.5.4, 6.1.5.5, 6.1.5.6, 6.1.5.7, 6.1.5.8, 6.1.5.9, 6.1.5.10, 6.1.6.1, 6.1.6.2, 6.1.6.3, 6.1.6.4, 6.1.6.5, 6.1.6.6, 6.1.6.7, 6.1.6.8, 6.1.6.9, 6.1.6.10, 6.1.7.1, 6.1.7.2, 6.1.7.3, 6.1.7.4, 6.1.7.5, 6.1.7.6, 6.1.7.7, 6.1.7.8, 6.1.7.9, 6.1.7.10, 6.1.8.1, 6.1.8.2, 6.1.8.3, 6.1.8.4, 6.1.8.5, 6.1.8.6, 6.1.8.7, 6.1.8.8, 6.1.8.9, 6.1.8.10, 6.1.9.1, 6.1.9.2, 6.1.9.3, 6.1.9.4, 6.1.9.5, 6.1.9.6, 6.1.9.7, 6.1.9.8, 6.1.9.9, 6.1.9.10, 6.1.10.1, 6.1.10.2, 6.1.10.3, 6.1.10.4, 6.1.10.5, 6.1.10.6, 6.1.10.7, 6.1.10.8, 6.1.10.9, 6.1.10.10, 6.2.1.1, 6.2.1.2, 6.2.1.3, 6.2.1.4, 6.2.1.5, 6.2.1.6, 6.2.1.7, 6.2.1.8, 6.2.1.9, 6.2.1.10, 6.2.2.1, 6.2.2.2, 6.2.2.3, 6.2.2.4, 6.2.2.5, 6.2.2.6, 6.2.2.7, 6.2.2.8, 6.2.2.9, 6.2.2.10, 6.2.3.1, 6.2.3.2, 6.2.3.3, 6.2.3.4, 6.2.3.5, 6.2.3.6, 6.2.3.7, 6.2.3.8, 6.2.3.9, 6.2.3.10, 6.2.4.1, 6.2.4.2, 6.2.4.3, 6.2.4.4, 6.2.4.5, 6.2.4.6, 6.2.4.7, 6.2.4.8, 6.2.4.9, 6.2.4.10, 6.2.5.1, 6.2.5.2, 6.2.5.3, 6.2.5.4, 6.2.5.5, 6.2.5.6, 6.2.5.7, 6.2.5.8, 6.2.5.9, 6.2.5.10, 6.2.6.1, 6.2.6.2, 6.2.6.3, 6.2.6.4, 6.2.6.5, 6.2.6.6, 6.2.6.7, 6.2.6.8, 6.2.6.9, 6.2.6.10, 6.2.7.1, 6.2.7.2, 6.2.7.3, 6.2.7.4, 6.2.7.5, 6.2.7.6, 6.2.7.7, 6.2.7.8, 6.2.7.9, 6.2.7.10, 6.2.8.1, 6.2.8.2, 6.2.8.3, 6.2.8.4, 6.2.8.5, 6.2.8.6, 6.2.8.7, 6.2.8.8, 6.2.8.9, 6.2.8.10, 6.2.9.1, 6.2.9.2, 6.2.9.3, 6.2.9.4, 6.2.9.5, 6.2.9.6, 6.2.9.7, 6.2.9.8, 6.2.9.9, 6.2.9.10, 6.2.10.1, 6.2.10.2, 6.2.10.3, 6.2.10.4, 6.2.10.5, 6.2.10.6, 6.2.10.7, 6.2.10.8, 6.2.10.9, 6.2.10.10, 6.3.1.1, 6.3.1.2, 6.3.1.3, 6.3.1.4, 6.3.1.5, 6.3.1.6, 6.3.1.7, 6.3.1.8, 6.3.1.9, 6.3.1.10, 6.3.2.1, 6.3.2.2, 6.3.2.3, 6.3.2.4, 6.3.2.5, 6.3.2.6, 6.3.2.7, 6.3.2.8, 6.3.2.9, 6.3.2.10, 6.3.3.1, 6.3.3.2, 6.3.3.3, 6.3.3.4, 6.3.3.5, 6.3.3.6, 6.3.3.7, 6.3.3.8, 6.3.3.9, 6.3.3.10, 6.3.4.1, 6.3.4.2, 6.3.4.3, 6.3.4.4, 6.3.4.5, 6.3.4.6, 6.3.4.7, 6.3.4.8, 6.3.4.9, 6.3.4.10, 6.3.5.1, 6.3.5.2, 6.3.5.3, 6.3.5.4, 6.3.5.5, 6.3.5.6, 6.3.5.7, 6.3.5.8, 6.3.5.9, 6.3.5.10, 6.3.6.1, 6.3.6.2, 6.3.6.3, 6.3.6.4, 6.3.6.5, 6.3.6.6, 6.3.6.7, 6.3.6.8, 6.3.6.9, 6.3.6.10, 6.3.7.1, 6.3.7.2, 6.3.7.3, 6.3.7.4, 6.3.7.5, 6.3.7.6, 6.3.7.7, 6.3.7.8, 6.3.7.9, 6.3.7.10, 6.3.8.1, 6.3.8.2, 6.3.8.3, 6.3.8.4, 6.3.8.5, 6.3.8.6, 6.3.8.7, 6.3.8.8, 6.3.8.9, 6.3.8.10, 6.3.9.1, 6.3.9.2, 6.3.9.3, 6.3.9.4, 6.3.9.5, 6.3.9.6, 6.3.9.7, 6.3.9.8, 6.3.9.9, 6.3.9.10, 6.3.10.1, 6.3.10.2, 6.3.10.3, 6.3.10.4, 6.3.10.5, 6.3.10.6, 6.3.10.7, 6.3.10.8, 6.3.10.9, 6.3.10.10, 6.4.1.1, 6.4.1.2, 6.4.1.3, 6.4.1.4, 6.4.1.5, 6.4.1.6, 6.4.1.7, 6.4.1.8, 6.4.1.9, 6.4.1.10, 6.4.2.1, 6.4.2.2, 6.4.2.3, 6.4.2.4, 6.4.2.5, 6.4.2.6, 6.4.2.7, 6.4.2.8, 6.4.2.9, 6.4.2.10, 6.4.3.1, 6.4.3.2, 6.4.3.3, 6.4.3.4, 6.4.3.5, 6.4.3.6, 6.4.3.7, 6.4.3.8, 6.4.3.9, 6.4.3.10, 6.4.4.1, 6.4.4.2, 6.4.4.3, 6.4.4.4, 6.4.4.5, 6.4.4.6, 6.4.4.7, 6.4.4.8, 6.4.4.9, 6.4.4.10, 6.4.5.1, 6.4.5.2, 6.4.5.3, 6.4.5.4, 6.4.5.5, 6.4.5.6, 6.4.5.7, 6.4.5.8, 6.4.5.9, 6.4.5.10, 6.4.6.1, 6.4.6.2, 6.4.6.3, 6.4.6.4, 6.4.6.5, 6.4.6.6, 6.4.6.7, 6.4.6.8, 6.4.6.9, 6.4.6.10, 6.4.7.1, 6.4.7.2, 6.4.7.3, 6.4.7.4, 6.4.7.5, 6.4.7.6, 6.4.7.7, 6.4.7.8, 6.4.7.9, 6.4.7.10, 6.4.8.1, 6.4.8.2, 6.4.8.3, 6.4.8.4, 6.4.8.5, 6.4.8.6, 6.4.8.7, 6.4.8.8, 6.4.8.9, 6.4.8.10, 6.4.9.1, 6.4.9.2, 6.4.9.3, 6.4.9.4, 6.4.9.5, 6.4.9.6, 6.4.9.7, 6.4.9.8, 6.4.9.9, 6.4.9.10, 6.4.10.1, 6.4.10.2, 6.4.10.3, 6.4.10.4, 6.4.10.5, 6.4.10.6, 6.4.10.7, 6.4.10.8, 6.4.10.9, 6.4.10.10, 6.5.1.1, 6.5.1.2, 6.5.1.3, 6.5.1.4, 6.5.1.5, 6.5.1.6, 6.5.1.7, 6.5.1.8, 6.5.1.9, 6.5.1.10, 6.5.2.1, 6.5.2.2, 6.5.2.3, 6.5.2.4, 6.5.2.5, 6.5.2.6, 6.5.2.7, 6.5.2.8, 6.5.2.9, 6.5.2.10, 6.5.3.1, 6.5.3.2, 6.5.3.3, 6.5.3.4, 6.5.3.5, 6.5.3.6, 6.5.3.7, 6.5.3.8, 6.5.3.9, 6.5.3.10, 6.5.4.1, 6.5.4.2, 6.5.4.3, 6.5.4.4, 6.5.4.5, 6.5.4.6, 6.5.4.7, |
| 6.5.4.8, 6.5.4.9, 6.5.4.10, 6.5.5.1, 6.5.5.2, 6.5.5.3, 6.5.5.4, 6.5.5.5, 6.5.5.6, 6.5.5.7, 6.5.5.8, 6.5.5.9, 6.5.5.10, 6.5.6.1, 6.5.6.2, 6.5.6.3, 6.5.6.4, 6.5.6.5, 6.5.6.6, 6.5.6.7, 6.5.6.8, 6.5.6.9, 6.5.6.10, 6.5.7.1, 6.5.7.2, 6.5.7.3, 6.5.7.4, 6.5.7.5, 6.5.7.6, 6.5.7.7, 6.5.7.8, 6.5.7.9, 6.5.7.10, 6.5.8.1, 6.5.8.2, 6.5.8.3, 6.5.8.4, 6.5.8.5, 6.5.8.6, 6.5.8.7, 6.5.8.8, 6.5.8.9, 6.5.8.10, 6.5.9.1, 6.5.9.2, 6.5.9.3, 6.5.9.4, 6.5.9.5, 6.5.9.6, 6.5.9.7, 6.5.9.8, 6.5.9.9, 6.5.9.10, 6.5.10.1, 6.5.10.2, 6.5.10.3, 6.5.10.4, 6.5.10.5, 6.5.10.6, 6.5.10.7, 6.5.10.8, 6.5.10.9, 6.5.10.10, 6.6.1.1, 6.6.1.2, 6.6.1.3, 6.6.1.4, 6.6.1.5, 6.6.1.6, 6.6.1.7, 6.6.1.8, 6.6.1.9, 6.6.1.10, 6.6.2.1, 6.6.2.2, 6.6.2.3, 6.6.2.4, 6.6.2.5, 6.6.2.6, 6.6.2.7, 6.6.2.8, 6.6.2.9, 6.6.2.10, 6.6.3.1, 6.6.3.2, 6.6.3.3, 6.6.3.4, 6.6.3.5, 6.6.3.6, 6.6.3.7, 6.6.3.8, 6.6.3.9, 6.6.3.10, 6.6.4.1, 6.6.4.2, 6.6.4.3, 6.6.4.4, 6.6.4.5, 6.6.4.6, 6.6.4.7, 6.6.4.8, 6.6.4.9, 6.6.4.10, 6.6.5.1, 6.6.5.2, 6.6.5.3, 6.6.5.4, 6.6.5.5, 6.6.5.6, 6.6.5.7, 6.6.5.8, 6.6.5.9, 6.6.5.10, 6.6.6.1, 6.6.6.2, 6.6.6.3, 6.6.6.4, 6.6.6.5, 6.6.6.6, 6.6.6.7, 6.6.6.8, 6.6.6.9, 6.6.6.10, 6.6.7.1, 6.6.7.2, 6.6.7.3, 6.6.7.4, 6.6.7.5, 6.6.7.6, 6.6.7.7, 6.6.7.8, 6.6.7.9, 6.6.7.10, 6.6.8.1, 6.6.8.2, 6.6.8.3, 6.6.8.4, 6.6.8.5, 6.6.8.6, 6.6.8.7, 6.6.8.8, 6.6.8.9, 6.6.8.10, 6.6.9.1, 6.6.9.2, 6.6.9.3, 6.6.9.4, 6.6.9.5, 6.6.9.6, 6.6.9.7, 6.6.9.8, 6.6.9.9, 6.6.9.10, 6.6.10.1, 6.6.10.2, 6.6.10.3, 6.6.10.4, 6.6.10.5, 6.6.10.6, 6.6.10.7, 6.6.10.8, 6.6.10.9, 6.6.10.10, 6.7.1.1, 6.7.1.2, 6.7.1.3, 6.7.1.4, 6.7.1.5, 6.7.1.6, 6.7.1.7, 6.7.1.8, 6.7.1.9, 6.7.1.10, 6.7.2.1, 6.7.2.2, 6.7.2.3, 6.7.2.4, 6.7.2.5, 6.7.2.6, 6.7.2.7, 6.7.2.8, 6.7.2.9, 6.7.2.10, 6.7.3.1, 6.7.3.2, 6.7.3.3, 6.7.3.4, 6.7.3.5, 6.7.3.6, 6.7.3.7, 6.7.3.8, 6.7.3.9, 6.7.3.10, 6.7.4.1, 6.7.4.2, 6.7.4.3, 6.7.4.4, 6.7.4.5, 6.7.4.6, 6.7.4.7, 6.7.4.8, 6.7.4.9, 6.7.4.10, 6.7.5.1, 6.7.5.2, 6.7.5.3, 6.7.5.4, 6.7.5.5, 6.7.5.6, 6.7.5.7, 6.7.5.8, 6.7.5.9, 6.7.5.10, 6.7.6.1, 6.7.6.2, 6.7.6.3, 6.7.6.4, 6.7.6.5, 6.7.6.6, 6.7.6.7, 6.7.6.8, 6.7.6.9, 6.7.6.10, 6.7.7.1, 6.7.7.2, 6.7.7.3, 6.7.7.4, 6.7.7.5, 6.7.7.6, 6.7.7.7, 6.7.7.8, 6.7.7.9, 6.7.7.10, 6.7.8.1, 6.7.8.2, 6.7.8.3, 6.7.8.4, 6.7.8.5, 6.7.8.6, 6.7.8.7, 6.7.8.8, 6.7.8.9, 6.7.8.10, 6.7.9.1, 6.7.9.2, 6.7.9.3, 6.7.9.4, 6.7.9.5, 6.7.9.6, 6.7.9.7, 6.7.9.8, 6.7.9.9, 6.7.9.10, 6.7.10.1, 6.7.10.2, 6.7.10.3, 6.7.10.4, 6.7.10.5, 6.7.10.6, 6.7.10.7, 6.7.10.8, 6.7.10.9, 6.7.10.10, 6.8.1.1, 6.8.1.2, 6.8.1.3, 6.8.1.4, 6.8.1.5, 6.8.1.6, 6.8.1.7, 6.8.1.8, 6.8.1.9, 6.8.1.10, 6.8.2.1, 6.8.2.2, 6.8.2.3, 6.8.2.4, 6.8.2.5, 6.8.2.6, 6.8.2.7, 6.8.2.8, 6.8.2.9, 6.8.2.10, 6.8.3.1, 6.8.3.2, 6.8.3.3, 6.8.3.4, 6.8.3.5, 6.8.3.6, 6.8.3.7, 6.8.3.8, 6.8.3.9, 6.8.3.10, 6.8.4.1, 6.8.4.2, 6.8.4.3, 6.8.4.4, 6.8.4.5, 6.8.4.6, 6.8.4.7, 6.8.4.8, 6.8.4.9, 6.8.4.10, 6.8.5.1, 6.8.5.2, 6.8.5.3, 6.8.5.4, 6.8.5.5, 6.8.5.6, 6.8.5.7, 6.8.5.8, 6.8.5.9, 6.8.5.10, 6.8.6.1, 6.8.6.2, 6.8.6.3, 6.8.6.4, 6.8.6.5, 6.8.6.6, 6.8.6.7, 6.8.6.8, 6.8.6.9, 6.8.6.10, 6.8.7.1, 6.8.7.2, 6.8.7.3, 6.8.7.4, 6.8.7.5, 6.8.7.6, 6.8.7.7, 6.8.7.8, 6.8.7.9, 6.8.7.10, 6.8.8.1, 6.8.8.2, 6.8.8.3, 6.8.8.4, 6.8.8.5, 6.8.8.6, 6.8.8.7, 6.8.8.8, 6.8.8.9, 6.8.8.10, 6.8.9.1, 6.8.9.2, 6.8.9.3, 6.8.9.4, 6.8.9.5, 6.8.9.6, 6.8.9.7, 6.8.9.8, 6.8.9.9, 6.8.9.10, 6.8.10.1, 6.8.10.2, 6.8.10.3, 6.8.10.4, 6.8.10.5, 6.8.10.6, 6.8.10.7, 6.8.10.8, 6.8.10.9, 6.8.10.10, 6.9.1.1, 6.9.1.2, 6.9.1.3, 6.9.1.4, 6.9.1.5, 6.9.1.6, 6.9.1.7, 6.9.1.8, 6.9.1.9, 6.9.1.10, 6.9.2.1, 6.9.2.2, 6.9.2.3, 6.9.2.4, 6.9.2.5, 6.9.2.6, 6.9.2.7, 6.9.2.8, 6.9.2.9, 6.9.2.10, 6.9.3.1, 6.9.3.2, 6.9.3.3, 6.9.3.4, 6.9.3.5, 6.9.3.6, 6.9.3.7, 6.9.3.8, 6.9.3.9, 6.9.3.10, 6.9.4.1, 6.9.4.2, 6.9.4.3, 6.9.4.4, 6.9.4.5, 6.9.4.6, 6.9.4.7, |
| 6.9.4.8, 6.9.4.9, 6.9.4.10, 6.9.5.1, 6.9.5.2, 6.9.5.3, 6.9.5.4, 6.9.5.5, 6.9.5.6, 6.9.5.7, 6.9.5.8, 6.9.5.9, 6.9.5.10, 6.9.6.1, 6.9.6.2, 6.9.6.3, 6.9.6.4, 6.9.6.5, 6.9.6.6, 6.9.6.7, 6.9.6.8, 6.9.6.9, 6.9.6.10, 6.9.7.1, 6.9.7.2, 6.9.7.3, 6.9.7.4, 6.9.7.5, 6.9.7.6, 6.9.7.7, 6.9.7.8, 6.9.7.9, 6.9.7.10, 6.9.8.1, 6.9.8.2, 6.9.8.3, 6.9.8.4, 6.9.8.5, 6.9.8.6, 6.9.8.7, 6.9.8.8, 6.9.8.9, 6.9.8.10, 6.9.9.1, 6.9.9.2, 6.9.9.3, 6.9.9.4, 6.9.9.5, 6.9.9.6, 6.9.9.7, 6.9.9.8, 6.9.9.9, 6.9.9.10, 6.9.10.1, 6.9.10.2, 6.9.10.3, 6.9.10.4, 6.9.10.5, 6.9.10.6, 6.9.10.7, 6.9.10.8, 6.9.10.9, 6.9.10.10, 6.10.1.1, 6.10.1.2, 6.10.1.3, 6.10.1.4, 6.10.1.5, 6.10.1.6, 6.10.1.7, 6.10.1.8, 6.10.1.9, 6.10.1.10, 6.10.2.1, 6.10.2.2, 6.10.2.3, 6.10.2.4, 6.10.2.5, 6.10.2.6, 6.10.2.7, 6.10.2.8, 6.10.2.9, 6.10.2.10, 6.10.3.1, 6.10.3.2, 6.10.3.3, 6.10.3.4, 6.10.3.5, 6.10.3.6, 6.10.3.7, 6.10.3.8, 6.10.3.9, 6.10.3.10, 6.10.4.1, 6.10.4.2, 6.10.4.3, 6.10.4.4, 6.10.4.5, 6.10.4.6, 6.10.4.7, 6.10.4.8, 6.10.4.9, 6.10.4.10, 6.10.5.1, 6.10.5.2, 6.10.5.3, 6.10.5.4, 6.10.5.5, 6.10.5.6, 6.10.5.7, 6.10.5.8, 6.10.5.9, 6.10.5.10, 6.10.6.1, 6.10.6.2, 6.10.6.3, 6.10.6.4, 6.10.6.5, 6.10.6.6, 6.10.6.7, 6.10.6.8, 6.10.6.9, 6.10.6.10, 6.10.7.1, 6.10.7.2, 6.10.7.3, 6.10.7.4, 6.10.7.5, 6.10.7.6, 6.10.7.7, 6.10.7.8, 6.10.7.9, 6.10.7.10, 6.10.8.1, 6.10.8.2, 6.10.8.3, 6.10.8.4, 6.10.8.5, 6.10.8.6, 6.10.8.7, 6.10.8.8, 6.10.8.9, 6.10.8.10, 6.10.9.1, 6.10.9.2, 6.10.9.3, 6.10.9.4, 6.10.9.5, 6.10.9.6, 6.10.9.7, 6.10.9.8, 6.10.9.9, 6.10.9.10, 6.10.10.1, 6.10.10.2, 6.10.10.3, 6.10.10.4, 6.10.10.5, 6.10.10.6, 6.10.10.7, 6.10.10.8, 6.10.10.9, 6.10.10.10, 7.1.1.1, 7.1.1.2, 7.1.1.3, 7.1.1.4, 7.1.1.5, 7.1.1.6, 7.1.1.7, 7.1.1.8, 7.1.1.9, 7.1.1.10, 7.1.2.1, 7.1.2.2, 7.1.2.3, 7.1.2.4, 7.1.2.5, 7.1.2.6, 7.1.2.7, 7.1.2.8, 7.1.2.9, 7.1.2.10, 7.1.3.1, 7.1.3.2, 7.1.3.3, 7.1.3.4, 7.1.3.5, 7.1.3.6, 7.1.3.7, 7.1.3.8, 7.1.3.9, 7.1.3.10, 7.1.4.1, 7.1.4.2, 7.1.4.3, 7.1.4.4, 7.1.4.5, 7.1.4.6, 7.1.4.7, 7.1.4.8, 7.1.4.9, 7.1.4.10, 7.1.5.1, 7.1.5.2, 7.1.5.3, 7.1.5.4, 7.1.5.5, 7.1.5.6, 7.1.5.7, 7.1.5.8, 7.1.5.9, 7.1.5.10, 7.1.6.1, 7.1.6.2, 7.1.6.3, 7.1.6.4, 7.1.6.5, 7.1.6.6, 7.1.6.7, 7.1.6.8, 7.1.6.9, 7.1.6.10, 7.1.7.1, 7.1.7.2, 7.1.7.3, 7.1.7.4, 7.1.7.5, 7.1.7.6, 7.1.7.7, 7.1.7.8, 7.1.7.9, 7.1.7.10, 7.1.8.1, 7.1.8.2, 7.1.8.3, 7.1.8.4, 7.1.8.5, 7.1.8.6, 7.1.8.7, 7.1.8.8, 7.1.8.9, 7.1.8.10, 7.1.9.1, 7.1.9.2, 7.1.9.3, 7.1.9.4, 7.1.9.5, 7.1.9.6, 7.1.9.7, 7.1.9.8, 7.1.9.9, 7.1.9.10, 7.1.10.1, 7.1.10.2, 7.1.10.3, 7.1.10.4, 7.1.10.5, 7.1.10.6, 7.1.10.7, 7.1.10.8, 7.1.10.9, 7.1.10.10, 7.2.1.1, 7.2.1.2, 7.2.1.3, 7.2.1.4, 7.2.1.5, 7.2.1.6, 7.2.1.7, 7.2.1.8, 7.2.1.9, 7.2.1.10, 7.2.2.1, 7.2.2.2, 7.2.2.3, 7.2.2.4, 7.2.2.5, 7.2.2.6, 7.2.2.7, 7.2.2.8, 7.2.2.9, 7.2.2.10, 7.2.3.1, 7.2.3.2, 7.2.3.3, 7.2.3.4, 7.2.3.5, 7.2.3.6, 7.2.3.7, 7.2.3.8, 7.2.3.9, 7.2.3.10, 7.2.4.1, 7.2.4.2, 7.2.4.3, 7.2.4.4, 7.2.4.5, 7.2.4.6, 7.2.4.7, 7.2.4.8, 7.2.4.9, 7.2.4.10, 7.2.5.1, 7.2.5.2, 7.2.5.3, 7.2.5.4, 7.2.5.5, 7.2.5.6, 7.2.5.7, 7.2.5.8, 7.2.5.9, 7.2.5.10, 7.2.6.1, 7.2.6.2, 7.2.6.3, 7.2.6.4, 7.2.6.5, 7.2.6.6, 7.2.6.7, 7.2.6.8, 7.2.6.9, 7.2.6.10, 7.2.7.1, 7.2.7.2, 7.2.7.3, 7.2.7.4, 7.2.7.5, 7.2.7.6, 7.2.7.7, 7.2.7.8, 7.2.7.9, 7.2.7.10, 7.2.8.1, 7.2.8.2, 7.2.8.3, 7.2.8.4, 7.2.8.5, 7.2.8.6, 7.2.8.7, 7.2.8.8, 7.2.8.9, 7.2.8.10, 7.2.9.1, 7.2.9.2, 7.2.9.3, 7.2.9.4, 7.2.9.5, 7.2.9.6, 7.2.9.7, 7.2.9.8, 7.2.9.9, 7.2.9.10, 7.2.10.1, 7.2.10.2, |
| 7.2.10.3, 7.2.10.4, 7.2.10.5, 7.2.10.6, 7.2.10.7, 7.2.10.8, 7.2.10.9, 7.2.10.10, 7.3.1.1, 7.3.1.2, 7.3.1.3, 7.3.1.4, 7.3.1.5, 7.3.1.6, 7.3.1.7, 7.3.1.8, 7.3.1.9, 7.3.1.10, 7.3.2.1, 7.3.2.2, 7.3.2.3, 7.3.2.4, 7.3.2.5, 7.3.2.6, 7.3.2.7, 7.3.2.8, 7.3.2.9, 7.3.2.10, 7.3.3.1, 7.3.3.2, 7.3.3.3, 7.3.3.4, 7.3.3.5, 7.3.3.6, 7.3.3.7, 7.3.3.8, 7.3.3.9, 7.3.3.10, 7.3.4.1, 7.3.4.2, 7.3.4.3, 7.3.4.4, 7.3.4.5, 7.3.4.6, 7.3.4.7, 7.3.4.8, 7.3.4.9, 7.3.4.10, 7.3.5.1, 7.3.5.2, 7.3.5.3, 7.3.5.4, 7.3.5.5, 7.3.5.6, 7.3.5.7, 7.3.5.8, 7.3.5.9, 7.3.5.10, 7.3.6.1, 7.3.6.2, 7.3.6.3, 7.3.6.4, 7.3.6.5, 7.3.6.6, 7.3.6.7, 7.3.6.8, 7.3.6.9, 7.3.6.10, 7.3.7.1, 7.3.7.2, 7.3.7.3, 7.3.7.4, 7.3.7.5, 7.3.7.6, 7.3.7.7, 7.3.7.8, 7.3.7.9, 7.3.7.10, 7.3.8.1, 7.3.8.2, 7.3.8.3, 7.3.8.4, 7.3.8.5, 7.3.8.6, 7.3.8.7, 7.3.8.8, 7.3.8.9, 7.3.8.10, 7.3.9.1, 7.3.9.2, 7.3.9.3, 7.3.9.4, 7.3.9.5, 7.3.9.6, 7.3.9.7, 7.3.9.8, 7.3.9.9, 7.3.9.10, 7.3.10.1, 7.3.10.2, 7.3.10.3, 7.3.10.4, 7.3.10.5, 7.3.10.6, 7.3.10.7, 7.3.10.8, 7.3.10.9, 7.3.10.10, 7.4.1.1, 7.4.1.2, 7.4.1.3, 7.4.1.4, 7.4.1.5, 7.4.1.6, 7.4.1.7, 7.4.1.8, 7.4.1.9, 7.4.1.10, 7.4.2.1, 7.4.2.2, 7.4.2.3, 7.4.2.4, 7.4.2.5, 7.4.2.6, 7.4.2.7, 7.4.2.8, 7.4.2.9, 7.4.2.10, 7.4.3.1, 7.4.3.2, 7.4.3.3, 7.4.3.4, 7.4.3.5, 7.4.3.6, 7.4.3.7, 7.4.3.8, 7.4.3.9, 7.4.3.10, 7.4.4.1, 7.4.4.2, 7.4.4.3, 7.4.4.4, 7.4.4.5, 7.4.4.6, 7.4.4.7, 7.4.4.8, 7.4.4.9, 7.4.4.10, 7.4.5.1, 7.4.5.2, 7.4.5.3, 7.4.5.4, 7.4.5.5, 7.4.5.6, 7.4.5.7, 7.4.5.8, 7.4.5.9, 7.4.5.10, 7.4.6.1, 7.4.6.2, 7.4.6.3, 7.4.6.4, 7.4.6.5, 7.4.6.6, 7.4.6.7, 7.4.6.8, 7.4.6.9, 7.4.6.10, 7.4.7.1, 7.4.7.2, 7.4.7.3, 7.4.7.4, 7.4.7.5, 7.4.7.6, 7.4.7.7, 7.4.7.8, 7.4.7.9, 7.4.7.10, 7.4.8.1, 7.4.8.2, 7.4.8.3, 7.4.8.4, 7.4.8.5, 7.4.8.6, 7.4.8.7, 7.4.8.8, 7.4.8.9, 7.4.8.10, 7.4.9.1, 7.4.9.2, 7.4.9.3, 7.4.9.4, 7.4.9.5, 7.4.9.6, 7.4.9.7, 7.4.9.8, 7.4.9.9, 7.4.9.10, 7.4.10.1, 7.4.10.2, 74.10.3, 7.4.10.4, 7.4.10.5, 7.4.10.6, 7.4.10.7, 7.4.10.8, 7.4.10.9, 7.4.10.10, 7.5.1.1, 7.5.1.2, 7.5.1.3, 7.5.1.4, 7.5.1.5, 7.5.1.6, 7.5.1.7, 7.5.1.8, 7.5.1.9, 7.5.1.10, 7.5.2.1, 7.5.2.2, 7.5.2.3, 7.5.2.4, 7.5.2.5, 7.5.2.6, 7.5.2.7, 7.5.2.8, 7.5.2.9, 7.5.2.10, 7.5.3.1, 7.5.3.2, 7.5.3.3, 7.5.3.4, 7.5.3.5, 7.5.3.6, 7.5.3.7, 7.5.3.8, 7.5.3.9, 7.5.3.10, 7.5.4.1, 7.5.4.2, 7.5.4.3, 7.5.4.4, 7.5.4.5, 7.5.4.6, 7.5.4.7, 7.5.4.8, 7.5.4.9, 7.5.4.10, 7.5.5.1, 7.5.5.2, 7.5.5.3, 7.5.5.4, 7.5.5.5, 7.5.5.6, 7.5.5.7, 7.5.5.8, 7.5.5.9, 7.5.5.10, 7.5.6.1, 7.5.6.2, 7.5.6.3, 7.5.6.4, 7.5.6.5, 7.5.6.6, 7.5.6.7, 7.5.6.8, 7.5.6.9, 7.5.6.10, 7.5.7.1, 7.5.7.2, 7.5.7.3, 7.5.7.4, 7.5.7.5, 7.5.7.6, 7.5.7.7, 7.5.7.8, 7.5.7.9, 7.5.7.10, 7.5.8.1, 7.5.8.2, 7.5.8.3, 7.5.8.4, 7.5.8.5, 7.5.8.6, 7.5.8.7, 7.5.8.8, 7.5.8.9, 7.5.8.10, 7.5.9.1, 7.5.9.2, 7.5.9.3, 7.5.9.4, 7.5.9.5, 7.5.9.6, 7.5.9.7, 7.5.9.8, 7.5.9.9, 7.5.9.10, 7.5.10.1, 7.5.10.2, 7.5.10.3, 7.5.10.4, 7.5.10.5, 7.5.10.6, 7.5.10.7, 7.5.10.8, 7.5.10.9, 7.5.10.10, 7.6.1.1, 7.6.1.2, 7.6.1.3, 7.6.1.4, 7.6.1.5, 7.6.1.6, 7.6.1.7, 7.6.1.8, 7.6.1.9, 7.6.1.10, 7.6.2.1, 7.6.2.2, 7.6.2.3, 7.6.2.4, 7.6.2.5, 7.6.2.6, 7.6.2.7, 7.6.2.8, 7.6.2.9, 7.6.2.10, 7.6.3.1, 7.6.3.2, 7.6.3.3, 7.6.3.4, 7.6.3.5, 7.6.3.6, 7.6.3.7, 7.6.3.8, 7.6.3.9, 7.6.3.10, 7.6.4.1, 7.6.4.2, 7.6.4.3, 7.6.4.4, 7.6.4.5, 7.6.4.6, 7.6.4.7, 7.6.4.8, 7.6.4.9, 7.6.4.10, 7.6.5.1, 7.6.5.2, 7.6.5.3, 7.6.5.4, 7.6.5.5, 7.6.5.6, 7.6.5.7, 7.6.5.8, 7.6.5.9, 7.6.5.10, 7.6.6.1, 7.6.6.2, 7.6.6.3, 7.6.6.4, 7.6.6.5, 7.6.6.6, 7.6.6.7, 7.6.6.8, 7.6.6.9, 7.6.6.10, 7.6.7.1, 7.6.7.2, |
| 7.6.7.3, 7.6.7.4, 7.6.7.5, 7.6.7.6, 7.6.7.7, 7.6.7.8, 7.6.7.9, 7.6.7.10, 7.6.8.1, 7.6.8.2, 7.6.8.3, 7.6.8.4, 7.6.8.5, 7.6.8.6, 7.6.8.7, 7.6.8.8, 7.6.8.9, 7.6.8.10, 7.6.9.1, 7.6.9.2, 7.6.9.3, 7.6.9.4, 7.6.9.5, 7.6.9.6, 7.6.9.7, 7.6.9.8, 7.6.9.9, 7.6.9.10, 7.6.10.1, 7.6.10.2, 7.6.10.3, 7.6.10.4, 7.6.10.5, 7.6.10.6, 7.6.10.7, 7.6.10.8, 7.6.10.9, 7.6.10.10, 7.7.1.1, 7.7.1.2, 7.7.1.3, 7.7.1.4, 7.7.1.5, 7.7.1.6, 7.7.1.7, 7.7.1.8, 7.7.1.9, 7.7.1.10, 7.7.2.1, 7.7.2.2, 7.7.2.3, 7.7.2.4, 7.7.2.5, 7.7.2.6, 7.7.2.7, 7.7.2.8, 7.7.2.9, 7.7.2.10, 7.7.3.1, 7.7.3.2, 7.7.3.3, 7.7.3.4, 7.7.3.5, 7.7.3.6, 7.7.3.7, 7.7.3.8, 7.7.3.9, 7.7.3.10, 7.7.4.1, 7.7.4.2, 7.7.4.3, 7.7.4.4, 7.7.4.5, 7.7.4.6, 7.7.4.7, 7.7.4.8, 7.7.4.9, 7.7.4.10, 7.7.5.1, 7.7.5.2, 7.7.5.3, 7.7.5.4, 7.7.5.5, 7.7.5.6, 7.7.5.7, 7.7.5.8, 7.7.5.9, 7.7.5.10, 7.7.6.1, 7.7.6.2, 7.7.6.3, 7.7.6.4, 7.7.6.5, 7.7.6.6, 7.7.6.7, 7.7.6.8, 7.7.6.9, 7.7.6.10, 7.7.7.1, 7.7.7.2, 7.7.7.3, 7.7.7.4, 7.7.7.5, 7.7.7.6, 7.7.7.7, 7.7.7.8, 7.7.7.9, 7.7.7.10, 7.7.8.1, 7.7.8.2, 7.7.8.3, 7.7.8.4, 7.7.8.5, 7.7.8.6, 7.7.8.7, 7.7.8.8, 7.7.8.9, 7.7.8.10, 7.7.9.1, 7.7.9.2, 7.7.9.3, 7.7.9.4, 7.7.9.5, 7.7.9.6, 7.7.9.7, 7.7.9.8, 7.7.9.9, 7.7.9.10, 7.7.10.1, 7.7.10.2, 7.7.10.3, 7.7.10.4, 7.7.10.5, 7.7.10.6, 7.7.10.7, 7.7.10.8, 7.7.10.9, 7.7.10.10, 7.8.1.1, 7.8.1.2, 7.8.1.3, 7.8.1.4, 7.8.1.5, 7.8.1.6, 7.8.1.7, 7.8.1.8, 7.8.1.9, 7.8.1.10, 7.8.2.1, 7.8.2.2, 7.8.2.3, 7.8.2.4, 7.8.2.5, 7.8.2.6, 7.8.2.7, 7.8.2.8, 7.8.2.9, 7.8.2.10, 7.8.3.1, 7.8.3.2, 7.8.3.3, 7.8.3.4, 7.8.3.5, 7.8.3.6, 7.8.3.7, 7.8.3.8, 7.8.3.9, 7.8.3.10, 7.8.4.1, 7.8.4.2, 7.8.4.3, 7.8.4.4, 7.8.4.5, 7.8.4.6, 7.8.4.7, 7.8.4.8, 7.8.4.9, 7.8.4.10, 7.8.5.1, 7.8.5.2, 7.8.5.3, 7.8.5.4, 7.8.5.5, 7.8.5.6, 7.8.5.7, 7.8.5.8, 7.8.5.9, 7.8.5.10, 7.8.6.1, 7.8.6.2, 7.8.6.3, 7.8.6.4, 7.8.6.5, 7.8.6.6, 7.8.6.7, 7.8.6.8, 7.8.6.9, 7.8.6.10, 7.8.7.1, 7.8.7.2, 7.8.7.3, 7.8.7.4, 7.8.7.5, 7.8.7.6, 7.8.7.7, 7.8.7.8, 7.8.7.9, 7.8.7.10, 7.8.8.1, 7.8.8.2, 7.8.8.3, 7.8.8.4, 7.8.8.5, 7.8.8.6, 7.8.8.7, 7.8.8.8, 7.8.8.9, 7.8.8.10, 7.8.9.1, 7.8.9.2, 7.8.9.3, 7.8.9.4, 7.8.9.5, 7.8.9.6, 7.8.9.7, 7.8.9.8, 7.8.9.9, 7.8.9.10, 7.8.10.1, 7.8.10.2, 7.8.10.3, 7.8.10.4, 7.8.10.5, 7.8.10.6, 7.8.10.7, 7.8.10.8, 7.8.10.9, 7.8.10.10, 7.9.1.1, 7.9.1.2, 7.9.1.3, 7.9.1.4, 7.9.1.5, 7.9.1.6, 7.9.1.7, 7.9.1.8, 7.9.1.9, 7.9.1.10, 7.9.2.1, 7.9.2.2, 7.9.2.3, 7.9.2.4, 7.9.2.5, 7.9.2.6, 7.9.2.7, 7.9.2.8, 7.9.2.9, 7.9.2.10, 7.9.3.1, 7.9.3.2, 7.9.3.3, 7.9.3.4, 7.9.3.5, 7.9.3.6, 7.9.3.7, 7.9.3.8, 7.9.3.9, 7.9.3.10, 7.9.4.1, 7.9.4.2, 7.9.4.3, 7.9.4.4, 7.9.4.5, 7.9.4.6, 7.9.4.7, 7.9.4.8, 7.9.4.9, 7.9.4.10, 7.9.5.1, 7.9.5.2, 7.9.5.3, 7.9.5.4, 7.9.5.5, 7.9.5.6, 7.9.5.7, 7.9.5.8, 7.9.5.9, 7.9.5.10, 7.9.6.1, 7.9.6.2, 7.9.6.3, 7.9.6.4, 7.9.6.5, 7.9.6.6, 7.9.6.7, 7.9.6.8, 7.9.6.9, 7.9.6.10, 7.9.7.1, 7.9.7.2, 7.9.7.3, 7.9.7.4, 7.9.7.5, 7.9.7.6, 7.9.7.7, 7.9.7.8, 7.9.7.9, 7.9.7.10, 7.9.8.1, 7.9.8.2, 7.9.8.3, 7.9.8.4, 7.9.8.5, 7.9.8.6, 7.9.8.7, 7.9.8.8, 7.9.8.9, 7.9.8.10, 7.9.9.1, 7.9.9.2, 7.9.9.3, 7.9.9.4, 7.9.9.5, 7.9.9.6, 7.9.9.7, 7.9.9.8, 7.9.9.9, 7.9.9.10, 7.9.10.1, 7.9.10.2, 7.9.10.3, 7.9.10.4, 7.9.10.5, 7.9.10.6, 7.9.10.7, 7.9.10.8, 7.9.10.9, 7.9.10.10, 7.10.1.1, 7.10.1.2, 7.10.1.3, 7.10.1.4, 7.10.1.5, 7.10.1.6, 7.10.1.7, 7.10.1.8, 7.10.1.9, 7.10.1.10, 7.10.2.1, 7.10.2.2, 7.10.2.3, 7.10.2.4, 7.10.2.5, 7.10.2.6, 7.10.2.7, 7.10.2.8, 7.10.2.9, 7.10.2.10, 7.10.3.1, 7.10.3.2, 7.10.3.3, 7.10.3.4, 7.10.3.5, 7.10.3.6, 7.10.3.7, 7.10.3.8, 7.10.3.9, 7.10.3.10, 7.10.4.1, 7.10.4.2, 7.10.4.3, 7.10.4.4, 7.10.4.5, 7.10.4.6, 7.10.4.7, 7.10.4.8, 7.10.4.9, 7.10.4.10, 7.10.5.1, 7.10.5.2, 7.10.5.3, 7.10.5.4, 7.10.5.5, 7.10.5.6, 7.10.5.7, 7.10.5.8, 7.10.5.9, 7.10.5.10, 7.10.6.1, 7.10.6.2, 7.10.6.3, 7.10.6.4, 7.10.6.5, |
| 7.10.6.6, 7.10.6.7, 7.10.6.8, 7.10.6.9, 7.10.6.10, 7.10.7.1, 7.10.7.2, 7.10.7.3, 7.10.7.4, 7.10.7.5, 7.10.7.6, 7.10.7.7, 7.10.7.8, 7.10.7.9, 7.10.7.10, 7.10.8.1, 7.10.8.2, 7.10.8.3, 7.10.8.4, 7.10.8.5, 7.10.8.6, 7.10.8.7, 7.10.8.8, 7.10.8.9, 7.10.8.10, 7.10.9.1, 7.10.9.2, 7.10.9.3, 7.10.9.4, 7.10.9.5, 7.10.9.6, 7.10.9.7, 7.10.9.8, 7.10.9.9, 7.10.9.10, 7.10.10.1, 7.10.10.2, 7.10.10.3, 7.10.10.4, 7.10.10.5, 7.10.10.6, 7.10.10.7, 7.10.10.8, 7.10.10.9, 7.10.10.10, 8.1.1.1, 8.1.1.2, 8.1.1.3, 8.1.1.4, 8.1.1.5, 8.1.1.6, 8.1.1.7, 8.1.1.8, 8.1.1.9, 8.1.1.10, 8.1.2.1, 8.1.2.2, 8.1.2.3, 8.1.2.4, 8.1.2.5, 8.1.2.6, 8.1.2.7, 8.1.2.8, 8.1.2.9, 8.1.2.10, 8.1.3.1, 8.1.3.2, 8.1.3.3, 8.1.3.4, 8.1.3.5, 8.1.3.6, 8.1.3.7, 8.1.3.8, 8.1.3.9, 8.1.3.10, 8.1.4.1, 8.1.4.2, 8.1.4.3, 8.1.4.4, 8.1.4.5, 8.1.4.6, 8.1.4.7, 8.1.4.8, 8.1.4.9, 8.1.4.10, 8.1.5.1, 8.1.5.2, 8.1.5.3, 8.1.5.4, 8.1.5.5, 8.1.5.6, 8.1.5.7, 8.1.5.8, 8.1.5.9, 8.1.5.10, 8.1.6.1, 8.1.6.2, 8.1.6.3, 8.1.6.4, 8.1.6.5, 8.1.6.6, 8.1.6.7, 8.1.6.8, 8.1.6.9, 8.1.6.10, 8.1.7.1, 8.1.7.2, 8.1.7.3, 8.1.7.4, 8.1.7.5, 8.1.7.6, 8.1.7.7, 8.1.7.8, 8.1.7.9, 8.1.7.10, 8.1.8.1, 8.1.8.2, 8.1.8.3, 8.1.8.4, 8.1.8.5, 8.1.8.6, 8.1.8.7, 8.1.8.8, 8.1.8.9, 8.1.8.10, 8.1.9.1, 8.1.9.2, 8.1.9.3, 8.1.9.4, 8.1.9.5, 8.1.9.6, 8.1.9.7, 8.1.9.8, 8.1.9.9, 8.1.9.10, 8.1.10.1, 8.1.10.2, 8.1.10.3, 8.1.10.4, 8.1.10.5, 8.1.10.6, 8.1.10.7, 8.1.10.8, 8.1.10.9, 8.1.10.10, 8.2.1.1, 8.2.1.2, 8.2.1.3, 8.2.1.4, 8.2.1.5, 8.2.1.6, 8.2.1.7, 8.2.1.8, 8.2.1.9, 8.2.1.10, 8.2.2.1, 8.2.2.2, 8.2.2.3, 8.2.2.4, 8.2.2.5, 8.2.2.6, 8.2.2.7, 8.2.2.8, 8.2.2.9, 8.2.2.10, 8.2.3.1, 8.2.3.2, 8.2.3.3, 8.2.3.4, 8.2.3.5, 8.2.3.6, 8.2.3.7, 8.2.3.8, 8.2.3.9, 8.2.3.10, 8.2.4.1, 8.2.4.2, 8.2.4.3, 8.2.4.4, 8.2.4.5, 8.2.4.6, 8.2.4.7, 8.2.4.8, 8.2.4.9, 8.2.4.10, 8.2.5.1, 8.2.5.2, 8.2.5.3, 8.2.5.4, 8.2.5.5, 8.2.5.6, 8.2.5.7, 8.2.5.8, 8.2.5.9, 8.2.5.10, 8.2.6.1, 8.2.6.2, 8.2.6.3, 8.2.6.4, 8.2.6.5, 8.2.6.6, 8.2.6.7, 8.2.6.8, 8.2.6.9, 8.2.6.10, 8.2.7.1, 8.2.7.2, 8.2.7.3, 8.2.7.4, 8.2.7.5, 8.2.7.6, 8.2.7.7, 8.2.7.8, 8.2.7.9, 8.2.7.10, 8.2.8.1, 8.2.8.2, 8.2.8.3, 8.2.8.4, 8.2.8.5, 8.2.8.6, 8.2.8.7, 8.2.8.8, 8.2.8.9, 8.2.8.10, 8.2.9.1, 8.2.9.2, 8.2.9.3, 8.2.9.4, 8.2.9.5, 8.2.9.6, 8.2.9.7, 8.2.9.8, 8.2.9.9, 8.2.9.10, 8.2.10.1, 8.2.10.2, 8.2.10.3, 8.2.10.4, 8.2.10.5, 8.2.10.6, 8.2.10.7, 8.2.10.8, 8.2.10.9, 8.2.10.10, 8.3.1.1, 8.3.1.2, 8.3.1.3, 8.3.1.4, 8.3.1.5, 8.3.1.6, 8.3.1.7, 8.3.1.8, 8.3.1.9, 8.3.1.10, 8.3.2.1, 8.3.2.2, 8.3.2.3, 8.3.2.4, 8.3.2.5, 8.3.2.6, 8.3.2.7, 8.3.2.8, 8.3.2.9, 8.3.2.10, 8.3.3.1, 8.3.3.2, 8.3.3.3, 8.3.3.4, 8.3.3.5, 8.3.3.6, 8.3.3.7, 8.3.3.8, 8.3.3.9, 8.3.3.10, 8.3.4.1, 8.3.4.2, 8.3.4.3, 8.3.4.4, 8.3.4.5, 8.3.4.6, 8.3.4.7, 8.3.4.8, 8.3.4.9, 8.3.4.10, 8.3.5.1, 8.3.5.2, 8.3.5.3, 8.3.5.4, 8.3.5.5, 8.3.5.6, 8.3.5.7, 8.3.5.8, 8.3.5.9, 8.3.5.10, 8.3.6.1, 8.3.6.2, 8.3.6.3, 8.3.6.4, 8.3.6.5, 8.3.6.6, 8.3.6.7, 8.3.6.8, 8.3.6.9, 8.3.6.10, 8.3.7.1, 8.3.7.2, 8.3.7.3, 8.3.7.4, 8.3.7.5, 8.3.7.6, 8.3.7.7, 8.3.7.8, 8.3.7.9, 8.3.7.10, 8.3.8.1, 8.3.8.2, 8.3.8.3, 8.3.8.4, 8.3.8.5, 8.3.8.6, 8.3.8.7, 8.3.8.8, 8.3.8.9, 8.3.8.10, 8.3.9.1, 8.3.9.2, 8.3.9.3, 8.3.9.4, 8.3.9.5, 8.3.9.6, 8.3.9.7, 8.3.9.8, 8.3.9.9, |
| 8.3.9.10, 8.3.10.1, 8.3.10.2, 8.3.10.3, 8.3.10.4, 8.3.10.5, 8.3.10.6, 8.3.10.7, 8.3.10.8, 8.3.10.9, 8.3.10.10, 8.4.1.1, 8.4.1.2, 8.4.1.3, 8.4.1.4, 8.4.1.5, 8.4.1.6, 8.4.1.7, 8.4.1.8, 8.4.1.9, 8.4.1.10, 8.4.2.1, 8.4.2.2, 8.4.2.3, 8.4.2.4, 8.4.2.5, 8.4.2.6, 8.4.2.7, 8.4.2.8, 8.4.2.9, 8.4.2.10, 8.4.3.1, 8.4.3.2, 8.4.3.3, 8.4.3.4, 8.4.3.5, 8.4.3.6, 8.4.3.7, 8.4.3.8, 8.4.3.9, 8.4.3.10, 8.4.4.1, 8.4.4.2, 8.4.4.3, 8.4.4.4, 8.4.4.5, 8.4.4.6, 8.4.4.7, 8.4.4.8, 8.4.4.9, 8.4.4.10, 8.4.5.1, 8.4.5.2, 8.4.5.3, 8.4.5.4, 8.4.5.5, 8.4.5.6, 8.4.5.7, 8.4.5.8, 8.4.5.9, 8.4.5.10, 8.4.6.1, 8.4.6.2, 8.4.6.3, 8.4.6.4, 8.4.6.5, 8.4.6.6, 8.4.6.7, 8.4.6.8, 8.4.6.9, 8.4.6.10, 8.4.7.1, 8.4.7.2, 8.4.7.3, 8.4.7.4, 8.4.7.5, 8.4.7.6, 8.4.7.7, 8.4.7.8, 8.4.7.9, 8.4.7.10, 8.4.8.1, 8.4.8.2, 8.4.8.3, 8.4.8.4, 8.4.8.5, 8.4.8.6, 8.4.8.7, 8.4.8.8, 8.4.8.9, 8.4.8.10, 8.4.9.1, 8.4.9.2, 8.4.9.3, 8.4.9.4, 8.4.9.5, 8.4.9.6, 8.4.9.7, 8.4.9.8, 8.4.9.9, 8.4.9.10, 8.4.10.1, 8.4.10.2, 8.4.10.3, 8.4.10.4, 8.4.10.5, 8.4.10.6, 8.4.10.7, 8.4.10.8, 8.4.10.9, 8.4.10.10, 8.5.1.1, 8.5.1.2, 8.5.1.3, 8.5.1.4, 8.5.1.5, 8.5.1.6, 8.5.1.7, 8.5.1.8, 8.5.1.9, 8.5.1.10, 8.5.2.1, 8.5.2.2, 8.5.2.3, 8.5.2.4, 8.5.2.5, 8.5.2.6, 8.5.2.7, 8.5.2.8, 8.5.2.9, 8.5.2.10, 8.5.3.1, 8.5.3.2, 8.5.3.3, 8.5.3.4, 8.5.3.5, 8.5.3.6, 8.5.3.7, 8.5.3.8, 8.5.3.9, 8.5.3.10, 8.5.4.1, 8.5.4.2, 8.5.4.3, 8.5.4.4, 8.5.4.5, 8.5.4.6, 8.5.4.7, 8.5.4.8, 8.5.4.9, 8.5.4.10, 8.5.5.1, 8.5.5.2, 8.5,5.3, 8.5.5.4, 8.5.5.5, 8.5.5.6, 8.5.5.7, 8.5.5.8, 8.5.5.9, 8.5.5.10, 8.5.6.1, 8.5.6.2, 8.5.6.3, 8.5.6.4, 8.5.6.5, 8.5.6.6, 8.5.6.7, 8.5.6.8, 8.5.6.9, 8.5.6.10, 8.5.7.1, 8.5.7.2, 8.5.7.3, 8.5.7.4, 8.5.7.5, 8.5.7.6, 8.5.7.7, 8.5.7.8, 8.5.7.9, 8.5.7.10, 8.5.8.1, 8.5.8.2, 8.5.8.3, 8.5.8.4, 8.5.8.5, 8.5.8.6, 8.5.8.7, 8.5.8.8, 8.5.8.9, 8.5.8.10, 8.5.9.1, 8.5.9.2, 8.5.9.3, 8.5.9.4, 8.5.9.5, 8.5.9.6, 8.5.9.7, 8.5.9.8, 8.5.9.9, 8.5.9.10, 8.5.10.1, 8.5.10.2, 8.5.10.3, 8.5.10.4, 8.5.10.5, 8.5.10.6, 8.5.10.7, 8.5.10.8, 8.5.10.9, 8.5.10.10, 8.6.1.1, 8.6.1.2, 8.6.1.3, 8.6.1.4, 8.6.1.5, 8.6.1.6, 8.6.1.7, 8.6.1.8, 8.6.1.9, 8.6.1.10, 8.6.2.1, 8.6.2.2, 8.6.2.3, 8.6.2.4, 8.6.2.5, 8.6.2.6, 8.6.2.7, 8.6.2.8, 8.6.2.9, 8.6.2.10, 8.6.3.1, 8.6.3.2, 8.6.3.3, 8.6.3.4, 8.6.3.5, 8.6.3.6, 8.6.3.7, 8.6.3.8, 8.6.3.9, 8.6.3.10, 8.6.4.1, 8.6.4.2, 8.6.4.3, 8.6.4.4, 8.6.4.5, 8.6.4.6, 8.6.4.7, 8.6.4.8, 8.6.4.9, 8.6.4.10, 8.6.5.1, 8.6.5.2, 8.6.5.3, 8.6.5.4, 8.6.5.5, 8.6.5.6, 8.6.5.7, 8.6.5.8, 8.6.5.9, 8.6.5.10, 8.6.6.1, 8.6.6.2, 8.6.6.3, 8.6.6.4, 8.6.6.5, 8.6.6.6, 8.6.6.7, 8.6.6.8, 8.6.6.9, 8.6.6.10, 8.6.7.1, 8.6.7.2, 8.6.7.3, 8.6.7.4, 8.6.7.5, 8.6.7.6, 8.6.7.7, 8.6.7.8, 8.6.7.9, 8.6.7.10, 8.6.8.1, 8.6.8.2, 8.6.8.3, 8.6.8.4, 8.6.8.5, 8.6.8.6, 8.6.8.7, 8.6.8.8, 8.6.8.9, 8.6.8.10, 8.6.9.1, 8.6.9.2, 8.6.9.3, 8.6.9.4, 8.6.9.5, 8.6.9.6, 8.6.9.7, 8.6.9.8, 8.6.9.9, 8.6.9.10, 8.6.10.1, 8.6.10.2, 8.6.10.3, 8.6.10.4, 8.6.10.5, 8.6.10.6, 8.6.10.7, 8.6.10.8, 8.6.10.9, 8.6.10.10, 8.7.1.1, 8.7.1.2, 8.7.1.3, 8.7.1.4, 8.7.1.5, 8.7.1.6, 8.7.1.7, 8.7.1.8, 8.7.1.9, 8.7.1.10, 8.7.2.1, 8.7.2.2, 8.7.2.3, 8.7.2.4, 8.7.2.5, 8.7.2.6, 8.7.2.7, 8.7.2.8, 8.7.2.9, 8.7.2.10, 8.7.3.1, 8.7.3.2, 8.7.3.3, 8.7.3.4, 8.7.3.5, 8.7.3.6, 8.7.3.7, 8.7.3.8, 8.7.3.9, 8.7.3.10, 8.7.4.1, 8.7.4.2, 8.7.4.3, 8.7.4.4, 8.7.4.5, 8.7.4.6, 8.7.4.7, 8.7.4.8, 8.7.4.9, 8.7.4.10, 8.7.5.1, 8.7.5.2, 8.7.5.3, 8.7.5.4, 8.7.5.5, 8.7.5.6, 8.7.5.7, 8.7.5.8, 8.7.5.9, 8.7.5.10, 8.7.6.1, 8.7.6.2, 8.7.6.3, 8.7.6.4, 8.7.6.5, 8.7.6.6, 8.7.6.7, 8.7.6.8, 8.7.6.9, 8.7.6.10, 8.7.7.1, 8.7.7.2, 8.7.7.3, 8.7.7.4, 8.7.7.5, 8.7.7.6, 8.7.7.7, 8.7.7.8, 8.7.7.9, 8.7.7.10, 8.7.8.1, 8.7.8.2, 8.7.8.3, 8.7.8.4, 8.7.8.5, 8.7.8.6, 8.7.8.7, 8.7.8.8, 8.7.8.9, 8.7.8.10, 8.7.9.1, 8.7.9.2, 8.7.9.3, 8.7.9.4, 8.7.9.5, 8.7.9.6, 8.7.9.7, 8.7.9.8, 8.7.9.9, 8.7.9.10, 8.7.10.1, 8.7.10.2, 8.7.10.3, 8.7.10.4, 8.7.10.5, 8.7.10.6, 8.7.10.7, 8.7.10.8, 8.7.10.9, 8.7.10.10, 8.8.1.1, 8.8.1.2, 8.8.1.3, 8.8.1.4, 8.8.1.5, 8.8.1.6, 8.8.1.7, 8.8.1.8, 8.8.1.9, 8.8.1.10, 8.8.2.1, 8.8.2.2, 8.8.2.3, 8.8.2.4, 8.8.2.5, 8.8.2.6, 8.8.2.7, 8.8.2.8, 8.8.2.9, 8.8.2.10, 8.8.3.1, |
| 8.8.3.2, 8.8.3.3, 8.8.3.4, 8.8.3.5, 8.8.3.6, 8.8.3.7, 8.8.3.8, 8.8.3.9, 8.8.3.10, 8.8.4.1, 8.8.4.2, 8.8.4.3, 8.8.4.4, 8.8.4.5, 8.8.4.6, 8.8.4.7, 8.8.4.8, 8.8.4.9, 8.8.4.10, 8.8.5.1, 8.8.5.2, 8.8.5.3, 8.8.5.4, 8.8.5.5, 8.8.5.6, 8.8.5.7, 8.8.5.8, 8.8.5.9, 8.8.5.10, 8.8.6.1, 8.8.6.2, 8.8.6.3, 8.8.6.4, 8.8.6.5, 8.8.6.6, 8.8.6.7, 8.8.6.8, 8.8.6.9, 8.8.6.10, 8.8.7.1, 8.8.7.2, 8.8.7.3, 8.8.7.4, 8.8.7.5, 8.8.7.6, 8.8.7.7, 8.8.7.8, 8.8.7.9, 8.8.7.10, 8.8.8.1, 8.8.8.2, 8.8.8.3, 8.8.8.4, 8.8.8.5, 8.8.8.6, 8.8.8.7, 8.8.8.8, 8.8.8.9, 8.8.8.10, 8.8.9.1, 8.8.9.2, 8.8.9.3, 8.8.9.4, 8.8.9.5, 8.8.9.6, 8.8.9.7, 8.8.9.8, 8.8.9.9, 8.8.9.10, 8.8.10.1, 8.8.10.2, 8.8.10.3, 8.8.10.4, 8.8.10.5, 8.8.10.6, 8.8.10.7, 8.8.10.8, 8.8.10.9, 8.8.10.10, 8.9.1.1, 8.9.1.2, 8.9.1.3, 8.9.1.4, 8.9.1.5, 8.9.1.6, 8.9.1.7, 8.9.1.8, 8.9.1.9, 8.9.1.10, 8.9.2.1, 8.9.2.2, 8.9.2.3, 8.9.2.4, 8.9.2.5, 8.9.2.6, 8.9.2.7, 8.9.2.8, 8.9.2.9, 8.9.2.10, 8.9.3.1, 8.9.3.2, 8.9.3.3, 8.9.3.4, 8.9.3.5, 8.9.3.6, 8.9.3.7, 8.9.3.8, 8.9.3.9, 8.9.3.10, 8.9.4.1, 8.9.4.2, 8.9.4.3, 8.9.4.4, 8.9.4.5, 8.9.4.6, 8.9.4.7, 8.9.4.8, 8.9.4.9, 8.9.4.10, 8.9.5.1, 8.9.5.2, 8.9.5.3, 8.9.5.4, 8.9.5.5, 8.9.5.6, 8.9.5.7, 8.9.5.8, 8.9.5.9, 8.9.5.10, 8.9.6.1, 8.9.6.2, 8.9.6.3, 8.9.6.4, 8.9.6.5, 8.9.6.6, 8.9.6.7, 8.9.6.8, 8.9.6.9, 8.9.6.10, 8.9.7.1, 8.9.7.2, 8.9.7.3, 8.9.7.4, 8.9.7.5, 8.9.7.6, 8.9.7.7, 8.9.7.8, 8.9.7.9, 8.9.7.10, 8.9.8.1, 8.9.8.2, 8.9.8.3, 8.9.8.4, 8.9.8.5, 8.9.8.6, 8.9.8.7, 8.9.8.8, 8.9.8.9, 8.9.8.10, 8.9.9.1, 8.9.9.2, 8.9.9.3, 8.9.9.4, 8.9.9.5, 8.9.9.6, 8.9.9.7, 8.9.9.8, 8.9.9.9, 8.9.9.10, 8.9.10.1, 8.9.10.2, 8.9.10.3, 8.9.10.4, 8.9.10.5, 8.9.10.6, 8.9.10.7, 8.9.10.8, 8.9.10.9, 8.9.10.10, 8.10.1.1, 8.10.1.2, 8.10.1.3, 8.10.1.4, 8.10.1.5, 8.10.1.6, 8.10.1.7, 8.10.1.8, 8.10.1.9, 8.10.1.10, 8.10.2.1, 8.10.2.2, 8.10.2.3, 8.10.2.4, 8.10.2.5, 8.10.2.6, 8.10.2.7, 8.10.2.8, 8.10.2.9, 8.10.2.10, 8.10.3.1, 8.10.3.2, 8.10.3.3, 8.10.3.4, 8.10.3.5, 8.10.3.6, 8.10.3.7, 8.10.3.8, 8.10.3.9, 8.10.3.10, 8.10.4.1, 8.10.4.2, 8.10.4.3, 8.10.4.4, 8.10.4.5, 8.10.4.6, 8.10.4.7, 8.10.4.8, 8.10.4.9, 8.10.4.10, 8.10.5.1, 8.10.5.2, 8.10.5.3, 8.10.5.4, 8.10.5.5, 8.10.5.6, 8.10.5.7, 8.10.5.8, 8.10.5.9, 8.10.5.10, 8.10.6.1, 8.10.6.2, 8.10.6.3, 8.10.6.4, 8.10.6.5, 8.10.6.6, 8.10.6.7, 8.10.6.8, 8.10.6.9, 8.10.6.10, 8.10.7.1, 8.10.7.2, 8.10.7.3, 8.10.7.4, 8.10.7.5, 8.10.7.6, 8.10.7.7, 8.10.7.8, 8.10.7.9, 8.10.7.10, 8.10.8.1, 8.10.8.2, 8.10.8.3, 8.10.8.4, 8.10.8.5, 8.10.8.6, 8.10.8.7, 8.10.8.8, 8.10.8.9, 8.10.8.10, 8.10.9.1, 8.10.9.2, 8.10.9.3, 8.10.9.4, 8.10.9.5, 8.10.9.6, 8.10.9.7, 8.10.9.8, 8.10.9.9, 8.10.9.10, 8.10.10.1, 8.10.10.2, 8.10.10.3, 8.10.10.4, 8.10.10.5, 8.10.10.6, 8.10.10.7, 8.10.10.8, 8.10.10.9, 8.10.10.10, 9.1.1.1, 9.1.1.2, 9.1.1.3, 9.1.1.4, 9.1.1.5, 9.1.1.6, 9.1.1.7, 9.1.1.8, 9.1.1.9, 9.1.1.10, 9.1.2.1, 9.1.2.2, 9.1.2.3, 9.1.2.4, 9.1.2.5, 9.1.2.6, 9.1.2.7, 9.1.2.8, 9.1.2.9, 9.1.2.10, 9.1.3.1, 9.1.3.2, 9.1.3.3, 9.1.3.4, 9.1.3.5, 9.1.3.6, 9.1.3.7, 9.1.3.8, 9.1.3.9, 9.1.3.10, 9.1.4.1, 9.1.4.2, 9.1.4.3, 9.1.4.4, 9.1.4.5, 9.1.4.6, 9.1.4.7, 9.1.4.8, 9.1.4.9, 9.1.4.10, 9.1.5.1, 9.1.5.2, 9.1.5.3, 9.1.5.4, 9.1.5.5, 9.1.5.6, 9.1.5.7, 9.1.5.8, 9.1.5.9, 9.1.5.10, 9.1.6.1, 9.1.6.2, 9.1.6.3, 9.1.6.4, 9.1.6.5, 9.1.6.6, 9.1.6.7, 9.1.6.8, 9.1.6.9, 9.1.6.10, 9.1.7.1, 9.1.7.2, 9.1.7.3, 9.1.7.4, 9.1.7.5, 9.1.7.6, 9.1.7.7, 9.1.7.8, 9.1.7.9, 9.1.7.10, 9.1.8.1, 9.1.8.2, 9.1.8.3, 9.1.8.4, 9.1.8.5, 9.1.8.6, 9.1.8.7, 9.1.8.8, 9.1.8.9, 9.1.8.10, 9.1.9.1, 9.1.9.2, 9.1.9.3, 9.1.9.4, 9.1.9.5, 9.1.9.6, 9.1.9.7, 9.1.9.8, 9.1.9.9, 9.1.9.10, 9.1.10.1, 9.1.10.2, 9.1.10.3, 9.1.10.4, 9.1.10.5, 9.1.10.6, 9.1.10.7, 9.1.10.8, 9.1.10.9, 9.1.10.10, 9.2.1.1, 9.2.1.2, 9.2.1.3, 9.2.1.4, 9.2.1.5, |
| 9.2.1.6, 9.2.1.7, 9.2.1.8, 9.2.1.9, 9.2.1.10, 9.2.2.1, 9.2.2.2, 9.2.2.3, 9.2.2.4, 9.2.2.5, 9.2.2.6, 9.2.2.7, 9.2.2.8, 9.2.2.9, 9.2.2.10, 9.2.3.1, 9.2.3.2, 9.2.3.3, 9.2.3.4, 9.2.3.5, 9.2.3.6, 9.2.3.7, 9.2.3.8, 9.2.3.9, 9.2.3.10, 9.2.4.1, 9.2.4.2, 9.2.4.3, 9.2.4.4, 9.2.4.5, 9.2.4.6, 9.2.4.7, 9.2.4.8, 9.2.4.9, 9.2.4.10, 9.2.5.1, 9.2.5.2, 9.2.5.3, 9.2.5.4, 9.2.5.5, 9.2.5.6, 9.2.5.7, 9.2.5.8, 9.2.5.9, 9.2.5.10, 9.2.6.1, 9.2.6.2, 9.2.6.3, 9.2.6.4, 9.2.6.5, 9.2.6.6, 9.2.6.7, 9.2.6.8, 9.2.6.9, 9.2.6.10, 9.2.7.1, 9.2.7.2, 9.2.7.3, 9.2.7.4, 9.2.7.5, 9.2.7.6, 9.2.7.7, 9.2.7.8, 9.2.7.9, 9.2.7.10, 9.2.8.1, 9.2.8.2, 9.2.8.3, 9.2.8.4, 9.2.8.5, 9.2.8.6, 9.2.8.7, 9.2.8.8, 9.2.8.9, 9.2.8.10, 9.2.9.1, 9.2.9.2, 9.2.9.3, 9.2.9.4, 9.2.9.5, 9.2.9.6, 9.2.9.7, 9.2.9.8, 9.2.9.9, 9.2.9.10, 9.2.10.1, 9.2.10.2, 9.2.10.3, 9.2.10.4, 9.2.10.5, 9.2.10.6, 9.2.10.7, 9.2.10.8, 9.2.10.9, 9.2.10.10, 9.3.1.1, 9.3.1.2, 9.3.1.3, 9.3.1.4, 9.3.1.5, 9.3.1.6, 9.3.1.7, 9.3.1.8, 9.3.1.9, 9.3.1.10, 9.3.2.1, 9.3.2.2, 9.3.2.3, 9.3.2.4, 9.3.2.5, 9.3.2.6, 9.3.2.7, 9.3.2.8, 9.3.2.9, 9.3.2.10, 9.3.3.1, 9.3.3.2, 9.3.3.3, 9.3.3.4, 9.3.3.5, 9.3.3.6, 9.3.3.7, 9.3.3.8, 9.3.3.9, 9.3.3.10, 9.3.4.1, 9.3.4.2, 9.3.4.3, 9.3.4.4, 9.3.4.5, 9.3.4.6, 9.3.4.7, 9.3.4.8, 9.3.4.9, 9.3.4.10, 9.3.5.1, 9.3.5.2, 9.3.5.3, 9.3.5.4, 9.3.5.5, 9.3.5.6, 9.3.5.7, 9.3.5.8, 9.3.5.9, 9.3.5.10, 9.3.6.1, 9.3.6.2, 9.3.6.3, 9.3.6.4, 9.3.6.5, 9.3.6.6, 9.3.6.7, 9.3.6.8, 9.3.6.9, 9.3.6.10, 9.3.7.1, 9.3.7.2, 9.3.7.3, 9.3.7.4, 9.3.7.5, 9.3.7.6, 9.3.7.7, 9.3.7.8, 9.3.7.9, 9.3.7.10, 9.3.8.1, 9.3.8.2, 9.3.8.3, 9.3.8.4, 9.3.8.5, 9.3.8.6, 9.3.8.7, 9.3.8.8, 9.3.8.9, 9.3.8.10, 9.3.9.1, 9.3.9.2, 9.3.9.3, 9.3.9.4, 9.3.9.5, 9.3.9.6, 9.3.9.7, 9.3.9.8, 9.3.9.9, 9.3.9.10, 9.3.10.1, 9.3.10.2, 9.3.10.3, 9.3.10.4, 9.3.10.5, 9.3.10.6, 9.3.10.7, 9.3.10.8, 9.3.10.9, 9.3.10.10, 9.4.1.1, 9.4.1.2, 9.4.1.3, 9.4.1.4, 9.4.1.5, 9.4.1.6, 9.4.1.7, 9.4.1.8, 9.4.1.9, 9.4.1.10, 9.4.2.1, 9.4.2.2, 9.4.2.3, 9.4.2.4, 9.4.2.5, 9.4.2.6, 9.4.2.7, 9.4.2.8, 9.4.2.9, 9.4.2.10, 9.4.3.1, 9.4.3.2, 9.4.3.3, 9.4.3.4, 9.4.3.5, 9.4.3.6, 9.4.3.7, 9.4.3.8, 9.4.3.9, 9.4.3.10, 9.4.4.1, 9.4.4.2, 9.4.4.3, 9.4.4.4, 9.4.4.5, 9.4.4.6, 9.4.4.7, 9.4.4.8, 9.4.4.9, 9.4.4.10, 9.4.5.1, 9.4.5.2, 9.4.5.3, 9.4.5.4, 9.4.5.5, 9.4.5.6, 9.4.5.7, 9.4.5.8, 9.4.5.9, 9.4.5.10, 9.4.6.1, 9.4.6.2, 9.4.6.3, 9.4.6.4, 9.4.6.5, 9.4.6.6, 9.4.6.7, 9.4.6.8, 9.4.6.9, 9.4.6.10, 9.4.7.1, 9.4.7.2, 9.4.7.3, 9.4.7.4, 9.4.7.5, 9.4.7.6, 9.4.7.7, 9.4.7.8, 9.4.7.9, 9.4.7.10, 9.4.8.1, 9.4.8.2, 9.4.8.3, 9.4.8.4, 9.4.8.5, 9.4.8.6, 9.4.8.7, 9.4.8.8, 9.4.8.9, 9.4.8.10, 9.4.9.1, 9.4.9.2, 9.4.9.3, 9.4.9.4, 9.4.9.5, 9.4.9.6, 9.4.9.7, 9.4.9.8, 9.4.9.9, 9.4.9.10, 9.4.10.1, 9.4.10.2, 9.4.10.3, 9.4.10.4, 9.4.10.5, 9.4.10.6, 9.4.10.7, 9.4.10.8, 9.4.10.9, 9.4.10.10, 9.5.1.1, 9.5.1.2, 9.5.1.3, 9.5.1.4, 9.5.1.5, 9.5.1.6, 9.5.1.7, 9.5.1.8, 9.5.1.9, 9.5.1.10, 9.5.2.1, 9.5.2.2, 9.5.2.3, 9.5.2.4, 9.5.2.5, 9.5.2.6, 9.5.2.7, 9.5.2.8, 9.5.2.9, 9.5.2.10, 9.5.3.1, 9.5.3.2, 9.5.3.3, 9.5.3.4, 9.5.3.5, 9.5.3.6, 9.5.3.7, 9.5.3.8, 9.5.3.9, 9.5.3.10, 9.5.4.1, 9.5.4.2, 9.5.4.3, 9.5.4.4, 9.5.4.5, 9.5.4.6, 9.5.4.7, 9.5.4.8, 9.5.4.9, 9.5.4.10, 9.5.5.1, 9.5.5.2, 9.5.5.3, 9.5.5.4, 9.5.5.5, 9.5.5.6, 9.5.5.7, 9.5.5.8, 9.5.5.9, 9.5.5.10, 9.5.6.1, 9.5.6.2, 9.5.6.3, 9.5.6.4, 9.5.6.5, 9.5.6.6, 9.5.6.7, 9.5.6.8, 9.5.6.9, 9.5.6.10, 9.5.7.1, 9.5.7.2, 9.5.7.3, 9.5.7.4, 9.5.7.5, 9.5.7.6, 9.5.7.7, 9.5.7.8, 9.5.7.9, 9.5.7.10, 9.5.8.1, 9.5.8.2, 9.5.8.3, 9.5.8.4, 9.5.8.5, 9.5.8.6, |
| 9.5.8.7, 9.5.8.8, 9.5.8.9, 9.5.8.10, 9.5.9.1, 9.5.9.2, 9.5.9.3, 9.5.9.4, 9.5.9.5, 9.5.9.6, 9.5.9.7, 9.5.9.8, 9.5.9.9, 9.5.9.10, 9.5.10.1, 9.5.10.2, 9.5.10.3, 9.5.10.4, 9. 5.10.5, 9.5.10.6, 9.5.10.7, 9.5.10.8, 9.5.10.9, 9.5.10.10, 9.6.1.1, 9.6.1.2, 9.6.1.3, 9.6.1.4, 9.6.1.5, 9.6.1.6, 9.6.1.7, 9.6.1.8, 9.6.1.9, 9.6.1.10, 9.6.2.1, 9.6.2.2, 9.6.2.3, 9.6.2.4, 9.6.2.5, 9.6.2.6, 9.6.2.7, 9.6.2.8, 9.6.2.9, 9.6.2.10, 9.6.3.1, 9.6.3.2, 9.6.3.3, 9.6.3.4, 9.6.3.5, 9.6.3.6, 9.6.3.7, 9.6.3.8, 9.6.3.9, 9.6.3.10, 9.6.4.1, 9.6.4.2, 9.6.4.3, 9.6.4.4, 9.6.4.5, 9.6.4.6, 9.6.4.7, 9.6.4.8, 9.6.4.9, 9.6.4.10, 9.6.5.1, 9.6.5.2, 9.6.5.3, 9.6.5.4, 9.6.5.5, 9.6.5.6, 9.6.5.7, 9.6.5.8, 9.6.5.9, 9.6.5.10, 9.6.6.1, 9.6.6.2, 9.6.6.3, 9.6.6.4, 9.6.6.5, 9.6.6.6, 9.6.6.7, 9.6.6.8, 9.6.6.9, 9.6.6.10, 9.6.7.1, 9.6.7.2, 9.6.7.3, 9.6.7.4, 9.6.7.5, 9.6.7.6, 9.6.7.7, 9.6.7.8, 9.6.7.9, 9.6.7.10, 9.6.8.1, 9.6.8.2, 9.6.8.3, 9.6.8.4, 9.6.8.5, 9.6.8.6, 9.6.8.7, 9.6.8.8, 9.6.8.9, 9.6.8.10, 9.6.9.1, 9.6.9.2, 9.6.9.3, 9.6.9.4, 9.6.9.5, 9.6.9.6, 9.6.9.7, 9.6.9.8, 9.6.9.9, 9.6.9.10, 9.6.10.1, 9.6.10.2, 9.6.10.3, 9.6.10.4, 9.6.10.5, 9.6.10.6, 9.6.10.7, 9.6.10.8, 9.6.10.9, 9.6.10.10, 9.7.1.1, 9.7.1.2, 9.7.1.3, 9.7.1.4, 9.7.1.5, 9.7.1.6, 9.7.1.7, 9.7.1.8, 9.7.1.9, 9.7.1.10, 9.7.2.1, 9.7.2.2, 9.7.2.3, 9.7.2.4, 9.7.2.5, 9.7.2.6, 9.7.2.7, 9.7.2.8, 9.7.2.9, 9.7.2.10, 9.7.3.1, 9.7.3.2, 9.7.3.3, 9.7.3.4, 9.7.3.5, 9.7.3.6, 9.7.3.7, 9.7.3.8, 9.7.3.9, 9.7.3.10, 9.7.4.1, 9.7.4.2, 9.7.4.3, 9.7.4.4, 9.7.4.5, 9.7.4.6, 9.7.4.7, 9.7.4.8, 9.7.4.9, 9.7.4.10, 9.7.5.1, 9.7.5.2, 9.7.5.3, 9.7.5.4, 9.7.5.5, 9.7.5.6, 9.7.5.7, 9.7.5.8, 9.7.5.9, 9.7.5.10, 9.7.6.1, 9.7.6.2, 9.7.6.3, 9.7.6.4, 9.7.6.5, 9.7.6.6, 9.7.6.7, 9.7.6.8, 9.7.6.9, 9.7.6.10, 9.7.7.1, 9.7.7.2, 9.7.7.3, 9.7.7.4, 9.7.7.5, 9.7.7.6, 9.7.7.7, 9.7.7.8, 9.7.7.9, 9.7.7.10, 9.7.8.1, 9.7.8.2, 9.7.8.3, 9.7.8.4, 9.7.8.5, 9.7.8.6, 9.7.8.7, 9.7.8.8, 9.7.8.9, 9.7.8.10, 9.7.9.1, 9.7.9.2, 9.7.9.3, 9.7.9.4, 9.7.9.5, 9.7.9.6, 9.7.9.7, 9.7.9.8, 9.7.9.9, 9.7.9.10, 9.7.10.1, 9.7.10.2, 9.7.10.3, 9.7.10.4, 9.7.10.5, 9.7.10.6, 9.7.10.7, 9.7.10.8, 9.7.10.9, 9.7.10.10, 9.8.1.1, 9.8.1.2, 9.8.1.3, 9.8.1.4, 9.8.1.5, 9.8.1.6, 9.8.1.7, 9.8.1.8, 9.8.1.9, 9.8.1.10, 9.8.2.1, 9.8.2.2, 9.8.2.3, 9.8.2.4, 9.8.2.5, 9.8.2.6, 9.8.2.7, 9.8.2.8, 9.8.2.9, 9.8.2.10, 9.8.3.1, 9.8.3.2, 9.8.3.3, 9.8.3.4, 9.8.3.5, 9.8.3.6, 9.8.3.7, 9.8.3.8, 9.8.3.9, 9.8.3.10, 9.8.4.1, 9.8.4.2, 9.8.4.3, 9.8.4.4, 9.8.4.5, 9.8.4.6, 9.8.4.7, 9.8.4.8, 9.8.4.9, 9.8.4.10, 9.8.5.1, 9.8.5.2, 9.8.5.3, 9.8.5.4, 9.8.5.5, 9.8.5.6, 9.8.5.7, 9.8.5.8, 9.8.5.9, 9.8.5.10, 9.8.6.1, 9.8.6.2, 9.8.6.3, 9.8.6.4, 9.8.6.5, 9.8.6.6, 9.8.6.7, 9.8.6.8, 9.8.6.9, 9.8.6.10, 9.8.7.1, 9.8.7.2, 9.8.7.3, 9.8.7.4, 9.8.7.5, 9.8.7.6, 9.8.7.7, 9.8.7.8, 9.8.7.9, 9.8.7.10, 9.8.8.1, 9.8.8.2, 9.8.8.3, 9.8.8.4, 9.8.8.5, 9.8.8.6, 9.8.8.7, 9.8.8.8, 9.8.8.9, 9.8.8.10, 9.8.9.1, 9.8.9.2, 9.8.9.3, 9.8.9.4, 9.8.9.5, 9.8.9.6, 9.8.9.7, 9.8.9.8, 9.8.9.9, 9.8.9.10, 9.8.10.1, 9.8.10.2, 9.8.10.3, 9.8.10.4, 9.8.10.5, 9.8.10.6, 9.8.10.7, 9.8.10.8, 9.8.10.9, 9.8.10.10, 9.9.1.1, 9.9.1.2, 9.9.1.3, 9.9.1.4, 9.9.1.5, 9.9.1.6, 9.9.1.7, 9.9.1.8, 9.9.1.9, 9.9.1.10, 9.9.2.1, 9.9.2.2, 9.9.2.3, 9.9.2.4, 9.9.2.5, 9.9.2.6, 9.9.2.7, 9.9.2.8, 9.9.2.9, 9.9.2.10, 9.9.3.1, 9.9.3.2, 9.9.3.3, 9.9.3.4, 9.9.3.5, 9.9.3.6, 9.9.3.7, 9.9.3.8, 9.9.3.9, 9.9.3.10, 9.9.4.1, 9.9.4.2, 9.9.4.3, 9.9.4.4, 9.9.4.5, 9.9.4.6, 9.9.4.7, 9.9.4.8, 9.9.4.9, 9.9.4.10, 9.9.5.1, 9.9.5.2, 9.9.5.3, 9.9.5.4, 9.9.5.5, 9.9.5.6, 9.9.5.7, 9.9.5.8, 9.9.5.9, 9.9.5.10, 9.9.6.1, 9.9.6.2, 9.9.6.3, 9.9.6.4, 9.9.6.5, 9.9.6.6, 9.9.6.7, 9.9.6.8, 9.9.6.9, 9.9.6.10, 9.9.7.1, 9.9.7.2, 9.9.7.3, 9.9.7.4, 9.9.7.5, 9.9.7.6, 9.9.7.7, 9.9.7.8, 9.9.7.9, 9.9.7.10, 9.9.8.1, 9.9.8.2, 9.9.8.3, 9.9.8.4, 9.9.8.5, 9.9.8.6, |
| 9.9.8.7, 9.9.8.8, 9.9.8.9, 9.9.8.10, 9.9.9.1, 9.9.9.2, 9.9.9.3, 9.9.9.4, 9.9.9.5, 9.9.9.6, 9.9.9.7, 9.9.9.8, 9.9.9.9, 9.9.9.10, 9.9.10.1, 9.9.10.2, 9.9.10.3, 9.9.10.4, 9.9.10.5, 9.9.10.6, 9.9.10.7, 9.9.10.8, 9.9.10.9, 9.9.10.10, 9.10.1.1, 9.10.1.2, 9.10.1.3, 9.10.1.4, 9.10.1.5, 9.10.1.6, 9.10.1.7, 9.10.1.8, 9.10.1.9, 9.10.1.10, 9.10.2.1, 9.10.2.2, 9.10.2.3, 9.10.2.4, 9.10.2.5, 9.10.2.6, 9.10.2.7, 9.10.2.8, 9.10.2.9, 9.10.2.10, 9.10.3.1, 9.10.3.2, 9.10.3.3, 9.10.3.4, 9.10.3.5, 9.10.3.6, 9.10.3.7, 9.10.3.8, 9.10.3.9, 9.10.3.10, 9.10.4.1, 9.10.4.2, 9.10.4.3, 9.10.4.4, 9.10.4.5, 9.10.4.6, 9.10.4.7, 9.10.4.8, 9.10.4.9, 9.10.4.10, 9.10.5.1, 9.10.5.2, 9.10.5.3, 9.10.5.4, 9.10.5.5, 9.10.5.6, 9.10.5.7, 9.10.5.8, 9.10.5.9, 9.10.5.10, 9.10.6.1, 9.10.6.2, 9.10.6.3, 9.10.6.4, 9.10.6.5, 9.10.6.6, 9.10.6.7, 9.10.6.8, 9.10.6.9, 9.10.6.10, 9.10.7.1, 9.10.7.2, 9.10.7.3, 9.10.7.4, 9.10.7.5, 9.10.7.6, 9.10.7.7, 9.10.7.8, 9.10.7.9, 9.10.7.10, 9.10.8.1, 9.10.8.2, 9.10.8.3, 9.10.8.4, 9.10.8.5, 9.10.8.6, 9.10.8.7, 9.10.8.8, 9.10.8.9, 9.10.8.10, 9.10.9.1, 9.10.9.2, 9.10.9.3, 9.10.9.4, 9.10.9.5, 9.10.9.6, 9.10.9.7, 9.10.9.8, 9.10.9.9, 9.10.9.10, 9.10.10.1, 9.10.10.2, 9.10.10.3, 9.10.10.4, 9.10.10.5, 9.10.10.6, 9.10.10.7, 9.10.10.8, 9.10.10.9, 9.10.10.10, 10.1.1.1, 10.1.1.2, 10.1.1.3, 10.1.1.4, 10.1.1.5, 10.1.1.6, 10.1.1.7, 10.1.1.8, 10.1.1.9, 10.1.1.10, 10.1.2.1, 10.1.2.2, 10.1.2.3, 10.1.2.4, 10.1.2.5, 10.1.2.6, 10.1.2.7, 10.1.2.8, 10.1.2.9, 10.1.2.10, 10.1.3.1, 10.1.3.2, 10.1.3.3, 10.1.3.4, 10.1.3.5, 10.1.3.6, 10.1.3.7, 10.1.3.8, 10.1.3.9, 10.1.3.10, 10.1.4.1, 10.1.4.2, 10.1.4.3, 10.1.4.4, 10.1.4.5, 10.1.4.6, 10.1.4.7, 10.1.4.8, 10.1.4.9, 10.1.4.10, 10.1.5.1, 10.1.5.2, 10.1.5.3, 10.1.5.4, 10.1.5.5, 10.1.5.6, 10.1.5.7, 10.1.5.8, 10.1.5.9, 10.1.5.10, 10.1.6.1, 10.1.6.2, 10.1.6.3, 10.1.6.4, 10.1.6.5, 10.1.6.6, 10.1.6.7, 10.1.6.8, 10.1.6.9, 10.1.6.10, 10.1.7.1, 10.1.7.2, 10.1.7.3, 10.1.7.4, 10.1.7.5, 10.1.7.6, 10.1.7.7, 10.1.7.8, 10.1.7.9, 10.1.7.10, 10.1.8.1, 10.1.8.2, 10.1.8.3, 10.1.8.4, 10.1.8.5, 10.1.8.6, 10.1.8.7, 10.1.8.8, 10.1.8.9, 10.1.8.10, 10.1.9.1, 10.1.9.2, 10.1.9.3, 10.1.9.4, 10.1.9.5, 10.1.9.6, 10.1.9.7, 10.1.9.8, 10.1.9.9, 10.1.9.10, 10.1.10.1, 10.1.10.2, 10.1.10.3, 10.1.10.4, 10.1.10.5, 10.1.10.6, 10.1.10.7, 10.1.10.8, 10.1.10.9, 10.1.10.10, 10.2.1.1, 10.2.1.2, 10.2.1.3, 10.2.1.4, 10.2.1.5, 10.2.1.6, 10.2.1.7, 10.2.1.8, 10.2.1.9, 10.2.1.10, 10.2.2.1, 10.2.2.2, 10.2.2.3, 10.2.2.4, 10.2.2.5, 10.2.2.6, 10.2.2.7, 10.2.2.8, 10.2.2.9, 10.2.2.10, 10.2.3.1, 10.2.3.2, 10.2.3.3, 10.2.3.4, 10.2.3.5, 10.2.3.6, 10.2.3.7, 10.2.3.8, 10.2.3.9, 10.2.3.10, 10.2.4.1, 10.2.4.2, 10.2.4.3, 10.2.4.4,10.2.4.5,10.2.4.6,10.2.4.7,10.2.4.8, 10.2.4.9, 10.2.4.10, 10.2.5.1, 10.2.5.2, 10.2.5.3,10.2.5.4, 10.2.5.5, 10.2.5.6, 10.2.5.7, 10.2.5.8, 10.2.5.9, 10.2.5.10, 10.2.6.1, 10.2.6.2, 10.2.6.3, 10.2.6.4, 10.2.6.5, 10.2.6.6, 10.2.6.7, 10.2.6.8, 10.2.6.9, 10.2.6.10, 10.2.7.1, 10.2.7.2, 10.2.7.3, 10.2.7.4, 10.2.7.5, 10.2.7.6, 10.2.7.7, 10.2.7.8, 10.2.7.9, 10.2.7.10, 10.2.8.1, 10.2.8.2, 10.2.8.3, 10.2.8.4, 10.2.8.5, 10.2.8.6, 10.2.8.7, 10.2.8.8, 10.2.8.9, 10.2.8.10, 10.2.9.1, 10.2.9.2, 10.2.9.3, 10.2.9.4, 10.2.9.5, 10.2.9.6, 10.2.9.7, 10.2.9.8, 10.2.9.9, 10.2.9.10, 10.2.10.1, 10.2.10.2, 10.2.10.3, 10.2.10.4, 10.2.10.5, 10.2.10.6, |
| 10.2.10.7, 10.2.10.8, 10.2.10.9, 10.2.10.10, 10.3.1.1, 10.3.1.2, 10.3.1.3, 10.3.1.4, 10.3.1.5, 10.3.1.6, 10.3.1.7, 10.3.1.8, 10.3.1.9, 10.3.1.10, 10.3.2.1, 10.3.2.2, 10.3.2.3, 10.3.2.4, 10.3.2.5, 10.3.2.6, 10.3.2.7, 10.3.2.8, 10.3.2.9, 10.3.2.10, 10.3.3.1, 10.3.3.2, 10.3.3.3, 10.3.3.4, 10.3.3.5, 10.3.3.6, 10.3.3.7, 10.3.3.8, 10.3.3.9, 10.3.3.10, 10.3.4.1, 10.3.4.2, 10.3.4.3, 10.3.4.4, 10.3.4.5, 10.3.4.6, 10.3.4.7, 10.3.4.8, 10.3.4.9, 10.3.4.10, 10.3.5.1, 10.3.5.2, 10.3.5.3, 10.3.5.4, 10.3.5.5, 10.3.5.6, 10.3.5.7, 10.3.5.8, 10.3.5.9, 10.3.5.10, 10.3.6.1, 10.3.6.2, 10.3.6.3, 10.3.6.4, 10.3.6.5, 10.3.6.6, 10.3.6.7, 10.3.6.8, 10.3.6.9, 10.3.6.10, 10.3.7.1, 10.3.7.2, 10.3.7.3, 10.3.7.4, 10.3.7.5, 10.3.7.6, 10.3.7.7, 10.3.7.8, 10.3.7.9, 10.3.7.10, 10.3.8.1, 10.3.8.2, 10.3.8.3, 10.3.8.4, 10.3.8.5, 10.3.8.6, 10.3.8.7, 10.3.8.8, 10.3.8.9, 10.3.8.10, 10.3.9.1, 10.3.9.2, 10.3.9.3, 10.3.9.4, 10.3.9.5, 10.3.9.6, 10.3.9.7, 10.3.9.8, 10.3.9.9, 10.3.9.10, 10.3.10.1, 10.3.10.2, 10.3.10.3, 10.3.10.4, 10.3.10.5, 10.3.10.6, 10.3.10.7, 10.3.10.8, 10.3.10.9, 10.3.10.10, 10.4.1.1, 10.4.1.2, 10.4.1.3, 10.4.1.4, 10.4.1.5, 10.4.1.6, 10.4.1.7, 10.4.1.8, 10.4.1.9, 10.4.1.10, 10.4.2.1, 10.4.2.2, 10.4.2.3, 10.4.2.4, 10.4.2.5, 10.4.2.6, 10.4.2.7, 10.4.2.8, 10.4.2.9, 10.4.2.10, 10.4.3.1, 10.4.3.2, 10.4.3.3, 10.4.3.4, 10.4.3.5, 10.4.3.6, 10.4.3.7, 10.4.3.8, 10.4.3.9, 10.4.3.10, 10.4.4.1, 10.4.4.2, 10.4.4.3, 10.4.4.4, 10.4.4.5, 10.4.4.6, 10.4.4.7, 10.4.4.8, 10.4.4.9, 10.4.4.10, 10.4.5.1, 10.4.5.2, 10.4.5.3, 10.4.5.4, 10.4.5.5, 10.4.5.6, 10.4.5.7, 10.4.5.8, 10.4.5.9, 10.4.5.10, 10.4.6.1, 10.4.6.2, 10.4.6.3, 10.4.6.4, 10.4.6.5, 10.4.6.6, 10.4.6.7, 10.4.6.8, 10.4.6.9, 10.4.6.10, 10.4.7.1, 10.4.7.2, 10.4.7.3, 10.4.7.4, 10.4.7.5, 10.4.7.6, 10.4.7.7, 10.4.7.8, 10.4.7.9, 10.4.7.10, 10.4.8.1, 10.4.8.2, 10.4.8.3, 10.4.8.4, 10.4.8.5, 10.4.8.6, 10.4.8.7, 10.4.8.8, 10.4.8.9, 10.4.8.10, 10.4.9.1, 10.4.9.2, 10.4.9.3, 10.4.9.4, 10.4.9.5, 10.4.9.6, 10.4.9.7, 10.4.9.8, 10.4.9.9, 10.4.9.10, 10.4.10.1, 10.4.10.2, 10.4.10.3, 10.4.10.4, 10.4.10.5, 10.4.10.6, 10.4.10.7, 10.4.10.8, 10.4.10.9, 10.4.10.10, 10.5.1.1, 10.5.1.2, 10.5.1.3, 10.5.1.4, 10.5.1.5, 10.5.1.6, 10.5.1.7, 10.5.1.8, 10.5.1.9, 10.5.1.10, 10.5.2.1, 10.5.2.2, 10.5.2.3, 10.5.2.4, 10.5.2.5, 10.5.2.6, 10.5.2.7, 10.5.2.8, 10.5.2.9, 10.5.2.10, 10.5.3.1, 10.5.3.2, 10.5.3.3, 10.5.3.4, 10.5.3.5, 10.5.3.6, 10.5.3.7, 10.5.3.8, 10.5.3.9, 10.5.3.10, 10.5.4.1, 10.5.4.2, 10.5.4.3, 10.5.4.4, 10.5.4.5, 10.5.4.6, 10.5.4.7, 10.5.4.8, 10.5.4.9, 10.5.4.10, 10.5.5.1, 10.5.5.2, 10.5.5.3, 10.5.5.4, 10.5.5.5, 10.5.5.6, 10.5.5.7, 10.5.5.8, 10.5.5.9, 10.5.5.10, 10.5.6.1, 10.5.6.2, 10.5.6.3, 10.5.6.4, 10.5.6.5, 10.5.6.6, 10.5.6.7, 10.5.6.8, 10.5.6.9, 10.5.6.10, 10.5.7.1, 10.5.7.2, 10.5.7.3, 10.5.7.4, 10.5.7.5, 10.5.7.6, 10.5.7.7, 10.5.7.8, 10.5.7.9, 10.5.7.10, 10.5.8.1, 10.5.8.2, 10.5.8.3, 10.5.8.4, 10.5.8.5, 10.5.8.6, 10.5.8.7, 10.5.8.8, 10.5.8.9, 10.5.8.10, 10.5.9.1, 10.5.9.2, 10.5.9.3, 10.5.9.4, 10.5.9.5, 10.5.9.6, 10.5.9.7, 10.5.9.8, 10.5.9.9, 10.5.9.10, 10.5.10.1, 10.5.10.2, 10.5.10.3, 10.5.10.4, 10.5.10.5, 10.5.10.6, 10.5.10.7, 10.5.10.8, 10.5.10.9, 10.5.10.10, 10.6.1.1, 10.6.1.2, 10.6.1.3, 10.6.1.4, 10.6.1.5, 10.6.1.6, 10.6.1.7, 10.6.1.8, 10.6.1.9, 10.6.1.10, 10.6.2.1, 10.6.2.2, 10.6.2.3, 10.6.2.4, 10.6.2.5, 10.6.2.6, 10.6.2.7, 10.6.2.8, 10.6.2.9, 10.6.2.10, 10.6.3.1, 10.6.3.2, 10.6.3.3, 10.6.3.4, 10.6.3.5, 10.6.3.6, 10.6.3.7, 10.6.3.8, 10.6.3.9, 10.6.3.10, 10.6.4.1, 10.6.4.2, 10.6.4.3, 10.6.4.4, 10.6.4.5, 10.6.4.6, 10.6.4.7, 10.6.4.8, 10.6.4.9, 10.6.4.10, 10.6.5.1, 10.6.5.2, 10.6.5.3, 10.6.5.4, |
| 10.6.5.5, 10.6.5.6, 10.6.5.7, 10.6.5.8, 10.6.5.9, 10.6.5.10, 10.6.6.1, 10.6.6.2, 10.6.6.3, 10.6.6.4, 10.6.6.5, 10.6.6.6, 10.6.6.7, 10.6.6.8, 10.6.6.9, 10.6.6.10, 10.6.7.1, 10.6.7.2, 10.6.7.3, 10.6.7.4, 10.6.7.5, 10.6.7.6, 10.6.7.7, 10.6.7.8, 10.6.7.9, 10.6.7.10, 10.6.8.1, 10.6.8.2, 10.6.8.3, 10.6.8.4, 10.6.8.5, 10.6.8.6, 10.6.8.7, 10.6.8.8, 10.6.8.9, 10.6.8.10, 10.6.9.1, 10.6.9.2, 10.6.9.3, 10.6.9.4, 10.6.9.5, 10.6.9.6, 10.6.9.7, 10.6.9.8, 10.6.9.9, 10.6.9.10, 10.6.10.1, 10.6.10.2, 10.6.10.3, 10.6.10.4, 10.6.10.5, 10.6.10.6, 10.6.10.7, 10.6.10.8, 10.6.10.9, 10.6.10.10, 10.7.1.1, 10.7.1.2, 10.7.1.3, 10.7.1.4, 10.7.1.5, 10.7.1.6, 10.7.1.7, 10.7.1.8, 10.7.1.9, 10.7.1.10, 10.7.2.1, 10.7.2.2, 10.7.2.3, 10.7.2.4, 10.7.2.5, 10.7.2.6, 10.7.2.7, 10.7.2.8, 10.7.2.9, 10.7.2.10, 10.7.3.1, 10.7.3.2, 10.7.3.3, 10.7.3.4, 10.7.3.5, 10.7.3.6, 10.7.3.7, 10.7.3.8, 10.7.3.9, 10.7.3. 10, 10.7.4.1, 10.7.4.2, 10.7.4.3, 10.7.4.4, 10.7.4.5, 10.7.4.6, 10.7.4.7, 10.7.4.8, 10.7.4.9, 10.7.4.10, 10.7.5.1, 10.7.5.2, 10.7.5.3, 10.7.5.4, 10.7.5.5, 10.7.5.6, 10.7.5.7, 10.7.5.8, 10.7.5.9, 10.7.5.10, 10.7.6.1, 10.7.6.2, 10.7.6.3, 10.7.6.4, 10.7.6.5, 10.7.6.6, 10.7.6.7, 10.7.6.8, 10.7.6.9, 10.7.6.10, 10.7.7.1, 10.7.7.2, 10.7.7.3, 10.7.7.4, 10.7.7.5, 10.7.7.6, 10.7.7.7, 10.7.7.8, 10.7.7.9, 10.7.7.10, 10.7.8.1, 10.7.8.2, 10.7.8.3, 10.7.8.4, 10.7.8.5, 10.7.8.6, 10.7.8.7, 10.7.8.8, 10.7.8.9, 10.7.8.10, 10.7.9.1, 10.7.9.2, 10.7.9.3, 10.7.9.4, 10.7.9.5, 10.7.9.6, 10.7.9.7, 10.7.9.8, 10.7.9.9, 10.7.9.10, 10.7.10.1, 10.7.10.2, 10.7.10.3, 10.7.10.4, 10.7.10.5, 10.7.10.6, 10.7.10.7, 10.7.10.8, 10.7.10.9, 10.7.10.10, 10.8.1.1, 10.8.1.2, 10.8.1.3, 10.8.1.4, 10.8.1.5, 10.8.1.6, 10.8.1.7, 10.8.1.8, 10.8.1.9, 10.8.1.10, 10.8.2.1, 10.8.2.2, 10.8.2.3, 10.8.2.4, 10.8.2.5, 10.8.2.6, 10.8.2.7, 10.8.2.8, 10.8.2.9, 10.8.2.10, 10.8.3.1, 10.8.3.2, 10.8.3.3, 10.8.3.4, 10.8.3.5, 10.8.3.6, 10.8.3.7, 10.8.3.8, 10.8.3.9, 10.8.3.10, 10.8.4.1, 10.8.4.2, 10.8.4.3, 10.8.4.4, 10.8.4.5, 10.8.4.6, 10.8.4.7, 10.8.4.8, 10.8.4.9, 10.8.4.10, 10.8.5.1, 10.8.5.2, 10.8.5.3, 10.8.5.4, 10.8.5.5, 10.8.5.6, 10.8.5.7, 10.8.5.8, 10.8.5.9, 10.8.5.10, 10.8.6.1, 10.8.6.2, 10.8.6.3, 10.8.6.4, 10.8.6.5, 10.8.6.6, 10.8.6.7, 10.8.6.8, 10.8.6.9, 10.8.6.10, 10.8.7.1, 10.8.7.2, 10.8.7.3, 10.8.7.4, 10.8.7.5, 10.8.7.6, 10.8.7.7, 10.8.7.8, 10.8.7.9, 10.8.7.10, 10.8.8.1, 10.8.8.2, 10.8.8.3, 10.8.8.4, 10.8.8.5, 10.8.8.6, 10.8.8.7, 10.8.8.8, 10.8.8.9, 10.8.8.10, 10.8.9.1, 10.8.9.2, 10.8.9.3, 10.8.9.4, 10.8.9.5, 10.8.9.6, 10.8.9.7, 10.8.9.8, 10.8.9.9, 10.8.9.10, 10.8.10.1, 10.8.10.2, 10.8.10.3, 10.8.10.4, 10.8.10.5, 10.8.10.6, 10.8.10.7, 10.8.10.8, 10.8.10.9, 10.8.10.10, 10.9.1.1, 10.9.1.2, 10.9.1.3, 10.9.1.4, 10.9.1.5, 10.9.1.6, 10.9.1.7, 10.9.1.8, 10.9.1.9, 10.9.1.10, 10.9.2.1, 10.9.2.2, 10.9.2.3, 10.9.2.4, 10.9.2.5, 10.9.2.6, 10.9.2.7, 10.9.2.8, 10.9.2.9, 10.9.2.10, 10.9.3.1, 10.9.3.2, 10.9.3.3, 10.9.3.4, 10.9.3.5, 10.9.3.6, 10.9.3.7, 10.9.3.8, 10.9.3.9, 10.9.3.10, 10.9.4.1, 10.9.4.2, 10.9.4.3, 10.9.4.4, 10.9.4.5, 10.9.4.6, 10.9.4.7, 10.9.4.8, 10.9.4.9, 10.9.4.10, 10.9.5.1, 10.9.5.2, 10.9.5.3, 10.9.5.4, 10.9.5.5, 10.9.5.6, 10.9.5.7, 10.9.5.8, 10.9.5.9, 10.9.5.10, 10.9.6.1, 10.9.6.2, 10.9.6.3, |
| 10.9.6.4, 10.9.6.5, 10.9.6.6, 10.9.6.7, 10.9.6.8, 10.9.6.9, 10.9.6.10, 10.9.7.1, 10.9.7.2, 10.9.7.3, 10.9.7.4, 10.9.7.5, 10.9.7.6, 10.9.7.7, 10.9.7.8, 10.9.7.9, 10.9.7.10, 10.9.8.1, 10.9.8.2, 10.9.8.3, 10.9.8.4, 10.9.8.5, 10.9.8.6, 10.9.8.7, 10.9.8.8, 10.9.8.9, 10.9.8.10, 10.9.9.1, 10.9.9.2, 10.9.9.3, 10.9.9.4, 10.9.9.5, 10.9.9.6, 10.9.9.7, 10.9.9.8, 10.9.9.9, 10.9.9.10, 10.9.10.1, 10.9.10.2, 10.9.10.3, 10.9.10.4, 10.9.10.5, 10.9.10.6, 10.9.10.7, 10.9.10.8, 10.9.10.9, 10.9.10.10, 10.10.1.1, 10.10.1.2, 10.10.1.3, 10.10.1.4, 10.10.1.5, 10.10.1.6, 10.10.1.7, 10.10.1.8, 10.10.1.9, 10.10.1.10, 10.10.2.1, 10.10.2.2, 10.10.2.3, 10.10.2.4, 10.10.2.5, 10.10.2.6, 10.10.2.7, 10.10.2.8, 10.10.2.9, 10.10.2.10, 10.10.3.1, 10.10.3.2, 10.10.3.3, 10.10.3.4, 10.10.3.5, 10.10.3.6, 10.10.3.7, 10.10.3.8, 10.10.3.9, 10.10.3.10, 10.10.4.1, 10.10.4.2, 10.10.4.3, 10.10.4.4, 10.10.4.5, 10.10.4.6, 10.10.4.7, 10.10.4.8, 10.10.4.9, 10.10.4.10, 10.10.5.1, 10.10.5.2, 10.10.5.3, 10.10.5.4, 10.10.5.5, 10.10.5.6, 10.10.5.7, 10.10.5.8, 10.10.5.9, 10.10.5.10, 10.10.6.1, 10.10.6.2, 10.10.6.3, 10.10.6.4, 10.10.6.5, 10.10.6.6, 10.10.6.7, 10.10.6.8, 10.10.6.9, 10.10.6.10, 10.10.7.1, 10.10.7.2, 10.10.7.3, 10.10.7.4, 10.10.7.5, 10.10.7.6, 10.10.7.7, 10.10.7.8, 10.10.7.9, 10.10.7.10, 10.10.8.1, 10.10.8.2, 10.10.8.3, 10.10.8.4, 10.10.8.5, 10.10.8.6, 10.10.8.7, 10.10.8.8, 10.10.8.9, 10.10.8.10, 10.10.9.1, 10.10.9.2, 10.10.9.3, 10.10.9.4, 10.10.9.5, 10.10.9.6, 10.10.9.7, 10.10.9.8, 10.10.9.9, 10.10.9.10, 10.10.10.1, 10.10.10.2, 10.10.10.3, 10.10.10.4, 10.10.10.5, 10.10.10.6, 10.10.10.7, 10.10.10.8, 10.10.10.9, 10.10.10.10 |

Additional exemplary formula B compound groups include the following compound groups disclosed below. Unless otherwise specified, the configurations of all hydrogen atoms and variable or "R" groups for the following compound groups are as defined for the group 1 compounds of formula B above.

**Group 2.** This group comprises compounds named in Table B having R¹, R², R³ and R⁴ substituents that are defined in Table A wherein the R¹, R², R³ and R⁴ substituents are bonded to the steroid nucleus as described for group 1 compounds, except that a double bond at the 5-6 position is present. Thus, group 2 compound 1.3.1.1 has the structure

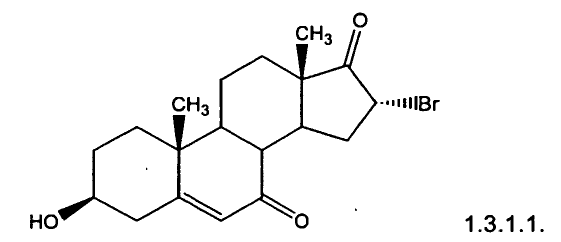

**Group 3.** This group comprises compounds named in Table B having R¹, R², R³ and R⁴ substituents defined in Table A wherein the R¹, R², R³ and R⁴ substituents are bonded to the steroid nucleus as described for group 1 compounds, except that double bonds at the 1-2- and 5-6 positions are present. Thus, group 3 compound 2.2.5.1 has the structure

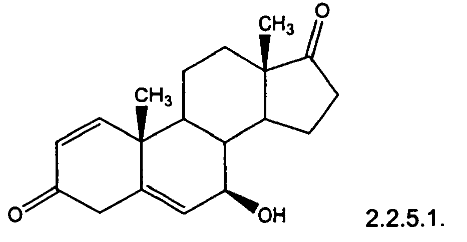

**Group 4.** This group comprises compounds named in Table B having R¹, R², R³ and R⁴ substituents defined in Table A wherein the R¹, R², R³ and R⁴ substituents are bonded to the steroid nucleus described for group 1 compounds, except that a double bond at the 1-2 position is present. Thus, group 4 compound 5.2.7.8 has the structure

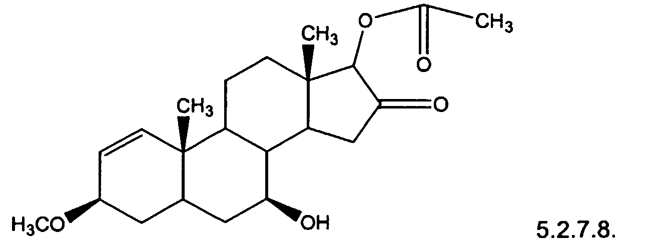

**Group 5.** This group comprises compounds named in Table B having R¹, R², R³ and R⁴ substituents defined in Table A wherein the R¹, R², R³ and R⁴ substituents are bonded to the steroid nucleus described for group 1 compounds, except that a double bond at the 4-5 position is present.

**Group 6.** This group comprises compounds named in Table B having R¹, R², R³ and R⁴ substituents defined in Table A wherein the R¹, R², R³ and R⁴ substituents are bonded to the steroid nucleus described for group 1 compounds, except that double bonds at both the 1-2 and 4-5 positions are present.

**Groups 7-1 through 7-6.** These groups comprise the 6 compound groups described above, except that R⁵ is hydrogen instead of methyl. Thus, group 7-1 has the same steroid nucleus as group 1 above, i.e., no double bond is present, but R⁵ is -H. Group 7-2 comprises the same steroid nucleus as group 2 above, i.e., a double bond is present at the 5-6-position, but R⁵ is -H. Compound groups 7-3 through 7-6 are assigned a steroid nucleus in the same manner. Thus, the group 7-1 through group 7-6 compounds named 1.2.1.9 have the structures 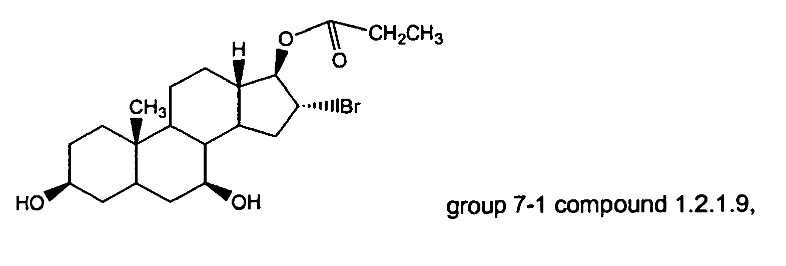 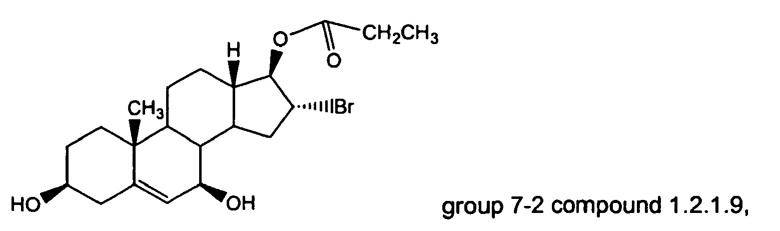 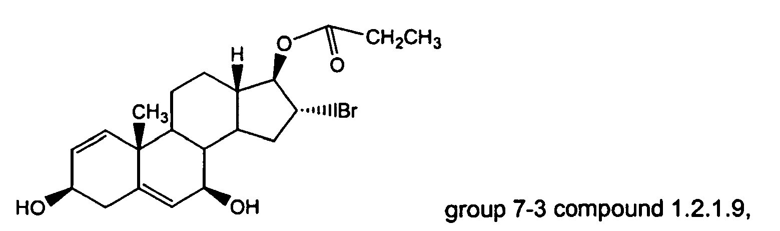 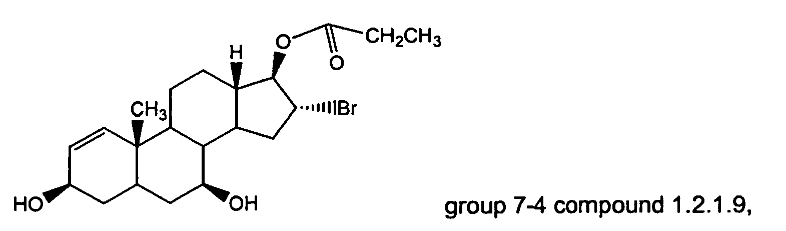 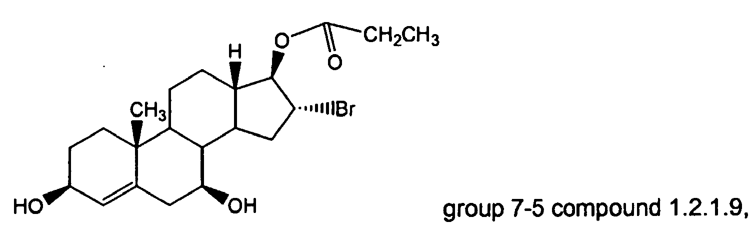 and

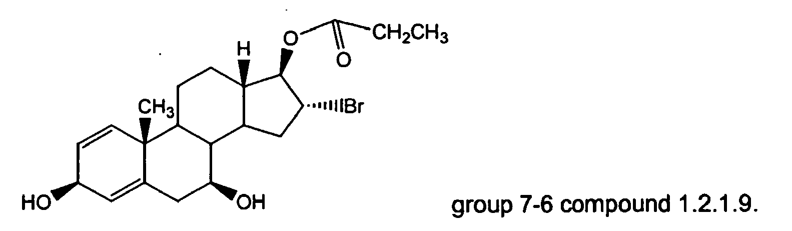

**Groups 8-1 through 8-6.** These groups comprise each compound named in groups 1-6, except that R⁵ of formula B is -CH₂OH instead of methyl. The groups 8-1 through group 8-6 compounds have structures that are named in the same manner as group 1-6 compounds, except that -CH₂OH instead of methyl is present at R⁵. These groups are named in the same manner as groups 7-1 through 7-6. Thus, group 8-1 and group 8-2 compounds named 1.2.1.9 have the structures 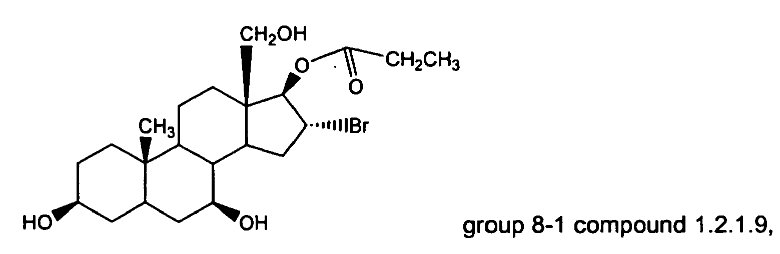 and

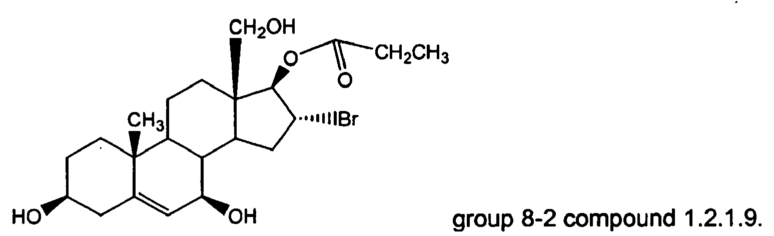

**Groups 9-1 through 9-6.** These groups comprise each compound named in compound groups 1-6, except that R⁶ of formula B is hydrogen instead of methyl. The groups 9-1 through group 9-6 compounds have structures that are named in the same manner as group 7-1 through 7-6 compounds, except that -H instead of methyl is present at R⁶. Thus, group 9-1 and group 9-2 compounds named 1.2.1.9 have the structures 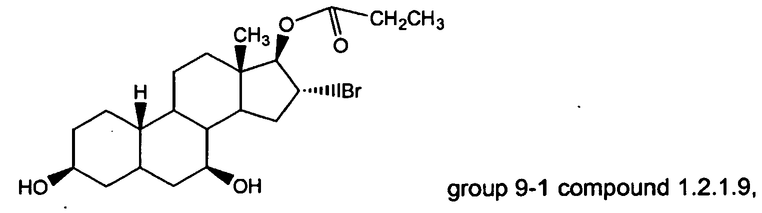 and

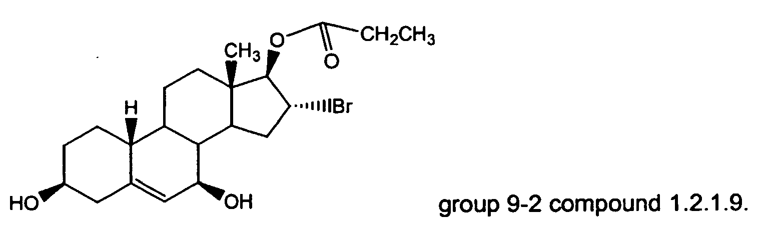

**Groups 10-1 through 10-6.** These groups comprise each compound named in compound groups 1-6 where R⁶ of formula 1 is -CH₂OH instead of methyl. The groups 10-1 through group 10-6 compounds have structures that are named in the same manner as group 7-1 through 7-6 compounds, except that -CH₂OH instead of methyl is present at R⁶. Thus, group 10-1 and group 10-2 compounds named 1.2.1.9 have the structures 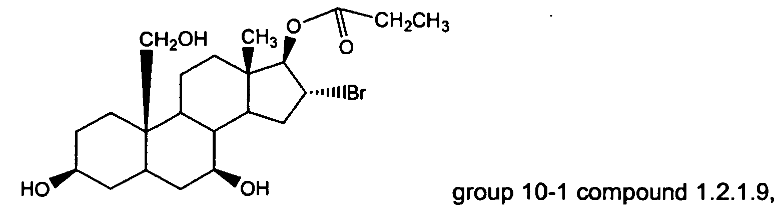 and

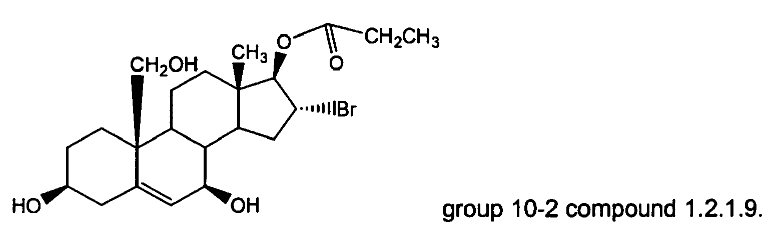

**Groups 11-1 through 11-10-6.** These groups comprise each compound named in compound groups 1 through 10-6 where R¹ substituents 1-10 listed in Table A are replaced with the following substituents:
1 -O-C(O)-CH₂CH₂CH₂CH₃ (-O-C(O)-CH₂CH₂CH₂CH₃ replaces -OH, which is R¹ substituent 1 in Table A)
2 -O-C(O)-CH₂CH₂CH₂CH₂CH₂CH₃
3 -O-C(O)-CH₂CH₂OCH₂CH₃
4 -O-C(O)-CH₂CH₂OCH₂CH₂OCH₂CH₃
5 -O-C(O)-CH₂CH₂CH₂CH₂OCH₂CH₃
6 -O-C(O)-CH₂CH₂OCH₂CH₂CH₂CH₃
7 -O-C₆H₄Cl
8 -O-C₆H₃F₂
9 -O-C₆H₄-O(CH₂)₂-O-CH₂CH₃
10 -O-C₆H₄-C(O)O(CH₂)₀₋₉CH₃

The group 11-1 through group 11-6 compounds have structures that are named in the same manner as group 7-1 through 7-6 compounds, except that substituents 1-10 of table A are replaced by the substituents 1-10 at R¹ listed above. Thus group 11-1 and 11-2 compounds named 1.2.1.9 have the structures

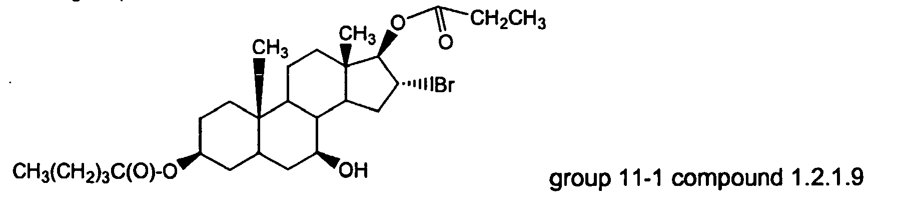

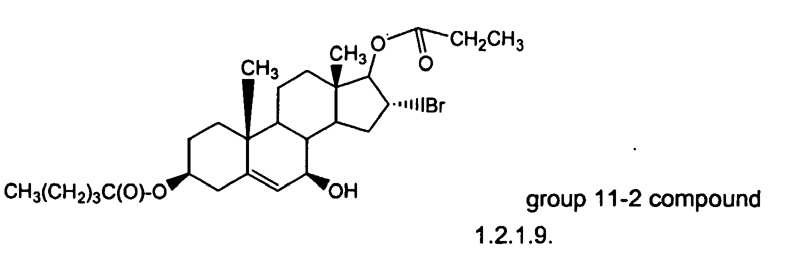

Group 11-7-1 and 11-7-2 compounds named 1.2.1.9 have the structures

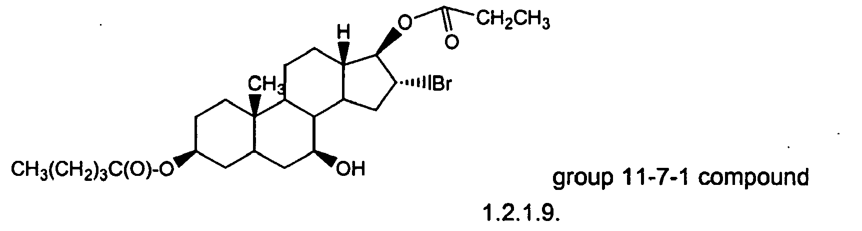

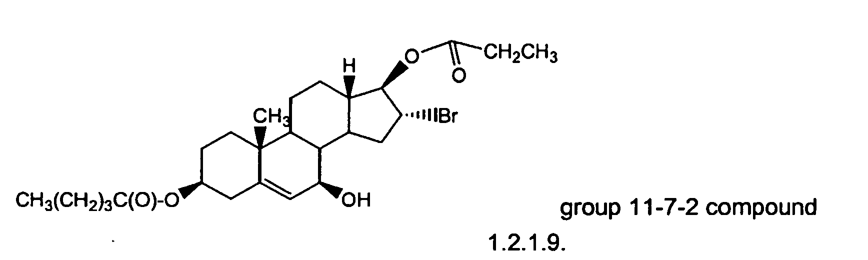

Group 11-8-1 and 11-8-2 compounds named 1.2.1.9 have the structures

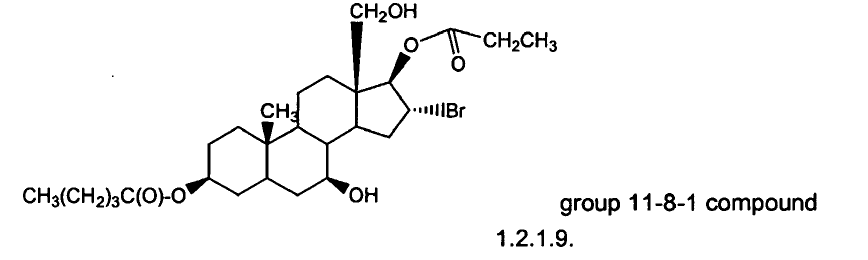

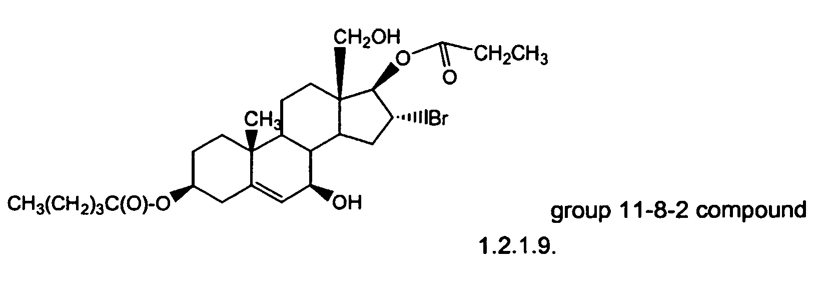

**Groups 12-1 through 12-10-6.** These groups comprise each compound named in groups 1 through 10-6 where R¹ substituents 1-10 listed in Table A are replaced with the following groups:
1 -O-P(O)(O)-OCH₂CH(CH₃)CH₃ (-O-P(O)(O)-OCH₂CH(CH₃)CH₃ replaces -OH, which is R¹ substituent 1 in Table A)
2 -O-P(O)(O)-OCH₂CH₂CH₂CH₂CH₃
3 -O-P(O)(O)-OCH₂CH₂CH₂CH₂CH₂CH₃
4 -O-P(O)(O)-OCH₂CH₂CH(CH₂CH₂)CH₃
5 -O-CH₂CH₂CH₂CH₂CH₂CH₃
6 -O-C₂H₅
7 -O-CH₂CH₂CH₃
8 -O-CH₂CH₂CH₂CH₃
9 -O-CH(CH₃)CHCH₃
10 -O-C(CH₃)₃

**Groups 13-1 through 13-10-6.** These groups comprise each compound named in groups 1 through 10-6 where R¹ substituents 1-10 listed in Table A are replaced with the following groups:
1 -O-(CH₂)₄CH₃ (-O-(CH₂)₄CH₃ replaces -OH, which is R¹ substituent 1 in Table A)
2 -O-C(O)-NH₂
3 -O-C(O)-NHCH₃
4 -O-C(O)-NHC₂H₅
5 -O-C(O)-NHCH₂CH₂CH₃
6 -O-C(O)-NHCH₂CH₂OCH₂CH₃
7 -O-C(O)-CH₃
8 -O-C(O)-C₂H₅
9 -O-C(O)-CH₂CH₂CH₃
10 -O-C(O)-CH₂CH₂CH₂CH₃

**Groups 14-1 through 14-10-6.** These groups comprise each compound named in groups 1 through 10-6 where R¹ substituents 1-10 listed in Table A are replaced with the following groups:
1 -O-CH₂C₆H₅
2 -O-CH₂C₆H₅
3 -O-CH₂C₆H₄OCH₃
4 -O-CH₂C₆H₄OCH₃
5 -O-CH₂C₆H₄F
6 -O-CH₂C₆H₄F
7 -O-CH₂C₆H₃(OCH₃)₂
8 -O-CH₂C₆H₃(OCH₃)₂
9 -O-CH₂C₆H₄OCH₂CH₃
10 -O-CH₂C₆H₄OCH₂CH₃

**Groups 15-1 through 15-10-6.** These groups comprise each compound named in groups 1 through 10-6 where R¹ substituents 1-10 listed in Table A are replaced with the following groups:
1 -O-C(O)-CH₂CH₂NH₂ (-O-C(O)-CH₂CH₂NH₂ replaces -OH, which is R¹ substituent 1 in Table A)
2 -O-C(O)-CH₂CH₂CH₂NH₂
3 -O-C(O)-CH₂OH
4 -O-C(O)-CH₂CH₂OH
5 -O-C(O)-CH₂CH₂CH₂OH
6 -O-C(O)-CH₂SH
7 -O-C(O)-CH₂CH₂SH
8 -O-C(O)-CH₂CH₂CH₂SH
9 -O-S(O)(O)-O-CH₂-CH(O-C(O)-OH)-CH₂O-C(O)-C₂H₅
10 -O-P(O)(O)-O-CH₂-CH(O-C(O(OH)-CH₂-O-C(O)-C₂H₅

**Groups 16-1 through 16-10-6.** These groups comprise each compound named in groups 1 through 10-6 where R¹ substituents 1-10 listed in Table A are replaced with the following groups:
1 -O-C(O)-A4-NH₂, where A4-NH₂ is a 4 carbon alkyl group substituted with -NH₂ (-O-C(O)-A4-NH₂ replaces -OH, which is R¹ substituent 1 in Table A)
2 -O-C(O)-A6-NH₂, where A6-NH₂ is a 6 carbon alkyl group substituted with -NH₂
3 -O-C(O)-A8-NH₂, where A8-NH₂ is a 8 carbon alkyl group substituted with -NH₂
4 -O-C(O)-A4-OH, where A4-OH is a 4 carbon alkyl group substituted with -OH or - O-
5 -O-C(O)-A6-OH, where A6-OH is a 6 carbon alkyl group substituted with -OH or - O-
6 -O-C(O)-A8-OH, where A8-OH is a 8 carbon alkyl group substituted with -OH or - O-
7 -O-S(O)(O)-O-CH₂-CH(O-C(O)-OH)-CH₂-O-C(O)-C₃H₇
8 -O-P(O)(O)-O-CH₂-CH(O-C(O)-OH)-CH₂-O-C(O)-C₃H₇
9 -O-S(O)(O)-O-CH₂-CH(O-C(O)-OH)-CH₂-O-C(O)-C₄H₉
10 -O-P(O)(O)-O-CH₂-CH(O-C(O)-OH)-CH₂-O-C(O)-C₄H₉

**Groups 17-1 through 17-10-6.** These groups comprise each compound named in compound groups 1 through 10-6 where R¹ substituents 1-10 listed in Table A are replaced with the following groups:
1 -O-S(O)(O)-O-CH₂-CH(O-C(O)-OH)-CH₂-O-C(O)-C₆H₁₃
2 -O-P(O)(O)-O-CH₂-CH(O-C(O)-OH)-CH₂-O-C(O)-C₆H₁₃
3 -O-S(O)(O)-O-CH₂-CH(O-C(OFOH)-CH₂-O-C(O)-C₈H₁₇
4 -O-P(O)(O)-O-CH₂-CH(O-C(O)-OH)-CH₂-O-C(O)-C₈H₁₇
5 -O-S(O)(O)-O-CH₂-CH(O-C(O)-OH)-CH₂-O-C(O)-CH₂C₅H₁₀OH
6 -O-P(O)(O)-O-CH₂-CH(O-C(O)-OH)-CH₂-O-C(O)-CH₂C₅H₁₀OH
7 -O-S(O)(O)-O-CH₂-CH(O-C(O)-OH)-CH₂-O-C(O)-CH₂C₃H₆OH
8 -O-P(O)(O)-O-CH₂-CH(O-C(O)-OH)-CH₂-O-C(O)- CH₂C₃H₆OH
9 -monosaccharide, e.g., -glucose, -rhamnose
10 oligosaccharide, e.g., a disaccharide

**Groups 18-1 through 18-10-6.** These groups comprise each compound named in groups 1 through 10-6 where R⁴ substituents 1-10 listed in Table A are replaced with the following groups:
1 -O-C(O)CH₂NH₂
2 -O-C(O)C(CH₃)H-NH₂
3 -O-C(O)C(CH₂C₆H₅)H-NH₂
4 -O-C(O)-O-NHC(CH₃)H-CO₂H
5 -O-C(O)-O-NHCH₂-CO₂H
6 -O-C(O)-O-NH(CH₂C₆H₅)H-CO₂H
7 -O-C(O)-CF₃
8 -O-C(O)-CH₂CF₃
9 -O-C(O)-(CH₂)₃CF₃
10 -O-C(O)-(CH₂)₅CH₃

**Groups 19-1 through 19-10-6.** These groups comprise each compound named in compound groups 1 through 10-6 where R⁴ substituents 1-10 listed in Table A are replaced with the following groups:
1 -O-C(O)-O-CH₃
2 -O-C(O)-O-CH₂CH₃
3 -O-C(O)-O-C₃H₇
4 -O-C(O)-O-C₄H₉
5 -O-C(O)-O-C₆H₁₃
6 -O-C(O)-O-C₆H₅
7 -O-C(O)-O-C₆H₄OH
8 -O-C(O)-O-C₆H₄OCH₃
9 -monosaccharide, e.g., -glucose, -rhamnose
10 oligosaccharide, e.g., a disaccharide

**Groups 20-1 through 20-10-6.** These groups comprise each compound named in groups 1 through 10-6 where R⁴ substituents 1-10 listed in Table A are replaced with the following groups:
1 -O-C(O)-S-CH₃
2 -O-C(O)-S-CH₂CH₃
3 -O-C(O)-S-C₃H₇
4 -O-C(O)-S-C₄H₉
5 -O-C(O)-S-C₆H₁₃
6 -O-C(O)-S-C₆H₅
7 -O-C(O)-S-C₆H₄OH
8 -O-C(O)-S-C₆H₄OCH₃
9 -O-C(O)-S-C₆H₄OCH₂CH₃
10 -O-C(O)-S-C₆H₄F

**Groups 21-1 through 21-10-6.** These groups comprise each compound named in compound groups 1 through 10-6 where R⁴ substituents 1-10 listed in Table A are replaced with the following groups:
1 -O-C(S)-O-CH₃
2 -O-C(S)-O-CH₂CH₃
3 -SH
4 =S
5 -O-C(S)-O-C₆H₁₃
6 -O-C(O)-O-CH₂C₆H₅
7 -O-C(O)-O-CH₂C₆H₄OH
8 -O-C(O)-O-CH₂C₆H₄OCH₃
9 -O-C(O)-O-CH₂C₆H₄OCH₂CH₃
10 -O-C(O)-O-CH₂C₆H₄F

**Groups 22-1 through 22-10-6.** These groups comprise each compound named in compound groups 1 through 10-6 where R² substituents 1-10 listed in Table A are replaced with the following groups:
1 -O-C(S)-O-CH₃
2 -O-C(S)-O-CH₂CH₃
3 -O-C(S)-O-C₃H₇
4 -O-C(S)-O-C₄H₉
5 -O-C(S)-O-C₆H₁₃
6 -O-C(O)-O-CH₂C₆H₅
7 -O-C(O)-O-CH₂C₆H₄OH
8 -O-C(O)-O-CH₂C₆H₄OCH₃
9 -monosaccharide, e.g., -glucose, -rhamnose
10 oligosaccharide, e.g., a disaccharide

**Groups 23-1 through 23-10-6.** These groups comprise each compound named in compound groups 1 through 10-6 where R³ substituents 1-10 listed in Table A are replaced with the following groups:
1 -O-C(S)-O-CH₃
2 -O-C(S)-O-CH₂CH₃
3 -O-C(S)-O-C₃H₇
4 -O-C(S)-O-C₄H₉
5 -O-C(S)-O-C₆H₁₃
6 -O-C(O)-O-CH₂C₆H₅
7 -O-C(O)-O-CH₂C₆H₄OH
8 -O-C(O)-O-CH₂C₆H₄OCH₃
9 -monosaccharide, e.g., -glucose, -rhamnose
10 oligosaccharide, e.g., a disaccharide

**Groups 24-1 through 24-10-6.** These groups comprise each compound named in compound groups 1 through 10-6 where R² substituents 1-10 listed in Table A are replaced with the following groups:
1 -O-C(O)-O-C₆H₅
2 -O-C(O)-O-C₆H₄OCH₃
3 -SH
4 =S
5 -O-CHR²⁴-C(O)-OR²⁵
6 -O-CHR²⁴-C(O)-R²⁵
7 -O-CHR²⁴-C(O)-N(R²⁵)₂
8 -O-CHR²⁴-C(O)-NHR²⁵
9 -O-CHR²⁴-C(O)-NH₂
10 -O-CHR²⁴-C(O)-OC₆H₅

**Groups 25-1 through 25-10-6.** These groups comprise each compound named in compound groups 1 through 10-6 where R³ substituents 1-10 listed in Table A are replaced with the following groups:
1 -O-C(O)-O-C₆H₅
2 -O-C(O)-O-C₆H₄OCH₃
3 -O-CH₂CH₂-O-CH₃
4 -S-CH₂CH₂-O-CH₃
5 -O-CHR²⁴-C(O)-OR²⁵
6 -O-CHR²⁴-C(O)-R²⁵
7 -O-CHR²⁴-C(O)-N(R²⁵)₂
8 -O-CHR²⁴-C(O)-NHR²⁵
9 -O-CHR²⁴-C(O)-NH₂
10 -O-CHR²⁴-C(O)-OC₆H₅.

**Groups 26-1 through 26-25-10-6.** These groups comprise each compound named in compound groups 1 through 25-10-6 wherein R⁷ in formula B is -O-, instead of -CH₂-. Thus the 26-1 and 26-2 compounds named 1.2.5.9 have the structures 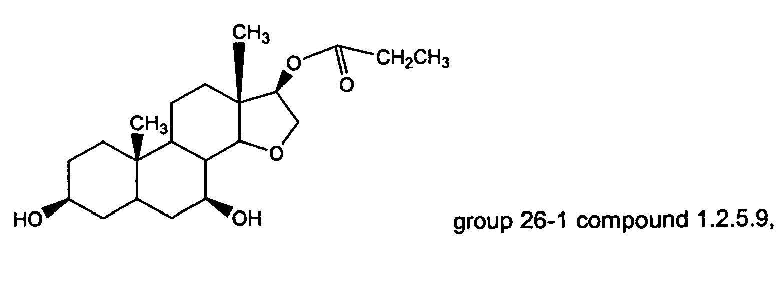 and

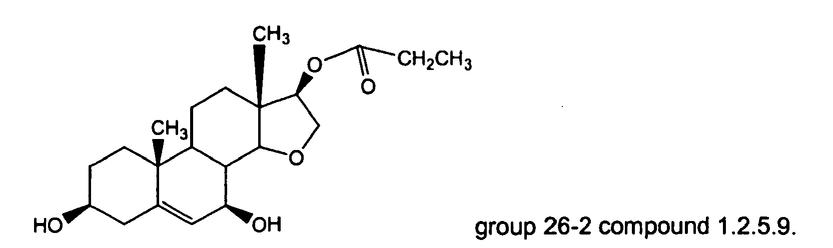

The compound group 26-8-1 and compound group 26-8-2 compounds named 1.2.5.9 have the structures 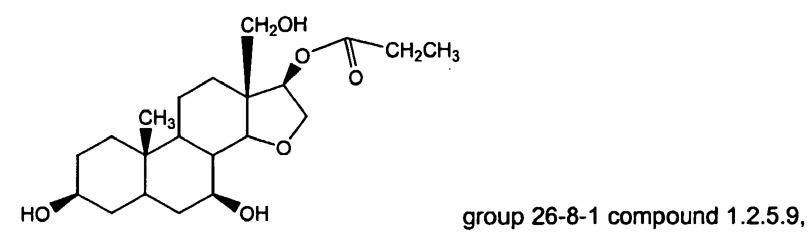 and

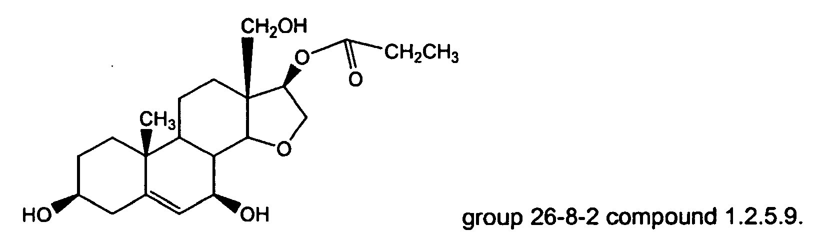

The group 26-11-1 and 26-11-2 compounds named 1.2.5.9 have the structures

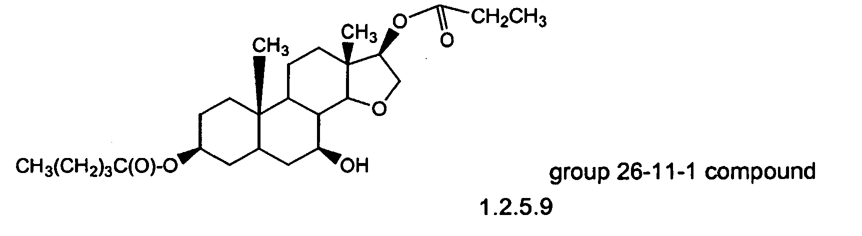

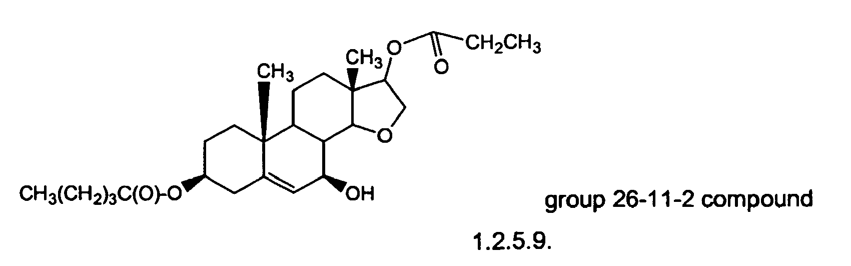

**Groups 27-1 through 27-25-10-6.** These groups comprise each compound named in compound groups 1 through 25-10-6 wherein R⁸ in formula B is -O-, instead of -CH₂-. Thus, the 27-1 and 27-2 compounds named 1.2.5.9 have the structures 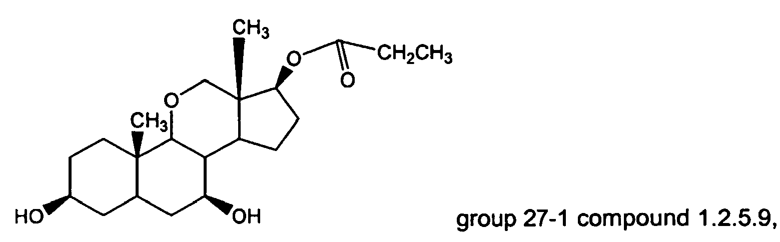 and

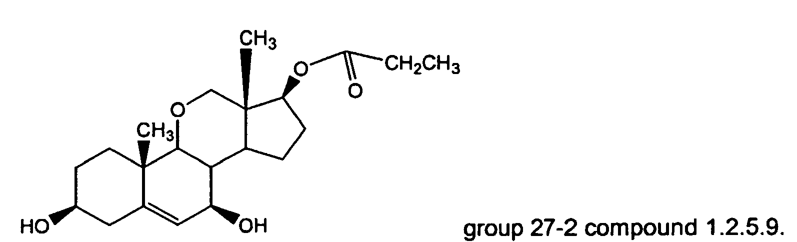

The group 27-8-1 and group 27-8-2 compounds named 1.2.5.9 have the structures 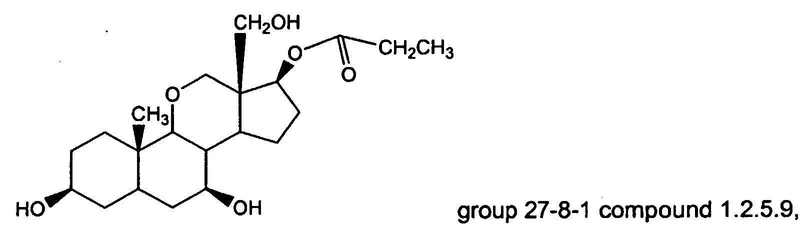 and

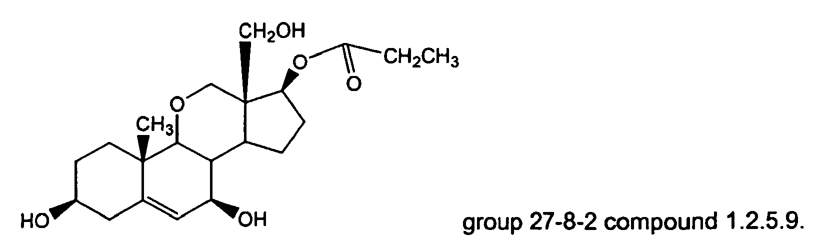

The group 27-11-1 and 27-11-2 compounds named 1.2.5.9 have the structures

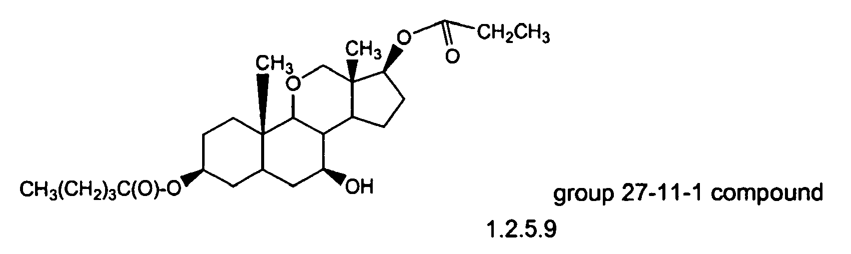

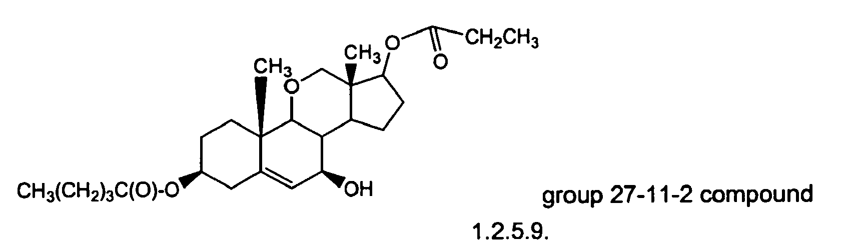

**Groups 28-1 through 28-25-10-6.** These groups comprise each compound named in compound groups 1 through 25-10-6 wherein R⁹ in formula B is -O-, instead of -CH₂- and no double bond is present at the 1-2 position. Thus, there is, e.g., no group 28-3, 28-4, 28-6, 28-8-3, 28-8-4 or 28-8-6, since a 1-2 double bond is present in these compounds and a ring oxygen at the 2 position would be charged. The 28-1, 28-2 and 28-5 compounds named 1.2.5.9 have the structures 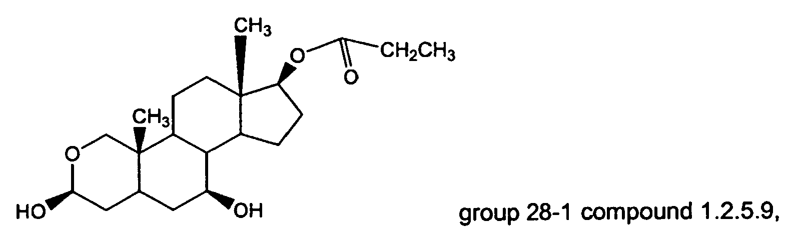 and

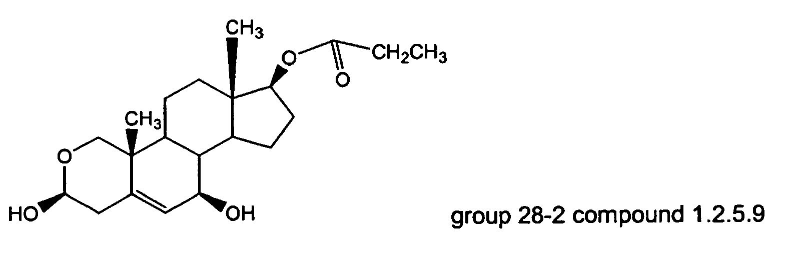

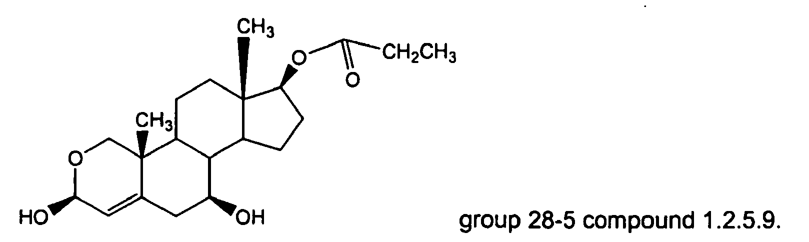

The group 28-8-1 and group 28-8-2 compounds named 1.2.5.9 have the structures 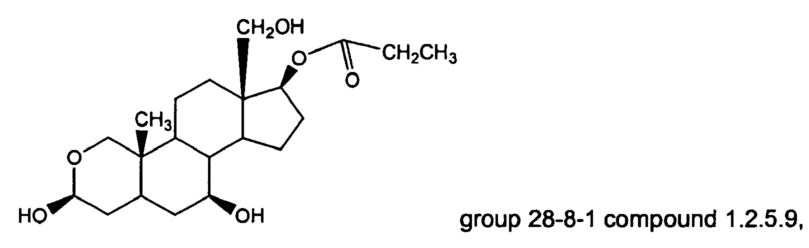 and

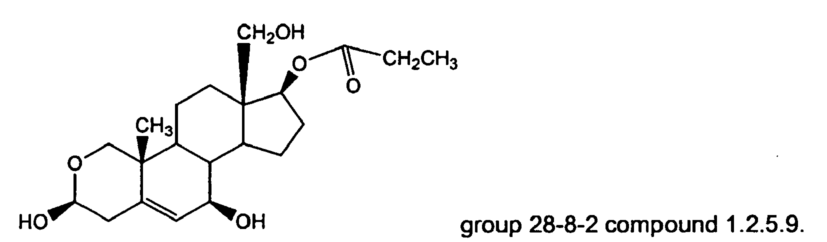

The group 28-11-1 and 28-11-2 compounds named 1.2.5.9 have the structures

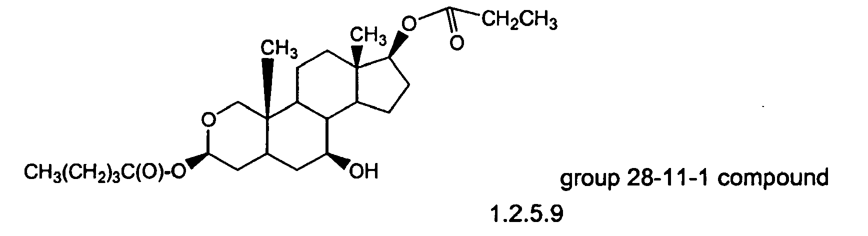

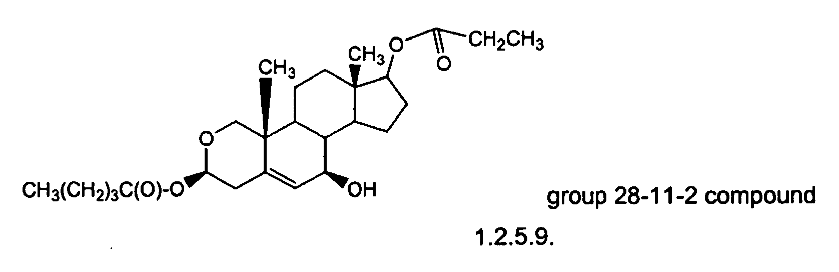

**Groups 29-1 through 29-25-10-6.** These groups comprise each compound named in compound groups 1 through 25-10-6 wherein R⁷ is -NH-, instead of -CH₂-. The compounds are named as described for compound groups 26-1 through 26-25-10-6.

**Groups 30-1 through 30-25-10-6.** These groups comprise each compound named in compound groups 1 through 25-10-6 wherein R⁸ is -NH-, instead of -CH₂-. The compounds are named as described for compound groups 26-1 through 26-25-10-6.

**Groups 31-1 through 31-25-10-6.** These groups comprise each compound named in compound groups 1 through 25-10-6 wherein R⁹ is -NH-, instead of -CH₂-. and no double bond is present at the 1-2 position. Thus, there is , e.g., no group 31-3, 31-4, 31-6, 31-8-3, 31-8-4 or 31-8-6. The compounds are named as described for compound groups 26-1 through 26-25-10-6.

**Groups 32-1 through 32-25-10-6.** These groups comprise each compound named in compound groups 1 through 25-10-6 wherein two of R⁷ R⁸ and R⁹ independently are -NH-, -O- or -S- instead of -CH₂-. The compounds are named as described for compound groups 26-1 through 26-25-10-6.

**Groups 33-1 through 33-25-10-6.** These groups comprise each compound named in compound groups 1 through 25-10-6 wherein each of R⁷ R⁸ and R⁹ independently are -NH-, -O- or -S- instead of -CH₂-. The compounds are named as described for compound groups 26-1 through 26-25-10-6.

**Groups 34-1 through 34-25-10-6.** These groups comprise each compound named in compound groups 1 through 25-10-6 wherein R⁷ is -S-, instead of -CH₂-. The compounds are named as described for compound groups 26-1 through 26-25-10-6.

**Groups 35-1 through 35-25-10-6.** These groups comprise each compound named in compound groups 1 through 25-10-6 wherein R⁸ is -S-, instead of -CH₂-. The compounds are named as described for compound groups 26-1 through 26-25-10-6.

**Groups 36-1 through 36-25-10-6.** These groups comprise each compound named in compound groups 1 through 25-10-6 wherein R⁹ is -S-, instead of -CH₂- and no double bond is present at the 1-2 position. There is , e.g., no group 36-3, 36-4, 36-6, 36-8-3, 36-8-4 or 36-8-6. The compounds are named as described for compound groups 26-1 through 26-25-10-6.

**Groups 37-1 through 37-25-10-6.** These groups comprise each compound named in all of the compound groups 1 through 36-25-10-6 described above wherein R¹ is not divalent, e.g., is not =O, and it is in the α-configuration, instead of the β-configuration as shown in formula B.

**Groups 38-1 through 38-25-10-6.** These groups comprise each compound named in all of the compound groups 1 through 36-25-10-6 described above wherein R² is not divalent, e.g., is not =O, and it is in the α-configuration, instead of the β-configuration as shown in formula B.

**Groups 39-1 through 39-25-10-6.** These groups comprise each compound named in all of the compound groups 1 through 36-25-10-6 described above wherein R³ is not divalent, e.g., is not =O, and it is in the β-configuration, instead of the α-configuration as shown in formula B.

**Groups 40-1 through 40-25-10-6.** These groups comprise each compound named in all of the compound groups 1 through 36-25-10-6 described above wherein R⁴ is not divalent, e.g., is not =O, and it is in the α-configuration, instead of the β-configuration as shown in formula B.

**Groups 41-1 through 41-25-10-6.** These groups comprise each compound named in all of the compound groups 1 through 36-25-10-6 described above wherein R² and R⁴ is not divalent, e.g., they is not =O, and they are both in the α-configuration, instead of the β-configuration as shown in formula B.

**Groups 42-1 through 42-25-10-6.** These groups comprise each compound named in all of the compound groups 1 through 36-25-10-6 described above wherein, when hydrogen is present at the 5-position, it is in the β-configuration, instead of the α-configuration as shown in formula B.

In other embodiments, the formula 1 compounds include one or more of the following: (1) one R¹ is as described in the compound groups described above and the second R¹ is a moiety other than hydrogen, e.g., -OH, -SH, an ester, an ether, optionally substituted alkyl, a heterocycle or an optionally substituted monosaccharide; (2) one R² is as described in the compound groups described above and the second R² is a moiety other than hydrogen, e.g., -OH, -SH, an ester, an ether, optionally substituted alkyl, a heterocycle or an optionally substituted monosaccharide; (3) one R³ is as described in the compound groups described above and the second R³ is a moiety other than hydrogen, e.g., -OH, -SH, an ester, an ether, optionally substituted alkyl, a heterocycle or an optionally substituted monosaccharide; (4) one R⁴ is as described in the compound groups described above and the second R⁴ is a moiety other than hydrogen, e.g., -OH, -SH, an ester, an ether, optionally substituted alkyl, a heterocycle or an optionally substituted monosaccharide; (5) 1 and 2 applies, i.e., one R¹ and one R² are as described in the compound groups described above and the second R¹ and R² are a moiety other than hydrogen, e.g., independently selected -OH, -SH, ester, ether, optionally substituted alkyl, heterocycle or optionally substituted monosaccharide; (6) 1 and 3 applies; (7) 1 and 4 applies; (8) 2 and 3 applies; (9) 2 and 4 applies; (10) 3 and 4 applies; (11) 1, 2 and 3 applies; (12) 1, 2 and 4 applies; or (13) 2, 3 and 4 applies.

Any of the compounds or genera of compounds that are named herein, including those named in compound groups 1 through 42-25-10-6 are sutiable for use in any of the methods described herein.

In some embodiments, one, or more of R¹-R⁶, R¹⁰, R¹⁵, R¹⁷ and R¹⁸ independently have the structure(s) and/or independently comprise the named compounds, -H, -OH, =O, -SH, =S, -NH₂, -CN, -N₃, halogen, =CH₂, =NOH, =NOC(O)CH₃, -C(O)-CH₃, -C(O)-(CH₂)₁₋₄-CH₃, -CCH, -CCCH₃, -CH=CH₂, -CH=CH₂CH₃, -O-C(O)-(CH₂)ₘ-(CF₂)ₙ-CH₃, -O-C(O)-(CH₂)ₘ-(CF₂)ₙ-CF₃, -O-C(O)-(CH₂)ₘ-(CF₂)ₙ-CH₂F, -O-C(OFO-(CH₂)ₘ-(CF₂)ₙ-CH₃, -O-C(O)-O-(CH₂)ₘ-(CF₂)ₙ-CF₃, - O-C(O)-O-(CH₂)ₘ-(CF₂)ₙ-CH₂F, -O-C(O)-NH-(CH₂)ₘ-(CF₂)ₙ-CH₃, -O-C(O)-NH-(CH₂)ₘ-(CF₂)ₙ-CF₃, -O-C(O)-NH-(CH₂)ₘ-(CF₂)ₙ-CH₂F (where m is 1, 2, 3, 4, 5, 6, 7, 8, 9 or 10, n is 0, 1, 2, 3, 4, 5, 6, 7, 8, 9 or 10, usually n is 0), -CH(CH₃)-(CH₂)₂-C(O)NH-CH₂COOH, -CH(CH₃)-(CH₂)₂-C(O)NH-CH₂SO₃H, -OSi(CH₃)₂C(CH₃)₃, - C(OH)=CHCH₃, =CH(CH₂)₀₋₁₅CH₃, -(CH₂)₀₋₁₄CH₂F, -(CH₂)₀₋₁₄CH₂Cl, -(CH₂)₀₋₁₄CH₂Br, -(CH₂)₀₋₁₄CH₂I, -(CH₂)₂₋₁₀-O-(CH₂)₀₋₄CH₃, -(CH₂)₂₋₁₀-S-(CH₂)₀₋₄CH₃, -(CH₂)₂₋₁₀-NH-(CH₂)₀₋₄CH₃, -O-(CH₂)₀₋₁₄CH₂F, -O-(CH₂)₀₋₁₄CH₂Cl, -O-(CH₂)₀₋₁₄CH₂Br, -O-(CH₂)₀₋₁₄CH₂I, -O-(CH₂)₂₋₁₀-O-(CH₂)₀₋₄CH₃, -O-(CH₂)₂₋₁₀-S-(CH₂)₀₋₄CH₃, -O-(CH₂)₂₋₁₀-NH-(CH₂)₀₋₄CH₃, -O-C(O)-(CH₂)₀₋₁₄CH₂F, -O-C(O)-(CH₂)₀₋₁₄CH₂Cl, -O-C(O)-(CH₂)₀₋₁₄CH₂Br, -O-C(O)-(CH₂)₀₋₁₄CH₂I, -O-C(O)-(CH₂)₂₋₁₀-O-(CH₂)₀₋₄CH₃, -O-C(O)-(CH₂)₂₋₁₀-S-(CH₂)₀₋₄CH₃, -O-C(O)-(CH₂)₂₋₁₀-NH-(CH₂)₀₋₄CH₃, -O-C(S)-(CH₂)₀₋₁₄CH₂F, -O-C(S)-(CH₂)₀₋₁₄CH₂Cl, -O-C(S)-(CH₂)₀₋₁₄CH₂Br, -O-C(S)-(CH₂)₀₋₁₄CH₂I, -O-C(S)-(CH₂)₂₋₁₀-O-(CH₂)₀₋₄CH₃, -O-C(S)-(CH₂)₂₋₁₀-S-(CH₂)₀₋₄CH₃, -O-C(S)-(CH₂)₂₋₁₀-NH-(CH₂)₀₋₄CH₃, -(CH₂)₀₋₁₆NH₂, -(CH₂)₀₋₁₅CH₃, -(CH₂)₀₋₁₅CN, -(CH₂)₀₋₁₅CH=CH₂, -(CH₂)₀₋₁₅NHCH(O), -(CH₂)₀₋₁₆NH-(CH₂)₀₋₁₅CH₃, -(CH₂)₀₋₁₅CCH, -(CH₂)₀₋₁₅OC(O)CH₃, -(-CH₂)₀₋₁₅OCH(OH)CH₃, -(CH₂)₀₋₁₅C(O)OCH₃, -(CH₂)₀₋₁₅C(O)OCH₂CH₃, -(CH₂)₀₋₁₅C(O)(CH₂)₀₋₁₅CH₃, -(CH₂)₀₋₁₅C(O)(CH₂)₀₋₁₅CH₂OH, -O(CH₂)₁₋₁₆NH₂, -O(CH₂)₁₋₁₅CH₃, -O(CH₂)₁₋₁₅CN, -O(CH₂)₁₋₁₅CH=CH₂, -O(CH₂)₁₋₁₅NHCH(O), -O(CH₂)₁₋₁₆NH-(CH₂)₁₋₁₅CH₃, -O(CH₂)₁₋₁₅CCH, -O(CH₂)₁₋₁₅OC(O)CH₃, -O(CH₂)₁₋₁₅OCH(OH)CH₃, -O(CH₂)₁₋₁₅C(O)OCH₃, -O(CH₂)₁₋₁₅C(O)OCH₂CH₃, -O(CH₂)₁₋₁₅C(O)(CH₂)₀₋₁₅CH₃, -O(CH₂)₁₋₁₅C(O)(CH₂)₀₋₁₅CH₂OH, -OC(O)(CH₂)₁₋₁₆NH₂, -OC(O)(CH₂)₁₋₁₅CH₃, -C(O)O(CH₂)₁₋₁₅CN, -C(O)O(CH₂)₁₋₁₅CH=CH₂, -OC(O)(CH₂)₁₋₁₅NHCH(O), -OC(O)(CH₂)₁₋₁₆NH-(CH₂)₁₋₁₅CH₃, -OC(O)(CH₂)₁₋₁₅CCH, -OC(O)(CH₂)₁₋₁₅OC(O)CH₃, -OC(O)(CH₂)₁₋₁₅OCH(OH)CH₃, -OC(O)(CH₂)₁₋₁₅C(O)OCH₃, -OC(O)(CH₂)₁₋₁₅C(O)OCH₂CH₃,-OC(O)(CH₂)₁₋₁₅C(O)(CH₂)₀₋₁₅CH₃, -OC(O)(CH₂)₁₋₁₅C(O)(CH₂)₀₋₁₅CH₂OH, phosphoenolpyruvate, D-glucosamine, glucholic acid, glucuronic acid, pantothenic acid, pyruvic acid, glucose, fructose, mannose, sucrose, lactose, fucose, rhamnose, galactose, ribose, 2'-deoxyribose, 3'-deoxyribose, glycerol, 3-phosphoglycerate, a PEG (PEG-20, PEG-100, PEG-200, PEG-10000), a polyoxyalkylene polymer, glycine, alanine, phenylalanine, threonine, proline, 4-hydroxyproline or an oligonucleotide or analog that comprises about 4 to about 21 monomers.

When a substituent is an oligonucleotide or a polymer usually only a one of these is bonded to the formula 1 compound. Typically, when R¹-R² and R⁴-R⁶ comprise one or more of these substituents (or others described herein), the substituent is present in the β-configuration, while R³ typically comprises a substituent in the β-configuration. In some embodiments, R² is in the α-configuration.

In some embodiments, one or more of R¹-R⁶, R¹⁰, R¹⁵, R¹⁷ and R¹⁸ independently comprise a nucleoside, a nucleotide, an oligonucleotide or an analog of any of these moieties. Typically, such moieties are linked to the steroid nucleus through a terminal hydroxyl, thiol, acyl moiety or amine at the 5', 3' or 2' positions, when a hydroxyl, thiol, acyl moiety or amine is present at that position. For oligonucleotides and oligonucleotide analogs, the linkage to the steroid occasionally is through a sugar hydroxyl at an internal 2' position.

Analogs of phosphodiester linkages include phosphorothioate linkages and others as described in the cited references. Oligonucleotide coupling groups means any moiety suitable for generating a phosphodiester linkage or phosphodiester analog linkage between adjacent nucleotides or their analogs. Suitable oligonucleotide coupling groups include -OH, H-phosphonate, alkylphosphonamidites or phosphoramidites such as β-cyanoethyl-phosphoramidite, N, N-diisopropylamino-β-cyanoethoxyphosphine and others as described in the cited references. Suitable purine and pyrimidine bases include adenine, guanine, cytosine, thymine, uracil and others as described in the cited references. Suitable nucleosides, nucleotides, oligonucleotides and their analogs have been described, see e.g., U.S. patents 4725677, 4973679, 4997927, 4415732, 4458066, 5047524, 4959463, 5212295, 5386023, 5489677, 5594121, 5614622, 5624621; and PCT publication Nos. WO 92/07864, WO 96/29337, WO 97/14706, WO 97/14709, WO 97/31009, WO 98/04585 and WO 98/04575 all of which are incorporated herein by reference. The formula 1 compounds, e.g., those named in any of the compound groups 1 through 42-25-10-6, are suitable for linkage to oligonucleotides modulate the lipophilicity of oligonucleotides or the transport or permeation of an oligonucleotide into cells. Such linkages may be biologically labile to facilitate release of the steroid from the oligonucleotide once the conjugate has entered the cell.

Table 2 shows these and other exemplary moieties that one or more of R¹-R⁶, R¹⁰, R¹⁵, R¹⁷ and R¹⁸ independently can comprise. Pr means a protecting group. These moieties are often bonded to one or more of the R¹, R² and R⁴ positions, usually to one or two of those positions. For structures with more than one of a given variable, e.g., X in structure A3 or A5, each is independently selected.

**TABLE 2**

| | |
|---|---|
| 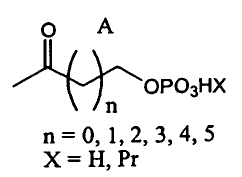 | 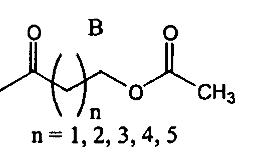 |
| | |
| 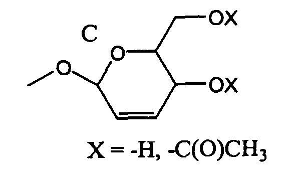 | 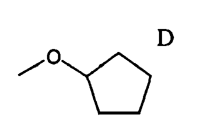 |
| 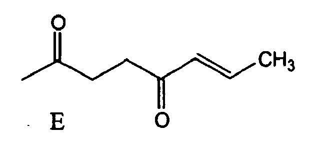 | 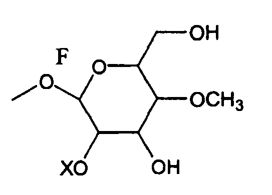 |
| 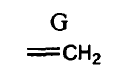 | 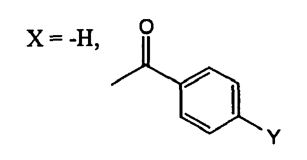 |
| 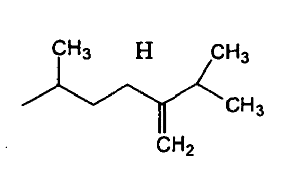 | Y = -CH₃, -OCH₃, Br, Cl, F I |
| 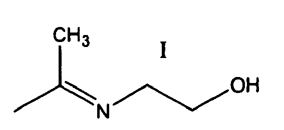 | 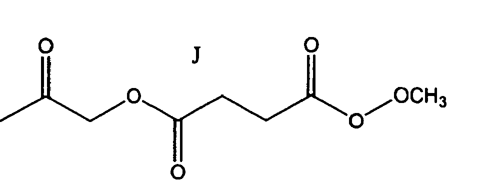 |
| 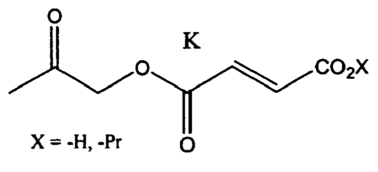 | 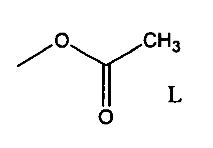 |
| 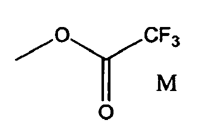 | 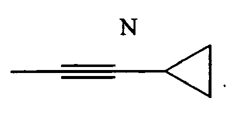 |
| 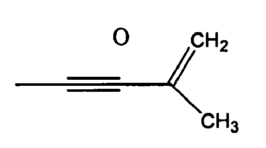 | 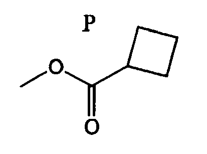 |
| 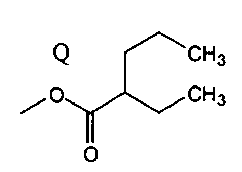 | 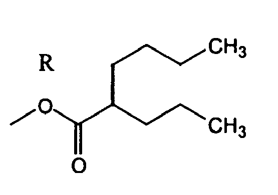 |
| 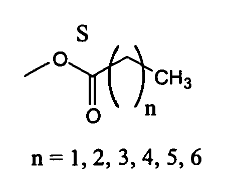 | 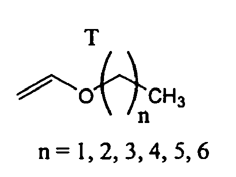 |
| 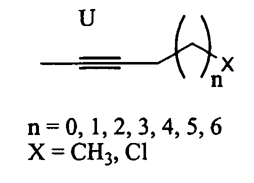 | 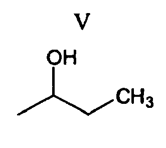 |
| 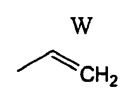 | 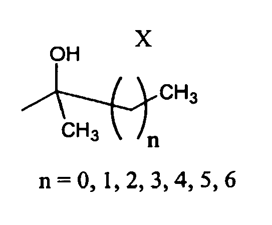 |
| 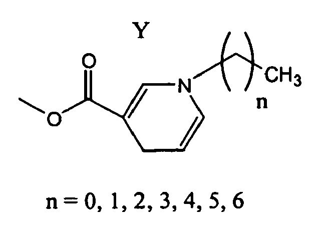 | 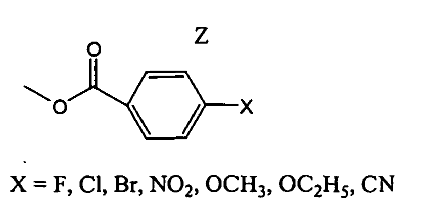 |
| 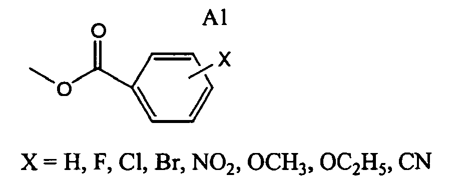 | 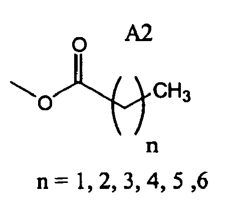 |
| 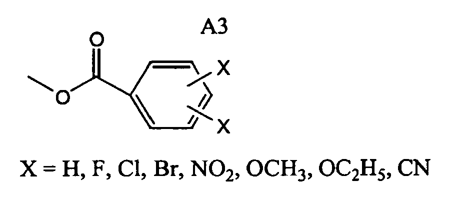 | 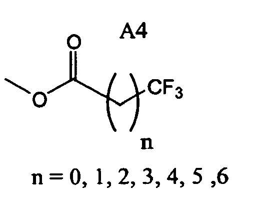 |
| 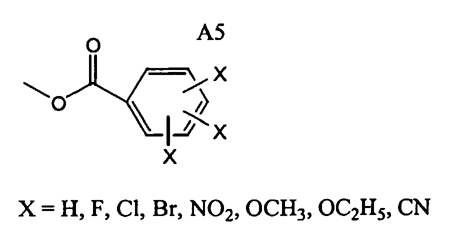 | 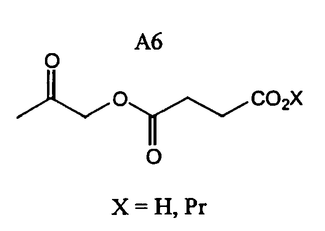 |

In some embodiments, an R³ and an R⁴ of the formula 1 compounds comprises a ring(s) structure. Exemplary compounds of formula 2 include the following structures, 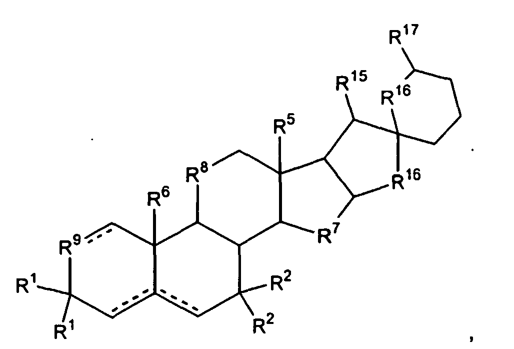 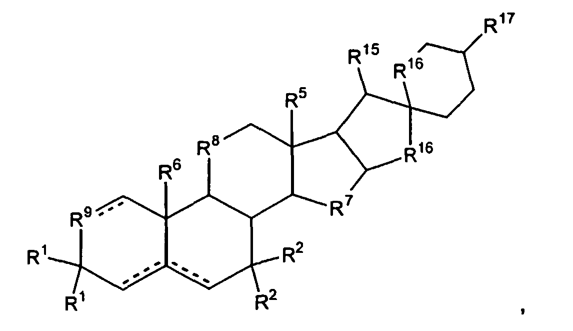 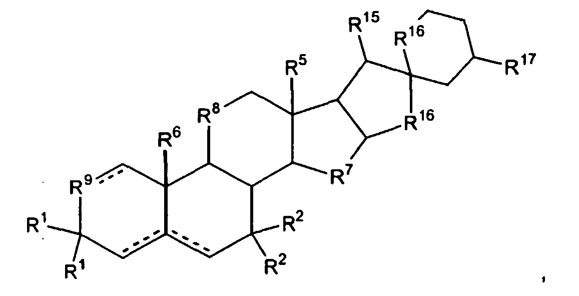 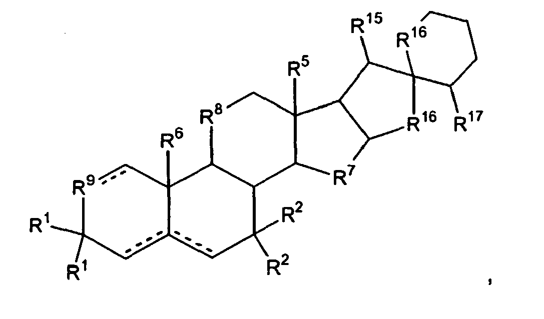 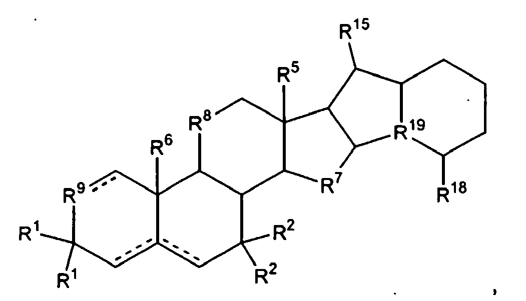 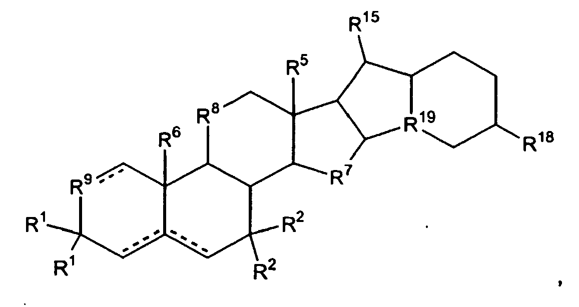 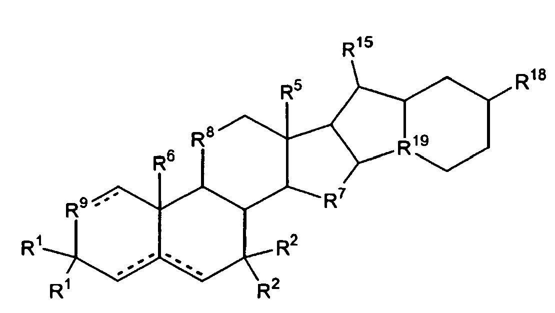 or 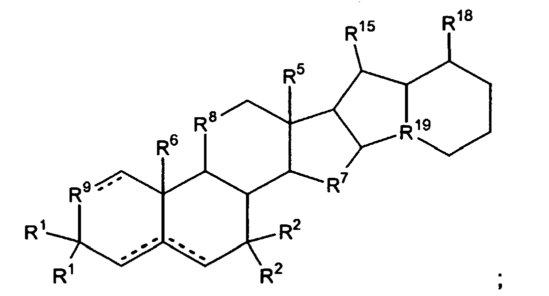 wherein, R¹⁶ independently are -CH₂-, -O-, -S- or -NH-; R¹⁵ , R¹⁷ and R¹⁸ independently are -H, -OR^{PR}, -SR^{PR}, -N(R^{PR})₂, -O-Si-(R¹³)₃, -CN, -NO₂, -OSO₃H, - OPO₃H, an ester, a thioester, a phosphoester, a phosphothioester, a phosphonoester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted monosaccharide, an optionally substituted oligosaccharide, a nucleoside, a nucleotide, an oligonucleotide, a polymer, or, one or more of R¹⁵, R¹⁷ and R¹⁸ independently are =O or =S and the hydrogen atom that is bonded to the same carbon atom is absent; and R¹⁹ is nitrogen or CH.

Such compounds include any of these structures wherein one, two or three of R⁷, R⁸ and R⁹ are independently -O-, -S-, or -NH- or wherein one or both of R⁵ and R⁶ independently are -H, -CH₃, -CH₂OR^{PR}, -CH₂OH, -CH₂SH, -CH₂SR^{PR}, -CH₂O-C(O)-C₁₋₁₀ alkyl, -CH₂S-C(O)-C₁₋₁₀ alkyl, -CH₂O-C(O)-C₁₋₁₀ alkenyl, -CH₂S-C(O)-C₁₋₁₀ alkenyl, -CH₂O-C(O)-C₀₋₄ alkyl-heterocycle, -CH₂S-C(O)-C₀₋₄ alkyl-heterocycle, - CH₂O-C(O)-C₀₋₄ alkyl-phenyl, -CH₂S-C(O)-C₀₋₄ alkyl-phenyl, wherein any C₁₋₁₀ alkyl, heterocycle or phenyl moiety is optionally substituted with one or more substituents, wherein the one or more substituents are one, two, three or more independently selected -O-, =O, -OR^{PR}, -S-, =S, -SR^{PR}, -NH-, -N(R^{PR})₂ or -C(O)-NH-, wherein each R^{PR} independently is -H or a protecting group.

Other embodiments of the invention and related subject matter include the following numbered embodiments.
1. A method to treat a blood cell deficiency in a subject comprising administering to the subject, or delivering to the subject's tissues, an effective amount of a compound of formula 1.
2. The method of embodiment 1 wherein the formula 1 compound has the structure 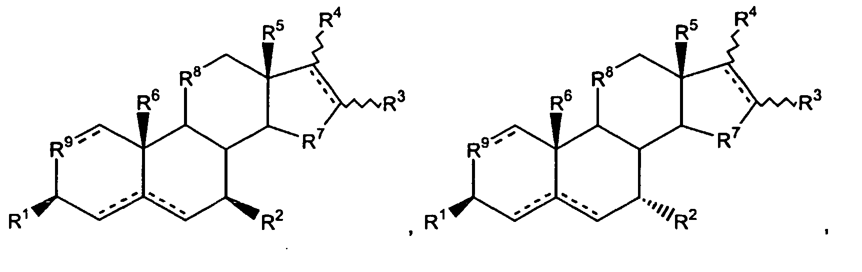 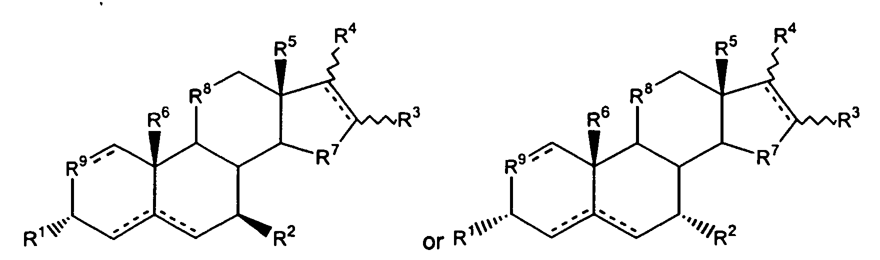 wherein one, two or three of R⁷, R⁸ and R⁹ are -CH₂- or -CH= and wherein the configuration of hydrogen atoms at the 5 (if present), 8, 9 and 14 positions respectively are α.α.α.α, α.α.α.β, α.α.β.α, α.β.α.α, β.α.α.α, α.α.β.β, α.β.α.β, β.α.α.β, β.α.β.α, β.β.α.α, α.β.β.α, α.β.β.β, β.α.β.β, β.β.α.β, β.β.β.α or β.β.β.β, typically α.α.β.α or β.α.β.α.
3. The method of embodiment 2 wherein the formula 1 compound has the structure 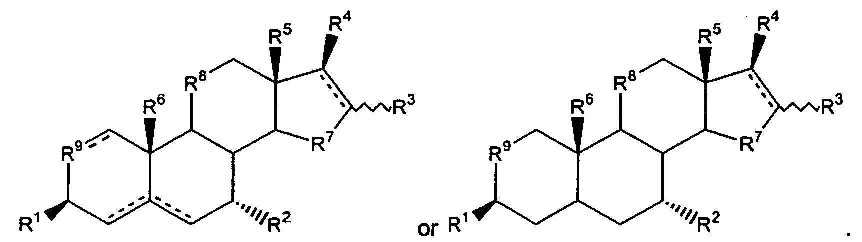
4. The method of embodiment 3 wherein R¹, R² and R⁴ independently are -OH, -SCN, a C2-C20 ester or C1-C20 alkoxy, R³ is -H and two or three of R⁷, R⁸ and R⁹ are -CH₂-.
5. The method of embodiment 3 or 4 wherein the formula 1 compound has the structure 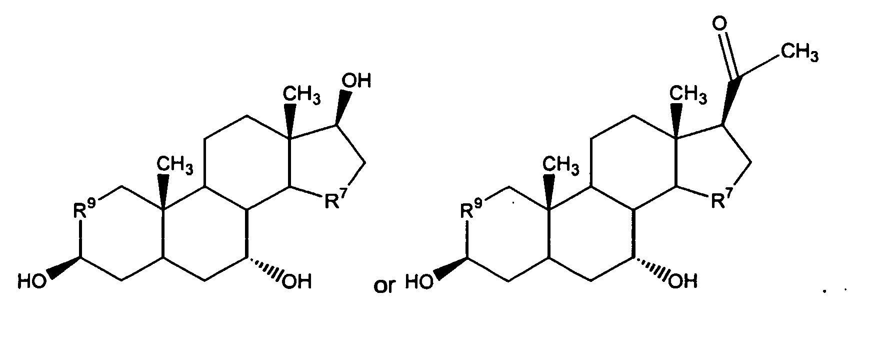
6. The method of any of embodiments 3-5 wherein the configuration of hydrogen atoms at the 5 (if present), 8, 9 and 14 positions respectively are α.α.β.α or β.α.β.α.
7. The method of embodiment 1 wherein the formula 1 compound has the structure 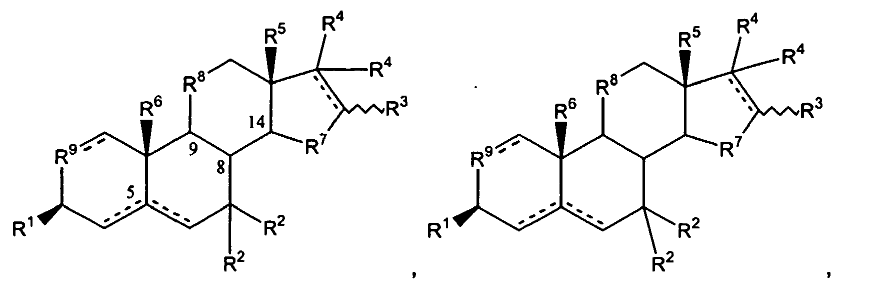 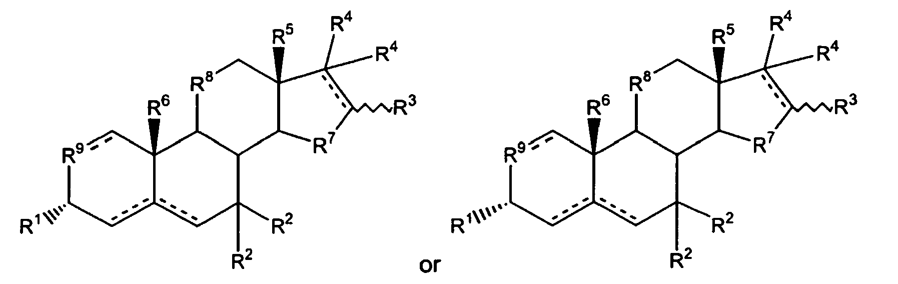 wherein one R⁴ is absent when there is a double bond at the 16-17 position and wherein R⁷, R⁸ and R⁹ are independently selected and wherein one, two or three of R⁷, R⁸ and R⁹ are not -CH₂- or -CH= and wherein hydrogen atoms at the 5 (if present), 8, 9 and 14 positions respectively are in the α.α.α.α α.α.α.β, α.α.β.α, α.β.α.α, β.α.α.α, α.α.β.β, α.β.α.β, β.α.α.β, β.α.β.α, β.β.α.α, α.β.β.α, α.β.β.β, β.α.β.β, β.β.α.β, β.β.β.α, or β.β.β.β configurations, typically α.α.β.α or β.α.β.α.
8. The method of embodiment 7 wherein R⁸ is -CH₂-, -O-, -S- or -NH-.
9. The method of embodiment 7 or 8 wherein R⁷ is -CH₂-CHR¹⁰-, -CH₂-, - O-CHR¹⁰- or -O-C(O)-.
10. The method of embodiment 7, 8 or 9 wherein R⁸ or R⁹ is absent.
11. The method of embodiment 7 or 8 wherein R⁷ and R⁹ independently are -CHR¹⁰-, -CH₂-, -CH=, -O-, -S- or -NH-, wherein R¹⁰ is -OH, -SH, a C₁₋₃₀ organic moiety, a C₁₋₃₀ ester, C₁₋₁₀ optionally substituted alkyl, C₁₋₁₀ optionally substituted alkoxy, C₁₋₁₀ optionally substituted alkenyl or C₁₋₁₀ optionally substituted alkynyl.
12. The method of embodiment 1 wherein the formula 1 method has the structure 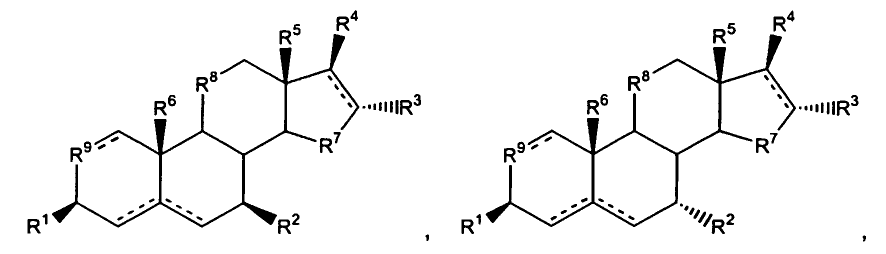 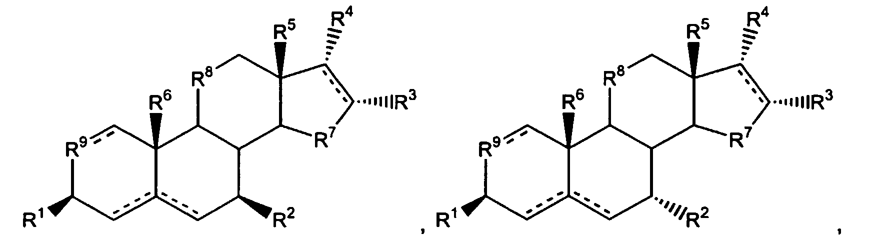 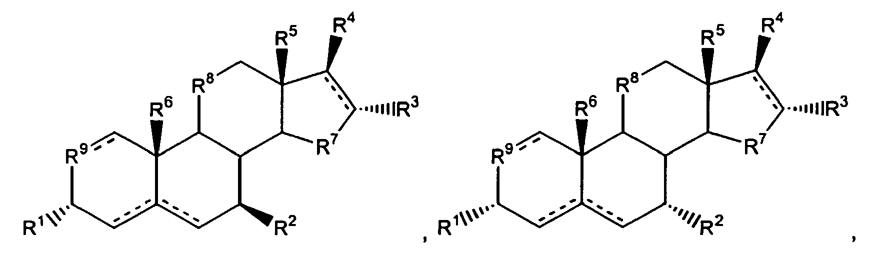 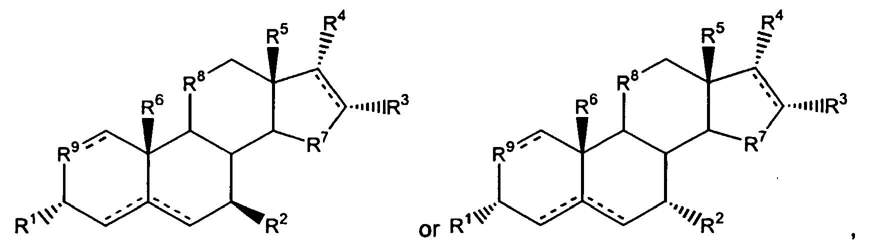 wherein hydrogen atoms at the 5 (if present), 8, 9 and 14 positions respectively are in the α.α.α.α, α.α.α.β, α.α.β.α, α.β.α.α, β.α.α.α, α.α.β.β, α.β.α.β, β.α.α.β, α.β.α.β, β.β.α.α, α.β.β.α, α.β.β.β, β.α.β.β, β.β.α.β, β.β.β.α, or β.β.β.β configurations, typically α.α.β.α or β.α.β.α.
13. The method of embodiment 12 wherein R⁴ is -OH, =O, -SH, -SCN, a C₁₋₃₀ ester or C₁₋₃₀ alkoxy, wherein the ester or alkoxy moiety is optionally substituted with one, two or more independently selected substituents, which are optionally selected from -F, -Cl, -Br, -I, -O-, =O, -S-, -NH-, -R^{PR}, -OR^{PR}, -SR^{PR} or -NHR^{PR}_{.}
14. The method of embodiment 12 or 13 wherein R¹ is -OH, =O, -SH, - SCN, a C₁₋₃₀ ester or C₁₋₃₀ alkoxy, wherein the ester or alkoxy moiety is optionally substituted with one, two or more independently selected substituents, which are optionally selected from -F, -Cl, -Br, -I, -O-, =O, -S-, -NH-, -R^{PR}, -OR^{PR}, -SR^{PR} or - NHR^{PR}.
15. The method of any of embodiments 1-14 wherein a second R¹ is present and it is a moiety other than hydrogen, e.g., -OH, -SH, -SCN, a C₁₋₃₀ ester, C₁₋₃₀ alkoxy, C₁₋₃₀ alkynyl or a monosaccharide wherein the ester, alkoxy, alkynyl or monosaccharide is optionally substituted with one, two or more independently selected substituents, which are optionally selected from -F, -Cl, -Br, -I, -O-, =O, -S-, -NH-, -R^{PR}, -OR^{PR}, -SR^{PR} or -NHR^{PR}_{.}
16. The method of any of embodiments 1-15 wherein a second R² is present and it is a moiety other than hydrogen, e.g., -OH, -SH, -SCN, a C₁₋₃₀ ester or C₁₋₃₀ alkoxy, C₁₋₃₀ alkynyl or a monosaccharide wherein the ester, alkoxy, alkynyl or monosaccharide is optionally substituted with one, two or more independently selected substituents, which are optionally selected from -F, -Cl, -Br, -I, -O-, =O, -S-, -NH-, -R^{PR}, -OR^{PR}, -SR^{PR} or -NHR^{PR}.
17. The method of any of embodiments 1-16 wherein a second R³ is present and it is a moiety other than hydrogen, e.g., -OH, -SH, -SCN, a C₁₋₃₀ ester or C₁₋₃₀ alkoxy, C₁₋₃₀ alkynyl or a monosaccharide wherein the ester, alkoxy, alkynyl or monosaccharide is optionally substituted with one, two or more independently selected substituents, which are optionally selected from -F, -Cl, -Br, -I, -O-, =O, -S-, -NH-, -R^{PR}, -OR^{PR}, -SR^{PR} or -NHR^{PR}.
18. The method of any of embodiments 1-17 wherein a second R⁴ is present and it is a moiety other than hydrogen, e.g., -OH, -SH, -SCN, a C₁₋₃₀ ester or C₁₋₃₀ alkoxy, C₁₋₃₀ alkynyl or a monosaccharide wherein the ester, alkoxy, alkynyl or monosaccharide is optionally substituted with one, two or more independently selected substituents, which are optionally selected from -F, -Cl, -Br, -I, -O-, =O, -S-, -NH-, -R^{PR}, -OR^{PR}, -SR^{PR} or -NHR^{PR}.
19. The method of any of embodiments 1-18 wherein there is a double bond at the 1-2 position.
20. The method of any of embodiments 1-18 wherein there is a double bond at the 4-5 position.
21. The method of any of embodiments 1-18 wherein there is a double bond at the 5-6 position.
22. The method of any of embodiments 1-18 wherein there is a double bond at the 16-17 position.
23. The method of any of embodiments 1-18 wherein there are double bonds at the 1-2 and 4-5 positions.
24. The method of any of embodiments 1-18 wherein there are double bonds at the 1-2 and 5-6 positions.
25. The method of any of embodiments 1-18 wherein there are double bonds at the 1-2 and 16-17 positions.
26. The method of any of embodiments 1-18 wherein there are double bonds at the 4-5 and 16-17 positions.
27. The method of any of embodiments 1-18 wherein there are double bonds at the 5-6 and 16-17 positions.
28. The method of any of embodiments 1-18 wherein there are double bonds at the 1-2, 4-5 and 16-17 positions.
29. The method of any of embodiments 1-18 wherein there are double bonds at the 1-2, 5-6 and 16-17 positions.
30. A compound of formula 1, e.g., a compound in any compound group or embodiment disclosed herein.
31. A composition comprising a compound of formula 1, e.g., a compound in any compound group or embodiment disclosed herein, and an excipient.
32. Use of a compound of formula 1, e.g., a compound in any compound group or embodiment disclosed herein, to manufacture a medicament for the treatment of a blood cell deficiency, e.g., NP of TP, in a subject, e.g., a mammal or a human.
33. A product produced by the process of contacting a formula 1 compound, e.g., a compound in any compound group or embodiment disclosed herein, and an excipient.
34. A kit comprising a formulation that comprises a unit dosage or a multiple dosage comprising a formula 1 compound, e.g., a compound in any compound group or embodiment disclosed herein, and one or more excipients wherein the formulation is dispensed in a suitable container, wherein the kit further comprises a label that provides information about one or more of (1) the formula 1 compound's chemical structure, (2) nany recommended dosing regimen, (3) any adverse effects of administering the formula 1 compound to a subject that are required to be disclosed and (4) the amount of the formula 1 compound that is present in each unit dose or in the entire container.

Exemplary synthesis methods. By way of exemplification and not limitation, the following methods are suitable to prepare the one or more of the compounds disclosed herein. Starting materials and straightforward variations of the schemes are found, e.g., in the following references, all of which are incorporated herein by reference: A. P. Davis, et al., Tetrahedron Lett., 33: 5111-5112, 1992; I. Takashi, el al., Chem. Pharm. Bull., 34: 1929-1933, 1986; I. Weisz, et al., Arch. Pharm., 319: 952-953, 1986; T. Watabe, et al., J. Med. Chem., 13: 311-312, 1970; M. Davis, et al., J. Chem.Soc. C., (11): 1045-1052, 1967; R. C. Cambie, et al., J. Chem. Soc., Perkin Trans. 1, (20): 2250-2257, 1977; L. Minale, et al., J. Chem Soc., Perkin Trans. 1, (20): 2380-2384, 1974; C. K. Lai, et al., Steroids, 42: 707-711, 1983; S. Irie, et al., Synthesis, (9): 1135-1138, 1996; E. J. Corey, J. Am. Chem. Soc., 118: 8765-8766, 1996; M. E. Annunziato, et al., Bioconjugate Chem., 4: 212-218, 1993; N. J. Cussans, et al., J. Chem. Soc., Perkin Trans. 1, (8): 1650-1653, 1980; D. H. R. Barton, et al., J. Chem. Soc., Chem. Commun., (9): 393-394, 1978; H. Loibner, et al., Helv. Chim. Acta, 59: 2100-2113, 1976; T. R. Kasturi, et al., Proc. Indian Acad. Sci., [Ser.]: Chem. Sci., 90: 281-290, 1981; T. Back, J. Org. Chem., 46: 1442-1446, 1981; A. Canovas, et al., Helv. Chim. Acta, 63: 486-487, 1980; R. J. Chorvat, et al., J. Org. Chem., 43: 966-972, 1978; M. Gumulka, et al., Can. J. Chem., 63: 766-772, 1985; H. Suginome, et al., J. Org. Chem., 55: 2170-2176, 1990; C. R. Engel, et al., Can. Heterocycles, 28: 905-922, 1989; H. Sugimone, et al., Bull, Chem. Soc. Jpn., 62: 193-197, 1989; V. S. Salvi, et al., Can. Steroids, 48: 47-53, 1986; C. R. Engel, et al., Can. Steroids, 47: 381-399, 1986; H. Suginome, et al., Chem. Lett., (5): 783-786, 1987; T. Iwadare, et al., J. Chem. Soc., Chem. Commun., (11): 705-706, 1985; H. Nagano, et al., J. Chem. Soc., Chem. Commun., (10): ;656-657, 1985; V. S. Salvi, et al., Steroids, 27: 717-725, 1976; C. H. Engel, et al, Steroids, 25: 781-790, 1975; M. Gobbini, et al., Steroids, 61: 572-582, 1996; A. G. Gonzalez, et al., Tetrahedron, 46: 1923-1930, 1990; S. C. Bobzin, et al., J. Org. Chem., 54: 3902-3907, 1989; B. Solaja, et al., Croat. Chem. Acta, 59: 1-17, 1986; Y. Kashman, et al., Tetrahedron, 27: 3437-3445, 1971; K. Yoshida, et al., Chem. Pharm. Bull. (Tokyo), 15: 1966-1978, 1967; P. B. Sollman, et al., Chem. Commun. (11): 552-554, 1967; H. Suginome, et al., J. Org. Chem., 55: 2170-2176, 1990; H. Suginome, et al., Journal Chem. Lett., (5): 783-786, 1987; G. A. Tolstikov, et al., Zh. Org. Khim., 22: 121-132, 1986; T. Terasawa, et al., J. Chem. Soc., Perkin Trans. 1, (4): 990-1003, 1979; Z. Zhuang, et al., Yougi Huaxue, (4): 281-285, 1986; W. T. Smith, et al., Trans. Ky. Acad. Sci., 45: 76-77, 1984; A. K. Batta, et al., Steroids, 64: 780-784, 1999; B. Ruan, et al., Steroids, 65: 29-39, 2000; L. Garrido,et al., Steroids, 65: 85-88, 2000; P. Ramesh, et al., Steroids, 64: 785-789, 1999; M. Numazawa, et al., Steroids, 64: 187-196, 1999; P. N. Rao, et al., Steroids, 64: 205-212, 1999; M. Numazawa, et al., Steroids, 64: 320-327, 1999; US patents 3281431, 3301872, 3325535, 3325536, 3952018, 4602008,5571795, 5627270, 5681964, 5744453; international publication numbers WO 9408588, WO 9508558, WO 9508559, WO 9638466, WO 9809450; United Kingdom patent numbers GB 1168227, GB 813529, GB 802618; French patent number 824529; Japan patent number JP 45010134; European patent applications EP 232788, EP 430078; and German patent number DE 19631189.

Scheme 1. For the structures shown in scheme 1, R⁵-R⁹ are as defined for formula 1 compounds. Thus, when R⁵ and R⁶ are both -CH₃ in the β-configuration, R⁷, R⁸ and R⁹ are all -CH₂-, H at the 9 and 14 positions are in the α-configuration, acetate at the 3-position is in the β-configuration, and H at the 8 position is in the β-configuration, the first compound in scheme 1 is DHEA acetate. The acetate groups at the 3, 7, 16, 17 or other positions in this scheme and in other schemes disclosed herein may independently be other ester moieties as described herein, e.g., C₂₋₅₀ esters including -C(O)-(CH₂)₀₋₄-(CF₂)₀₋₄-CF₃, including -C(O)-CF₃, -C(O)-C₂₋₂₉ optionally substituted alkyl, -C(O)-CH₂-C₂₋₂₈ optionally substituted alkenyl, -C(O)-CH₂-C₂₋₂₈ optionally substituted alkynyl, -C(O)-(CH₂)₀₋₆-optionally substituted phenyl, or -C(O)-(CH₂)₀₋₆-optionally substituted heterocycle or other organic moieties as disclosed herein or in the cited references.

Typical substituents for these organic moieties are as described herein, including one, two, three or more independently selected -O-, =O, optionally protected hydroxyl, -S-, optionally protected thiol, -NH-, optionally protected -NH₂, optionally protected -C(O)OH, -C(O)-NH-, -C(OFNH₂, -NH₂-C(O)-H, -NH₂-C(O)-C₀₋₄H₁₋₉, -NH₂-C(O)-O-C₀₋₄H₁₋₉, -CN, -NO₂, -N₃ or halogen. Reactive groups are protected as needed, e.g., =O would usually be protected in the LiCR reaction that is used to generate compound 1 in scheme 1 below.

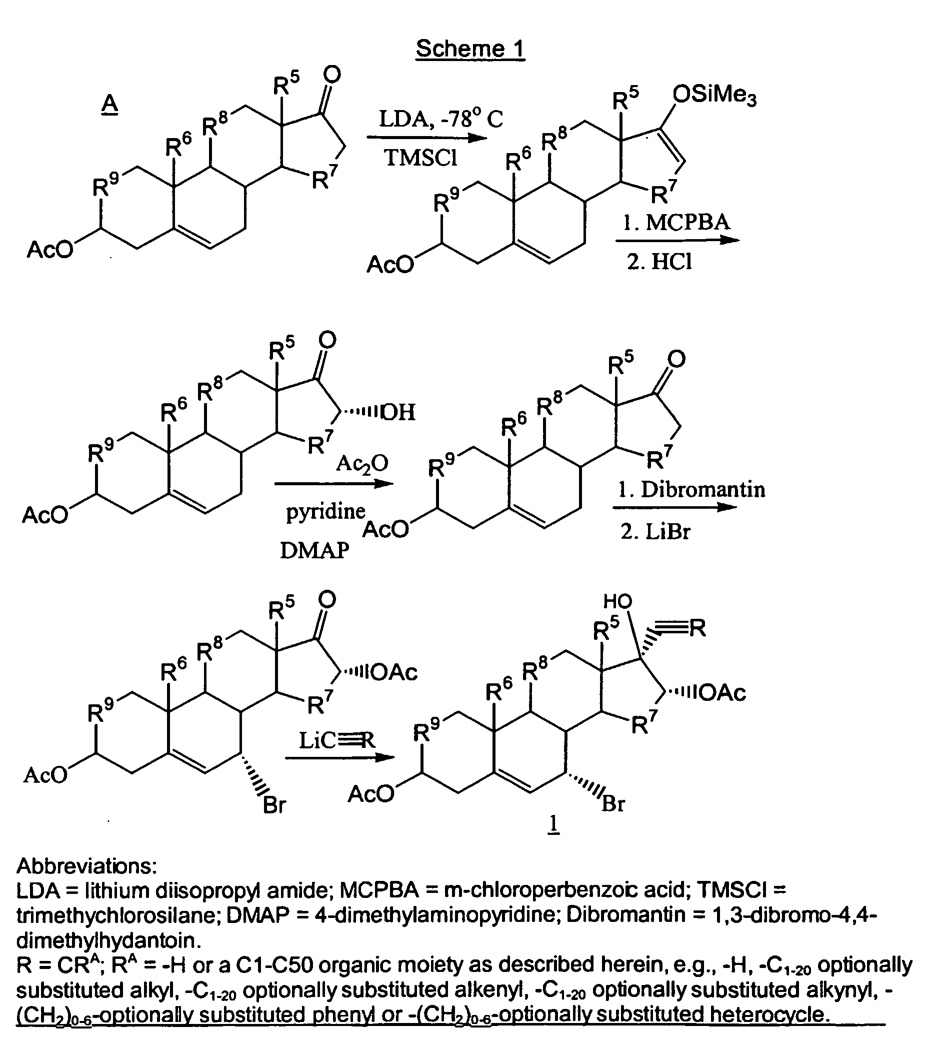

Scheme 2. Compounds of formula 2 are prepared from structure A compounds shown in scheme 1 using the last two steps of Scheme 1: (1) dibromantin, (2) LiBr, (3) Li-C-R, where R is CR^{A} and R^{A} is -H or -C₁₋₁₂ optionally substituted alkyl. When R⁷, R⁸ and R⁹ are all -CH₂-, H at the 9 and 14 positions are in the α-configuration and H at the 8 position is in the β-configuration the first compound in scheme 1 is DHEA acetate. Typical substituents for the R^{A} alkyl moiety includes one, two or more independently selected -O-, optionally protected =O, optionally protected hydroxyl, -S-, optionally protected thiol, -NH-, optionally protected -NH₂, optionally protected -C(O)OH, -C(O)-NH-, -C(O)-NH₂, -NH₂-C(O)-H, - NH2-C(O)-C₀₋₄H₁₋₉, -NH₂-C(O)-O-C₀₋₄H₁₋₉, -CN, -NO₂, -N₃ or halogen.

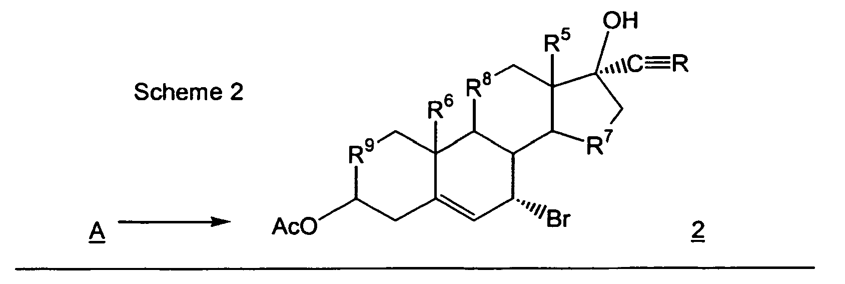

Scheme 3. The allylic bromination at C-7 is done as in Scheme 1. R and R^{A} are as defined in Schemes 1 and 2.

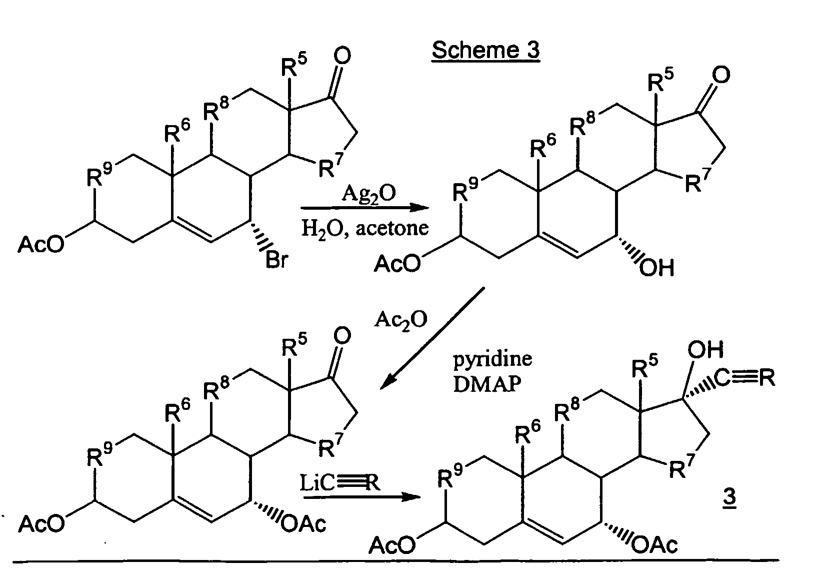

Scheme 4. The addition of lithium reagent (lithium acetylide when R is -CH) to the 17-position >C=O in the presence of the bromide at C-16 results in epoxide formation or in a pinacol rearrangement. Alternatively, compounds without of structure 3 can be dehydrated by mild acid catalysis to form compounds of formula 4 by treatment of the alkene with Br₂, H₂O. R and R^{A} are as defined in Schemes 1 and 2.

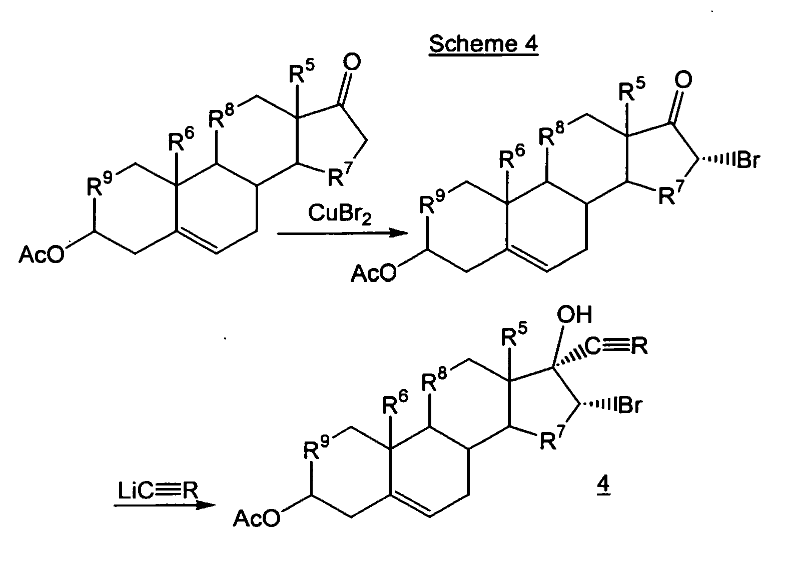

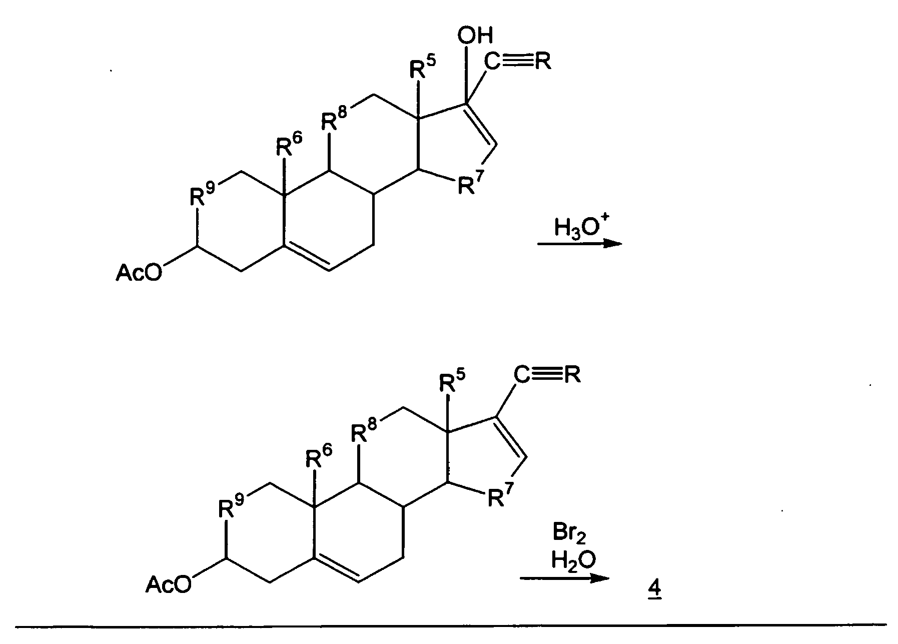

Scheme 5. Sodium borohydride gives a mixture of epimers at C-7, which may be separated by standard methods, e.g., HPLC, TLC or column chromatography. To obtain the pure 7α-OH compound, allylic bromination followed by hydrolysis is accomplished as described in Schemes 1 and 3.

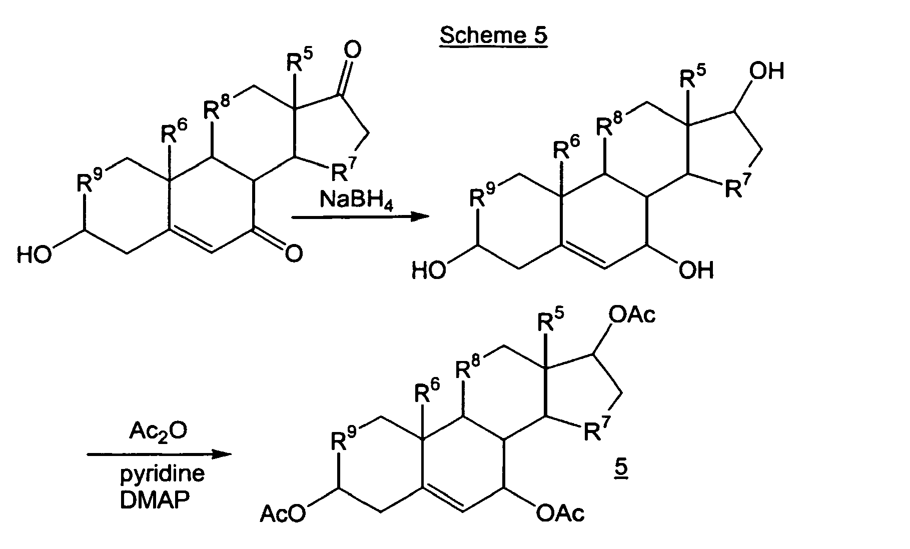

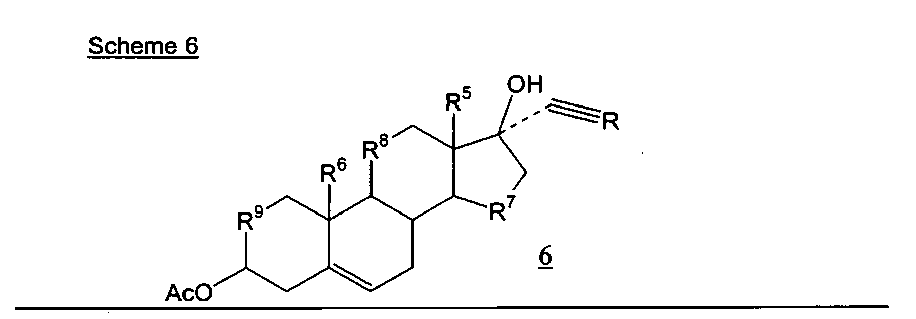

Scheme 6. Formula 6 compounds are prepared by treatment of the acetate with lithium acetylide as in Schemes 1, 2, 3 or 4. R and R^{A} are as defined in Schemes 1 and 2.

Scheme 7. Formula 7 compounds are prepared from the 3-acetate with reagents described in Schemes 1 and 4. R and R^{A} are as defined in Schemes 1 and 2.

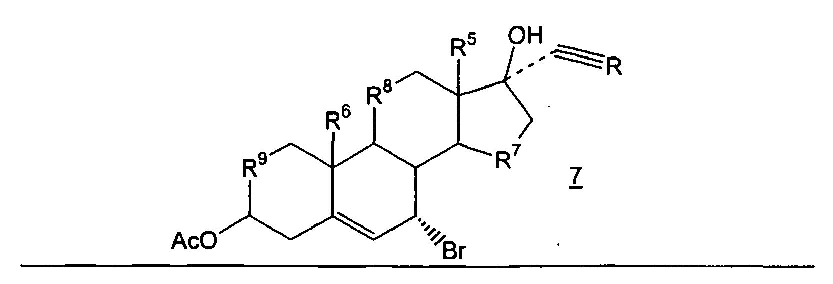

Scheme 8. Formula 8 compounds are prepared from the formula A compounds by sodium borohydride reduction at C-17 followed by acetylation.

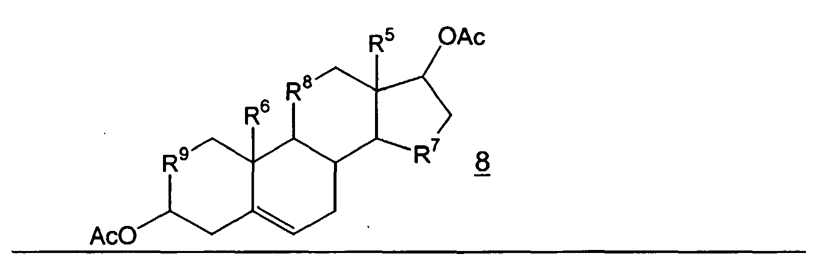

Scheme 9. The starting material is made using reactions described in Schemes 1 and 3.

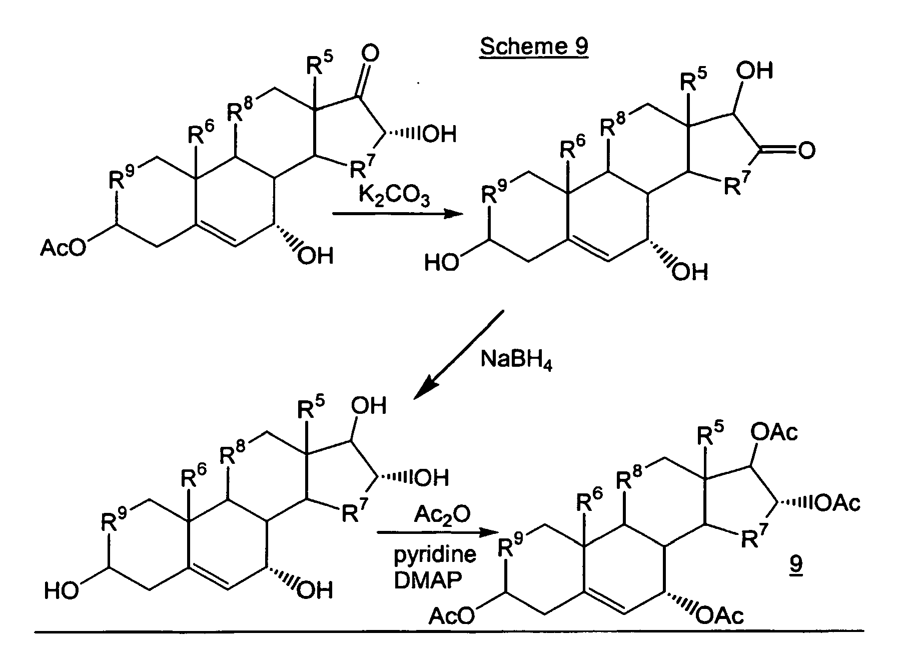

Scheme 10. Reduction and acetylation at C-3 and hydrolysis and oxidation at C-17 will allow formula 10a and 10b compounds to undergo functionalization as shown in Schemes 1-9 at C-3, C-16 and C-17. The 7-oxo acetate can be substituted for the formula A compound 3-acetate and functionalization at C-3, C-16 and C-17 is achieved similarly for 7-oxo compounds using the reactions shown in schemes 1-9.

Treatment of 10a with LDA, followed by alkylation of the enolate allows introduction of side chains such as R¹⁰, whiich may be, e.g., C1-C20 alkyl (methyl, ethyl), C1-C20 alkenyl (CH₂=CH-(CH₂)₀₋₆-), benzyl, -(CH₂)₁₋₄-O-(CH₂)₀₋₄-CH3.

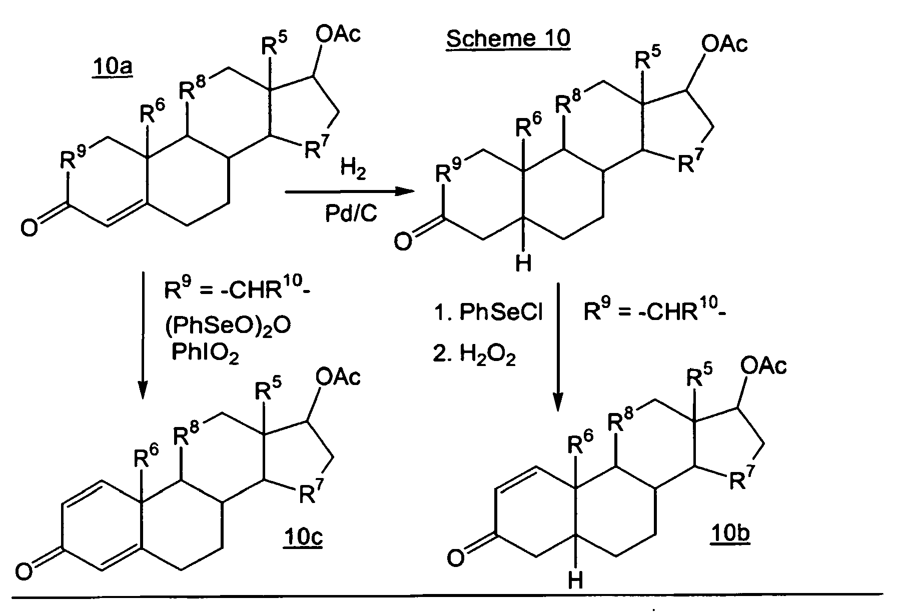

Schemes 1-9 show the introduction of the hydroxyl function at the positions shown. Methods to convert hydroxyl to other functional groups are accomplished essentially as described, e.g., in the references cited herein. For example, esters, of formula 1 - 10c compounds, such as -O-C(O)-R^{B} where R^{B} is a C₁₋₅₀ organic moiety, are prepared from the steroid alcohol by treatment with the appropriate acid anhydride or acid chloride (R^{B}-C(O)-Cl) to form any desired ester. Ethers, such as - O-R^{B}, are prepared from alcohols by formation of the alkaline metal alkoxide (Na⁺ or K⁺) followed by treatment with a primary or secondary iodide (R^{B}-I). Thionoesters, R^{B}-C(S)-O-, are prepared by treating the R^{B}-C(O)-O- ester with Lawesson's reagent.

Sulfates, NaO-S(O)(O)-O-, R^{B}-O-S(O)(O)-O-, e.g., CH₃(CH₂)₀₋₁₈-S(O)(O)-O-, are prepared by treatment of alcohols with chlorosulfonic acid followed by NaOH or alternatively by oxidation of sulfites using KMnO₄. If the alkyl (e.g., methyl) ester is desired alkylchloro-sulfonate (methylchloro-sulfonate) can be used. Sulfites HO-S(O)-O- and ammonium salts NH₄ O-S(O)-O, or R^{B} O-S(O)-O- esters (e.g., CH₃ O-S(O)-O-) are prepared by standard methods. The ammonium salts are prepared by treatment of alcohols with ammonia and sulfur dioxide. The esters such as alkyl, alkenyl and alkynyl esters (e.g., methyl ester) are obtained when alcohols are treated with alkylchlorosulfite (e.g., methycholorosulfite), alkenylchlorosulfite or alkynylchlorosulfite in the presence of a suitable base such as triethylamine. Phosphoesters, R^{B}O-P(OR^{PR})(O)-O- are prepared by treating the alcohol with diethylchlorophosphate in the presence of Na₂CO₃. Alternatively, if the alcohol is treated with phosphoric acid diesters in the presence of triphenylphospine (PPh₃) and diethylazodicaboxylate (DEAD) the corresponding triesters are formed with inversion (Mitsunobu reaction).

Phosphothioesters, R^{B}O-P(SR^{PR})(O)-O- are generated by treatment of alcohols with the monothio analog of diethylchlorophosphate as described for phosphoesters yielding the phosphothioesters. Carbonates, R^{B}O-C(O)-O- are generated from the corresponding steroid alcohol using the chloroformate (R^{B}-C(O)-CI), e.g., C₁₋₂₀ alkyl, alkenyl or alkynyl chloroformates (e.g. CH₃(CH₂)₀₋₅-C(O)Cl). Carbamates, R^{B}-NH-C(O)-O- are made from steroid alcohols by treatment with isocyanates (R^{B}N=C=O) or NaOCN in the presence of trifluroroacetic acid. Aminoacid esters, ZNX-CHY-C(O)-O- are generated by coupling the steroid alcohol with the acid chloride of the N-protected amino acid.

Oxidation of hydroxyl groups that are linked to the steroid nucleus is used to obtain ketones and related functionalities. For example, conversion of alcohols to ketones can be achieved using a variety of oxidizing agents such as CrO₃ in AcOH, or pyridinium cholorchromate, pyridinium dichromate or oxalyl chloride with triethylamine (Swern oxidation). Thioketones (=S) are prepared by treating ketones with Lawesson's reagent (2,4-bis(4-methoxyphenylyl,3,2,4-dithiadiphosphetane-2,4-disulfide; commercially available from Aldrich). Thioacetals, -C(SR^{B})(SR^{B})-, are prepared from ketones (-C(O)-) by treatment with R^{B}-SH thiols under acid catalysis conditions (e.g., HCl). Phosphonoesters, RO-P(OR^{PR})(O)-, are generated by addition of the phosphorus acid diester to ketones in the presence of KF to yield hydroxy phosphonoesters. One may optionally remove the hydroxy group using a dehydration and hydrogenation sequence.

Substitution of hydroxyl groups is used to generate a number of functionalities. For example, thiols, -SH, are prepared from alcohols by conversion of the alcohol with inversion to the bromide using PBr₃. Treatment of the bromide with thiourea followed by NaOH gives the thiol. Thioethers, R^{B}-S-, are prepared from thiols by treatment with NaOH and the required halide, e.g., alkyl halide. Alternatively, alcohol derivatives like tosylates or mesylates can be displaced by thiolate anions, R^{B}-S⁻, to yield the thioether. Thioesters, R-C(O)-S-, are prepared by treating the tosylate (mesylate) of the alcohol with the sodium salt of the thioacid.

Substitution of hydroxyl groups can be used to generate both esters, R^{B}O-C(O)-, and amides, NHR^{B}-C(O)-, linked to the steroid at carbon atoms. For amides and amines, R^{B} is -H, a protecting group or a C₁₋₅₀ organic moiety. These are synthesized from the steroid bromide with inversion by displacement with NaCN. The cyanide group can be hydrolyzed to the amide or the acid. The acid is esterified or treated by standard peptide coupling reactions with an O-protected amino acid in the presence of a suitable carboxyl activating agents such as dicyclohexylcarbodiimide (DCC) to form steroid -C(O)-NH-CHY-C(O)-OR, where Y is the side chain of an amino acid or a C1-C10 organic moiety and R is a protecting group (or hydrogen when deprotected).

Amines and derivatives of amines, e.g., R^{B}NH-, R^{B}-C(O)NH-, R^{B}OC(O)-NH- or R^{B}O-C(O)-CHR^{B}-NH- linked to steroid carbon atoms, are typically prepared by standard methods. For example, amines (NH₂-steroid) are generally prepared using the Hoffmann rearrangement (Br₂, NaOH) from the amide (NH₂-C(O)-steroid) or the Curtius rearrangement (NaN₃) from the acid chloride of the steroid. The R^{B} substituent can subsequently be introduced by alkylation. Steroid alcohols can be used as starting materials under standard Mitsunobu conditions (PPh₃, DEAD) to yield N-Boc sulfonamides using N-(t-butoxycarbonyl)-p-toluenesulfonamide. One can selectively remove either protecting group. Treatment with trifluoroacetic acid affords the sulfonamide (R^{B}-S(O)(O)-NH-steroid). Alternatively, sodium napthalenide deprotects to give the N-Boc compound. Amines (NH₂-steroid) can be converted to amides (R^{B}NH-C(O)-steroid) using acyl chlorides (R^{B}-C(O)-Cl). Treatment with ethyl chloroformate gives the N-carbamate (R^{B}O-C(O)-NH-steroid). The amine (NH₂-steroid) can be alkylated with an α-bromoester (R^{B}-C(O)-CHY-NH₂) to yield the amio acid substituted steroid (R^{B}-O-C(O)-CHY-NH-steroid).

Where reactions such as substitutions give a product mixture, the desired intermediate is optionally separated from other products or at least partially enriched (e.g., enriched at least about 10-fold, usually at least about 50-100-fold) from other products before subsequent reactions are conducted. Substitution at steroid carbon atoms will generally proceed with greatest efficiency at the 3-position, which is relatively sterically unhindered and C-17 is generally somewhat less accessible than the C-3 position. The relative reactivities of the C-3, C-7, C-17 and C-16 positions allows one to use their reactivities to control the sequential introduction of different functional groups into the same steroid molecule. Also, groups, such as hydroxyl at more reactive positions, C-3 or C-17, may be sequentially protected or deprotected to allow introduction of functional groups at other positions, such as C-7 or C-16.

Polymers such as PEG are linked to the compounds essentially as described above. For example, PEG200 or PEG300 is linked to the steroid at the 3, 7, 16, 17 or other positions by an ether linkage (PEG-O-steroid) using a PEG alkoxide (PEG-ONa), to displace the steroid bromide. Alternatively, PEG-Br can be treated with the steroid alkoxide. Polyethylene glycol esters such as those described in U.S. patent 5681964 can also be prepared using a suitable formula 1 compound and the methods described therein. Monosaccharides or polysaccharides and oligonucleotides are linked to steroid hydroxyl groups using known methods, see e.g., U.S. patent 5627270.

Formula 1 steroid analogs that comprise one or more ring heteroatoms are synthesized according to the following methods.

Scheme 11. Formula 1 compounds that comprise two or three ring heteroatoms are prepared as shown in the schemes. In the scheme, X is -CH₂-, - NH-, -O-, or -S-; R⁴⁰ is -H or -Br; R⁴¹ is an organic moiety having about 12 carbon atoms or less, typically C1 - C8 optionally substituted alkyl (e.g., methyl, hydroxymethyl, ethyl, propyl) or C2 - C8 optionally substituted alkenyl having a single double bond (e.g., vinyl) with 1, 2, 3 or more indepenently selected substituents (e.g., -OH, -COOH, -O-) and with any substituents that comprise a functional group generally being protected. Preparation of compound 20 from 19 is accomplished using a glycol such as HOC(CH₃)₂C(CH₃)₂OH in acid (H⁺) (B.H. Lipshutz et al., Synth. Commun. 12: 267, 1982). The use of a bulky protecting group facilitates generation of a double bond at the 5-6 position over the 4-5 position.

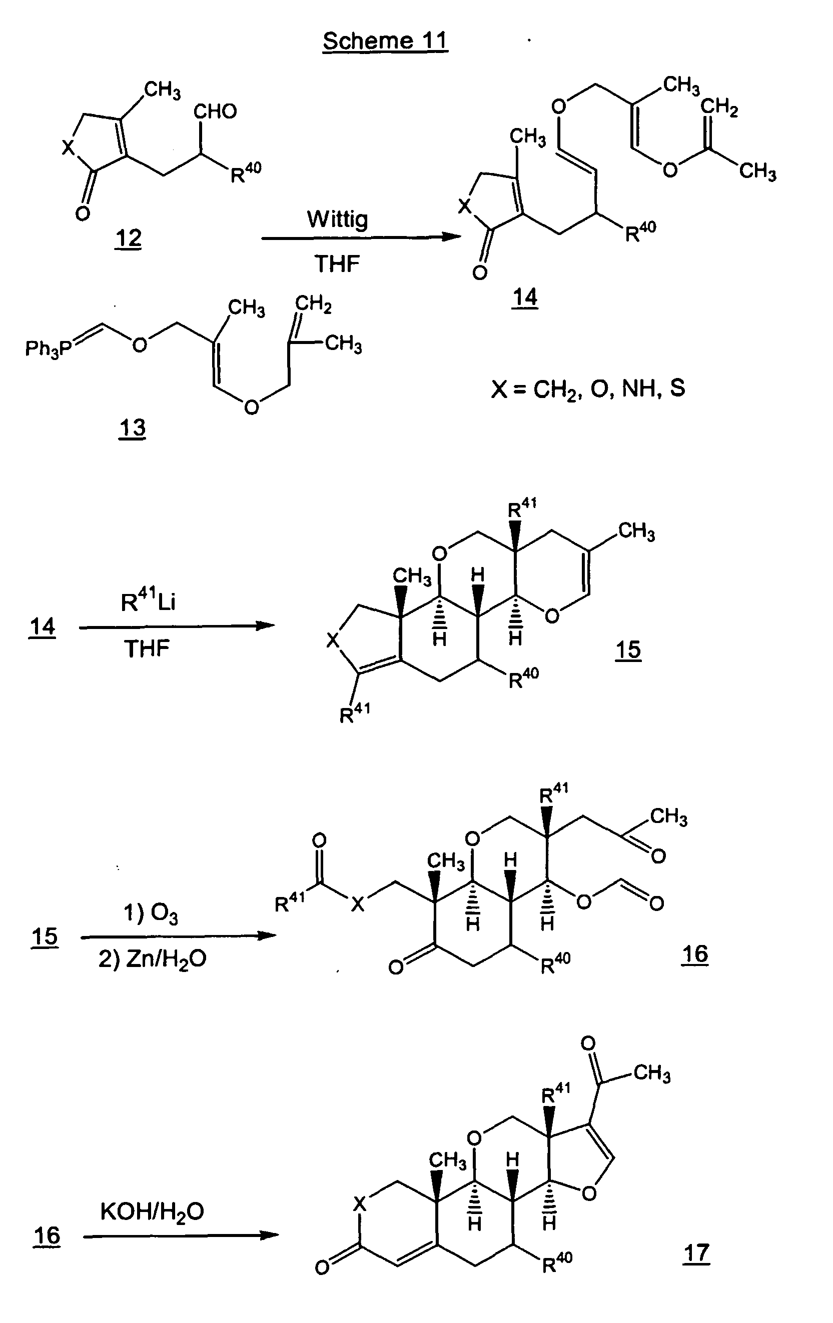

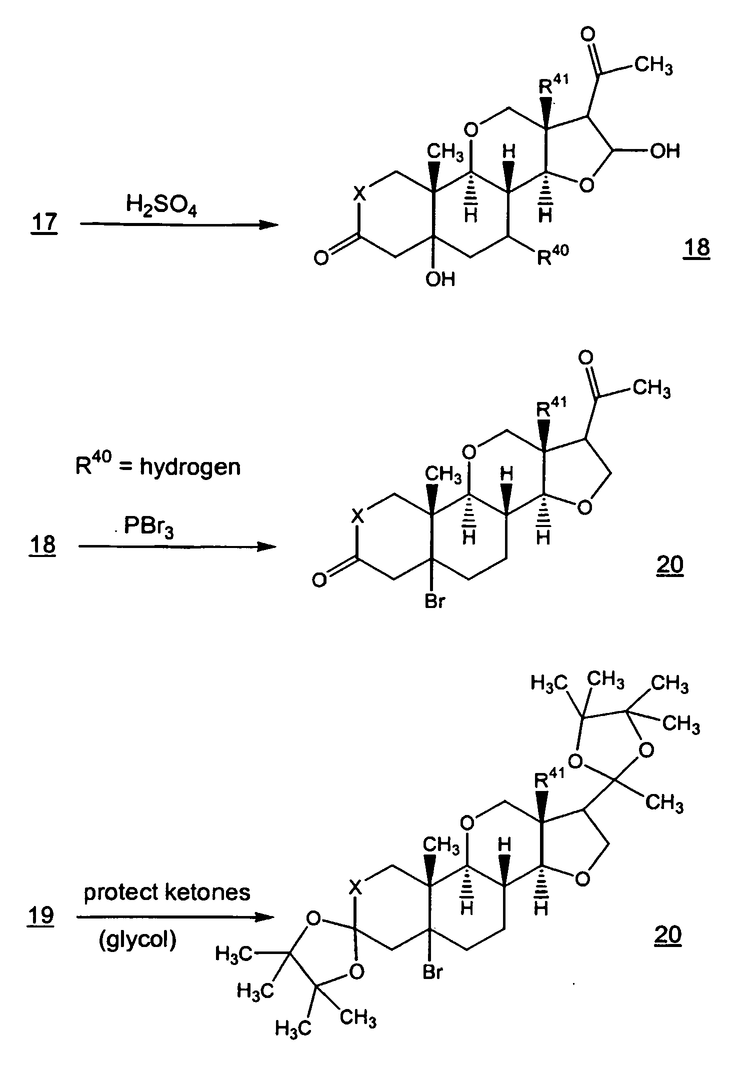

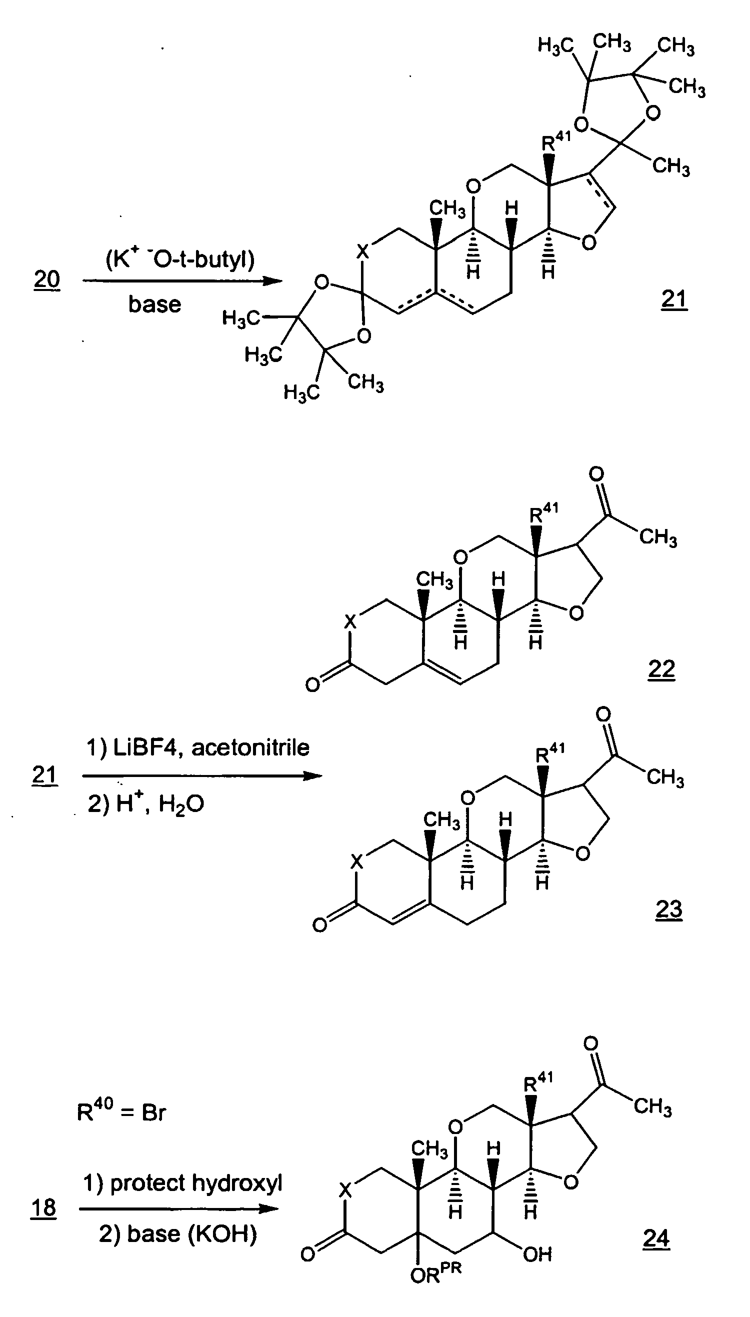

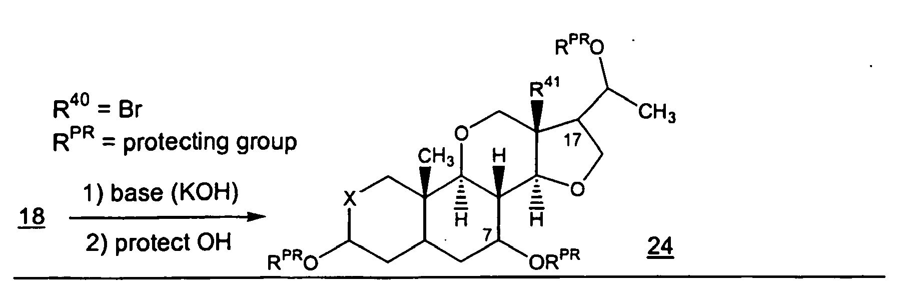

Schemes 12A-12D. Compounds of structure 12 are generated as shown in the schemes below. Most of the reactions are conducted essentially as described. See e.g., W.D. Langley, Org. Syn. I, 122, 1932 (compound 30); R. Ratcliffe et al., J. Org. Cham. 35: 4000, 1970 (compound 32); A.I. Meyers et al., J. Org. Chem. 39: 2787, 1974 (compound 33, 41); J.L. Isidor et al., J. Org. Chem. 38: 544, 1973 (compound 35); G. Wittig et al., Chem. Ber. 87: 1318, 1954 (compound 36); P.M. Pojer et al., Tet. Lett. 3067, 1976 (compound 38); A. Maercker, Org. React. 14: 270, 1965 (compound 37); E.J. Corey et al., Tet. Lett. 3269 1975 (compound 37); R.S. Tipson, J. Org. Chem. 9: 235, 1944 (compound 39); G.W. Kabalka, J. Org. Chem. 51: 2386, 1986; B.B. Carson et al., Org. Synth. 1: 179, 1941 (compound 43); H.J. Bestman et al., Justus Liebigs Ann. Chem. 693: 1321966 (compound 39); M. Miyano et al., J. Org. Chem. 37: 268, 1972 (compound 51); W.H. Glaze et al., J. Org. Chem. 33: 1987, 1968 (compound 52).

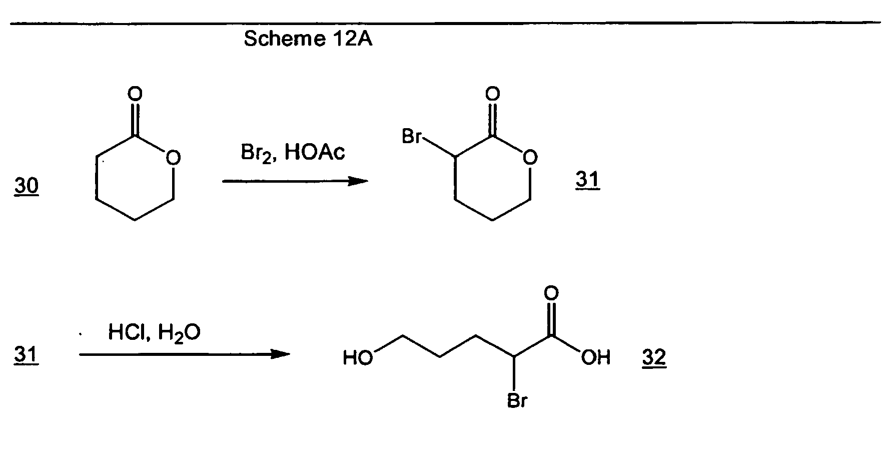

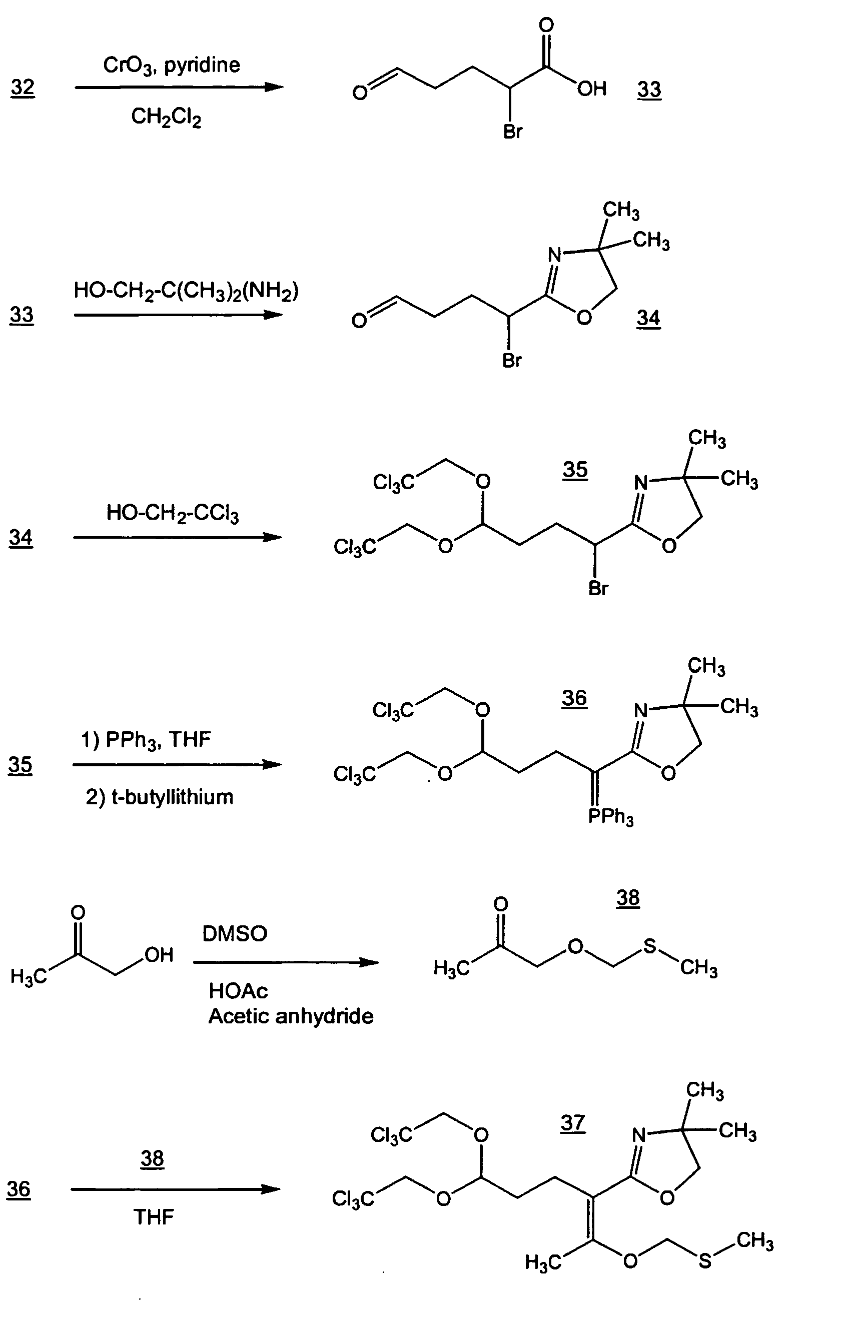

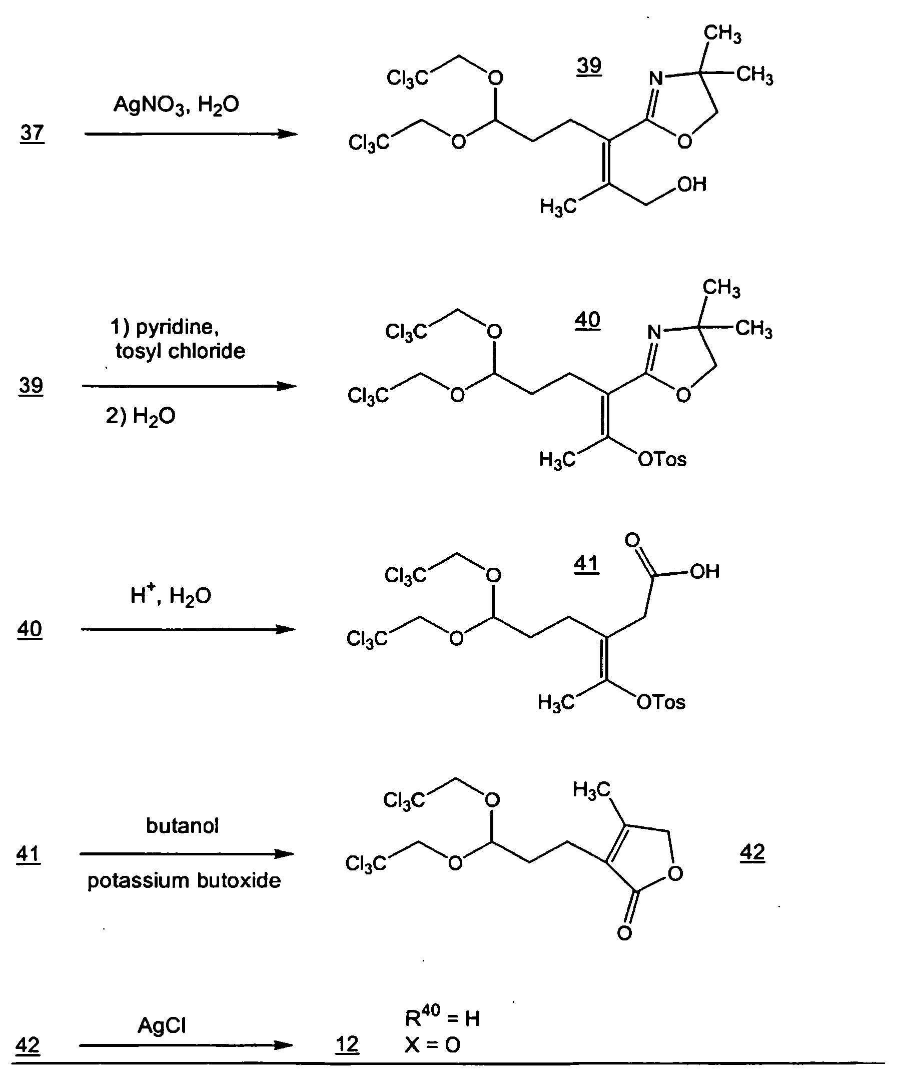

Compounds of structure 12 where X is NH, S and CH₂ are prepared as shown in schemes 12B, 12C and 12D respectively.

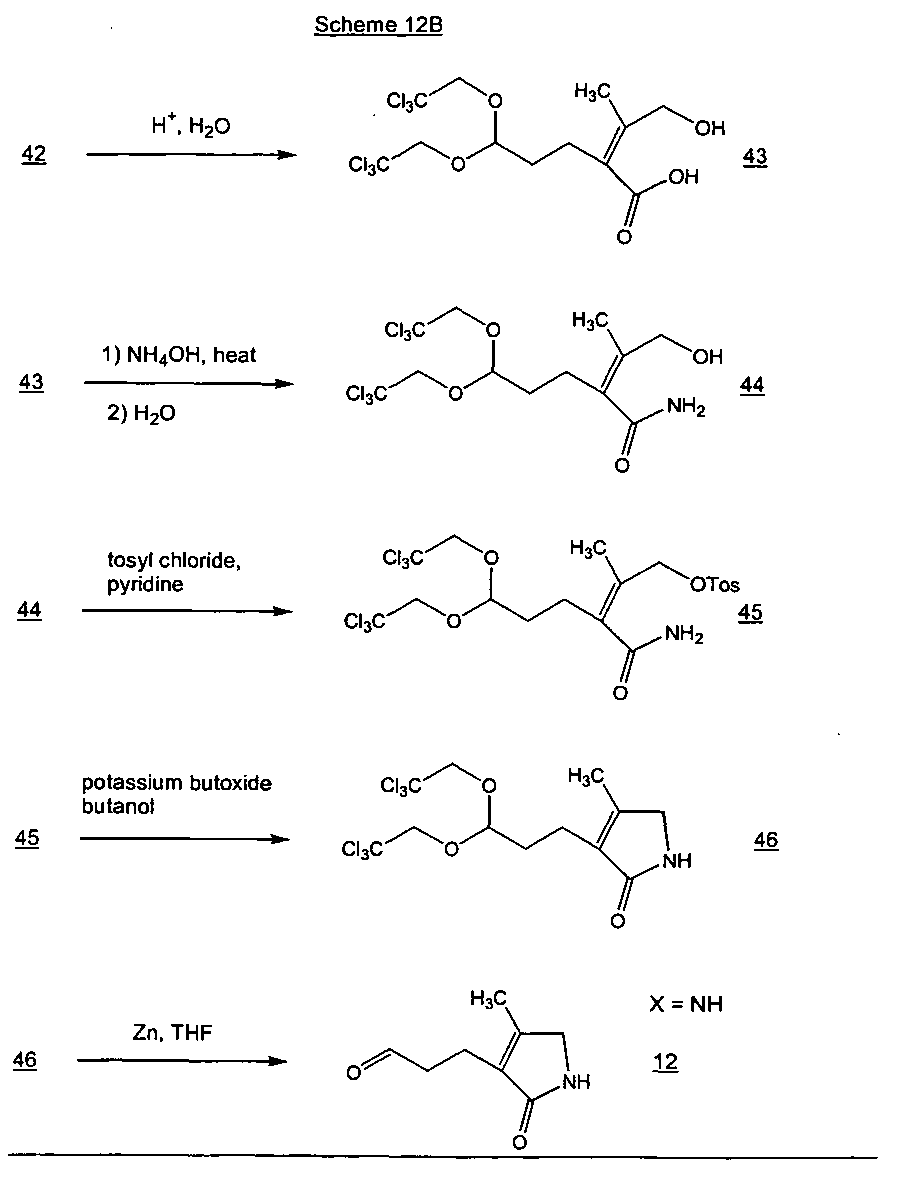

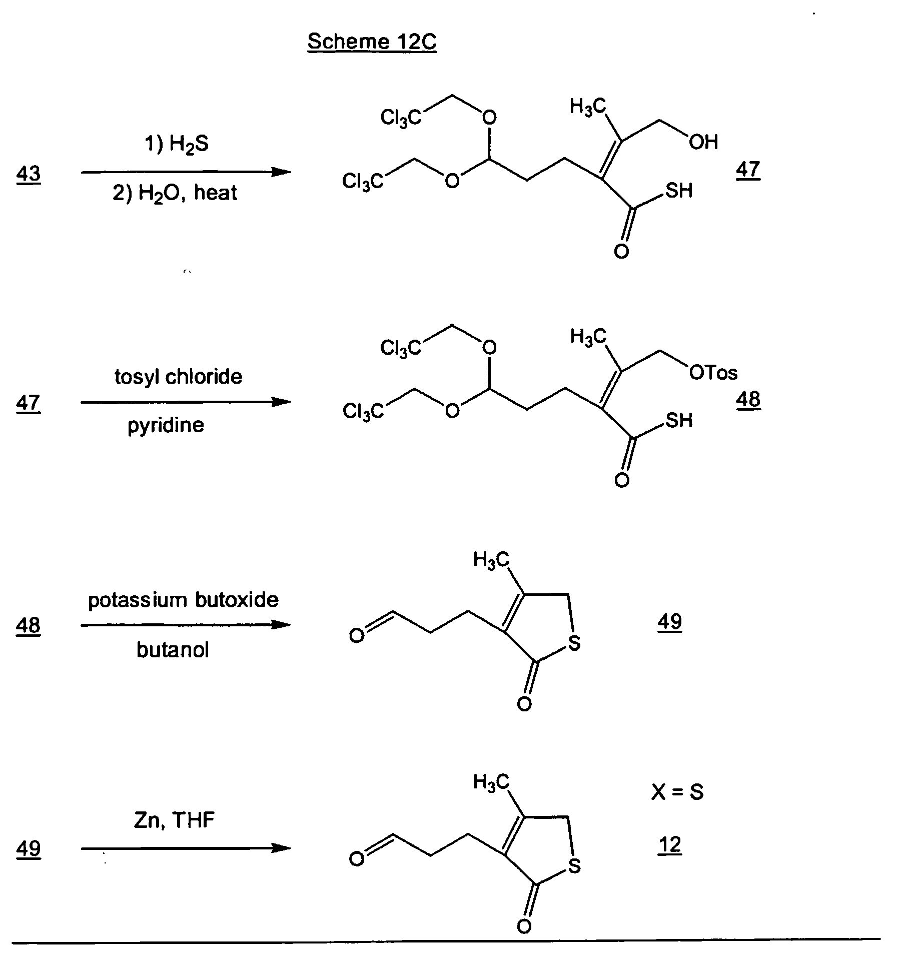

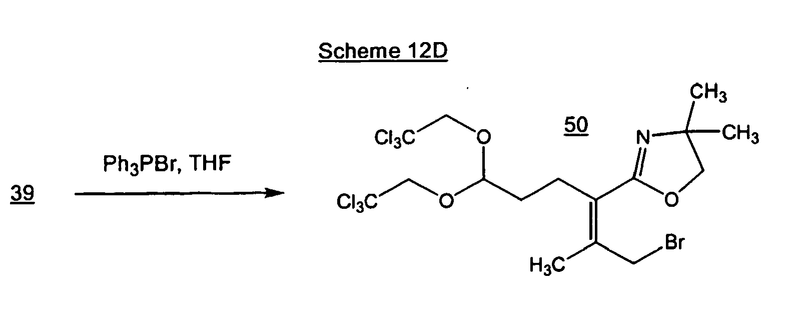

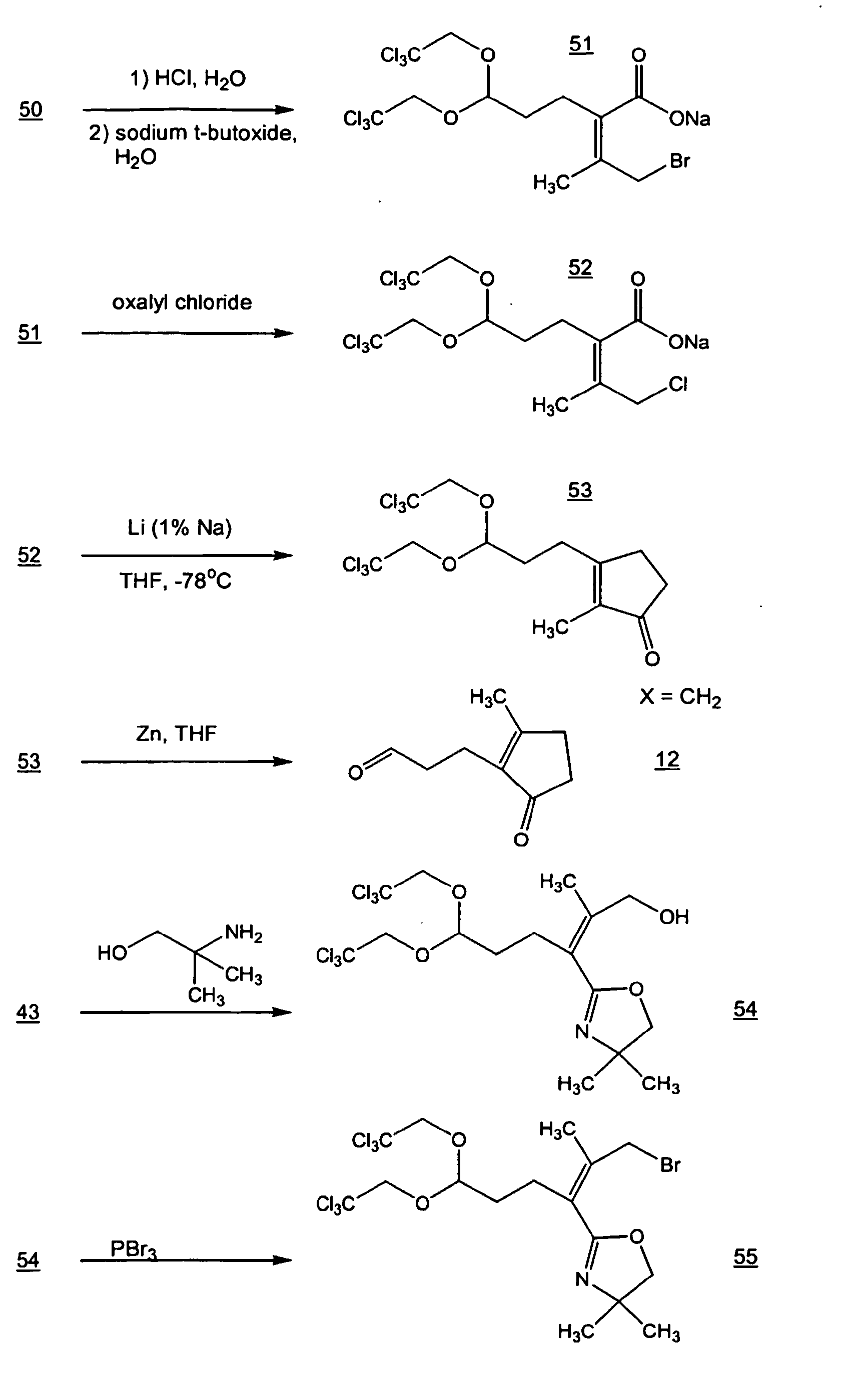

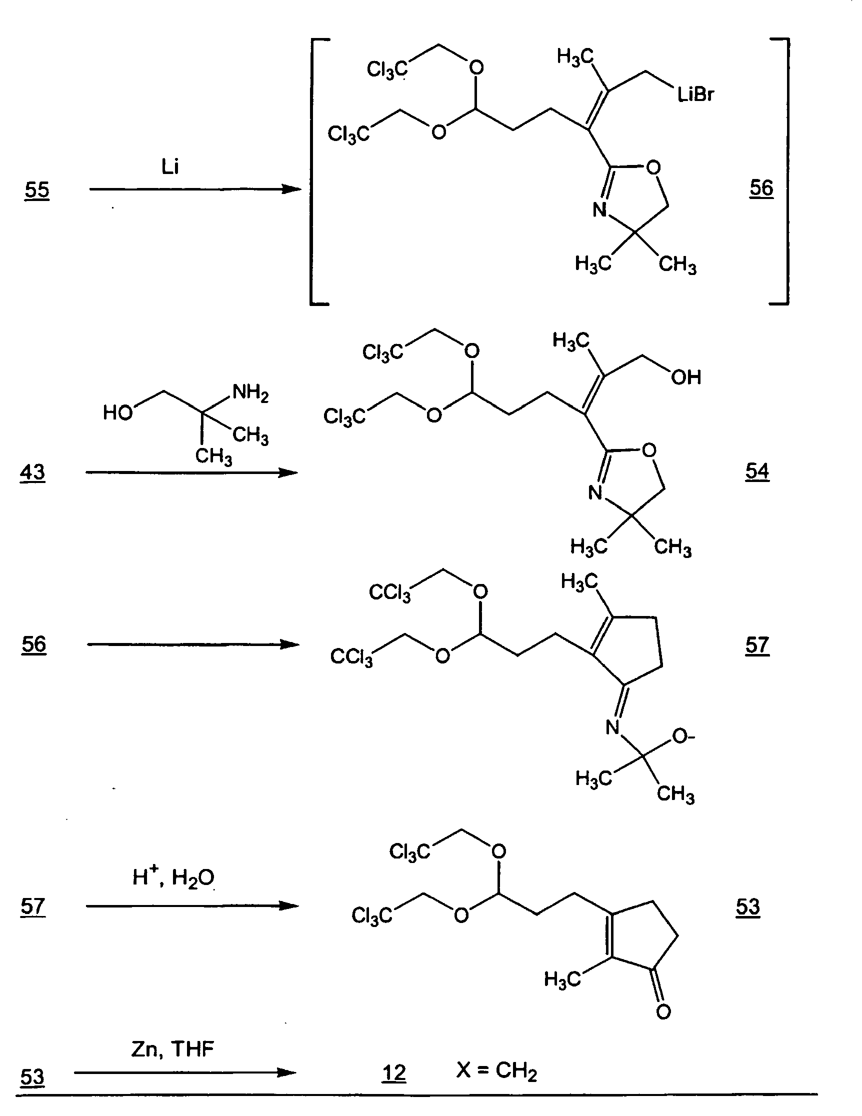

Scheme 13. The scheme and rections shown below are used to prepare the compound of structure 13 and related compounds that are used to introduce oxygen, carbon, nitrogen or sulfur into the R⁷ and R⁸ positions of formula 1 compounds. The reactant in the preparation of compound 63, 3-chloro-2-methylpropene (reg. No. 563-47-3), is available commercially (e.g., Aldrich, Fluka).

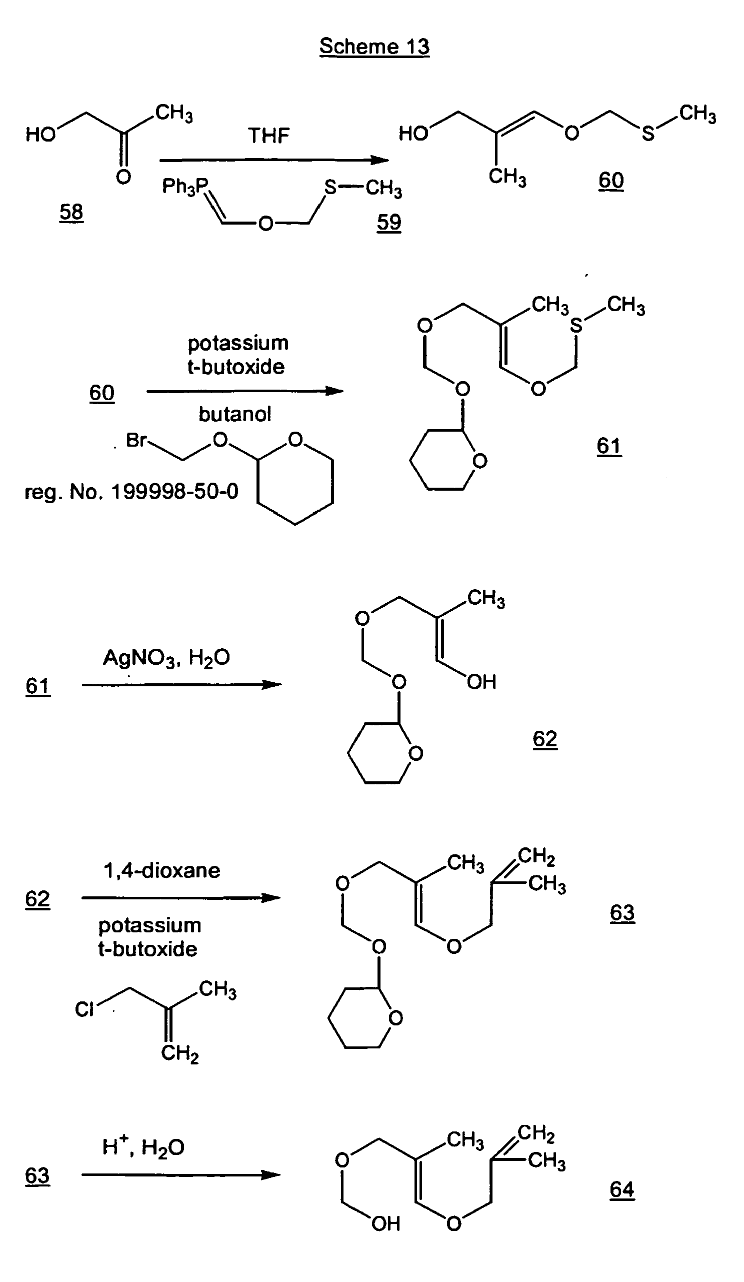

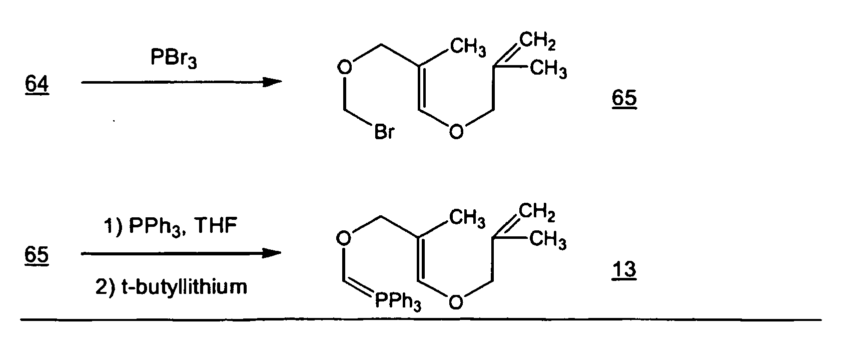

Compound 59 and analogs of compound 59 where CH₂, S or NH CH₂ replaces oxygen are preapred as shown in the following reactions. Conditions suitable for conversion of compound 106 to 107 have been described (T. Hamada et al., Heterocycles 12: 647, 1979; T. Hamada et al., J. Am. Chem. Soc. 108: 140, 1986).

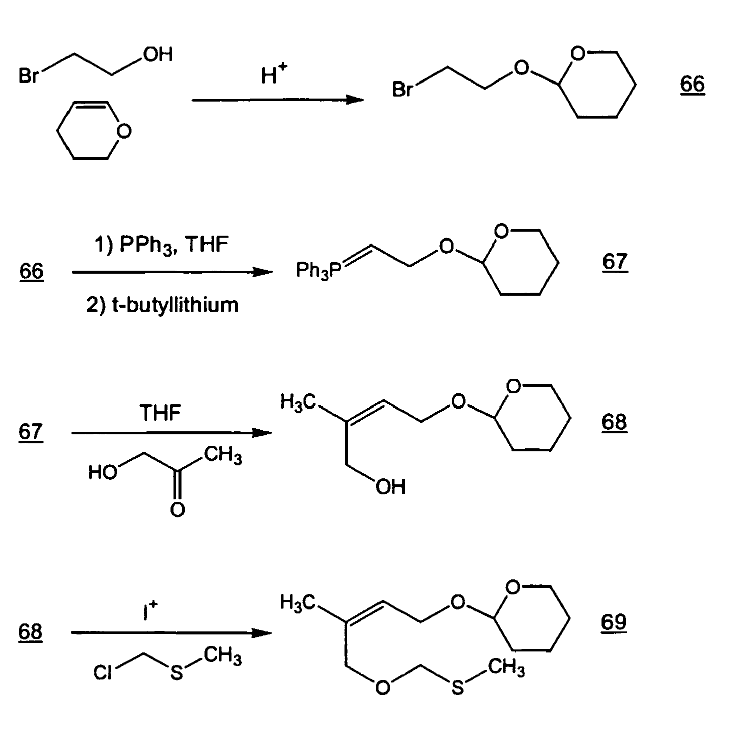

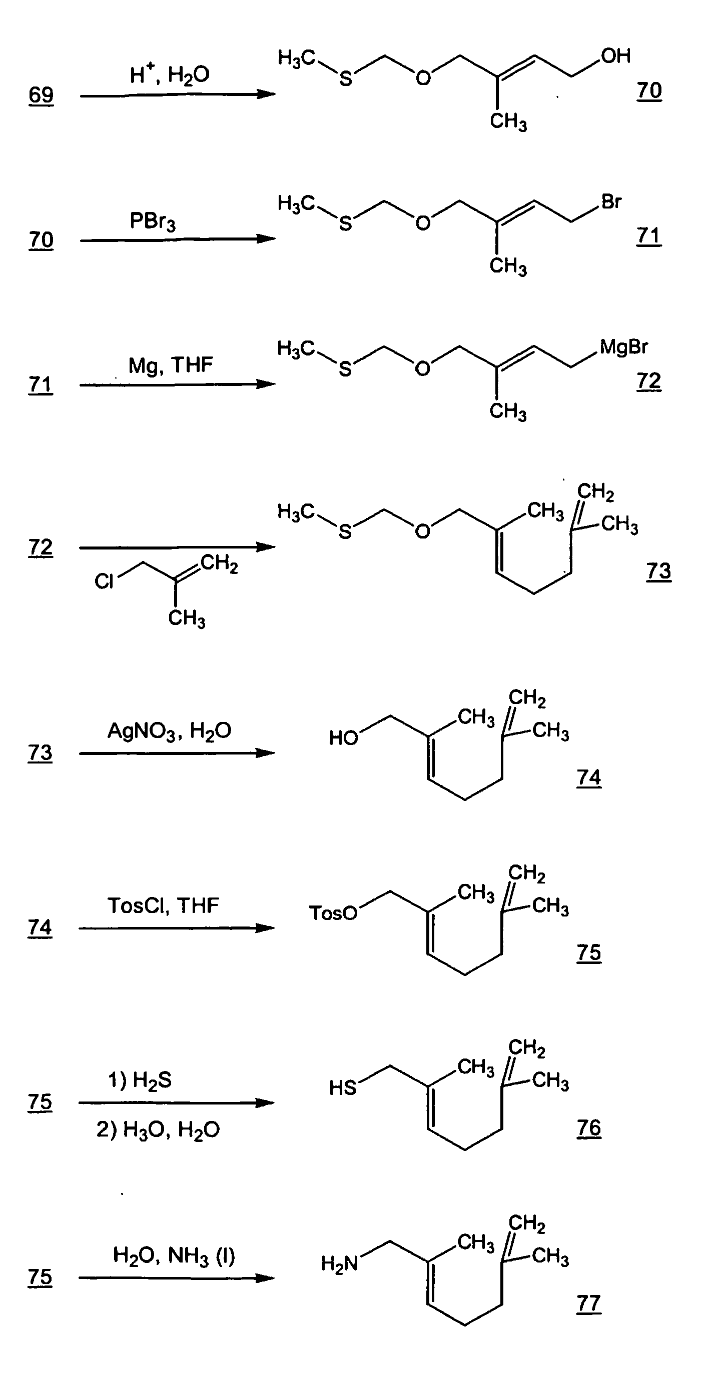

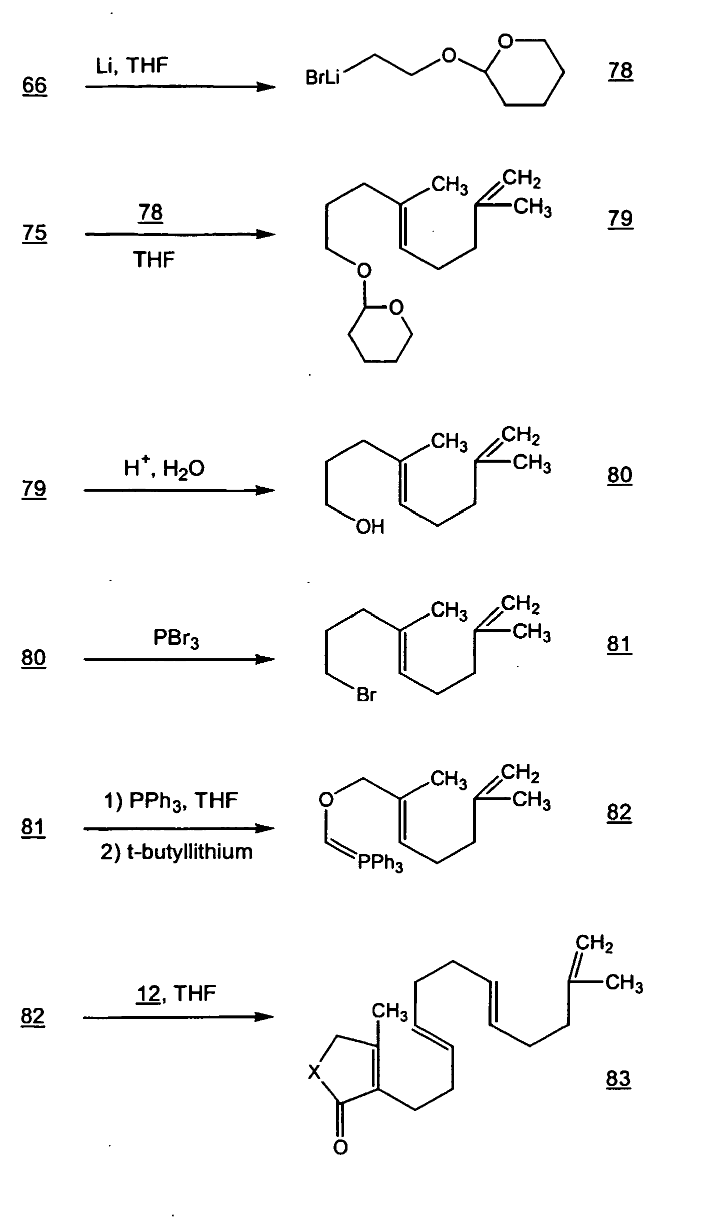

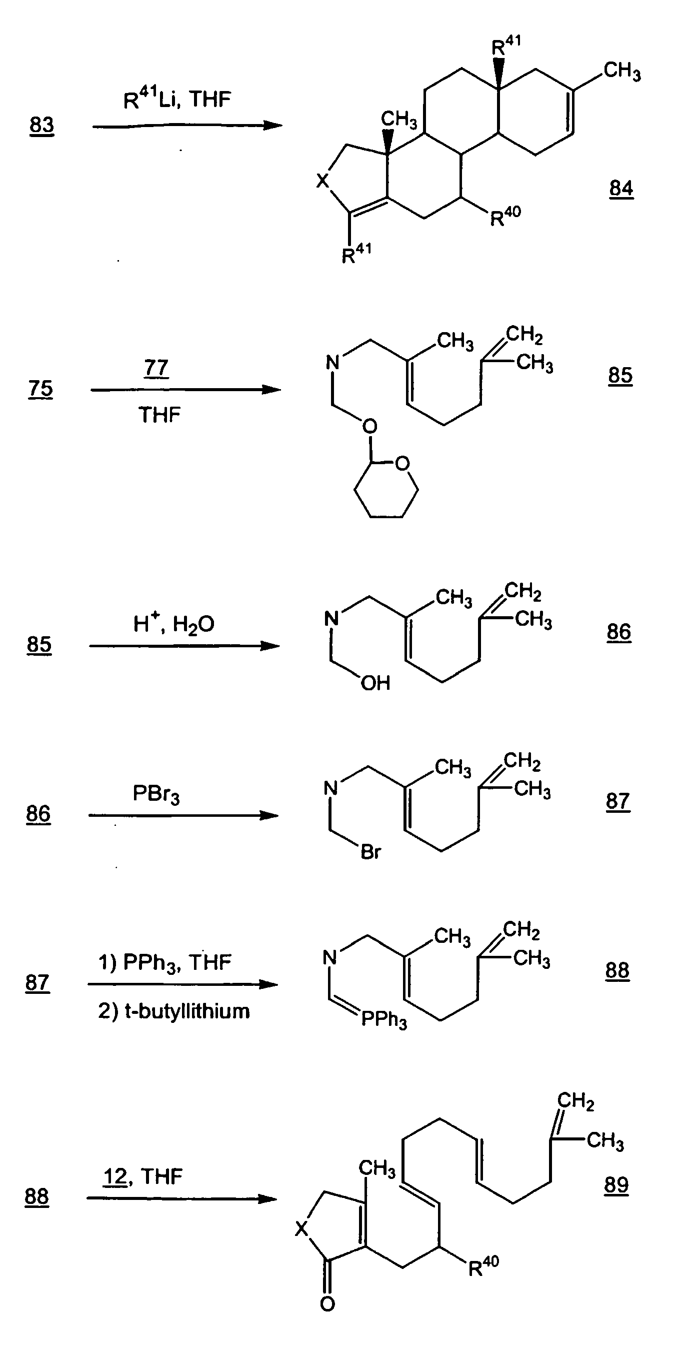

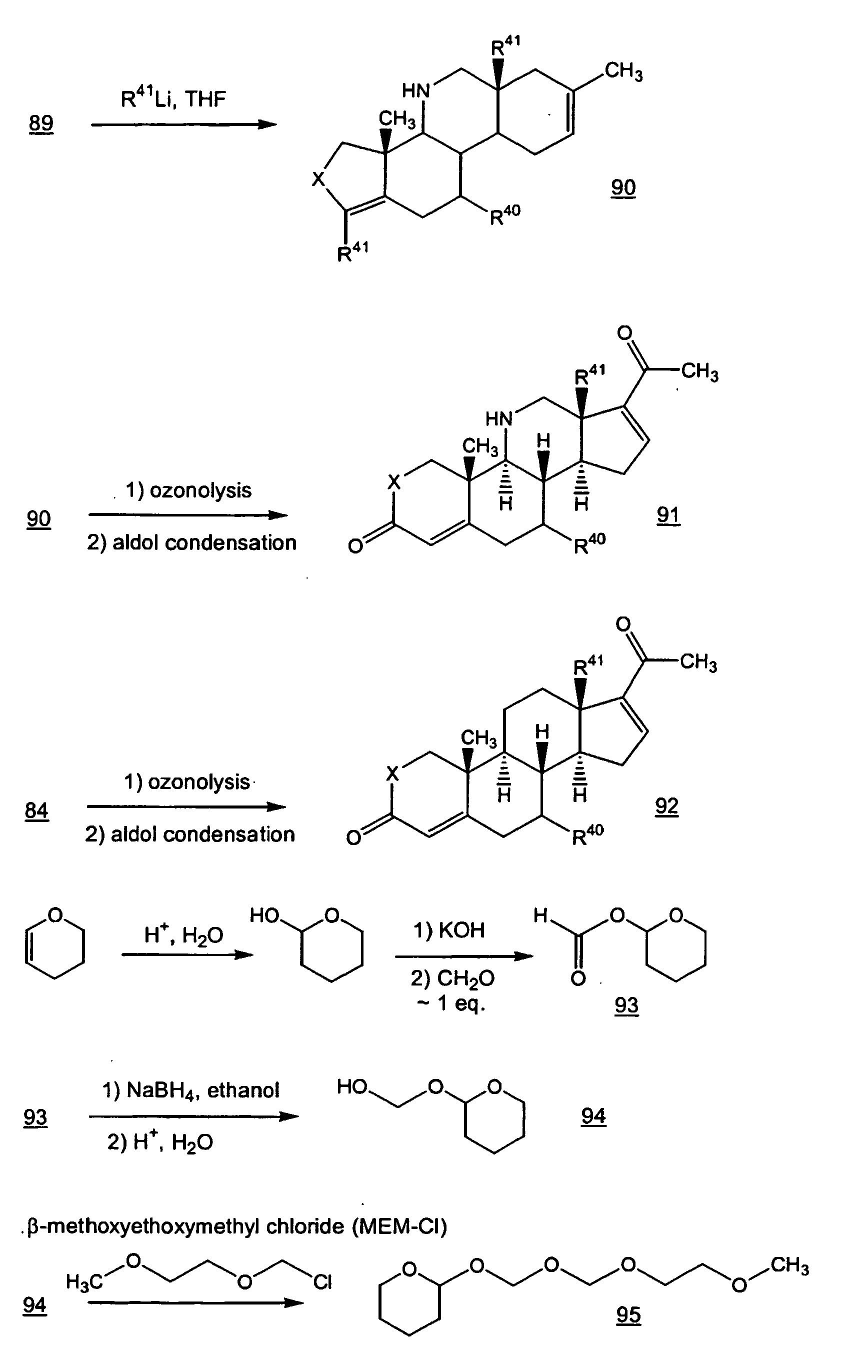

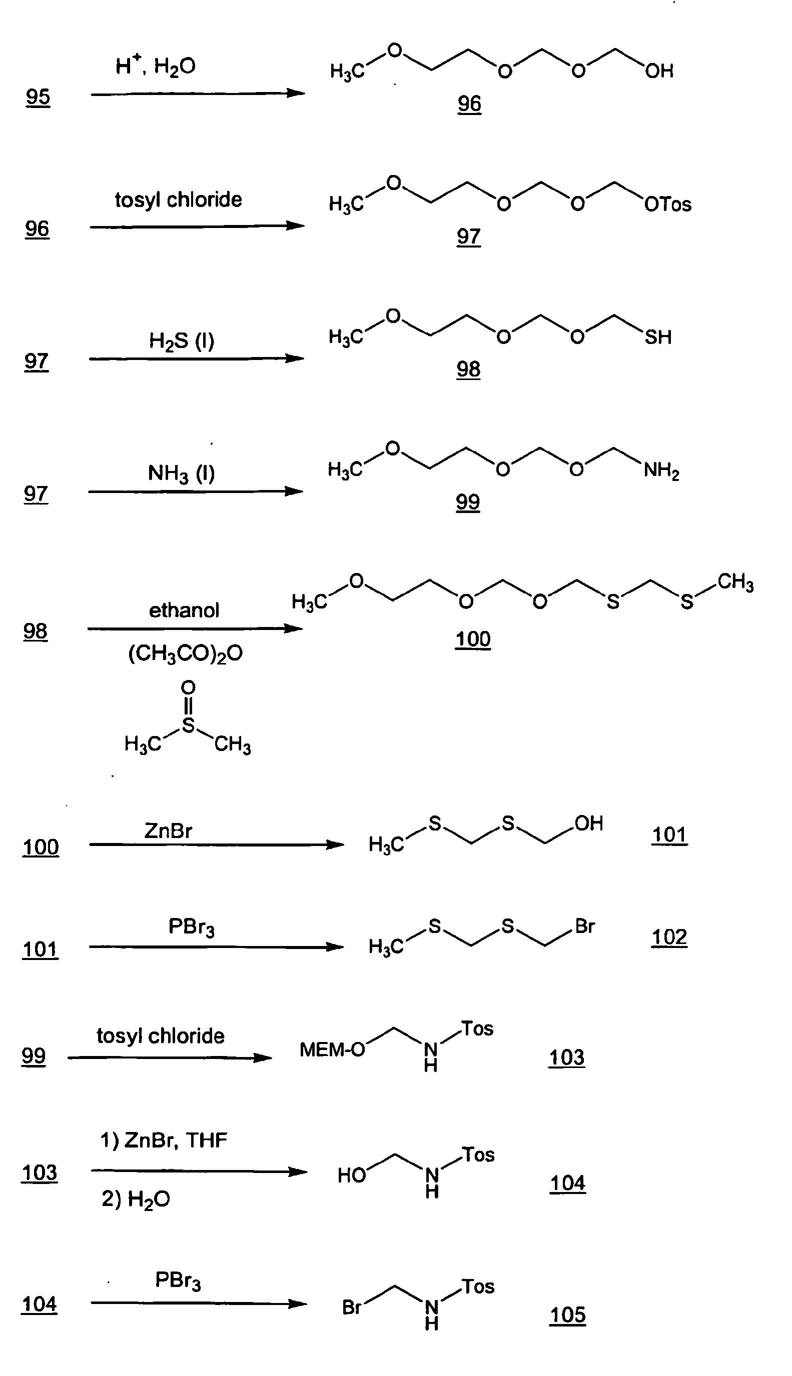

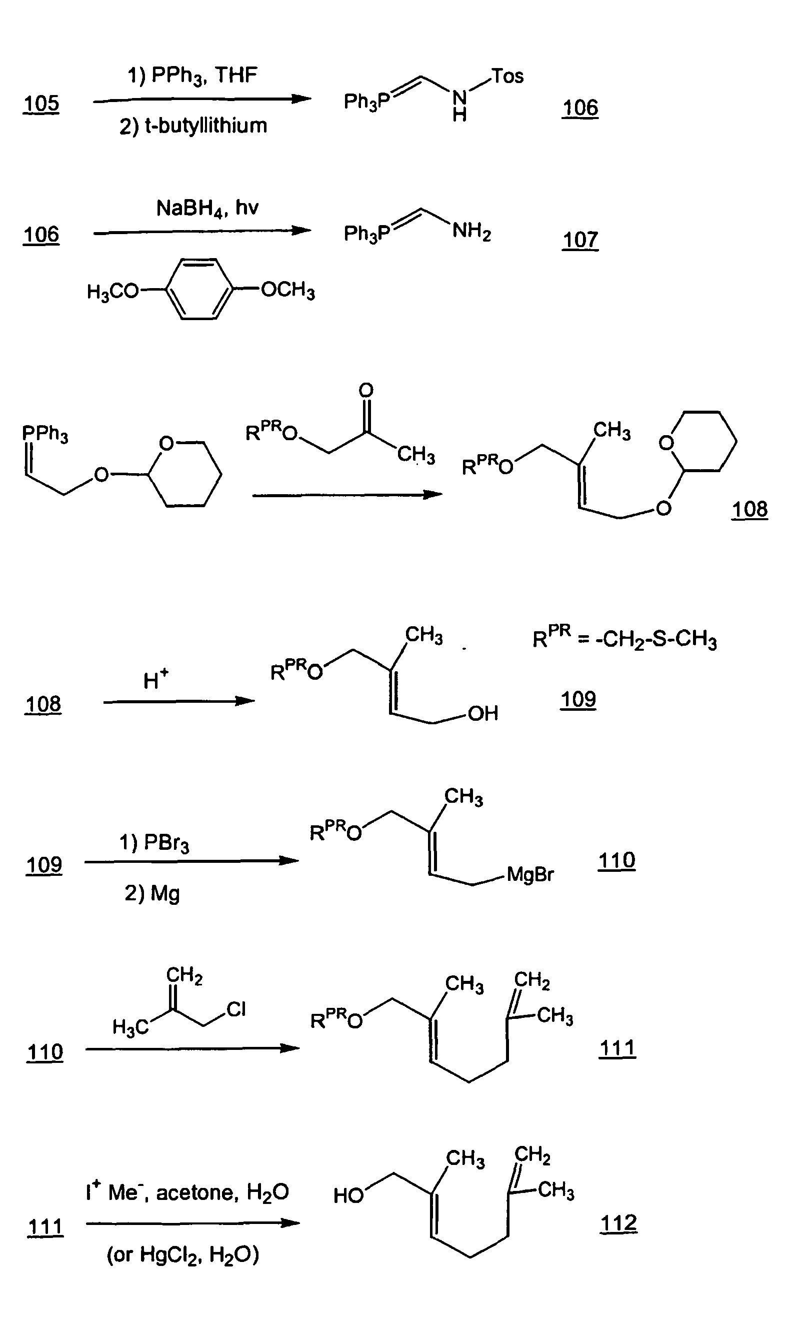

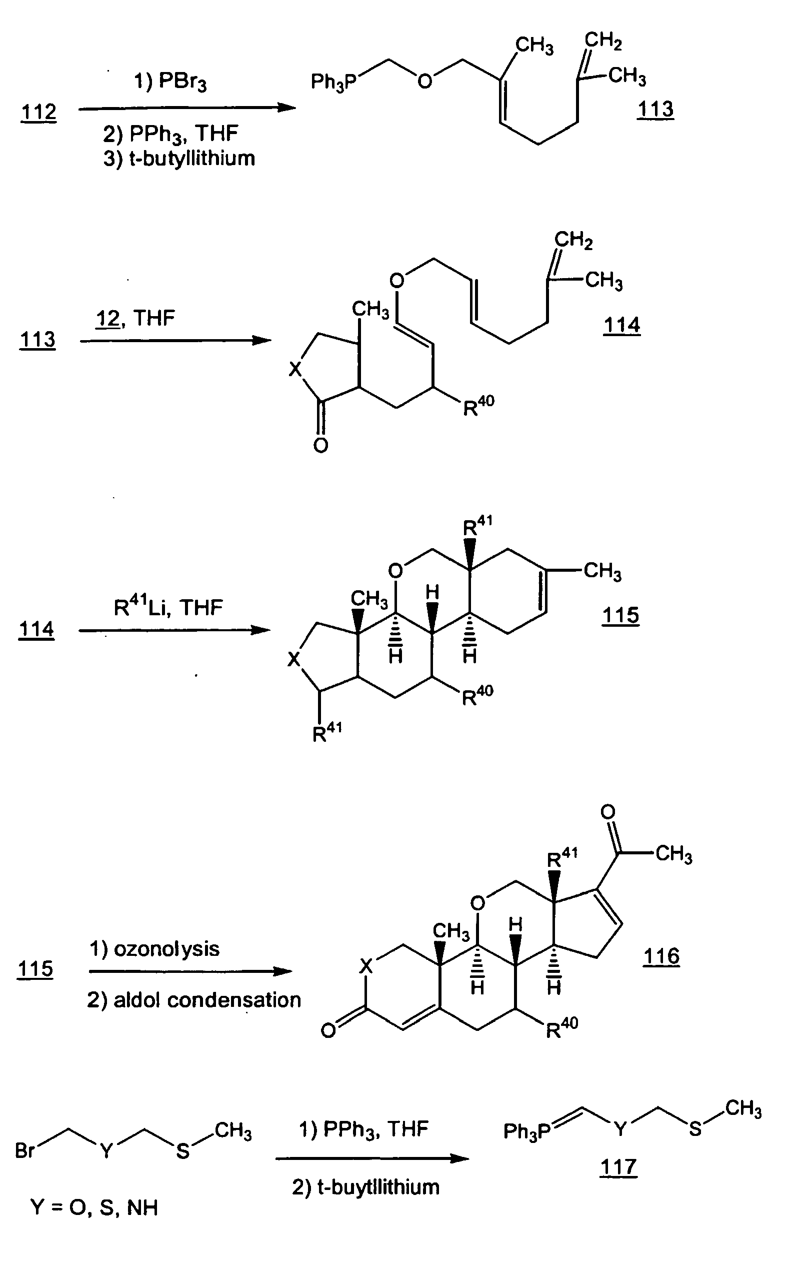

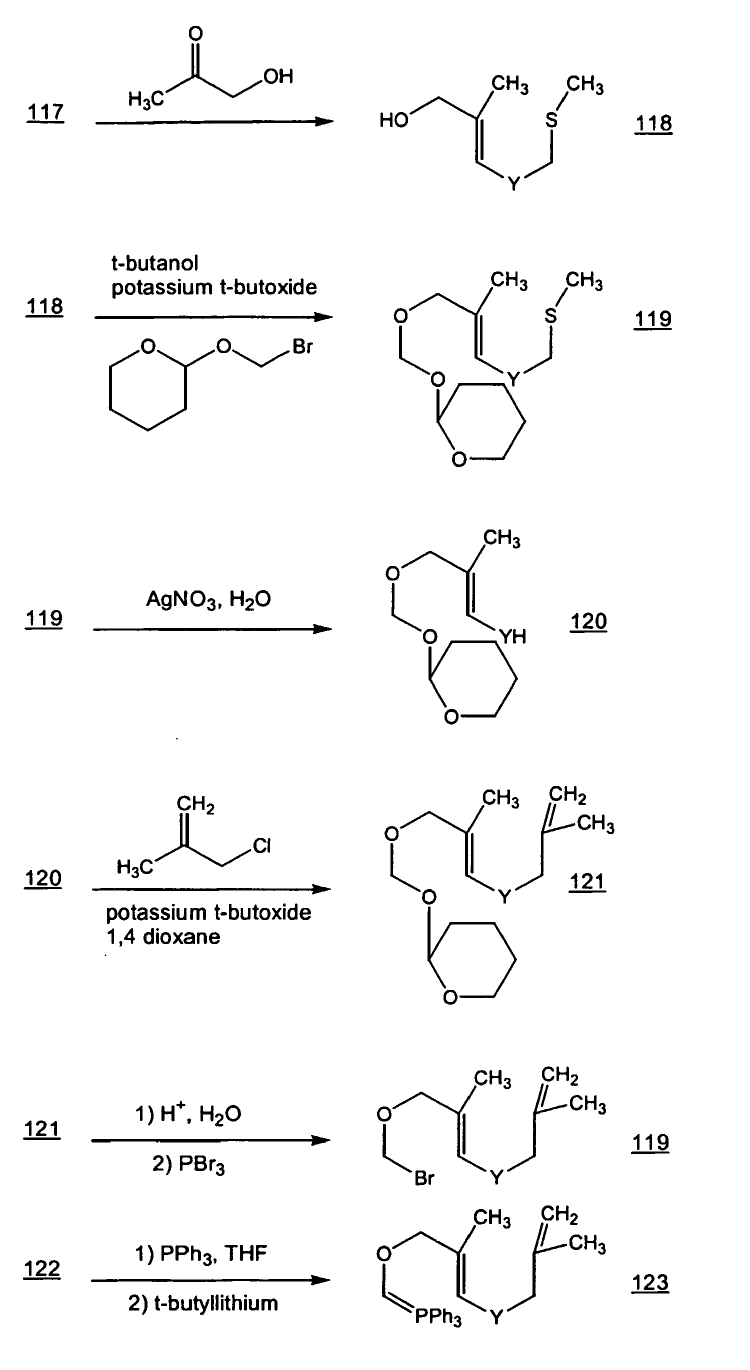

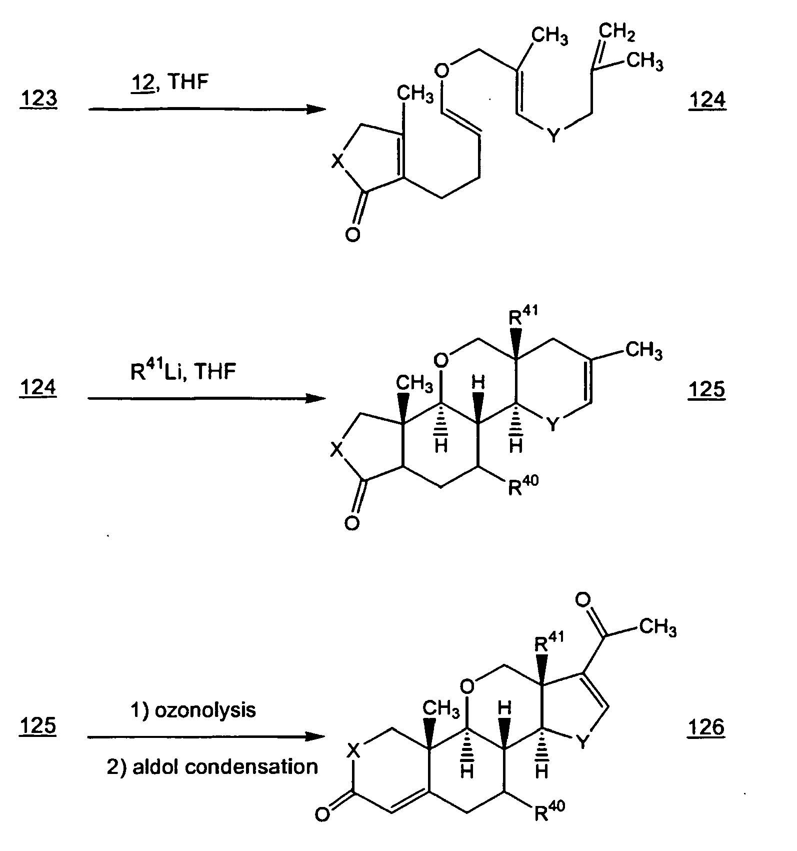

Conversion of the methyl ketone (-C(O)-CH₃) moiety in compounds having the structure 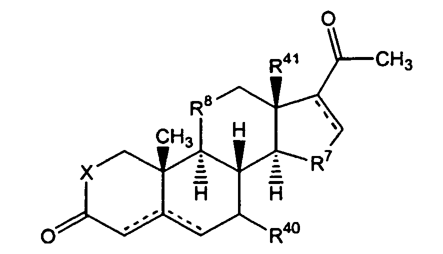 (R-C(O)-CH₃) to other functionalities is accomplished as follows. The methyl ketone is cleaved to yield a carboxyl moiety using, e.g., Br₂ or I₂ in base, followed by treatment with acid (H₃O⁺) essentially as described (S.J. Chakrabarty Oxidations in Organic Chemistry Part C, W. Trahnnousy, editor, Academic Press, NY, 1987, chapter 5; L.J. Smith et al., Org. Synth. III 302, 1953), to yield R-C(O)-OH. The carboxylic acid is reduced to the alcohol using LiAlH₄. Conversion of the carboxylic acid to the bromide is accomplished using, e.g., Br₂ in water, essentially as described (J.S. Meck et al., Org. Synth. V, 126, 1973; A. Mckillop et al., J. Org. Chem. 34: 1172, 1969).

Compounds of structure 11 are brominated using N-bromosuccinimide to obtain steroids and analogs with bromine at the 7-position. 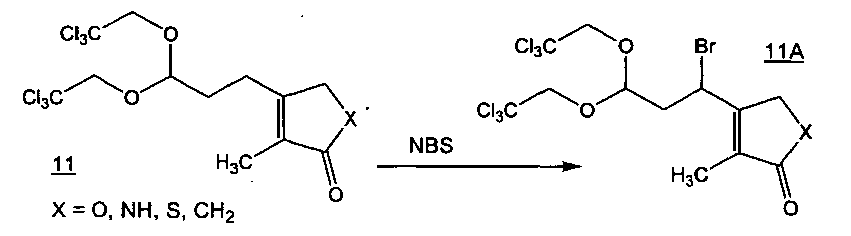 The 11A compounds are deprotected to yield the aldehyde compounds 12. As shown in scheme 11, the bromine atom is ultimately found at the 7-position. The bromine may be converted to a hydroxyl by reaction of the steroid with base (e.g., aqueous KOH), and the hydroxyl may in turn be protected using known methods, e.g., using C₆H₅-CH₂-Br and base (KOH). The alcohol is protected and deprotected essentially using described methods, see, e.g., W.H. Hartung et al., Org. React. 7: 263, 1953; E.J. Rerst et al., J. Org. Chem. 29: 3725, 1968; A.M. Felix et al., J. Org. Chem. 43: 4194, 1978; D.A. Evans et al., J. Am. Chem. Soc. 101: 6789, 1979; international publication number WO 98/02450. Similar reactions are used to convert a bromine at other positions to a hydroxyl. Other substituents are linked to the steroids as described in schemes 1-10.

Alternative routes to introduce a functional group into the 7-position are also suitable. For example, formula 1 compounds that have a double bond at the 5-6 position and are unsubstituted at the 7-position are optionally protected, e.g., hydroxyl groups are protected with acetate, and a ketone is introduced into the 7-position by oxidation with chromic acid essentially as described (U.S patent 2170124). The carbonyl (=O) at 7 is reduced to a hydroxyl using mild conditions, e.g., Al(Oi-Pr)₃, to avoid reducing the 5-6 double bond. The use of stronger reducing conditions, e.g., reduction with LiBH₄ in THF, leads to conversion of the 7-carbonyl to hydroxyl and to reduction of the 5-6 double bond and other double bonds that may be present in the molecule.

Selective hydrogenation of a double bond at the 16-17 position without reduction of a double bond at 5-6 is accomplished using H₂ and Pd. In general, ketones (=O) can be protected using a glycol, e.g., reaction with ethylene glycol in p-toluenesulfonic acid and benzene, before subsequent oxidation or reduction reactions are conducted.

Various groups that may comprise the formula 1 compounds described herein, e.g., hydroxyl groups or ketones bonded to the steroid nucleus, or substituted alkyl groups, substituted heterocycles, amino acids and peptides, can contain one or more reactive moieties such as hydroxyl, carboxyl, amino or thiol. Intermediates used to make formula 1 compounds may be protected as is apparent in the art. Noncyclic and cyclic protecting groups and corresponding cleavage reactions are described in "Protective Groups in Organic Chemistry", Theodora W. Greene (John Wiley & Sons, Inc., New York, 1991, ISBN 0-471-62301-6) (hereafter "Greene") and will not be detailed here. In the context of the present invention, these protecting groups are groups that can be removed from the molecule of the invention without irreversibly changing the covalent bond structure or oxidation/reduction state of the remainder of the molecule. For example, the protecting group, -R^{PR}, that is bonded to an -O- or -NH- group can be removed to form -OH or -NH₂, respectively, without affecting other covalent bonds in the molecule. At times, when desired, more than one protecting group can be removed at a time, or they can be removed sequentially. In compounds of the invention containing more than one protecting group, the protecting groups are the same or different.

Protecting groups are removed by known procedures, although it will be understood that the protected intermediates fall within the scope of this invention. The removal of the protecting group may be arduous or straight-forward, depending upon the economics and nature of the conversions involved. In general, one will use a protecting group with exocyclic amines or with carboxyl groups during synthesis of a formula 1 compound. For most therapeutic applications amine groups should be deprotected. Protecting groups commonly are employed to protect against covalent modification of a sensitive group in reactions such as alkylation or acylation. Ordinarily, protecting groups are removed by, e.g. hydrolysis, elimination or aminolysis. Thus, simple functional considerations will suffice to guide the selection of a reversible or an irreversible protecting group at a given locus on the invention compounds. Suitable protecting groups and criteria for their selection are described in T.W. Greene and P.G.M. Wuts, Eds. "Protective Groups in Organic Synthesis" 2nd edition, Wiley Press, at pps. 10-142, 143-174, 175-223, 224-276, 277-308, 309-405 and 406-454.

Determination of whether a group is a protecting group is made in the conventional manner, e.g., as illustrated by Kocienski, Philip J.; "Protecting Groups" (Georg Thieme Verlag Stuttgart, New York, 1994) (hereafter "Kocienski"), Section 1.1, page 2, and Greene Chapter 1, pages 1-9. In particular, a group is a protecting group if when, based on mole ratio, 90% of that protecting group has been removed by a deprotection reaction, no more than 50%, typically 25%, more typically 10%, of the deprotected product molecules of the invention have undergone changes to their covalent bond structure or oxidation/reduction state other than those occasioned by the removal of the protecting group. When multiple protecting groups of the same type are present in the molecule, the mole ratios are determined when all of the groups of that type are removed. When multiple protecting groups of different types are present in the molecule, each type of protecting group is treated (and the mole ratios are determined) independently or together with others depending on whether the deprotection reaction conditions pertinent to one type are also pertinent to the other types present. In one embodiment of the invention, a group is a protecting group if when, based on mole ratio determined by conventional techniques, 90% of that protecting group has been removed by a conventional deprotection reaction, no more than 50%, typically 25%, more typically 10%, of the deprotected product molecules of the invention have undergone irreversible changes to their covalent bond structure or oxidation/reduction state other than those occasioned by the removal of the protecting group. Irreversible changes require chemical reactions (beyond those resulting from aqueous hydrolysis, acid/base neutralization or conventional separation, isolation or purification) to restore the covalent bond structure or oxidation/reduction state of the deprotected molecule of the invention.

Protecting groups are also described in detail together with general concepts and specific strategies for their use in Kocienski, Philip J.; "Protecting Groups" (Georg Thieme Verlag Stuttgart, New York, 1994), which is incorporated by reference in its entirety herein. In particular Chapter 1, Protecting Groups: An Overview, pages 1-20, Chapter 2, Hydroxyl Protecting Groups, pages 21-94, Chapter 3, Diol Protecting Groups, pages 95-117, Chapter 4, Carboxyl Protecting Groups, pages 118-154, Chapter 5, Carbonyl Protecting Groups, pages 155-184, Chapter 6, Amino Protecting Groups, pages 185-243, Chapter 7, Epilog, pages 244-252, and Index, pages 253-260, are incorporated with specificity in the context of their contents. More particularly, Sections 2.3 Silyl Ethers, 2.4 Alkyl Ethers, 2.5 Alkoxyalkyl Ethers (Acetals), 2.6 Reviews (hydroxy and thiol protecting groups), 3.2 Acetals, 3.3 Silylene Derivatives, 3.4 1,1,3,3-Tetraisopropyldisiloxanylidene Derivatives, 3.5 Reviews (diol protecting groups), 4.2 Esters, 4.3 2,6,7-Trioxabicyclo[2.2.2]octanes [OBO] and Other Ortho Esters, 4.4 Oxazolines, 4.5 Reviews (carboxyl protecting groups), 5.2 O,O-Acetals, 5.3 S,S-Acetals, 5.4 O,S-Acetals, 5.5 Reviews (carbonyl protecting groups), 6.2 N-Acyl Derivatives, 6.3 N-Sulfonyl Derivatives, 6.4 N-Sulfenyl Derivatives, 6.5 N-Alkyl Derivatives, 6.6 N-Silyl Derivatives, 6.7 Imine Derivatives, and 6.8 Reviews (amino protecting groups), are each incorporated with specificity where protection/deprotection of the requisite functionalities is discussed. Further still, the tables "Index to the Principal Protecting Groups" appearing on the inside front cover and facing page, "Abbreviations" at page xiv, and "reagents and Solvents" at page xv are each incorporated in their entirety herein at this location.

Typical hydroxy protecting groups are described in Greene at pages 14-118 and include Ethers (Methyl); Substituted Methyl Ethers (Methoxymethyl, Methylthiomethyl, t-Butylthiomethyl, (Phenyldimethylsilyl)methoxymethyl, Benzyloxymethyl, p-Methoxybenzyloxymethyl, (4-Methoxyphenoxy)methyl, Guaiacolmethyl, t-Butoxymethyl, 4-Pentenyloxymethyl, Siloxymethyl, 2-Methoxyethoxymethyl, 2,2,2-Trichloroethoxymethyl, Bis(2-chloroethoxy)methyl, 2-(Trimethylsilyl)ethoxymethyl, Tetrahydropyranyl, 3-Bromotetrahydropyranyl, Tetrahydropthiopyranyl, 1-Methoxycyclohexyl, 4-methoxytetrahydropyranyl, 4-Methoxytetrahydrothiopyranyl, 4-Methoxytetrahydropthiopyranyl S,S-Dioxido, 1-[(2-Chloro-4-methyl)phenyl]-4-methoxypiperidin-4-yl, 1,4-Dioxan-2-yl, Tetrahydrofuranyl, Tetrahydrothiofuranyl, 2,3,3a,4,5,6,7,7a-Octahydro-7,8,8-trimethyl-4,7-methanobenzofuran-2-yl); Substituted Ethyl Ethers (1-Ethoxyethyl, 1-(2-Chloroethoxy)ethyl, 1-Methyl-1-methoxyethyl, 1-Methyl-1-benzyloxyethyl, 1-Methyl-1-benzyloxy-2-fluoroethyl, 2,2,2-Trichloroethyl, 2-Trimethylsilylethyl, 2-(Phenylselenyl)ethyl, t-Butyl, Allyl, p-Chlorophenyl, p-Methoxyphenyl, 2,4-Dinitrophenyl, Benzyl); Substituted Benzyl Ethers (p-Methoxybenzyl, 3,4-Dimethoxybenzyl, o-Nitrobenzyl, p-Nitrobenzyl, p-Halobenzyl, 2,6-Dichlorobenzyl, p-Cyanobenzyl, p-Phenylbenzyl, 2- and 4-Picolyl, 3-Methyl-2-picolyl N-Oxido, Diphenylmethyl, p, p'-Dinitrobenzhydryl, 5-Dibenzosuberyl, Triphenylmethyl, alpha-Naphthyldiphenylmethyl, p-methoxyphenyldiphenylmethyl, Di(p-methoxyphenyl)phenylmethyl, Tri(p-methoxyphenyl)methyl, 4-(4'-Bromophenacyloxy)phenyldiphenylmethyl, 4,4', 4"-Tris(4,5-dichlorophthalimidophenyl)methyl, 4,4', 4"-Tris(levulinoyloxyphenyl)methyl, 4,4', 4"-Tris(benzoyloxyphenyl)methyl, 3-(Imidazol-1-ylmethyl)bis(4', 4"-dimethoxyphenyl)methyl, 1,1-Bis(4-methoxyphenyl)-1'-pyrenylmethyl, 9-Anthryl, 9-(9-Phenyl)xanthenyl, 9-(9-Phenyl-10-oxo)anthryl, 1,3-Benzodithiolan-2-yl, Benzisothiazolyl, S,S-Dioxido); Silyl Ethers (Trimethylsilyl, Triethylsilyl, Triisopropylsilyl, Dimethylisopropylsilyl, Diethylisopropylsily, Dimethylthexylsilyl, t-Butyldimethylsilyl, t-Butyldiphenylsilyl, Tribenzylsilyl, Tri-p-xylylsilyl, Triphenylsilyl, Diphenylmethylsilyl, t-Butylmethoxyphenylsilyl); Esters (Formate, Benzoylformate, Acetate, Choroacetate, Dichloroacetate, Trichloroacetate, Trifluoroacetate, Methoxyacetate, Triphenyl-methoxyacetate, Phenoxyacetate, p-Chlorophenoxyacetate, p-poly-Phenylacetate, 3-Phenylpropionate, 4-Oxopentanoate (Levulinate), 4,4-(Ethylenedithio)pentanoate, Pivaloate, Adamantoate, Crotonate, 4-Methoxycrotonate, Benzoate, p-Phenylbenzoate, 2,4,6-Trimethylbenzoate (Mesitoate); Carbonates (Methyl, 9-Fluorenylmethyl, Ethyl, 2,2,2-Trichloroethyl, 2-(Trimethylsilyl)ethyl, 2-(Phenylsulfonyl)ethyl, 2-(Triphenylphosphonio)ethyl, Isobutyl, Vinyl, Allyl, p-Nitrophenyl, Benzyl, p-Methoxybenzyl, 3,4-Dimethoxybenzyl, o-Nitrobenzyl, p-Nitrobenzyl, S-Benzyl Thiocarbonate, 4-Ethoxy-1-naphthyl, Methyl Dithiocarbonate); Groups With Assisted Cleavage (2-lodobenzoate, 4-Azidobutyrate, 4-Nitro-4-methylpentanoate, o-(Dibromomethyl)benzoate, 2-Formylbenzenesulfonate, 2-(Methylthiomethoxy)ethyl Carbonate, 4-(Methylthiomethoxy)butyrate, 2-(Methylthiomethoxymethyl)benzoate); Miscellaneous Esters (2,6-Dichloro-4-methylphenoxyacetate, 2,6-Dichloro-4-(1,1,3,3-tetramethylbutyl)phenoxyacetate, 2,4-Bis(1,1-dimethylpropyl)phenoxyacetate, Chorodiphenylacetate, Isobutyrate, Monosuccinoate, (E)-2-Methyl-2-butenoate (Tigloate), o-(Methoxycarbonyl)benzoate, p-poly-Benzoate, α-Naphthoate, Nitrate, Alkyl N,N,N', N'-Tetramethylphosphorodiamidate, N-Phenylcarbamate, Borate, Dimethylphosphinothioyl, 2,4-Dinitro-phenylsulfenate); and Sulfonates (Sulfate, Methanesulfonate (Mesylate), Benzylsulfonate, Tosylate (Tos)).

More typically hydroxy protecting groups include subtituted methyl ethers, substituted benzyl ethers, silyl ethers, and esters including sulfonic acid esters, still more typically, trialkylsilyl ethers, tosylates and acetates.

Typical 1,2- and 1,3-diol protecting groups are described in Greene at pages 118-142 and include Cyclic Acetals and Ketals (Methylene, Ethylidene, 1-t-Butylethylidene, 1-Phenylethylidene, (4-Methoxyphenyl)ethylidene, 2,2,2-Trichloroethylidene, Acetonide (Isopropylidene), Cyclopentylidene, Cyclohexylidene, Cycloheptylidene, Benzylidene, p-Methoxybenzylidene, 2,4-Dimethoxybenzylidene, 3,4-Dimethoxybenzylidene, 2-Nitrobenzylidene); Cyclic Ortho Esters (Methoxymethylene, Ethoxymethylene, Dimethoxymethylene, 1-Methoxyethylidene, 1-Ethoxyethylidine, 1,2-Dimethoxyethylidene, alpha-Methoxybenzylidene, 1-(N,N-Dimethylamino)ethylidene Derivative, alpha-(N,N-Dimethylamino)benzylidene Derivative, 2-Oxacyclopentylidene); and Silyl Derivatives (Di-t-butylsilylene Group, 1,3-(1,1,3,3-Tetraisopropyldisiloxanylidene) Derivative, Tetra-t-butoxydisiloxane-1,3-diylidene Derivative, Cyclic Carbonates, Cyclic Boronates, Ethyl Boronate, Phenyl Boronate).

More typically, 1,2- and 1,3-diol protecting groups include epoxides and acetonides.

Typical amino protecting groups are described in Greene at pages 315-385 and include Carbamates (Methyl and Ethyl, 9-Fluorenylmethyl, 9(2-Sulfo)fluoroenylmethyl, 9-(2,7-Dibromo)fluorenylmethyl, 2,7-Di-t-buthyl-[9-(10,10-dioxo-10,10,10,10-tetrahydrothioxanthyl)]-methyl, 4-Methoxy-phenacyl); Substituted Ethyl (2,2,2-Trichoroethyl, 2-Trimethylsilylethyl, 2-Phenylethyl, 1-(1-Adamantyl)-1-methylethyl, 1,1-Dimethyl-2-haloethyl, 1,1-Dimethyl-2,2-dibromoethyl, 1,1-Dimethyl-2,2,2-trichloroethyl, 1-Methyl-1-(4-biphenylyl)ethyl, 1-(3,5-Di-t-butylphenylyl-methylethyl, 2-(2'- and 4'-Pyridyl)ethyl, 2-(N,N-Dicyclohexylcarboxamido)ethyl, t-Butyl, 1-Adamantyl, Vinyl, Allyl, 1-Isopropylallyl, Cinnamyl, 4-Nitrocinnamyl, 8-Quinolyl, N-Hydroxypiperidinyl, Alkyldithio, Benzyl, p-Methoxybenzyl, p-Nitrobenzyl, p-Bromobenzyl, p-Chorobenzyl, 2,4-Dichlorobenzyl, 4-Methylsulfinylbenzyl, 9-Anthrylmethyl, Diphenylmethyl); Groups With Assisted Cleavage (2-Methylthioethyl, 2-Methylsulfonylethyl, 2-(p-Toluenesulfonyl)ethyl, [2-(1,3-Dithianyl)]methyl, 4-Methylthiophenyl, 2,4-Dimethylthiophenyl, 2-Phosphonioethyl, 2-Triphenylphosphonioisopropyl, 1,1-Dimethyl-2-cyanoethyl, m-Choro-p-acyloxybenzyl, p-(Dihydroxyboryl)benzyl, 5-Benzisoxazolylmethyl, 2-(Trifluoromethyl)-6-chromonylmethyl); Groups Capable of Photolytic Cleavage (m-Nitrophenyl, 3,5-Dimethoxybenzyl, o-Nitrobenzyl, 3,4-Dimethoxy-6-nitrobenzyl, Phenyl(o-nitrophenyl)methyl); Urea-Type Derivatives (Phenothiazinyl-(10)-carbonyl Derivative, N'-p-Toluenesulfonylaminocarbonyl, N'-Phenylaminothiocarbonyl); Miscellaneous Carbamates (t-Amyl, S-Benzyl Thiocarbamate, p-Cyanobenzyl, Cyclobutyl, Cyclohexyl, Cyclopentyl, Cyclopropylmethyl, p-Decyloxybenzyl, Diisopropylmethyl, 2,2-Dimethoxycarbonylvinyl, o-(N,N-Dimethyl-carboxamido)benzyl, 1,1-Dimethyl-3-(N,N-dimethylcarboxamido)propyl, 1,1-Dimethylpropynyl, Di(2-pyridyl)methyl, 2-Furanylmethyl, 2-lodoethyl, Isobomyl, Isobutyl, Isonicotinyl, p-(p'-Methoxyphenylazo)benzyl, 1-Methylcyclobutyl, 1-Methylcyclohexyl, 1-Methyl-1-cyclopropylmethyl, 1-Methyl-1-(3,5-dimethoxyphenyl)ethyl, 1-Methyl-1-(p-phenylazophenyl)ethyl, 1-Methyl-1-phenylethyl, 1-Methyl-1-(4-pyridyl)ethyl, Phenyl, p-(Phenylazo)-benzyl, 2,4,6-Tri-t-butylphenyl, 4-(Trimethylammonium)benzyl, 2,4,6-Trimethylbenzyl); Amides (N-Formyl, N-Acetyl, N-Choroacetyl, N-Trichoroacetyl, N-Trifluoroacetyl, N-Phenylacetyl, N-3-Phenylpropionyl, N-Picolinoyl, N-3-Pyridylcarboxamide, N-Benzoylphenylalanyl Derivative, N-Benzoyl, N-p-Phenylbenzoyl); Amides With Assisted Cleavage (No-Nitrophenylacetyl, N-o-Nitrophenoxyacetyl, N-Acetoacetyl, (N'-Dithiobenzyloxycarbonylamino)acetyl, N-3-(p-Hydroxyphenyl)propionyl, N-3-(o-Nitrophenyl)propionyl, N-2-Methyl-2-(o-nitrophenoxy)propionyl, N-2-Methyl-2-(o-phenylazophenoxy)propionyl, N-4-Chlorobutyryl, N-3-Methyl-3-nitrobutyryl, N-o-Nitrocinnamoyl, N-Acetylmethionine Derivative, N-o-Nitrobenzoyl, N-o-(Benzoyloxymethyl)benzoyl, 4,5-Diphenyl-3-oxazolin-2-one); Cyclic Imide Derivatives (N-Phthalimide, N-Dithiasuccinoyl, N-2,3-Diphenylmaleoyl, N-2,5-Dimethylpyrrolyl, N-1,1,4,4-Tetramethyl-disilylazacyclopentane Adduct, 5-Substituted 1,3-Dimethyl-1,3,5-triazacyclo-hexan-2-one, 5-Substituted 1,3-Dibenzyl-1,3,5-triazacyclohexan-2-one, 1-Substituted 3,5-Dinitro-4-pyridonyl); N-Alkyl and N-Aryl Amines (N-Methyl, N-Allyl, N-[2-(Trimethylsilyl)ethoxy]methyl, N-3-Acetoxypropyl, N-(1-Isopropyl-4-nitro-2-oxo-3-pyrrolin-3-yl), Quaternary Ammonium Salts, N-Benzyl, N-Di(4-methoxyphenyl)methyl, N-5-Dibenzosuberyl, N-Triphenylmethyl, N-(4-Methoxyphenyl)diphenylmethyl, N-9-Phenylfluorenyl, N-2,7-Dichloro-9-fluorenylmethylene, N-Ferrocenylmethyl, N-2-Picolylamine N'-Oxide); Imine Derivatives (N-1,1-Dimethylthiomethylene, N-Benzylidene, N-p-methoxybenylidene, N-Diphenylmethylene, N-[(2-Pyridyl)mesityl]methylene, N,(N',N'-Dimethylaminomethylene, N,N'-Isopropylidene, N-p-Nitrobenzylidene, N-Salicylidene, N-5-Chlorosalicylidene, N-(5-Chloro-2-hydroxyphenyl)phenylmethylene, N-Cyclohexylidene); Enamine Derivative (N-(5,5-Dimethyl-3-oxo-1-cyclohexenyl)); N-Metal Derivatives (N-Borane Derivatives, N-Diphenylborinic Acid Derivative, N-[Phenyl(pentacarbonylchromium- or -tungsten)]carbenyl, N-Copper or N-Zinc Chelate); N-N Derivatives (N-Nitro, N-Nitroso, N-Oxide); N-P Derivatives (N-Diphenylphosphinyl, N-Dimethylthiophosphinyl, N-Diphenylthiophosphinyl, N-Dialkyl Phosphoryl, N-Dibenzyl Phosphoryl, N-Diphenyl Phosphoryl); N-Si Derivatives; N-S Derivatives; N-Sulfenyl Derivatives (N-Benzenesulfenyl, N-o-Nitrobenzenesulfenyl, N-2,4-Dinitrobenzenesulfenyl, N-Pentachlorobenzenesulfenyl, N-2-nitro-4-methoxybenzenesulfenyl, N-Triphenylmethylsulfenyl, N-3-Nitropyridinesulfenyl); andN-Sulfonyl Derivatives (N-p-Toluenesulfonyl, N-Benzenesulfonyl, N-2,3,6-Trimethyl-4-methoxybenzenesulfonyl, N-2,4,6-Trimethoxybenzenesulfonyl, N-2,6-Dimethyl-4-methoxybenzenesulfonyl, N-Pentamethylbenzenesulfonyl, N-2,3,5,6,-Tetramethyl-4-methoxybenzenesulfonyl, N-4-methoxybenzenesulfonyl, N-2,4,6-Trimethylbenzenesulfonyl, N-2,6-Dimethoxy-4-methylbenzenesulfonyl, N-2,2,5,7,8-Pentamethylchroman-6-sulfonyl, N-Methanesulfonyl, N-.beta.-Trimethylsilyethanesulfonyl, N-9-Anthracenesulfonyl, N-4-(4', 8'-Dimethoxynaphthylmethyl)benzenesulfonyl, N-Benzylsulfonyl, N-Trifluoromethylsulfonyl, N-Phenacylsulfonyl).

More typically, amino protecting groups include carbamates and amides, still more typically, N-acetyl groups.

Groups capable of biological cleavage typically include prodrugs. A large number of such groups are described in "Design of Prodrugs", Hans Bundgaard (Elsevier, N.Y., 1985, ISBN 0-444-80675-X) (Bundgaard) and will not be detailed here. In particular, Bundgaard, at pages 1-92, describes prodrugs and their biological cleavage reactions for a number of functional group types. Prodrugs for carboxyl and hydroxyl groups are detailed in Bundgaard at pages 3 to 10, for amides, imides and other NH-acidic compounds at pages 10 to 27, amines at pages 27 to 43, and cyclic prodrugs at pages 62 to 70. These moieties are optionally bonded to the steroid at one two or more of R¹-R⁶, R¹⁰, R¹⁵, R¹⁷ and R¹⁸.

Therapeutic applications. Aspects of the invention include methods to treat or prevent various blood cell deficiencies such as TP or NP. Without being bound to any theory, the treatment methods may result in enhanced hemopoiesis or the treatment methods may reduce the loss of cells such as platelets or neutrophils. Increased platelet or neutrophil production or reduced loss is typically observed as increased circulating blood cell counts. Thus, invention aspects comprise methods to treat or prevent neutropenia in a subject in need thereof, comprising administering to a subject in need, or delivering to the subject's tissues, an effective amount of a formula 1 compound.

Normal ranges of various white blood cells or blood components in adult (about 18-49 years of age) human blood are as follows. Total adult white blood cell counts average about 7500/mm³, with an approximate normal range of about 4.5 - 11.0 x 10³/mm³. The normal basophil level is about 35 mm⁻³, with a normal range of about 10-100/mm³. The normal adult neutrophil level is about 4400/mm³, with a normal range of about 2000-7700/mm⁻³. The normal eosinophil level is about 275 mm⁻³, with a normal range of about 150-300/mm³. The normal monocyte level is about 540 mm⁻³, with a normal range of about 300-600/mm³. The normal adult platelet level is about 2.5 x 10⁵/mm³, with a normal range of about 2.1 x 10⁵ - 2.9 x 10⁵/mm³. The normal adult red cell mass corresponds to about 4.6 x 10¹² red cells/L in females and about 5.2 x 10¹² red cells/L in males.

Thus, a human patient in need of treatment will typically have, or be subject to, a cell count below these values. As used herein, neutropenia means generally a circulating neutrophil count of less than about 1800/mm³, generally a count of about 1500/mm³ or less. Thrmobocytopenia generally means a circulating platelet count of less than about 1.9 x 10⁵/mm³, generally a count of less than about 1.2 x 10⁵/mm³. Anemia generally means a red cell mass corresponding to less than about 4.0 x 10¹² red cells/L in adult females and less than about 4.5 x 10¹² red cells/L in adult males (a hemoglobin level of less than about 12.0 g/dL in adult females and less than about 13.5 g/dL in adult males).

In some cases, the diagnosis of a deficiency may cover a cell count that falls outside these ranges, due, e.g., to individual variations in a subject's age, sex, race, animal strain or normal blood cell status for the individual. Such variations are identified by known means such as by identification of a change from the subject's normal status or by multiple cell measurements over time that reveal a deficiency. See, e.g., Hematology - Basic Principles and Practice, 2nd edition, R. Hoffman, E.J. Benz Jr. et al., editors, Churchill Livingstone, New York, 1995. Subjects with an identified or identifiable deficiency outside these standard ranges are included in the definition of a blood cell deficiency or a subject in need of treatment, as used herein.

Specific conditions that are amenable to prophylaxis or treatment by the invention methods include the acquired blood cell deficiencies. Exemplary deficiencies or groups of deficiencies are neonatal alloimmune TP, immune TP, immune thrombocytopenic purpura, thrombotic thrombocytopenic purpura, post-transfusion purpura, radiation associated TP, chemotherapy associated TP (e.g., NSAID treatments such as with indomethacin, ibuprofen, naproxen, phenylbutazone, piroxican or zompirac, or β-lactam antibiotic treatments such as with ampicillin, carbenacillin, penicillin G, ticarcillin, or cephalosporin treatments such as with cefazolin, cefoxitin or cephalothin, anticoagulant treatments such as heparin, hirudin, lepirudin or aspirin, treatment with plasma expanders or psychotropic drugs), amegakaryocitic TP, chemotherapy associated TP, radiation associated TP, TP associated with solid organ allograft or xenograft rejection or immune suppression therapy in solid organ or other tissue transplants (e.g., liver, lung, kidney, heart, bone marrow, hematopoietic stem cell or endothelial cell transplant, implant or transfusion), cardiopulmonary bypass surgery or chemotherapy associated TP (e.g., an anticancer, antiviral, antibacterial, antifungal or antiparasite therapy), cardiovascular disease or therapy associated TP (e.g., congenital cyanotic heart disease, valvular heart disease, pulmonary embolism, pulmonary hypertension disorders or diltiazem, nifedipine, nitroglycerin or nitroprusside therapy), TP associated with chronic or acute renal failure or treatment for these conditions (e.g., dialysis), TP associated with infection such as a virus or bacterial infection, postinfectious NP, drug-induced NP, autoimmune NP, chronic idiopathic NP, basophilic leukopenia, eosinophilic leukopenia, monocytic leukopenia, neutrophilic leukopenia, cyclic NP, periodic NP, chemotherapy associated NP, radiation associated NP, chemotherapy associated NP, radiation associated NP, NP associated with solid organ allograft or xenograft rejection or immune suppression therapy in solid organ or other tissue transplants (e.g., liver, lung, kidney, heart, bone marrow, hematopoietic stem cell or endothelial cell transplant, implant or transfusion), chemotherapy associated leukopenia, radiation associated leukopenia, leukopenia associated with solid organ allograft or xenograft rejection or immune suppression therapy in solid organ or other tissue transplants (e.g., liver, lung, kidney, heart, bone marrow, hematopoietic stem cell or endothelial cell transplant, implant or transfusion), immune hemolytic anemias, anemia associated with chronic or acute renal failure or treatment for these conditions (e.g., dialysis), anemia associated with chemotherapy (e.g., isoniazid, prednisone) or anemia associated with radiation therapy.

Some of the blood cell deficiencies are associated with, or caused by, other therapeutic treatments, e.g., cancer chemotherapy, anti-pathogen chemotherapy, radiation therapy and chemotherapy for suppression of autoimmunity or immune suppression therapy for organ or tissue transplantation or implantation. In many cases it would be medically sound to continue the treatment associated with causing or exacerbating the blood cell deficiency. Thus, one would generally conduct the invention methods with subjects who are undergoing another therapy at the same time or near the same time, e.g., within a few days to within about 1-6 months. Such subjects typically will have an identified blood cell deficiency such as a NP or a TP, e.g., as disclosed herein. However, the formula 1 compounds are generally suitable for preventing the onset of such deficiencies, and they can thus be used prophylactically in these indications. The invention includes all of these embodiments.

In some embodiments, the invention method is accomplished using an effective amount of one or more growth factors or cytokines as a means to further enhance the effect of the formula 1 compounds for their intended uses or to modulate their effects. Suitable growth factors and cytokines are as described herein or in the cited references. For example, when one administers the formula 1 compound to enhance generation of platelets in humansor other subjects, or their precursor cells such as BFU-Mk, CFU-Mk, immature megakaryocites or mature postmitotic megakaryocites, one can also administer one or more of G-CSF, GM-CSF, SCF, Steel factor ("SF"), leukemia inhibitory factor ("LIF"), interkeukin-1α, ("IL-1α"), IL-3, IL-6, IL-11, TPO, EPO, their isoforms, their derivatives (e.g., linked to a PEG or fusions such as PIXY321) or their homologs for other species. Similarly, administration of the formula 1 compound to enhance the generation or function of myelomonocytic cells such as neutrophils, basophils or monocytes in humans or other subjects, one can also administer one or more of G-CSF, GM-CSF, M-CSF, LIF, TPO, SF, interleukin-1 ("IL-1"), IL-2, IL-3, IL-4, interleukin-5 ("IL-5"), IL-6, IL-11, interleukin-12 ("IL-12"), interleukin-13 ("IL-13"), *FLT*3 ligand, their isoforms, homologs or derivatives (e.g., linked to a PEG or fusions such as PIXY321) or their homologs for other species. To enhance generation of red cells or their precursor cells such as CFU-GEMM, BFU-E or CFU-E in humans being treated with a formula 1 compound, one can co-administer one or more of G-CSF, GM-CSF, IL-1, IL-3, IL-6, TPO, EPO, transforming growth factor-β1, their isoforms, their derivatives (e.g., linked to a PEG or fusions such as PIXY321) or their homologs for other species. See, e.g., Hematology - Basic Principles and Practice, 3rd edition, R. Hoffman, E.J. Benz Jr. et al., editors, Churchill Livingstone, New York, 2000 (see, e.g., Chapters 14-17 at pages 154-260). The co-administration of such factors in these methods is intended to enhance the efficacy of the formula 1 compound treatment, which is optionally measured by taking suitable blood or tissue, e.g., bone marrow, samples at one or more times before and after the compounds have been administered. Such co-administration will generally be compatible with a subject's condition and other therapeutic treatments. Co-administration of such factors can precede, be simultaneous with, or follow the times of administration of the formula 1 compound(s) to the subject. Dosages of such growth factors would generally be similar to those previously described, e.g., typically an initial course of treatment comprises administering about 1.0 to about 20 µg/kg/d for about 1-10 days, or as described in, e.g., Hematology - Basic Principles and Practice, 3rd edition, R. Hoffman, E.J. Benz Jr. et al., editors, Churchill Livingstone, New York, 2000 (see, e.g., Chapter 51 at pages 939-979 and the references cited therein).

In cases where a subject's blood cell deficiency is caused by, or associated with another therapy, the invention contemplates that the other therapy will continue, if this is reasonable under the circumstances. The timing of other therapies can precede, be simultaneous with, or follow the times of administration of the formula 1 compound(s) to the subject. For example, chemotherapy for some malignancies is accompanied by myelosuppression or a deficiency in one or more blood cell types, e.g., TP or NP. Continued treatment would be called for in some cases, and then the invention methods would be employed to deliver to the subject an effective amount of a formula 1 compound. Thus, alkylating agents, antimicrotubule agents, antimetabolites, topoisomerase I or II inhibitors, or platinum compounds such as one or more of mechlorethamine, vincristine, vinblastine, bleomycin, doxorubicin, epirubicin, tamoxifen, cyclophosphamide, etoposide, methotrexate, ifosfamide, melphalan, chlorambucil, busulfan, carmustine, lomustine, streptozocin, dacarbazine, vinorelbine, paclitaxel (taxol), docetaxel, cytosine arabinoside, hydroxyurea, fludarabine, 2'-chlorodeoxyadenosine, 2'-deoxycoformycin, 6-thioguanine, 6-mercaptopurine, 5-azacytidine, gemcitabine, arabinofuranosylguanine, daunorubicin, mitoxantrone, amsacrine, topotecan, irinotecan, cisplatin, carboplatin, pilcamycin, procarbazine, aspariginase, aminoglutethimide, actinomycin D, azathioprine and gallium nitrate may be administered in conjunction with administration of any formula 1 compound(s) that is disclosed herein. Treatments with other therapeutic agents such as heparin or nucleoside analogs such as 3-thiacytosine, azidothymidine or dideoxycytosine, or other antimicrobials such as cephalosporin, quinine, quinidine, gold salts (e.g., aurothioglucose), ciprofloxacin, clarithromycin, fluconazole, fusidic acid, gentamycin, nalidixic acid, penicillins, pentamidine, rifampicin, sulfa antibiotics, suramin or vancomycin may result in a blood cell deficiency(s) and they can thus be combined with administration of a formula 1 compound to treat the deficiency, or to ameliorate a symptom thereof. Similarly, anti-inflammatory drugs (e.g., salicylates), cardiac drugs (e.g., digitoxin), β-blockers or antihypertensive drugs (e.g., oxprenolol or captopril), diuretics (e.g., spironolactone), benzodiazepines, (e.g., diazepam) or antidepressants (e.g., amitriptyline, doxepin). Any of these methods also optionally include co-administration of one or more of the growth factors described above, e.g., IL-3, G-CSF, GM-CSF or TPO.

In related embodiments, the activity or numbers of neutrophils or monocytes is enhanced by co-administering the formula 1 compound with an neutrophil or monocyte stimulator, which is an non-protein agent or molecule that can stimulate the activity or number of neutrophils or monocytes in a subject. This aspect of the present invention encompasses any technique to enhance neutrophil or monocyte counts or activity. Means to accomplish this include administering an effective amount of a formula 1 compound and an effective amount of one or more of lithium, e.g., in the form of a salt such as lithium carbonate or chloride, deuterium oxide, levamisole (an antihelminthic agent), lactoferrin, thyroxine, tri-iodothyromine, anthrax toxin, ascorbic acid, I-palmitoyl-lysophosphatidic acid, a calcium ionophore, e.g., A23187, cytochalasin B, sodium butyrate, piracetamine, micronized L-arginine, hydroxyurea and a bacterial lipopolysaccharide.

The neutrophil or monocyte stimulator can be administered at various time relative to administration of the formula 1 compound, including about 2-4 hours to about 1 or 2 weeks before administering the formula 1 compound and including administration that is essentially simultaneous with administering the formula 1 compound. Typically a neutrophil or monocyte stimulator will be dosed according to known methods, including daily dosing of about 0.01 mg/kg/day to about 25 mg/kg/day. For example, about 1 g/day of ascorbic acid (e.g., about 0.5 to about 1.5 g/day) can be administered to humans. When duterium oxide is used as a neutrophil or monocyte stimulator, liquid aqueous formulations may comprise a formula 1 compound and duterium oxide in place of some or all of the water. Naturally occurring water contains approximately 1 part of deuterium oxide per 6500 parts water. Thus, the water present in a formulation may comprise, e.g., at least 1 part D₂O in 6000 parts H₂O, or at least 1 part in 100 parts, or about 50 parts or more per 100 parts of water. These aqueous formulations may comprise one or more additional excipients such as a cyclodextrin such as β-hydroxypropylcyclodextrin. Formulations comprising cyclodextrin and deuterium oxide, or comprising cyclodextrin, deuterium oxide and water, may thus comprise deuterium oxide in an amount greater than 1 part per 6500 parts water, such as 1 part deuterium oxide per 1 - 100 parts water, e.g., 50 parts deuterium oxide per 100 parts water. The amount of cyclodextrin can be in the range of from about 2 to about 85 grams per liter of water and/or deuterium oxide, such as in the range of from about 5 to about 70 grams per liter of water and/or deuterium oxide, one example of a suitable amount being in the range of about 45 grams per liter of water and/or deuterium oxide.

In conducting any of the invention methods disclosed herein, one can monitor the subject's clinical condition at any relevant time before, during or after administration of the formula 1 compounds, which treatments are optionally combined with any of the other agents or treatments disclosed herein, such as cytokines, interleukins or an agent or molecule that can stimulate the activity or number of neutrophils or monocytes. The subject's blood can be drawn on one, two or more occasions in advance of treatment to, e.g., obtain a baseline or initial level of white or red blood cells, to verify a presumptive diagnosis of a blood cell deficiency or to determine a blood parameter such as circulating myelomonocyte counts, circulating neutrophil counts, circulating platelet counts or the myeloperoxidase index. Then, during the course of treatment or thereafter the subject's blood can be drawn on one, two or more occasions to follow the subject's response.

The formula 1 compounds are believed to be effective in facilitating release of myeloperoxidase from granulated neutrophils. The enzyme generates free hydrogen peroxide. Some of the formula 1 compounds, e.g., compounds with a halogen such as bromine or iodine at, e.g., the 16 position, can be metabolized to provide a source of halogen. In cases where the halogen is released, the released halogen can react with hydrogen peroxide (H₂O₂) to generate hypohalogenous acid such as hypobromous acid (HOBr). Exemplary compounds include a halogenated formula 1 compound such as 16-bromoepiandrosterone. Alternatively, a halogen salt, e.g., KBr, NaBr, KI or Nal, can be administered to the subject to provide a source of halogen. The halogen source can be administered to a subject as a component in a formulation that comprises a formula 1 compound or it can be administered separately. Hypohalogenous acid is a potent antimicrobial agent, which may be effective in reducing pathogens in the circulatory system of subjects with a blood cell deficiency who also have a pathogen infection. Hypohalogenous acid that is generated *in vivo* would provide benefits to such subject as shown by, e.g., a reduced quantitative circulating viral or bacterial culture measurement, without the toxicity that is normally associated with its direct administration to a subject. Biological activities of white blood peroxidase enzymes have been described, see, e.g., M. Saran et al., Free Radical Biol. Med. 1999 26:482-490, W. Wu et al., J. Clin. Invest. 2000 105:1455-1463 and Z. Shen et al., Biochemistry 2000 39:5474-5482.

Invention embodiments include methods that comprise administering to a subject in need thereof an effective amount of a formula 1 compound and an effective amount of at least one form of interferon, such as γ-Interferon or a growth factor or interleukin such as G-CSF or IL-6. Interferons can enhance the biological activity of the white cells that arise from increased hemopoiesis. This can be particularly useful when the subject's circulating blood cell deficiency is associated with, e.g., an infection or a chemotherapy that suppresses hemopoiesis. Administration of an growth factor or an interleukin such as IL-6 can facilitate hemopoiesis by stimulating quiescent stem cells or other progenitors that give rise to deficient cell types. Related embodiments replace growth factor or interferon administration partially or completely by increasing endogenous production in the subject using conventional methods, e.g., administering double stranded RNA to stimulate γ-IFN.

For cases where γ-IFN is administered, the administration is usually relatively constant, e.g., daily. This is because in patients in whom γ-IFN is not generated endogenously in significant amounts, there is a tendency for levels of γ-IFN to drop relatively quickly, i.e., within one day. In other words, in a patient in whom initially, γ-IFN levels are close to zero, it should be administered in an amount which is effective to bring γ-IFN levels to within normal levels, e.g., up to 10 nanograms per milliliter, and a similar amount of γ-IFN should be administered each day thereafter.

Suitable forms of γ-IFN and their biological properties and methods to obtain them have been described, see, e.g., U.S. patents 4,289,690, 4,314,935, 4,382,027, 4,376,821; 4,376,822, 4,460,685, 4,604,284 and 5,145,677, European patent publication nos. EP 063,482 EP 088 540, and EP 087 686, N. Fujii et al., J. Immunol., 1983 130:1683-86. A. Zlotnick et al., J. Immunol. 1983 131:794-80, M. deLey et al., Eur. J. Immunol. 1980 10:877-83, F. Dianzani et al., Infection and Immunity, 1980 29:561-63, G. H. Reem et al., Infection and Immunity 1982 37:216-21 (1982), R. Devos et al., Nucleic Acids Research 1982 10(8):2487-501, G. Simons et al., Gene 1984 28:55-64, P. W. Gray et al., Nature, 1982 295:503-508, D. Novick et al., EMBO Journal, 1983 2:1527-30.

In embodiments where the subject has, or is susceptible to, an infection, administration of the formula 1 compound is optionally accompanied by administration of an agent that can inhibit γ-glutamylcysteine synthetase in the subject. Such inhibition can enhance the capacity of the formula 1 compounds to inhibit replication of pathogens or to sensitize pathogens to metabolites derived from the formula 1 compounds. Suitable γ-glutamylcysteine synthetase inhibitors include buthionine-sulfoximine (BSO), which may be in the form of, e.g., L-buthionine-S-sulfoximine or L-buthionine-R-sulfoximine. Other compounds which can be used to perform this function include sesquiterpene lactones and butylated hydroxy anisole. All of these are commercially available. Such compounds, e.g., BSO, make infected cells more susceptible to the action of the formula 1 compounds, in particular, halogenated formula 1 compounds, e.g., 16-bromoepiandrosterone. The effects of hypohalogenous acid can be enhanced by the administration of an inhibitor of γ- glutamylcysteine synthetase, such as BSO, which, as discussed herein, can render infected cells more susceptible to hypohalogenous acid.

Some compounds that inhibit γ-glutamylcysteine synthetase have significant toxicity toward at least some subjects. Such compounds are suitable for this purpose if such toxicity can be counteracted or kept to levels which are acceptable. Such compounds include pentathionine-sulfoximine, hexathionine-sulfoximine, heptathionine-sulfoximine, prothionine-sulfoximine and methionine sulfoximine. When these compounds are used, they are used in amounts that limits their toxicity.

In any of the methods disclosed herein, a treatment may be interrupted briefly or for extended periods of time. The reason for such interruption can be any of a wide variety, e.g., patient non-compliance, apparent improvement in a subject's condition or by design. Any such interruption would not take a regimen outside the scope of the present invention. For example, a patient might miss a day or several days of administration. Similarly, the regimen might call for administration of one or more compounds for one or more day, and then non-administration of the one or more compounds for one or more day, and then resumption of the administration of the one or more compounds. Furthermore, a regimen according to the present invention can be altered in view of a patient's current condition, and can continue for any length of time, including the entire subject's lifetime.

Dosages of the formula 1 compounds and the other therapeutuic agents described herein are suitably determined depending on the individual case. Factors that affect dosing include a subject's symptoms, age, sex and the route of administration. The amount of a therapeutic agent that is incorporated into a pharmaceutical formulation varies with the dosage form, solubility and chemical properties of the compound, administration route, administration scheme and the like. An effective amount for a particular patient may vary depending on factors such as the condition being treated, the overall health of the patient, the method route and dose of administration and the severity of side effects. Dosages should be varied according to side effects (if any) and blood cell counts, which may be monitored frequently, e.g., every several days or at longer intervals such as weekly or monthly. In some aspects, the dose begins with an amount somewhat less than the optimum dose and it is increased by small increments thereafter until the desired or optimum effect is achieved.

For administration of γ-IFN, a volume of about 1 mL of a solid or liquid sublingual formulation that comprises about 100 micrograms of γ-IFN may be used. An exemplary liquid formulation comprises a saline solution containing 45 weight % β-hydroxypropylcyclodextrin. It would be expected that such a dosage would provide in the range of 30 to 40 micrograms of γ-IFN to the patient's blood. Such sublingual formulations would be held under the patient's tongue for a period of time sufficient to allow some or all of the γ-IFN to be delivered to the patient while held under the patient's tongue. Such administration has not been previously known in the art, in which conventionally, it has been thought that administration of γ-IFN must be by injection, e.g., subcutaneous injection. Subcutaneous injection of γ-IFN is associated with unwanted side effects, including fatigue, headache, night sweats, fever, local pain at the injection site, nausea, vomiting, diarrhea and others. The above-described sublingual γ-IFN formulations of the present invention is an aspect of the present invention, which can be of use in accordance with other aspects of the present invention as described herein. In general, however, a wide variety of routes of administration could be employed for γ-IFN in accordance with the present invention, including those disclosed in U.S. Patent No. 5,145,677.

In some aspects, the formula 1 compound is present in a formulation that comprises both micronized and non-micronized matereial. As used here, the expression "micronized" means that the compound has been micronized in accordance with any process for micronizing, a number of which are known in the art. The micronized particles preferably include a percentage which are less than about 10 µm in diameter, typically 5 µm or less. For example, at least about 80% of the formula 1 compound in a formulation of micronized comprises particles have a diameter of less than 5 microns. An alternative to micronizing a compound is to solubilize the compound and incorporate it into liposomes of appropriate size. The manufacture of liposomes and the insertion of active ingredients into such liposomes are well known in the art.

Methods to make invention formulations usually include the step of contacting a formula 1 compound with an excipient(s). In general the formulations are prepared by uniformly and intimately bringing into association the compound with liquid excipients or finely divided solid excipients or both, and then shaping the product as appropriate. Methods to make formulations and unit dosage forms suitable for the methods disclosed herein are generally known. See, e.g., Remington's Pharmaceutical Sciences, Mack Publishing Co., Easton, PA 1985, 17th edition, Nema et al., PDA J.Pharm. Sci. Tech. 1997 51:166-171, G. Cole, et al., editors, Pharmaceutical Coating Technology, 1995, Taylor & Francis, ISBN 0 136628915, H.A. Lieberman, et al., editors, Pharmaceutical Dosage Forms, 1992 2nd revised edition, volumes 1 and 2, Marcel Dekker, ISBN 0824793870, J.T. Carstensen. Pharmaceutical Preformulation, 1998, pages 1-306, Technomic Publishing Co. ISBN 1566766907.

In general, formulations suitable for oral administration are prepared as discrete units such as capsules, cachets or tablets each containing a predetermined amount of an active ingredient, e.g., a formula 1 compound, an interferon and/or an interleukin . Such formulations may comprise a powder, granules, a solution or a suspension in an aqueous liquid, a non-aqueous liquid or as an oil-in-water liquid emulsion or a water-in-oil liquid emulsion. The active ingredient(s) may also be presented as a bolus, electuary or paste.

A tablet or capsule may be made made by compression or molding, optionally with one or more excipients. Tablets may be prepared by compressing in a suitable machine a formula 1 compound in a free-flowing form such as a powder or granules, optionally mixed with a binder, lubricant, inert diluent, preservative, surface active or dispersing agent. Exemplary excipients for formulations such as tablets or parenteral solutions include lactose, sucrose, dextrose, a cellulose such as a microcrystalline cellulose or carboxymethyl cellulose, a lubricant such as magnesium stearate, a preservative such as EDTA, a starch such as corn starch and an α-, β- or γ-cyclodextrin such as hydroxypropyl-β-cyclodextrin. Molded tablets may be made by molding in a suitable machine a mixture of the powdered active ingredient(s) moistened with an inert liquid diluent. The tablets may optionally be coated or scored and optionally are formulated so as to provide slow or controlled release of the active ingredient(s) therefrom.

Formulations suitable for parenteral administration include aqueous sterile injection solutions which may contain anti-oxidants, buffers, bacteriostats and solutes which render the formulation isotonic with the blood of the intended recipient. Aqueous and non-aqueous sterile suspensions optionally include suspending agents, thickening agents or complexing agents, including an α-, β- or γ-cyclodextrin such as hydroxypropyl-β-cyclodextrin. Parenteral formulations may comprise a powder or granular material that contains a formula 1 compound and optionally one or more additional excipients, which is dispensed in a suitable container, e.g., a sterile hermetically sealed vial, to which one or more liquid excipients are added.

The formulations are presented in unit-dose or multi-dose containers, for example sealed ampules and vials, and may be stored in a freeze-dried (lyophilized) condition requiring only the addition of the sterile liquid excipient, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof.
Invention formulations include controlled release pharmaceutical formulations containing an active ingredient(s) ("controlled release formulations") in which the release of the active ingredient(s) is controlled and regulated to allow less frequency dosing or to improve the pharmacokinetic or toxicity profile of a given active ingredient(s).

An effective dose of active ingredient(s) depends at least on the nature of the condition being treated, toxicity, whether the compound(s) is being used prophylactically (lower doses) or against an active infection or condition, the method of delivery, and the pharmaceutical formulation, and will be determined by the clinician using conventional dose escalation studies. It can be expected to be from about 0.05 to about 30 mg/kg body weight for a daily dosage. For example, for topical delivery, the daily candidate dose for an adult human of approximately 70 kg body weight will typically range from about 3 mg to about 1200 mg, generally between about 10 mg and about 350 mg, and may take the form of a single dose or multiple subdoses using one or several administration routes or sites. Solid or liquid unit dosages may comprise about 10 mg, 25 mg, 50 mg, 100 mg, 200 mg, 300 mg, 400 mg or 500 mg of a formula 1 compound. Dosages for oral administration and parenteral administration will generally comprise about 10 to about 1000 mg/day of a formula 1 compound.

The formula 1 compounds can be administered daily or intermittently, i.e., dosing on one day, followed by no dosing for at least a day and then repeated dosing after that. An exemplary regimen is a weekly schedule where dosing is every other day for 3 doses, followed by no dosing for 2 days and then optionally followed by dosing is every other day for 3 doses again. Daily dosing regimens include dosing for 2, 3, 4, 5, 6, 7 or more consecutive days, optionally followed by no dosing for 1, 2, 3, 4 or more days (or about 4 to about 24 weeks) and then a repeated dosing for 2, 3, 4, 5, 6, 7 or more consecutive days.

In other related treatment regimens, the subject receives 1-5 daily formula 1 compound doses in the first week of treatment, followed by no dosing with a formula 1 comopound for about 1-30 weeks, typically about 4-20 weeks, and this is then optionally followed by another week with 1-5 daily doses.

At the end of a treatment regimen, dosing of the formula 1 compound may be tapered off over a period of days or several weeks, e.g., each week after the initial dosing regimen is complete, the daily dose is reduced by about 25-50% and this is administered for a week, optionally followed by another week of dosing with a further 25-50% reduced daily dose.

It should be understood that in addition to the ingredients particularly mentioned above the formulations of this invention may include agents or excipients that are conventional in the art having regard to the type of formulation in question, for example those suitable for oral administration may include flavoring agents.

The invention further provides for the use of veterinary compositions that comprise at least one formula 1 compound together with a veterinary excipient(s) therefor. Generally, veterinary excipients are materials useful for the purpose of administering the composition and may be solid, liquid or gaseous materials that are otherwise inert or acceptable in the veterinary art and are compatible with the formula 1 compound(s). These veterinary compositions may be administered orally, parenterally or by any other desired route.

Formulations that comprise a liposome or lipid complex that comprises a formula 1 compound(s), are also invention embodiments for treating subjects suffering from a blood cell deficiency such as TP or NP. Such formulations are prepared according to known methods, e.g., U.S. patents 4427649, 5043165, 5714163, 5744158, 5783211, 5795589, 5795987, 5798348, 5811118, 5820848, 5834016 and 5882678. The liposomes optionally contain an additional therapeutic agent(s), e.g., amphotericin B, cis-platin, adriamycin, a protease inhibitor, a nucleoside analog or a nucleotide analog, such as one of the agents mentioned herein. The liposomes can be delivered to a subject by any standard route, e.g., oral, aerosol or parenteral (e.g., i.v.) administration. Liposome formulations can be used to enhance delivery of the formula 1 compound(s) to certain cell types such as tumor cells (see e.g., U.S. patent 5714163) or to cells of the reticuloendothelial system ("RES"). The RES includes macrophages, mononuclear phagocytic cells, cells lining the sinusoids of the spleen, lymph nodes, and bone marrow, and the fibroblastic reticular cells of hematopoietic tissues.

The following examples further exemplify, but are not intended to limit the invention.

Example 1. Enhanced hemopoiesis in mammals with immune injury from radiation exposure. Animal experiments are used to demonstrate the effect of formula 1 compounds on hemopoiesis after immune system injury due to radiation. Hemopoiesis in the murine immune system after radiation is optionally examined because of the similar responses of murine and human hemopoiesis to drugs and toxic insults (see, e.g., J.H. Hendry and B.I. Lord, editors, Radiation toxicology: Bone marrow and leukaemia 1995 Taylor & Francis Inc., London).

In an exemplary protocol, B6D2F1/J female mice (Jackson Laboratory, Bar Harbor, ME), 18-24 weeks of age, 22-30 g body weight, are obtained and held in quarantine for two weeks. Up to 10 mice are housed in sanitized 46 x 24 x 15 cm polycarbonate boxes with filter covers (Microlsolator; Lab Products, Inc, Maywood, NJ) on autoclaved hardwood chip bedding. Mice are given feed and acidified (pH 2.5) water freely. The animal holding room is maintained with conditioned fresh air at approximately 21 °C and 50° (±10%) relative humidity and with a 12-h light/dark full spectrum lighting cycle.

Mice are placed in ventilated Plexiglas containers and exposed bilaterally to gamma-radiation from a ⁶⁰Co source. Exposure time is adjusted so that each animal received a midline tissue-absorbed dose of 1-12 Gy at a nominal dose rate of 0.4 Gy/min at ambient temperature. Using a standardized technique, the midline dose rate is measured by placing a 0.5 cc tissue-equivalent ionization chamber at the center of a 2.5-cm diameter cylindrical acrylic mouse phantom. The tissue-air ratio, defined as the ratio of the dose rate measured in the phantom to the dose rate in free air, for this array is about 0.96. Variation within the exposure field is less than about 4%. Dosimetric measurements are made in accordance with the American Association of Physicists in Medicine protocol for the determination of absorbed dose from high-energy photon and electron beams (Med. Phys. 1983 10:741-771). Sham-irradiated mice are treated in the same way as the irradiated animals, except that the animals are not irridiated.

Various formula 1 compounds e.g., compounds such as those in the compound groups described herein are formulated with a suitable vehicle (e.g., PEG-400) or sterile 0.9% NaCl (saline) optionally containing other excipients such as a cyclodextrin. The compounds are injected subcutaneously in a volume of about 0.1 mL or they are delivered orally or they are administered by another route. Doses typically range from about 1 mg/kg to about 350 mg/kg, e.g., about 1, 10, 20, 40, 80, 160 or 320 mg/ kg.

Blood (0.6-1.0 mL) is obtained from halothane-anesthetized mice by cardiac puncture using a heparinized syringe attached to a 21-gauge needle. Blood is collected in EDTA-containing sample tubes. Mice are euthanized by cervical dislocation after blood collection. White blood cell (WBC), red blood cell (RBC) and platelet (PLT) counts are performed using, e.g., a Hematology System 9000 (Biochem Immunosystems). Wright-stained blood smears from the same samples are made for differential counts of neutrophils and lymphocytes by light microscopy.

Hemopoietic progenitor cells committed to granulocyte-macrophage differentiation (GM-CFC) are assayed by a single-layer modification of a double-layer semisolid agar technique essentially as described (Patchen et al. Adv. Space Res. 1992 12:223-248). For example, femoral marrow is extracted and cell suspensions are prepared by flushing with 3 mL of McCoy's 5A medium containing 10% heat-inactivated fetal bovine serum (HIFBS; Hyclone, Logan, UT). Each cell suspension represented a pool of marrow from four femurs, i.e., both femurs from each of two mice. The total number of nucleated cells in each suspension is determined with, e.g., a Coulter counter. The agar-medium mixture consisted of equal volumes of 0.66% agar and double-strength supplemented CMRL 1066 medium (Gibco, Grand Island, NY). The medium is supplemented with final concentrations of 10% HIFBS, 5% tryptic soy broth, 5% heat-inactivated horse serum, antibiotics, and L-serine. One milliliter of the agar-medium mixture is added to each 35-mm plastic Petri dish (two dishes per suspension) and mixed with 50 µL of 0.1 ng/µL recombinant mouse GM-CSF (Genzyme, Cambridge, MA). Cell suspensions are then mixed into the agar-medium mixture to a final concentration of 0.5 x 10⁵ cells/mL for unirradiated animals, and 1.0 x 10⁵ or 1.5 x 10⁵ cells/mL for irradiated animals to ensure sufficient colonies per plate for quantitation. Control experiments are done to confirm linearity of colonies at cell concentrations of 0.5-1.5 x 10⁵ cells/ mL. Colonies (> 50 cells) are counted after seven days incubation in a 37 °C humidified environment containing 5% CO₂. The average of the counts for the two dishes is taken as the value for each pool. About six animals are used per group in each of two experiments.

For histological examination of myeloid hyperplasia in bone marrow after administration of the formula 1 compound, mice are euthanized with halothane, tissues are immersed in formalin, bones are decalcified and routine H&E-stained 6-µm paraffin sections are prepared.

For induced-infection studies, a clinical isolate of K. *pneumoniae,* capsule type 5 (strain AFRRI 7), that is kept frozen at 70 °C in skim milk, is grown overnight at 35 °C on Trypticase Soy Agar (Becton-Dickinson, Sparks, MD). Five typical colonies are inoculated into 8 mL of brain heart infusion broth (Becton-Dickinson) and incubated overnight at 35 °C. Two milliliters of this overnight suspension is inoculated into 95 mL of prewarmed brain heart infusion broth. The culture is incubated at 35 °C with shaking for approximately 2.5 h. The optical density of bacterial growth is monitored with a spectrophotometer at a wavelength of 550 nm. Late log-phase cells are ished and suspended in cold saline to yield 10⁹ viable bacteria per mL. Appropriate dilutions for inoculations are made in cold saline.

To induce a bacterial infection, all mice are injected sc with *K. pneumoniae* four days after sham-irradiation or irradiation when circulating leukocytes are depressed. Mice are inoculated sc rather than iv or ip, to establish infection leading to sepsis, but not rapid septic shock. After sc inoculations of *K*. *pneumoniae* in the mice, the infection remains largely localized to the injection site. *K. pneumoniae* are not detectable in blood of inoculated mice until a few hours before death.

Different doses of the bacteria are inoculated for each of three radiation dose levels (0, 1 or 3 Gy) to approximate the LD_{95/30}, because the effects of radiation on hemopoiesis and susceptibility to infection are dependent on the dose of radiation. The LD_{95/30} for bacteria at each radiation dose is calculated from probit analysis. The actual doses are estimated by dilution plating of inocula onto Trypticase Soy Agar and incubating overnight at 35 °C. Since different bacterial doses are expected to be needed for different radiation doses, the LD_{95/30} is estimated for each group and different mortality rates are observed in the vehicle-injected control groups. Bacterial doses for induced-infection experiments are prepared and calculated in the same manner.

Animals are checked frequently, e.g., once or twice daily, six or seven days per week, to monitor survival and to euthanize mice that are in a moribund state. To verify that mortality in the induced-infection experiments is associated with *K. pneumoniae* injection, heart blood from recently deceased animals (or moribund animals euthanized by cervical dislocation) is cultured overnight at 35 °C on Columbia sheep blood agar plates (Becton-Dickinson, Sparks, MD). Colonies are identified as *K. pneumoniae* by a suitable means, e.g., Biolog analysis.

For histological analysis of bone marrow, coded slides are scored blind using a five-level semiquantitative scale and the results analyzed with a randomization t-test to obtain exact P-values. Thirty-day survival values are compared using the generalized Savage (Mantel-Cox) procedure (BMDP Statistical Software, Inc, Los Angeles, CA). To calculate dose reduction factors (DRFs), probit analysis is performed on mortality data.

To test the ability of formula 1 compounds to ameliorate radiation-induced defects in hemopoiesis, mice are exposed to bilateral whole-body gamma-radiation and receive a dose of 3 Gy (or are sham-irradiated). One hour after irradiation or sham-irradiation, mice are injected with 320 mg/kg AED or PEG-400 vehicle. Between-group differences in blood cell elements, e.g., neutrophils, GM-CFC and platelets are generally determined. Irradiation results in a decrease in neutrophils at about four days after radiation compared to sham-irradiated animals.

Example 2. Enhanced hemopoiesis in mammals with immune injury from radiation exposure. In another exemplary protocol, C57BU6J male mice six week old are obtained from Jackson Bar Harbor Laboratory. Animals are weighed for three consecutive times to ascertain that they are gaining weight prior to experimentation. Animals are exposed to whole body irradiation of 6 to 8 Gy depending on the experiments, using, e.g., JL Sheperd & Associates Mark 68 ¹³⁷CS gamma unit. A single or several daily subcutaneous injections of a formula 1 compound is administered to the animals. Animals in survival experiments are monitored for about 21 days. A sublethal dose of about 2.0 X 10² pfu/mouse of human coxsackievirus B4 is administered to the animals to test for the capacity of the formula 1 compounds to enhance hemopoiesis and immune function. This virus dose is administered by e.g., intraperitoneal or subcutaneous injection about 1 to about 48 hours before or after whole body radiation exposure. Spleen and bone marrow preparation are stained for either one or two color FACS analysis essentially as described (e.g., M.E. Payette et al., J. Immunol. 1999 163:223).

Fluorescence activated cell sorting ("FACS") analysis is performed over a period of, e.g., about 21 days on spleen and bone marrow cells of mice exposed to about 6 Gy whole body irradiation and treated with a formula 1 compound and compared with untreated irradiated animals. Spleen cells are stained for cells such as CD4⁺ and CD8⁺ cells while bone marrow cells are stained for, e.g., CD11b /Mac-1 and B200.

Example 3. Enhancement of hemopoiesis in a human patient. A patient infected with the HIV virus was treated with 16α-bromoepiandrosterone ("BrEA"), using a physiological saline formulation containing 10 mg/mL of BrEA in solution, 36% w/v hydroxypropyl-β-cyclodextrin and 20 mg/ mL of micronized BrEA in suspension. The patient received two courses of treatment. The first course consisted of subcutaneous injection once per day of 80 mg of BrEA for 10 days. The second course of treatment commenced 74 days later and used the same formulation, except that deuterium oxide replaced water in the formulation. The second treatment course consisted of subcutaneous injection once per day of 80 mg of BrEA for 15 days. The patient's blood parameters were obtained at the start and at the end of each course of treatment. The results shown below indicated that the BrEA compound enhanced blood cell counts as shown by increased blood platelets and neutrophils after the second course of treatment or by increased NK cells after either treatment.

| Blood parameter | 1^{st} Course | | 2^{nd} Course | |
|---|---|---|---|---|
| | start | end | start | end |
| WBC (x 10⁹/L) | 2.38 | 2.47 | 3.57 | 5.66 |
| platelets (µL⁻¹) | 142 | 146 | 119 | 225 |
| neutrophils (x 10⁹/L) | 0.92 | 0.8 | 2.02 | 3.60 |
| monocytes (x 10⁹/L) | 0.35 | 0.3 | 0.34 | 0.54 |
| basophils (x 10⁹/L) | 0.02 | 0.01 | 0.01 | 0.06 |
| CD16⁺/56⁺ NK cells (mm⁻³) | 186 | 348 | 144 | 425 |
| CD4⁺ T cells (mm⁻³) | 93 | 116 | 113 | 125 |
| T suppressor cells (mm⁻³) | 344 | 452 | 351 | 513 |

Example 4. Parenteral formulation comprising micronized and non-micronized formula 1 compound. To1 L of physiological saline was added 450 g of hydroxypropyl-β-cyclodextrin to obtain a clear solution, which was brought to a volume of 1250 mL with saline. 12.5 g of 16α-bromoepiandrosterone was then added and the solution was stirred for 2 hours with a magnetic mixer. The solution was filter sterilized and 25 g of sterile micronized (average particle diameter of about 5 µm) 16α-bromoepiandrosterone was added. The solution was stirred for 20 hours at medium speed to obtain a solution with micronized material 16α-bromoepiandrosterone in suspension and non-micronized material in solution. Prior to use the solution is shaken and dispensed into sealed sterile vials for storage or it is directly dispensed into a syringe for immediate use by injection. A variation of this protocol would substitute duterium oxide for some or all of the water. Other variations would utilize a different formula 1 compound such as one of those named herein.

Example 5. Non-aqueous parenteral formulation containing a formula 1 compound. A formulation comprising 100 mg/mL 16α-bromoepiandrosterone ("BrEA"), ∼30% v/v PEG300, 30% v/v propylene glycol, 30% v/v benzyl benzoate and 2% v/v benzyl alcohol is prepared by suspending BrEA in polyethylene glycol 300, and sequentially adding propylene glycol and benzyl benzoate, to form a solution, which is diluted to the final desired volume with additional propylene glycol. An exemplary procedure is described below.

The calculated amount of polyethylene glycol 300 is added to a compounding vessel. Then, while mixing, the calculated amount of BrEA is added to the vessel, and mixed for at least 5 minutes to form a smooth, creamy liquid propylene glycol is added to the vessel, and mixed for a minimum of 5 minutes to form a uniform suspension. The calculated amount of benzyl benzoate is added to the vessel, and mixed for approximately 5 minutes to form a translucent liquid suspension. Propylene glycol is then added to achieve the desired final formulation, and mixed for approximately 5 minutes. The drug solution is transferred to a volume dispensing device set to deliver 1.2 mL per vial. Under nitrogen pressure, the solution is filtered through two 0.2 µm) polyvinylidene fluoride filters in series into 2 cc amber glass vials. The vials are capped with Teflon-coated, butyl-rubber stoppers and crimp sealed.

Example 6. Subcutaneous formulation. A BrEA formulation is prepared essentially as described above. The formulation contains 50 mg/mL BrEA, 40% v/v PEG 200, 2% v/v benzyl alcohol, 2% v/v benzyl benzoate and - 66% v/v propylene glycol (qs). The formulation is particularly suitable for subcutaneous administration of BrEA.

Example 7. Preparation of BrEA hemihydrate - procedure 1. Crude BrEA is prepared by bromination of epiandrosterone, followed by crystallization from methanol. BrEA hemihydrate, i.e., (BrEA)₂.H₂O, is prepared by dissolving 25 g of crude BrEA in 75 mL of refluxing ethanol with moderate agitation. To the BrEA solution 12.5 mL of water is slowly added while maintaining the solution at reflux with agitation. Agitation of the solution is maintained and the solution is then allowed to cool to about 20-25°C and kept at about 20-25°C for about 15 minutes to obtain a suspension of BrEA hemihydrate crystals. The crystals are recovered by filtration, ished with a solution of 25 mL of water:ethanol (5:1 v/v) at about 20-25°C and then vacuum dried for about 13 hours at 50-60°C until the product weight is constant. The crystals are primarily rod and needle shaped, with smaller amounts of other shapes such as tablets.

Example 8. Preparation of BrEA hemihydrate - procedure 2. The hemihydrate is prepared by dissolving 10 g of crude BrEA in 40 mL of refluxing acetone with moderate agitation. To the BrEA solution 4.0 mL of water is slowly added while maintaining the solution at reflux with agitation. Agitation of the solution is maintained and the solution is then allowed to cool to about 20-25°C and kept at about 20-25°C for about 15 minutes to obtain a suspension of BrEA hemihydrate crystals. The crystals are recovered by filtration, washed with a solution of 6.0 mL of water:acetone (10:1 v/v) at about 20-25°C and then vacuum dried overnight (about 13-15 hours) at 50-60°C until the product weight is constant.

Example 9. Liposome formulation. Liposomes suitable for parenteral administration are prepared as follows. 400 mg of phosphatidyl choline and 80 mg of BrEA are dissolved in chloroform and methanol (2:1 v/v) and the solution is dried by rotary evaporation under reduced presure. The resulting film is rehydrated by adding 8.0 mL of a 0.9% w/v NaCl solution and agitating the solution. The sizes of the liposomes are optionally measured, e.g., by photon correlation spectroscopy (Malvern Zetasizer 3000 or equivalent). The liposomes are optionally sized by, e.g., sonication to reduce the average size below 400 mn, or by filtration using suitable filters. Similar procedures are used to prepare liposome preparations that contain a formula 1 compound at about 15-100 mg/mL. The formulation is used to deliver the compound orally or parenterally (e.g., intramuscular, intravenoue or subcutaneous).

Example 10. BrEA formulation. Two lots of a non-aqueous BrEA formulation are made at a BrEA concentration of 50 mg/mL in 25% v/v polyethylene glycol 300, 12.5% v/v dehydrated ethyl alcohol, 5% v/v benzyl benzoate, and 57.5% v/v propylene glycol essentially as follows. The excipients are as follows. Propylene glycol, USP, Arco Chemical, polyethylene glycol, 300, NF, Union Carbide dehydrated ethanol, USP, McCormick Distilling, benzyl benzoate, USP, Spectrum Pharmaceuticals.

The formulation is prepared by suspending BrEA in polyethylene glycol 300, and sequentially adding propylene glycol, benzyl benzoate, and dehydrated ethyl alcohol to form a solution, which is diluted to the final desired volume with additional propylene glycol. An exemplary procedure is described below.

The calculated amount of polyethylene glycol 300 is added to a compounding vessel. Then , while mixing, the calculated amount of BrEA is added to the vessel, and mixed for at least 5 minutes to form a smooth, creamy liquid propylene glycol is added to the vessel, and mixed for a minimum of 5 minutes to form a uniform suspension. The calculated amount of benzyl benzoate is added to the vessel, and mixed for approximately 5 minutes to form a translucent liquid suspension. Dehydrated alcohol is added to the vessel, and mixed for approximately 5 minutes to form a clear, colorless solution. Propylene glycol is then added to achieve the desired final formulation, and mixed for approximately 5 minutes. The drug solution is transferred to a volume dispensing device set to deliver 1.2 mL per vial. Under nitrogen pressure, the solution is filtered through two 0.2 µm polyvinylidene fluoride filters in series into 2 cc amber glass vials, which are then sealed.

### References

Fliedner TM, Nothdurft W, Heit H. Biological factors affecting the occurrence of radiation syndromes. In: Broerse JJ, MacVittie TJ, editors. Response of different species to total body irradiation. Boston: Martinus Nijhoff Publishers, 1984. p. 209-19.
Hendry JH, Feng-Tong Y. Response of bone marrow to low LET irradiation. In: Hendry JH, Lord BI, editors. Radiation toxicology: bone marrow and leukaemia. London: Taylor and Francis, 1995. p. 91-116.
Mauch P, Constine L, Greenberger J, Knospe W, Sullivan J, Liesveld JL, Deeg HJ. Hematopoietic stem cell compartment: acute and late effects of radiation therapy and chemotherapy. Int J Radiat Oncol Biol Phys 1995;31: I 19-39.
Elliott TB, Madonna GS, Ledney GD, Brook I. Combined therapy for postirradiation infection. Microecol Ther 1989;19:105-8.
Madonna GS, Ledney GD, Elliott TB, Brook I, Ulrich JT, Myers KR, Patchen ML, Walker RI. Trehalose dimycolate enhances resistance to infection in neutropenic animals. Infect Immun 1989;57:2495-501.
Patchen ML, MacVittie TJ, Solberg BD, D'Alesandro MM, Brook 1. Radioprotection by polysaccharides alone and in combination with aminothiols. Adv Space Res 1992;12:(2)233-48.
Peterson VM, Adamovicz JJ, Elliott TB, Moore MM, Madonna GS, Jackson III WE, Ledney GD, Gause WC. Gene expression of hematoregulatory cytokines is elevated endogenously after sublethal gamma irradiation and is differentially enhanced by therapeutic administration of biologic response modifiers. J Immunol 1994;153:2321-30. Fedorocko P, Mackova O. Combined modality radiopro. tection: enhancement of survival and hematopoietic recovery in gamma-irradiated mice by the joint use of liposomal muramyl tripeptide phosphatidylethanolamine (MTP-PE) and indomethacin. Int J Immunopharmacol 1996;18:329-37.
Neta R, Oppenheim JJ, Douches SD. Interdependence of the radioprotective effects of human recombinant interleukin la, tumor necrosis factor a, granulocyte colony-stimulating factor, and murine recombinant granulocyte-macrophage colony-stimulating factor. J Immunol 1988;140:108-11.
Waddick KG, Song CW, Souza L, Uckun FM. Comparative analysis of the in vivo radioprotective effects of recombinant granulocyte colony-stimulating factor (G-CSF), recombinant granulocyte-macrophage CSF, and their combination. Blood 1991;77:2364-71.
MacVittie TJ, Weiss JF, Browne D. Advances in the treatment of radiation injuries. Tarrytown, NY: Pergamon/Elsevier Science Inc, 1996.
Kumar KS, Srinivasan V, Palazzolo D, Kendrick JM, Clark EP. Synergistic protection of irradiated mice by a combination of iloprost and misoprostol. In: Honn KV, Nigam S, Marnett LJ, editors. Eicosanoids and other bioactive lipids in cancer, inflammation, and radiation injury 2. New York: Plenum Press, 1997. p. 831-9.
Ben-Nathan D, Lachmi B, Lustig S, Feuerstein G. Protection by dehydroepiandrosterone in mice infected with viral encephalitis. Arch Virol 1991;120:263-71.
Loria RM, Padgett DA. Androstenediol regulates systemic resistance against lethal infections in mice. Arch Virol 1992;127:103-15.
Loria RM, Padgett DA. Mobilization of cutaneous immunity for systemic protection against infections. Ann N Y Acad Sci 1992;650:363-6.
Rasmussen KR, Martin EG, Healey MC. Effects of dehydroepiandrosterone in immunosuppressed rats infected with Cryptosporidium parvum. J Parasitol 1993;79:364-70.
Araneo B, Daynes R. Dehydroepiandrosterone functions as more than an antiglucocorticoid in preserving immunocompetence after thermal injury. Endocrinology 1995; I 36:393-401.
Padgett DA, Sheridan JA, Loria RM. Steroid hormone regulation of a polyclonal TH2 immune response. Ann N Y Acad Sci 1995;774:323-5.
Gianotti L, Alexander JW, Fukushima R, Pyles T. Steroid therapy can modulate gut barrier function, host defense, and survival in thermally injured mice. J. Surg. Res. 1996;62:53-8.
Padgett DA, Loria RM, Sheridan JF. Endocrine regulation of the immune response to influenza virus infection with a metabolite of DHEA-androstenediol. J. Neuroimmunol. 1997;78:203-11.
Suitters AI, Shaw S, Wales MR, Porter JP, Leonard J, Woodger R, Brand H, Bodmer M, Foulkes R. Immune enhancing effects of dehydro-epiandrosterone and dehydroepiandrosterone sulphate and the role of steroid sulphatase. Immunology 1997;91:314-21.
Carr DJ. Increased levels of IFN-gamma in the trigeminal ganglion correlate with protection against HSV-1 induced encephalitis following subcutaneous administration with androstenediol. J. Neuroimmunol. 1998;89:160-7.
Daigle J, Carr DJ. Androstenediol antagonizes herpes simplex virus type 1-induced encephalitis through the augmentation of type I IFN production. J. Immunol. 1998;160:3060-6.
Yen SSC, Morales AJ, Khorram O. Replacement of DHEA in aging men and women. Potential remedial effects. Ann N Y Acad Sci 1995 774:128-42.
Danenberg HD, Alpert G, Lustig S, Ben-Nathan D. Dehydroepiandrosterone protects mice from endotoxin toxicity and reduces tumor necrosis factor production. Antimicrob Agents Chemother 1992;36:2275-9.
Araghi-Niknam M, Zhang Z, Jiang S, Call O, Eskelson CD, Watson RR. Cytokine dysregulation and increased oxidation is prevented by dehydroepiandrosterone in mice infected with murine leukemia retrovirus. Proc Soc Exp Biol Med 1997;216:386-91.
Hernandez-Pando R, De La Luz Streber M, Orozco H, Arriaga K, Pavon L, AI-Nakhli SA, Rook GA. The effects of androstenediol and dehydroepiandrosterone on the course and cytokine profile of tuberculosis in BALB/c mice. Immunology 1998;95:234-41.
Inserra P, Zhang Z. Ardestani SK, Araghi-Niknam M, Liang B, Jiang S, Shaw D, Molitor M, Elliott K, Watson RR. Modulation of cytokine production by dehydroepiandrosterone (DHEA) plus melatonin (MLT) supplementation of old mice. Proc Soc Exp Biol Med 1998;218:76-82.
Kimura M, Tanaka S-I, Yamada Y, Kiuchi Y, Yamakawa T, Sekihara H. Dehydroepiandrosterone decreases serum tumor necrosis factora and restores insulin sensitivity: independent effect from secondary weight reduction in genetically obese Zucker fatty rats. Endocrinology 1998;139:3249-53.
Moynihan JA, Callahan TA, Kelley SP, Campbell LM. Adrenal hormone modulation of type 1 and type 2 cytokine production by spleen cells: dexamethasone and dehydroepiandrosterone suppress interleukin-2, interleukin-4, and interferon-gamma production in vitro. Cell. Immunol. 1998;184:58-64.
Padgett DA, Loria RM. Endocrine regulation of murine macrophage function: effects of dehydroepiandrosterone, androstenediol, and androstenetriol. J Neuroimmunol 1998;84:61-8.
MacVittie TJ, Farese AM. Experimental approaches to treatment of radiation-induced hemopoietic injury. In: Hendry JH, Lord BI, editors. Radiation toxicology: bone marrow and leukaemia. London: Taylor & Francis, 1995. p. 141-94.
Patchen ML. Single and combination cytokine therapies for the treatment of radiation-induced hemopoietic injury. Adv Biosci 1996;94:21-36.
Neta R. Modulation of radiation damage by cytokines. Stem Cells Dayt 1997;15(Suppl. 2):87-94.
Sonka J, Strakova M. Survival of irradiated mice pretreated by dehydroepiandosterone. Agressologie 1970;11:42 I-6.
Padgett DA, Loria RM. In vitro potentiation of lymphocyte activation by dehydroepiandrosterone, androstenediol, and androstenetriol. J Immunol 1994 153:1544-52.
McLachlan JA, Serkin CD, Bakouche O. Dehydroepiandrosterone modulation of lipopolysaccharide-stimulated monocyte cytotoxicity. J Immunol 1996;156:328-35.
Risdon G, Kumar V, Bennett M. Differential effects of dehydroepiandrosterone on murine lymphopoiesis and myelopoiesis. Exp. Hematol. 1991;19:128-31.
Hendry JH, Lord BI, editors. Radiation toxicology: bone marrow and leukaemia. London: Taylor & Francis Inc, 1995.
Myska JC, Adams TL, Bhatt RC. Broom JG. Pitcher CD, Sharpnack FM II, St. John TJ, Tomes BA, Vavrina G, Gerstenberg HM. Arrays for use at the cobalt irradiation facility. AFRRI Technical Report 97-2. Armed Forces Radiobiology Research Institute, September, 1997. ).
American Association of Physicists in Medicine (AAPM Task Group 21A protocol for the determination of absorbed dose from high-energy photon and electron beams. Med. Phys. 1983 10:741-71.
Hammer JG, Harris HA, Komm BS, Goodwin T, Goldberg K, Van Winkle T. Comparison of gross and histologic effects of six vehicles for subcutaneous injection of hydrophobic steriod mimetic compounds in rats. Contemp Top Lab Anim Sci 1996;35:72.
Seed TM, Fritz TE, Tolle DV, Poole CM, Lombard LS, Doyle DE, Kaspar LV, Cullen SM, Carnes BA. Survival patterns and hemopathological responses of dogs under continuous gamma irradiation. In: Broerse JJ, MacVittie TJ, editors. Response of different species to total body irradiation. Boston: Martinus Nijhoff Publishers, 1984. p. 137-159.
Fritz TE. Seed TM, Tolle DV, Lombard LS. Late effects of protracted whole-body irradiation of beagles by cobalt-60 gamma rays. In: Thompson RC, McGaffey JA, editors. Life-span radiation effects studies in animals: what can they tell us? Washington, DC: Office of Scientific and Technical Information, United States Department of Energy, 1986. p. 116-141.
Seed TM, Kaspar LV. Probing altered hematopoietic progenitor cells of preleukemic dogs with JANUS fission neutrons. Radiat Res 1991;128:581-S86.
Lord BI, Hendry JH. Radiation toxicology: bone marrow and leukaemia. In: Hendry JH, Lord BI, editors. Radiation toxicology: bone marrow and leukaemia. London: Taylor & Francis, 1995. p. 1-21.
Vial T, Descotes J. Clinical toxicity of cytokines used as haemopoietic growth factors. Drug Safety 1995;13:371-406.
Araneo BA, Shelby J, Li GZ, Ku W, Daynes RA. Administration of dehydroepiandrosterone to burned mice preserves normal immunologic competence. Arch Surg 1993;128:318-25.
Daynes RA, Araneo BA, Ershler WB, Maloney C, Li GZ, Ryu SY. Altered regulation of IL-6 production with normal aging. Possible linkage to the age-associated decline in dehydroepiandrosterone and its sulfated derivative. J Immunol 1993 150:5219-30.
Kim HR, Ryu SY, Kim HS, Choi BM, Lee EJ, Kim HM, Chung HT. Administration of dehydroepiandrosterone reverses the immune suppression induced by high dose antigen in mice. Immunol Invest 1995;24:583-93.
Seed TM, Cullen SM, Kaspar LV, Tolle DV, Fritz TE. Hemopathologic consequences of protracted gamma irradiation: alterations in granulocyte reserves and granulocyte mobilization. Blood 1980;56:42-51.
Fujikawa H, Okura F, Kuwano Y, Sekizawa A, Chiba H, Shimodaira K, Saito H, Yanaihara T. Steroid sulfatase activity in osteblast cells. Biochem Biophys Res Commun 1997;231:42-7.
Lea-Currie YR, Wu SM. McIntosh MK. Effects of acute administration of dehydroepiandrosteron-sulfate on adipose tissue mass and cellularity in male rats. Int J Obes Relat Metab Disord 1997 21:147-54.
Nakashima N, Haji M, Sakai Y, Ono Y, Umeda F, Nawata H. Effect of dehydroepiandrosterone on glucose uptake in cultured human fibroblasts. Metabolism 1995;44:543-8.
Meikle AW, Daynes RA, Araneo BA. Adrenal androgen secretion and biologic effects. Endocrinol Metab Clin North Am 1991;20:381-400. Mohan PF, Jacobson MS. Inhibition of macrophage superoxide generation by dehydroepiandrosterone. Am J Med Sci 1993;306:10-5.
Whitcomb JM, Schwartz AG. Dehydroepiandrosterone and 16 alpha-Br-epiandrosterone inhibit 120-tetradecanoylphorbol-13-acetate stimulation of superoxide radical production by human polymorphonuclear leukocytes. Carcinogenesis 1985;6:333-5.
Pascale R, Garcea R, Ruggiu ME, Daino L, Frassetto S, Vannini MG, Cozzolino P, Lenzerini L, Feo F, Schwartz AG. Decreased stimulation by 120-tetradecanoylphorbol-13-acetate of superoxide radical production by polymorphonuclear leukocytes carrying the Mediterranean variant of glucose-6-phosphate dehydrogenase. Carcinogenesis 1987;8:1567-70.
Suzuki T, Suzuki N, Daynes RA, Engleman EG. Dehydro-epiandrosterone enhances IL2 production and cytotoxic effector function of human T cells. Clin Immunol Immunopathol 1991;61:202-211.
Meikle AW, Dorchuck RW, Araneo BA, Stringham JD, Evans TG, Spruance SL, Daynes RA. The presence of a dehydroepiandrosterone specific receptor binding complex in murine T cells. J Steroid Biochem Mol Biol 1992;42:293-304.
Okabe T, Haji M, Takayanagi R, Adachi M, Imasaki K, Kurimoto F, Watanabe T, Nawata H. Up-regulation of high-affinity dehydro-epiandrosterone binding activity by dehydroepiandrostecone in activated human T lymphocytes. J Clin Endocrinol Metab 1995;80:2993-6.
Maradny E, Kanayama N, Maehara K, Kobayashi T, Terao T. Dehydroepiandrosterone sulfate potentiates the effect of interleukin-8 on the cervix. Gynecol Obstet Invest 1996;42:191-5. [63] Waxman DJ. Role of metabolism in the activation of dehydroepiandrosterone as a peroxisome proliferator. J Endocrinol 1996150:S129-S147.
Peters JM, Zhou YC, Ram PA, Lee SS, Gonzalez FJ, Waxman DJ. Peroxisome proliferator-activated receptor alpha required for gene induction by dehydroepiandrosterone-3 beta-sulfate. Molec Pharmacol 1996;50:67-74.
Loria RM, Padgett DA, Huynh PN. Regulation of the immune response by dehydroepiandrosterone and its metabolites. J Endocrinol 1996;150:S209-S220.
Morfin R, Courchay G. Pregnenolone and dehydroepiandrosterone as precursors of native 7-hydroxylated metabolites which increase the immune response in mice. J Steroid Biochem Mol Biol 1994;50:91-100.
Frenkel RA, Slaughter CA, Orth K, Moomaw CR, Hicks SH, Snyder JM, Bennett M, Prough RA, Putnam RS, Milewich L. Peroxisome proliferation and induction of peroxisomal enzymes in mouse and rat liver by dehydroepiandrosterone feeding. J Steroid Biochem 1990;35:333-42.
Yamada J, Sakuma M, Ikeda T, Fukuda K, Suga T. Characteristics of dehydroepiandrosterone as a peroxisome proliferator. Biochim Biophys Acta 1991;1092:23343.
Coffey DS. Androgen action and the sex accessory tissues. In: Knobil E, Neill J, editors. The physiology of reproduction. New York: Raven Press, 1988. p. 1081-119 [Chapter 24].
Angele MK, Ayala A, Cioffi WG, Bland KI, Chaudry IH. Testosterone: the culprit for producing splenocyte immune depression after trauma hemorrhage. Am J Physiol 1998 274:C1530-C1536.

## Claims

1. Use of a compound for the preparation of a medicament for the prophylaxis or treatment of neutropenia or thrombocytopenia in a human or primate subject, wherein the compound has the structure 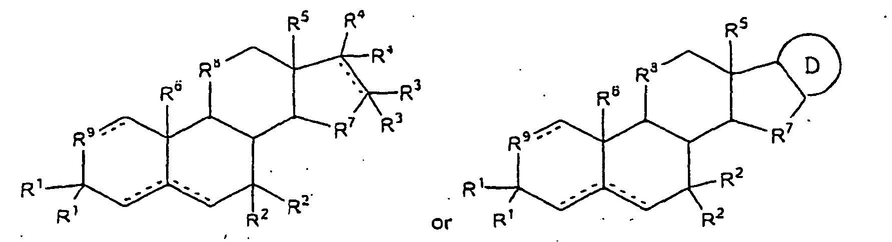
wherein, hydrogen at tne 5-position, if present is in the α-configuration;
one R¹ is -OR^{PR}, -SR^{PR}, -N(R^{PR})₂, -NO₂, -OPO₃H, an ester, a thioester, a phosphoester, a phosphothioester, a phosphonoester, a sulfite ester, a sulfate ester, an amide having the structure -NH-C(O)-organic moiety, an amino acid, a peptide, an ether, a thioether, a carbonate, a carbamate, a thioacetal, an optionally substituted monosaccharide, an optionally substituted oligosaccharide or a polymer;
the other R¹ is -H, -CN, acyl, thioacyl, optionally substituted alkyl, optionally substituted alkenyl, optionally substituted alkynyl, optionally substituted aryl, optionally substituted heteroaryl or an optionally substituted heterocycle;
R² independently are -H, -OR^{PR}, -SR^{PR}, -N(R^{PR})₂, =NOH, -CN, -NO₂,-OSO₃H, -OPO₃H, an ester, a thioester, a phosphoester, a phosphothioester, a phosphonoester, a sulfite ester, a sulfate ester, an amide, an amino acid, a peptide, an ether, a thioether, acyl, thioacyl, a carbonate, a carbamate, a thioacetal, a halogen, optionally substituted alkyl, optionally substituted alkenyl, optionally substituted alkynyl, optionally substituted ary, optionally substituted heteroaryl, an optionally substituted heterocycle or a polymer;
R³ independently are -H, -OR^{PR}, =O, -SR^{PR}, =S, -N(R^{PR})₂, =NOH, -CN,-NO₂, -OSO₃H, -OPO₃H, an ester, a thioester, a phosphoester, a phosphothioester, a phosphonoester, a sulfite ester, a sulfate ester, an amide, an amino acid, a peptide, an ether, a thioether, acyl, thioacyl, a carbonate, a carbamate, a thioacetal, a halogen, optionally substituted alky, optionally substituted alkenyl, optionally substituted alkynyl, optionally substituted aryl, optionally Substituted heteroaryl, an optionally substituted heterocycle or a polymer;
one R⁴ is -OR^{PR}, -SR^{PR}, -N(R^{PR})₂, -NO₂, -OPO₃H. an ester, a thioester, a phosphoester, a phosphothioester, a phosphonoester, a sulfite ester, a sulfate ester, an amide having the structure -NH-C(O)-organic moiety, an amino acid, a peptide, an ether, a thioether, a carbonate, a carbamate, a thioacetal, an optionally substituted monosaccharide, an optionally substituted oligosaccharide or a polymer;
the other R⁴ is -H, -CN, acyl, thioacyl, optionally substituted alky, optionally substituted alkenyl, optionally substituted alkynyl, optionally substituted aryl, optionally substituted heteroaryl or an optionally substituted heterocycle, or both R⁴ together are =NOH;
R⁵ is -H, optionally substituted alkyl, optionally substituted alkenyl or optionally substituted alkynyl;
R⁶ is optionally substituted alkyl, optionally substituted alkenyl or optionally substituted alkynyl;
R⁷ is -CHR¹⁰-, -CHR¹⁰-CHR¹⁰-, -CHR¹⁰-CHR¹⁰-CHR¹⁰-, -CHR¹⁰-O-CHR¹⁰-, -CHR¹⁰-S-CHR¹⁰-, -CHR¹⁰-NR^{PR}-CHR¹⁰-, -O-, -O-CHR¹⁰-, -S-, -S-CHR¹⁰-, -NR^{PR}- or -NR^{PR}-CHR¹⁰-;
R⁸ and R⁹ independently are -CHR¹⁰-, -CHR¹⁰-CHR¹⁰-, -O-, -O-CHR¹⁰-, -S--S-CHR¹⁰-, -NR^{PR}- or -NR^{PR}-CHR¹⁰-, or R⁸ or R⁹ independently is absent, leaving a 5-membered ring;
R¹⁰ independently are -H, -OR^{PR}, =O, -SR^{PR}, =S, -N(R^{PR})₂, -CN, -NO₂,-OSO₃H, -OPO₃H, an ester, a thioester, a phosphoester, a phosphothioester, a phosphonoester, a sulfite ester, a sulfate ester, an amide, an amino acid, a peptide, an ether, a thioether, acyl, thioacyl, a carbonate, a carbamate, a thioacetal, a halogen, optionally substituted alkyl, optionally substituted alkenyl, optionally substituted alkynyl, optionally substituted aryl, optionally substituted neteroaryl, an optionally substituted heterocycle, an optionally substituted monosaccharide, an optionally substituted oligosaccharide, a nucleoside, a nucleotide, an oligonucleotide, a polymer, or,
R^{PR} independently are -H or a protecting group;
D is a heterocycle or a 4-, 5-, 6- or 7-membered ring that comprises saturated carbon atoms, wherein 1, 2 or 3 ring carbon atoms of the 4-, 5-, 6- or 7-membered ring are optionally independently substituted with -O-, -S- or -NR^{PR}- or where 1, 2 or 3 hydrogen atoms of the heterocycle or where 1 or 2 hydrogen atoms of the 4-, 5-, 6- or 7-membered ring are substituted with -OR^{PR}, -SR^{PR}, - N(R^{PR})₂, -CN, -NO₂, an ester, a thioester, a phosphoester, a phospnothioester, a sulfite ester, a sulfate ester, an amide, an ammo acid, a peptide, an ether, a thioether, acyl, thioacyl, a carbonate, a carbamate, a thioacetal, a halogen, optionally substituted alkyl, optionally substituted alkenyl, optionally substituted alkynyl, optionally substituted aryl, optionally substituted heteroaryl, an optionally substituted monosaccharide, an optionally substituted oligosaccharide, a nucleoside, a nucleotide, an oligonucleotide or a polymer, or, one more of the ring carbons are substituted with =O or =S, or D comprises two 5- or 6-membered rings, wherein the rings are fused or are linked by 1 or 2 bonds, and
R¹⁶ independently are -CH₂-, -O-, -S- or -NH-.

2. Use according to claim 1 wherein R³ independently are -H, -OH, =O,-SH, =S, =NOH, -NO₂, -OSO₃H, -OPO₃H, an ester, a thioester, a phosphoester, a phosphothioester, an amino acid, a peptide, an einer, a thioether, a carbonate, a carbamate, halogen or a polymer.

3. Use according to claim 2 wherein the compound has the structure

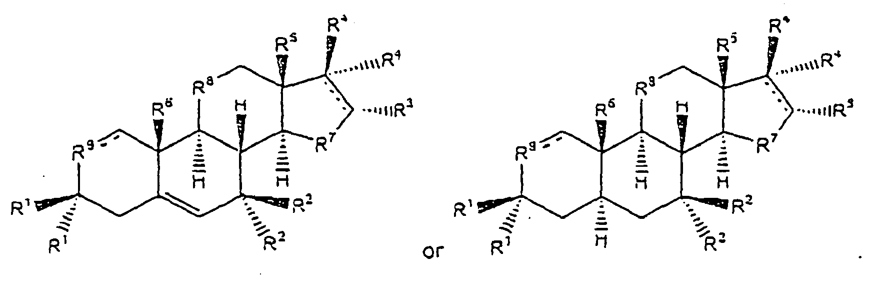

4. Use according to claim 3 wherein wherein one R¹ is -OH, -SH, an ester, a carbamate or a carbonate and the other R¹ is -H, optionally substituted alkyl, optionally substituted alkenyl or optionally substituted alkynyl, R² in the β-configuration is -H, -OH or -SH, R³ is -H, -OH or =O, R⁴ in the β-configuration is - OH or -SH and R⁴ in the α-configuration is -H, optionally substituted alkyl, optionally substituted alkenyl or optionally substituted alkynyl or R⁴ together are =NOH, R⁵ is -CH₃ or -C₂H₅ and R⁶ is -H, -CH₃ or -C₂l-H₅.

5. Use according to claim 4 wherein the subject's circulating platelets and neutrophils are detectably increased.

6. Use according to claim 1, 2 or 3 wherein the subject is a human having or subject to developing neutropenia and the formula 1 compound has the structure

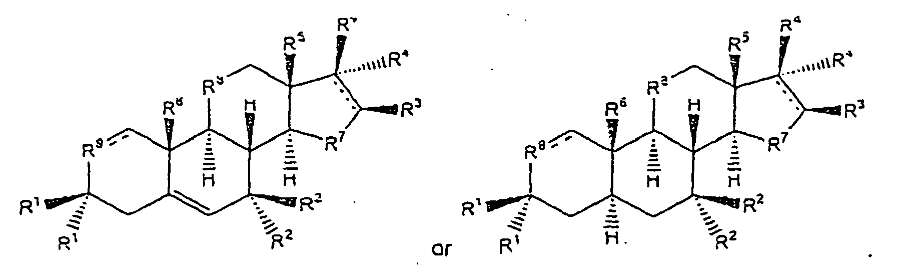

7. Use according to any preceeding claim wherein the use further comprises administration of an effective amount of G-CSF. GM-CSF, M-CSF, IL-3, IL-5, IL-6, IL-11 or thrombopoietin.

8. Use according to claim 1-7, wherein the compound is 3α, 16α, 17β-trihydroxyandrostane and/or 3β, 17β, dihydroxyandrost-5-ene.

9. Use according to claim 1-8 in an immune suppression therapie in solid organ or other tissue transplants.

10. 3α, 16α, 17β Trihydroxyandrostane as a medicament.

## Patentansprüche

1. Verwendung einer Verbindung zur Herstellung eines Medikaments zur Prophylaxe oder Behandlung von Neutropenie oder Thrombozytopenie in einem menschlichen oder primaten Subjekt, wobei die Verbindung die Struktur 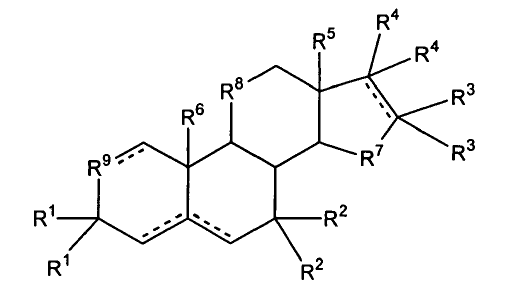 oder 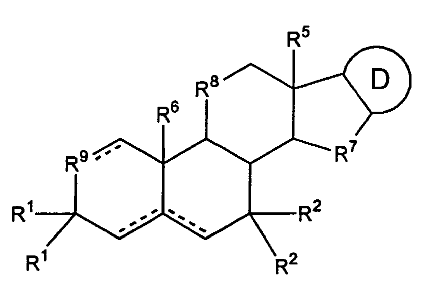 aufweist,
wobei Wasserstoff an der Position 5, falls vorhanden, in der α-Konfiguration vorliegt;
ein R¹ gleich -OR^{PR}, -SR^{PR}, -N(R^{PR})₂, -NO₂, -OPO₃H, ein Ester, ein Thioester, ein Phosphoester, ein Phosphothioester, ein Phosphonoester, ein Sulfitester, ein Sulfatester, ein Amid mit der Struktur-NH-C(O)-organische Gruppierung, eine Aminosäure, ein Peptid, ein Ether, ein Thioether, ein Carbonat, ein Carbamat, ein Thioacetal, ein gegebenenfalls substituiertes Monosaccharid, ein gegebenenfalls substituiertes Oligosaccharid oder ein Polymer ist;
das andere R¹ gleich -H, -CN, Acyl, Thiacyl, gegebenenfalls substituiertes Alkyl, gegebenenfalls substituiertes Alkenyl, gegebenenfalls substituiertes Alkinyl, gegebenenfalls substituiertes Aryl, gegebenenfalls substituiertes Heteroaryl oder ein gegebenenfalls substituierter Heterocyclus ist;
R² unabhängig -H, -OR^{PR}, -SR^{PR}, -N(R^{PR})₂, =NOH, -CN, -NO₂, -OSO₃H, -OPO₃H, ein Ester, ein Thioester, ein Phosphoester, ein Phosphothioester, ein Phosphonoester, ein Sulfitester, ein Sulfatester, ein Amid, eine Aminosäure, ein Peptid, ein Ether, ein Thioether, Acyl, Thioacyl, ein Carbonat, ein Carbamat, ein Thioacetal, ein Halogen, gegebenenfalls substituiertes Alkyl, gegebenenfalls substituiertes Alkenyl, gegebenenfalls substituiertes Alkinyl, gegebenenfalls substituiertes Aryl, gegebenenfalls substituiertes Heteroaryl, ein gegebenenfalls substituierter Heterocyclus oder ein Polymer sind;
R³ unabhängig -H, -OR^{PR}, =O, -SR^{PR}, =S, -N(R^{PR})₂, =NOH, -CN, -NO₂, -OSO₃H, -OPO₃H, ein Ester, ein Thioester, ein Phosphoester, ein Phosphothioester, ein Phosphonoester, ein Sulfitester, ein Sulfatester, ein Amid, eine Aminosäure, ein Peptid, ein Ether, ein Thioether, Acyl, Thioacyl, ein Carbonat, ein Carbamat, ein Thioacetal, ein Halogen, gegebenenfalls substituiertes Alkyl, gegebenenfalls substituiertes Alkenyl, gegebenenfalls substituiertes Alkinyl, gegebenenfalls substituiertes Aryl, gegebenenfalls substituiertes Heteroaryl, ein gegebenenfalls substituierter Heterocyclus oder ein Polymer sind;
ein R⁴ gleich -OR^{PR}, -SR^{PR}, -N(R^{PR})₂, -NO₂, -OPO₃H, ein Ester, ein Thioester, ein Phosphoester, ein Phosphothioester, ein Phosphonoester, ein Sulfitester, ein Sulfatester, ein Amid mit der Struktur -NH-C(O)-organische Gruppierung, eine Aminosäure, ein Peptid, ein Ether, ein Thioether, ein Carbonat, ein Carbamat, ein Thioacetal, ein gegebenenfalls substituiertes Monosaccharid, ein gegebenenfalls substituiertes Oligosaccharid oder ein Polymer ist;
das andere R⁴ gleich -H, -CN, Acyl, Thiacyl, gegebenenfalls substituiertes Alkyl, gegebenenfalls substituiertes Alkenyl, gegebenenfalls substituiertes Alkinyl, gegebenenfalls substituiertes Aryl, gegebenenfalls substituiertes Heteroaryl oder ein gegebenenfalls substituierter Heterocyclus ist oder beide R⁴ zusammen gleich =NOH sind;
R⁵ gleich -H, gegebenenfalls substituiertes Alkyl, gegebenenfalls substituiertes Alkenyl oder gegebenenfalls substituiertes Alkinyl ist;
R⁶ gleich gegebenenfalls substituiertes Alkyl, gegebenenfalls substituiertes Alkenyl oder gegebenenfalls substituiertes Alkinyl ist;
R⁷ gleich -CHR¹⁰-, -CHR¹⁰-CHR¹⁰-, -CHR¹⁰-CHR¹⁰-CHR¹⁰-, -CHR¹⁰-O-CHR¹⁰-, -CHR¹⁰-S-CHR¹⁰- -CHR¹⁰-NR^{PR}-CHR¹⁰-, -O-, -O-CHR¹⁰-, -S-, -S-CHR¹⁰- -NR^{PR}- oder -NR^{PR}-CHR¹⁰- ist;
R⁸ and R⁹ unabhängig -CHR¹⁰-, -CHR¹⁰-CHR¹⁰-, -O-, -O-CHR¹⁰-, -S-, -S-CHR¹⁰-, -NR^{PR}- oder -NR^{PR}-CHR¹⁰- sind oder R⁸ oder R⁹ unabhängig abwesend ist, so dass ein 5-gliedriger Ring zurückbleibt;
R¹⁰ unabhängig -H, -OR^{PR}, =O, -SR^{PR}, =S, -N(R^{PR})₂, -CN, -NO₂, -OSO₃H, -OPO₃H, ein Ester, ein Thioester, ein Phosphoester, ein Phosphothioester, ein Phosphonoester, ein Sulfitester, ein Sulfatester, ein Amid, eine Aminosäure, ein Peptid, ein Ether, ein Thioether, Acyl, Thioacyl, ein Carbonat, ein Carbamat, ein Thioacetal, ein Halogen, gegebenenfalls substituiertes Alkyl, gegebenenfalls substituiertes Alkenyl, gegebenenfalls substituiertes Alkinyl, gegebenenfalls substituiertes Aryl, gegebenenfalls substituiertes Heteroaryl, ein gegebenenfalls substituierter Heterocyclus, ein gegebenenfalls substituiertes Monosaccharid, ein gegebenenfalls substituiertes Oligosaccharid, ein Nukleosid, ein Nukleotid oder ein Polymer ist; oder
R^{PR} unabhängig -H oder eine Schutzgruppe sind,
D ein Heterocyclus oder ein 4-, 5-, 6- oder 7-gliedriger Ring ist, der gesättigte Kohlenstoffatome umfasst, wobei 1, 2 oder 3 Ringkohlenstoffatome des 4-, 5-, 6-oder 7-gliedrigen Rings gegebenenfalls unabhängig mit -O-, -S- oder -NR^{PR}-substituiert sind, oder wobei 1, 2 oder 3 Wasserstoffatome des Heterocyclus oder wobei 1 oder 2 Wasserstoffatome des 4-, 5-, 6- oder 7-gliedrigen Rings substituiert sind mit -OR^{PR}, -SR^{PR}, -N(R^{PR})₂, -CN, -NO₂, einem Ester, einem Thioester, einem Phosphoester, einem Phosphothioester, einem Sulfitester, einem Sulfatester, einem Amid, einer Aminosäure, einem Peptid, einem Ether, einem Thioether, Acyl, Thioacyl, einem Carbonat, einem Carbamat, einem Thioacetal, einem Halogen, gegebenenfalls substituiertem Alkyl, gegebenenfalls substituiertem Alkenyl, gegebenenfalls substituiertem Alkinyl, gegebenenfalls substituiertem Aryl, gegebenenfalls substituiertem Heteroaryl, einem gegebenenfalls substituierten Monosaccharid, einem gegebenenfalls substituierten Oligosaccharid, einem Nukleosid, einem Nukleotid, einem Oligonukleotid oder einem Polymer, oder ein weiteres der Ringkohlenstoffatome mit =O oder =S substituiert ist, oder D zwei 5- oder 6-gliedrige Ringe umfasst, wobei die Ringe kondensiert oder durch 2 oder 3 Bindungen verknüpft sind, und
R¹⁶ unabhängig -CH₂-, -O, -S-, oder -NH- sind.

2. Verwendung nach Anspruch 1, wobei R³ unabhängig -H, -OH, =O, -SH, =S, =NOH, -NO₂, -OSO₃H, -OPO₃H, ein Ester, ein Thioester, ein Phosphoester, ein Phosphothioester, eine Aminosäure, ein Peptid, ein Ether, ein Thioether, ein Carbonat, ein Carbamat, Halogen oder ein Polymer sind.

3. Verwendung nach Anspruch 2, wobei die Verbindung die Struktur 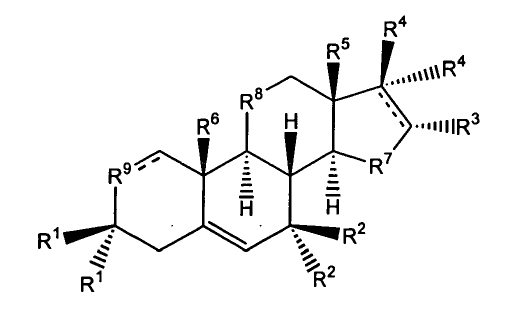 oder 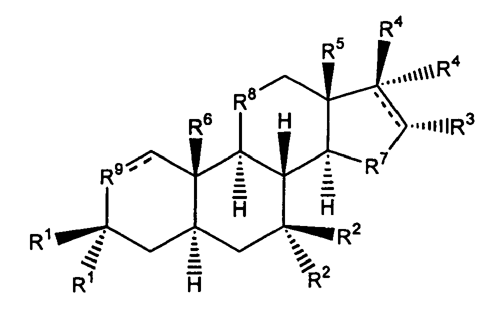 aufweist.

4. Verwendung nach Anspruch 3, wobei ein R¹ gleich -OH, -SH, ein Ester, ein Carbamat oder ein Carbonat ist, und der andere R¹ gleich -H, gegebenenfalls substituiertes Alkyl, gegebenenfalls substituiertes Alkenyl oder gegebenenfalls substituiertes Alkinyl ist, R² in der β-Konfiguration gleich -H, -OH oder -SH ist, R³ gleich -H, -OH oder =O ist, R⁴ in der β-Konfiguration gleich -OH oder -SH ist und R⁴ in der α-Konfiguration gleich -H, gegebenenfalls substituiertes Alkyl, gegebenenfalls substituiertes Alkenyl oder gegebenenfalls substituiertes Alkinyl ist oder R⁴ zusammen gleich =NOH sind, R⁵ gleich -CH₃ oder -C₂H₅ ist und R⁶ gleich -H, -CH₃ oder -C₂H₅ ist.

5. Verwendung nach Anspruch 4, wobei die zirkulierenden Thrombozyten und Neutrophilen des Subjekts messbar erhöht sind.

6. Verwendung nach Anspruch 1, 2 oder 3, wobei das Subjekt ein Mensch ist, der eine Neutropenie aufweist oder zu deren Entwicklung neigt und die Verbindung der Formel 1 die Struktur 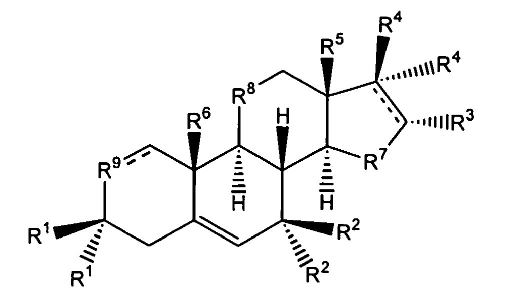 oder 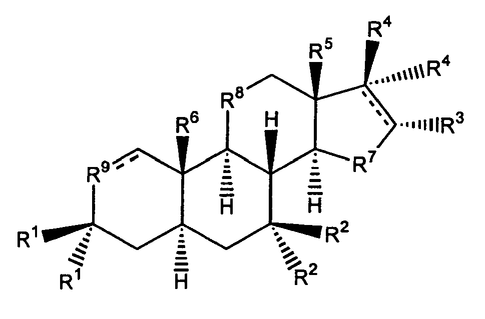 aufweist.

7. Verwendung nach einem der vorhergehenden Ansprüche, wobei die Verwendung ferner eine Verabreichung einer effektiven Menge an G-CSF, GM-CSF, M-CSF, IL-3, IL-5, IL-6, IL-11 oder Thrombopoietin umfasst.

8. Verwendung nach Anspruch 1-7, wobei die Verbindung 3α,16α,17β-Trihydroxyandrostan und/oder 3β,17β-Dihydroxyandrost-5-en ist.

9. Verwendung nach Anspruch 1-8 in einer Immunsuppressionstherapie in soliden Organ- oder anderen Gewebetransplantaten.

10. 3α,16α,17β-Trihydroxyandrostan als ein Medikament.

## Revendications

1. Utilisation d'un composé pour la préparation d'un médicament destiné à la prophylaxie ou au traitement de la neutropénie ou de la thrombocytopénie chez un sujet humain ou primate, le composé possédant la structure 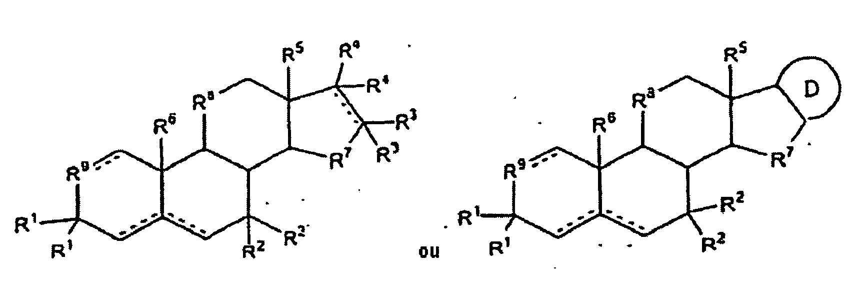
dans laquelle l'atome d'hydrogène en position 5, lorsqu'il est présent, se trouve dans la configuration α;
un radical R¹ représente un groupe -OR^{PR}, un groupe -SR^{PR}, un groupe -N(R^{PR})₂, un groupe -NO₂, un groupe -OPO₃H, un ester, un thioester, un phosphoester, un phosphothioester, un phosphonoester, un ester de sulfite, un ester de sulfate, un amide possédant la structure -NH-C(O)-fraction organique, un acide aminé, un peptide, un éther, un thioéther, un carbonate, un carbamate, un thioacétal, un monosaccharide le cas échéant substitué, un oligosaccharide le cas échéant substitué ou un polymère;
l'autre radical R¹ représente un atome d'hydrogène, un groupe -CN, un groupe acyle, un groupe thioacyle, un groupe alkyle le cas échéant substitué, un groupe alcényle le cas échéant substitué, un groupe alcynyle le cas échéant substitué, un groupe aryle le cas échéant substitué, un groupe hétéroaryle le cas échéant substitué ou un groupe hétérocyclique le cas échéant substitué;
les radicaux R² représentent, de manière indépendante, un atome d'hydrogène, un groupe -OR^{PR}, un groupe -SR^{PR}, un groupe -N(R^{PR})₂, un groupe =NOH, un groupe -CN, un groupe -NO₂, un groupe -OSO₃H, un groupe -OPO₃H, un ester, un thioester, un phosphoester, un phosphothioester, un phosphonoester, un ester de sulfite, un ester de sulfate, un amide, un acide aminé, un peptide, un éther, un thioéther, un groupe acyle, un groupe thioacyle, un carbonate, un carbamate, un thioacétal, un atome d'halogène, un groupe alkyle le cas échéant substitué, un groupe alcényle le cas échéant substitué, un groupe alcynyle le cas échéant substitué, un groupe aryle le cas échéant substitué, un groupe hétéroaryle le cas échéant substitué ou un groupe hétérocyclique le cas échéant substitué ou un polymère;
les radicaux R³ représentent, de manière indépendante, un atome d'hydrogène, un groupe -OR^{PR}, un atome d'oxygène, un groupe -SR^{PR}, un atome de soufre, un groupe -N(R^{PR})₂, un groupe =NOH, un groupe -CN, un groupe -NO₂, un groupe -OSO₃H, un groupe -OPO₃H, un ester, un thioester, un phosphoester, un phosphothioester, un phosphonoester, un ester de sulfite, un ester de sulfate, un amide, un acide aminé, un peptide, un éther, un thioéther, un groupe acyle, un groupe thioacyle, un carbonate, un carbamate, un thioacétal, un atome d'halogène, un groupe alkyle le cas échéant substitué, un groupe alcényle le cas échéant substitué, un groupe alcynyle le cas échéant substitué, un groupe aryle le cas échéant substitué, un groupe hétéroaryle le cas échéant substitué ou un groupe hétérocyclique le cas échéant substitué ou un polymère;
un radical R⁴ représente un groupe -OR^{PR}, un groupe -SR^{PR}, un groupe -N(R^{PR})₂, un groupe -NO₂, un groupe -OPO₃H, un ester, un thioester, un phosphoester, un phosphothioester, un phosphonoester, un ester de sulfite, un ester de sulfate, un amide possédant la structure -NH-C(O)-fraction organique, un acide aminé, un peptide, un éther, un thioéther, un carbonate, un carbamate, un thioacétal, un monosaccharide le cas échéant substitué, un oligosaccharide le cas échéant substitué ou un polymère;
l'autre radical R⁴ représente un atome d'hydrogène, un groupe -CN, un groupe acyle, un groupe thioacyle, un groupe alkyle le cas échéant substitué, un groupe alcényle le cas échéant substitué, un groupe alcynyle le cas échéant substitué, un groupe aryle le cas échéant substitué, un groupe hétéroaryle le cas échéant substitué ou un groupe hétérocyclique le cas échéant substitué ou bien les deux radicaux R⁴ forment ensemble un groupe =NOH;
R⁵ représente un atome d'hydrogène, un groupe alkyle le cas échéant substitué, un groupe alcényle le cas échéant substitué ou un groupe alcynyle le cas échéant substitué;
R⁶ représente un groupe alkyle le cas échéant substitué, un groupe alcényle le cas échéant substitué ou un groupe alcynyle le cas échéant substitué;
R⁷ représente un groupe -CHR¹⁰, un groupe -CHR¹⁰CHR¹⁰, un groupe -CHR¹⁰CHR¹⁰CHR¹⁰, un groupe -CHR¹⁰-O-CHR¹⁰, un groupe -CHR¹⁰-S-CHR¹⁰, un groupe -CHR¹⁰-NR^{PR}-CHR¹⁰, un atome d'oxygène, un groupe -O-CHR¹⁰, un atome de soufre, un groupe -S-CHR¹⁰, un groupe -NR^{PR}, ou un groupe -NR^{PR}-CHR¹⁰;
R⁸ et R⁹ représentent, indépendamment l'un de l'autre, un groupe -CHR¹⁰, un groupe -CHR¹⁰CHR¹⁰, un atome d'oxygène, un groupe -O-CHR¹⁰, un atome de soufre, un groupe -S-CHR¹⁰, un groupe -NR^{PR}, ou un groupe -NR^{PR}-CHR¹⁰, ou bien R⁸ ou R⁹, indépendamment l'un de l'autre, est absent, en laissant subsister un noyau pentagonal;
les radicaux R¹⁰ représentent, indépendamment l'un de l'autre, un atome d'hydrogène, un groupe -OR^{PR}, un atome d'oxygène, un groupe -SR^{PR}, un atome de soufre, un groupe -N(R^{PR})₂, un groupe -CN-, un groupe -NO₂, un groupe -OSO₃H, un groupe -OPO₃H, un ester, un thioester, un phosphoester, un phosphothioester, un phosphonoester, un ester de sulfite, un ester de sulfate, un amide possédant la structure -NH-C(O)-fraction organique, un acide aminé, un peptide, un éther, un thioéther, un groupe acyle, un groupe thioacyle, un carbonate, un carbamate, un thioacétal, un atome d'halogène, un groupe alkyle le cas échéant substitué, un groupe alcényle le cas échéant substitué, un groupe alcynyle le cas échéant substitué, un groupe aryle le cas échéant substitué, un groupe hétéroaryle le cas échéant substitué ou un groupe hétérocyclique le cas échéant substitué, un monosaccharide le cas échéant substitué, un oligosaccharide le cas échéant substitué, un nucléoside, un nucléotide, un oligonucléotide, un polymère ou bien
les radicaux R^{PR} représentent, indépendamment l'un de l'autre, un atome d'hydrogène ou un groupe de protection;
D représente un composé hétérocyclique ou un noyau carré, pentagonal, hexagonal ou heptagonal qui comprend des atomes de carbone saturés, 1, 2 ou 3 atomes de carbone nucléaire du noyau carré, pentagonal, hexagonal ou heptagonal étant, le cas échéant, substitué(s) de manière indépendante avec un atome d'oxygène, un atome de soufre ou un groupe -NR^{PR}, ou bien 1, 2 ou 3 atomes d'hydrogène du composé hétérocyclique ou 1 ou 2 atomes d'hydrogène du noyau carré, pentagonal, hexagonal ou heptagonal étant substitué(s) avec un groupe -OR^{PR}, un groupe -SR^{PR}, un groupe -N(R^{PR})₂, un groupe -CN-, un groupe -NO₂, un ester, un thioester, un phosphoester, un phosphothioester, un ester de sulfite, un ester de sulfate, un amide, un acide aminé, un peptide, un éther, un thioéther, un groupe acyle, un groupe thioacyle, un carbonate, un carbamate, un thioacétal, un atome d'halogène, un groupe alkyle le cas échéant substitué, un groupe alcényle le cas échéant substitué, un groupe alcynyle le cas échéant substitué, un groupe aryle le cas échéant substitué, un groupe hétéroaryle le cas échéant substitué ou un groupe hétérocyclique le cas échéant substitué, un monosaccharide le cas échéant substitué, un oligosaccharide le cas échéant substitué, un nucléoside, un nucléotide, un oligonucléotide, un polymère ou bien un atome de plus parmi les atomes nucléaires étant substitué avec un atome d'oxygène ou avec un atome de soufre, ou bien D comprend deux noyaux pentagonaux ou hexagonaux, les noyaux étant condensés ou étant reliés par 1 ou 2 liaisons, et
les radicaux R¹⁶ représentent, indépendamment l'un de l'autre, un groupe -CH₂, un atome d'oxygène, un atome de soufre ou un groupe -NH.

2. Utilisation selon la revendication 1, dans laquelle R³ représente un atome d'hydrogène, un groupe -OH, un atome d'oxygène, un groupe -SH, un atome de soufre, un groupe =NOH, un groupe -NO₂, un groupe -OSO₃H, un groupe -OPO₃H, un ester, un thioester, un phosphoester, un phosphothioester, un acide aminé, un peptide, un éther, un thioéther, un carbonate, un carbamate, un atome d'halogène ou un polymère.

3. Utilisation selon la revendication 2, dans laquelle le composé possède la structure

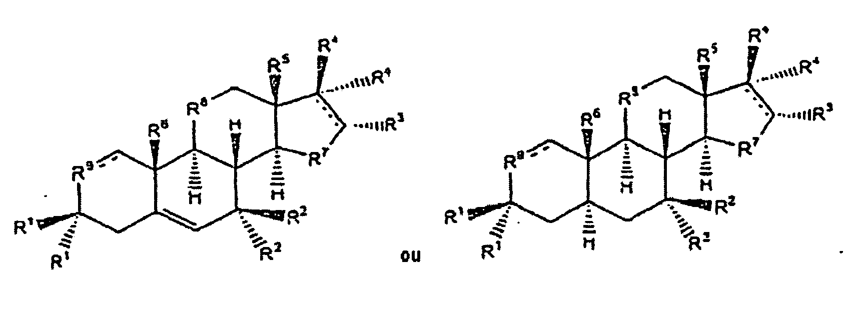

4. Utilisation selon la revendication 3, dans laquelle un des radicaux R¹ représente un groupe -OH, un groupe -SH, un ester, un carbamate ou un carbonate et l'autre radical R¹ représente un atome d'hydrogène, un groupe alkyle le cas échéant substitué, un groupe alcényle le cas échéant substitué ou un groupe alcynyle le cas échéant substitué, R² dans la configuration β représente un atome d'hydrogène, un groupe -OH ou un groupe -SH, R³ représente un atome d'hydrogène, un groupe -OH ou un atome d'oxygène, R⁴ dans la configuration β représente un groupe -OH ou un groupe -SH, et R⁴ dans la configuration α représente un atome d'hydrogène, un groupe alkyle le cas échéant substitué, un groupe alcényle le cas échéant substitué ou un groupe alcynyle le cas échéant substitué, ou bien les radicaux R⁴ représentent ensemble un groupe =NOH, R⁵ représente un groupe -CH₃ ou un groupe -C₂H₅, et R⁶ représente un atome d'hydrogène, un groupe -CH₃ ou un groupe -C₂H₅.

5. Utilisation selon la revendication 4, dans laquelle la numération des plaquettes et des neutrophiles circulants du sujet manifeste une augmentation que l'on peut détecter.

6. Utilisation selon la revendication 1, 2 ou 3, dans laquelle le sujet est un être humain présentant ou susceptible de développer une neutropénie et le composé répondant à la formule 1 possède la structure

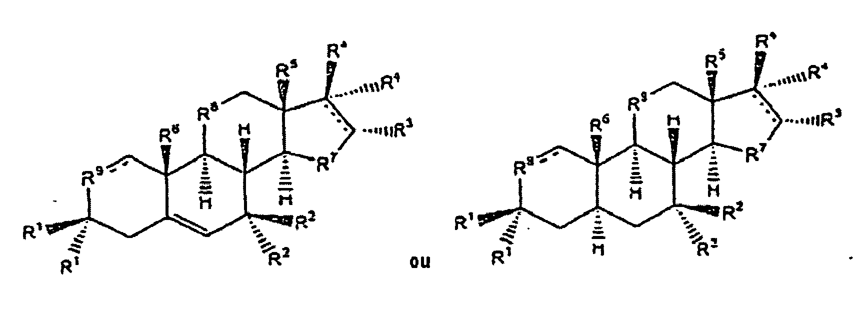

7. Utilisation selon l'une quelconque des revendications précédentes, dans laquelle l'utilisation comprend en outre l'administration d'une quantité efficace du G-CSF, du GM-CSF, du M-CSF, de IL-3, de IL-5, de IL-6, de IL-11 ou de thrombopoïétine.

8. Utilisation selon l'une quelconque des revendications 1 à 7, dans laquelle le composé est le 3α,16α,17β-trihydroxyandrostane et/ou le 3β,17β-dihydroxyandrost-5-ène.

9. Utilisation selon l'une quelconque des revendications 1 à 8, dans une thérapie d'immunosuppression dans des organes solides ou dans d'autres transplants tissulaires.

10. 3α,16α,17β-trihydroxyandrostane à titre de médicament.
